(19) Europäisches Patentamt
European Patent Office
Office européen des brevets

(11) EP 4 461 794 A2

(12) **EUROPEAN PATENT APPLICATION**

(43) Date of publication:
**13.11.2024 Bulletin 2024/46**

(21) Application number: **24203386.8**

(22) Date of filing: **09.12.2021**

(51) International Patent Classification (IPC):
***C11D 3/20*** *(2006.01)*

(52) Cooperative Patent Classification (CPC):
**A61Q 13/00; A61K 8/345; A61Q 15/00; A61Q 17/005; A61Q 17/02; A61Q 17/04; A61Q 19/008; A61Q 19/10; C11D 3/2044; C11D 3/48;** A61K 2800/5922; Y02A 50/30

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.12.2020 PCT/EP2020/085174**

(62) Document number(s) of the earlier application(s) in accordance with Art. 76 EPC:
**21831297.3 / 4 110 277**

(71) Applicant: **Symrise AG**
**37603 Holzminden (DE)**

(72) Inventors:
- **BUGDAHN, Nikolas**
**37603 Holzminden (DE)**
- **LANGE, Sabine**
**37603 Holzminden (DE)**
- **KOCH, Christin**
**32760 Detmold (DE)**
- **STRUEVER, Frank**
**31789 Hameln (DE)**
- **DROEGE, Dietmar**
**31868 Ottenstein (DE)**

(74) Representative: **Global IP Europe Patentanwaltskanzlei**
**Pfarrstraße 14**
**80538 München (DE)**

Remarks:
This application was filed on 27.09.2024 as a divisional application to the application mentioned under INID code 62.

# (54) MULTIFUNCTIONAL COMPOUND MIXTURES COMPRISING MULTIPLE (BIO)-ALKANEDIOLS

(57) The present invention relates to a cosmetic or pharmaceutical composition comprising an effective amount of at least one linear 1,2-alkanediol having a carbon chain of 5 to 13 carbon atoms and an effective amount of at least one linear 2,3-alkanediol having a carbon chain of 5 to 13 carbon atoms. Moreover, the present invention relates to a composition comprising an effective amount of at least one linear 1,2-alkanediol having a carbon chain of 5 to 13 carbon atoms and an effective amount of at least one linear 2,3-alkanediol having a carbon chain of 5 to 13 carbon atoms. Additionally, the present invention relates to the use of the compositions of the present invention for enhancing the antioxidative effect of an antioxidant, for improving the sensory properties, i.e. skin sensation, of a liquid lipophilic component, for sebum control, or for malodour management, or for modulating fragrance notes, or for modification of fragrance notes, or for suppression of fragrance notes, or for topical applications, or for coverage of the malodour of 1-octen-3-ol, or for masking insect alluring odours, or for repelling insects. Finally, the present invention relates to a cosmetic product, a pharmaceutical or a homecare product comprising a composition according to the present invention.

Figure 1

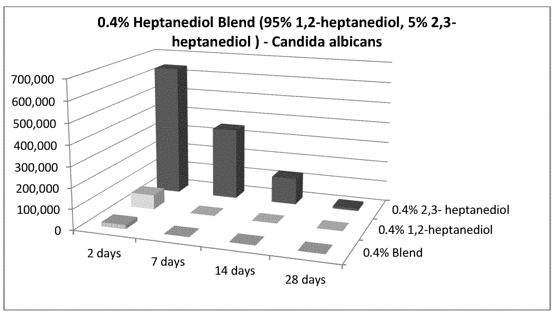


EP 4 461 794 A2

## Description

### Technical field

The present invention relates to a cosmetic or pharmaceutical composition or homecare product comprising or consisting of an effective amount of at least one linear 1,2-alkanediol having a carbon chain of 5 to 13 carbon atoms and/or an effective amount of at least one linear 2,3-alkanediol having a carbon chain of 5 to 13 carbon atoms. Moreover, the present invention relates to a composition comprising or consisting of an effective amount of at least one linear 1,2-alkanediol having a carbon chain of 5 to 13 carbon atoms and/or an effective amount of at least one linear 2,3-alkanediol having a carbon chain of 5 to 13 carbon atoms. Additionally, the present invention relates to the use of the compositions of the present invention for enhancing the antioxidative effect of an antioxidant, in particular in a cosmetic or pharmaceutical composition or homecare product, for improving the sensory properties, i.e. skin sensation, of a cosmetic or pharmaceutical composition comprising a liquid lipophilic component, for sebum control, or for malodour management, or for modulating fragrance notes, or for modification of fragrance notes, or for suppression of fragrance notes, or for topical applications, or for coverage of the malodour of 1-octen-3-ol, or for masking insect alluring odours, or for repelling insects. Finally, the present invention relates to a cosmetic or pharmaceutical composition or homecare product comprising a composition according to the present invention.

### Background Art

In recent years, the cosmetics industry has been seeking a nature-derived, biodegradable, ingredient with multiple benefits packed into a single molecule, which offers good economics and shows important properties at low concentrations.

Therefore, there is an ongoing need for multifunctionals, whose multiple key benefits, such as antioxidant enhancer, solubilizer of lipophilic cosmetic ingredients, product protection enhancer in synergy with many preservatives, reduce formulation complexity while improving finished product properties.

The skin is the human body's largest organ. It is colonized by diverse microorganisms, most of which are harmless or even beneficial to their host. Colonization is driven by the ecology of the skin surface, which is highly variable depending on topographical location, endogenous host factors and exogenous environmental factors. For instance, the armpits present a rather humid and nutrient-rich habitat that provides favorable conditions for growth of many microorganisms. Other habitats include sebum-rich habitats like the face and rather dry and nutrient-poor habitats like most other body regions.

Many common skin pathogens, such as *Staphylococcus aureus* and *Streptococcus pyogenes,* are inhibited by an acidic pH, thus the growth of coagulase-negative staphylococci and corynebacteria is favored. However, skin occlusion results in an elevated pH, which favors the growth of *S. aureus* and *S. pyogenes.*

Areas with a high density of sebaceous glands, such as the face, chest and back, encourage the growth of lipophilic microorganisms such as *Corynebacterium spp, Propionibacterium spp* and *Malassezia spp.*

Over-production of sebum causes the sebaceous gland follicles to be partly or completely filled with sebum. This sebum often has a very firm consistency and accordingly a low spreading, so that it can be released from the sebaceous gland follicles and can spread over the skin only with great difficulty. The sebum consequently accumulates to an undesirably high degree in the sebaceous glands and forms an ideal nutrient medium for microorganisms, such as, in particular, Propionibacterium acnes, a microorganism involved decisively in the development of acne. On greasy-oily skin, over-production of sebum leads to an undesirable greasy shine on the skin. In the area of a greasy-oily scalp, over-production of sebum leads to an undesirable greasy shine on the roots of the hair. On impure skin, over-production of sebum leads to irregularities of the skin, such as e.g. pimples and pustules. Acne is a pathologically changed clinical skin picture of impure skin.

In cases of acne, which usually occurs in puberty, the anaerobic microorganism *Propionibacterium acnes (P. acnes,* recently reclassified/renamed as *Cutibacerium acnes)* is involved decisively in the development of the condition and decomposes the sebum to glycerin and fatty acids, as a result of which the sebaceous glands are in turn stimulated to produce an increased amount of sebum, these degradation products attacking or destroying the follicle walls. This regularly causes inflammations in the skin (pimples, pustules, nodules, cysts), which often heal only with scarring, as a result of which the visual appearance of the person suffering from impure skin is permanently damaged.

Many skin disorders and infections can be caused by bacteria and fungi: Chronic wounds, affecting diabetic, elderly, and immobile individuals, are an example where commensal skin organisms invade and become pathogenic upon breach of the skin barrier. Although bacteria do not cause the initial wounding event, they are thought to contribute to the lack of healing and persistent inflammation that is associated with chronic wounds. Burn wounds commonly become infected with *S. pyogenes, Enterococcus spp.* or *Pseudomonas aeruginosa.*

A further common skin commensal is *Staphylococcus epidermidis.* It is also the most frequent cause of hospital-acquired infection on in-dwelling medical devices such as catheters or heart valves.

Hence, there is a need for products capable of controlling the skin microbiota, and in particular inhibit the growth of skin pathogens. Concomitant, there is a need for products capable for balancing or reducing or inhibiting sebum overproduction and/or reducing dandruff and thereby control the colonization of the skin with specific microbial species which are lipid dependent and are feed on human sebum.

WO 2008/046791 discloses the use of 1,2-decanediol for sebum reduction of the skin and a cosmetic or dermatological formulation for topical application comprising 1,2-decanediol. In addition to 1,2-decanediol, the formulations include optionally cleansing substances and antimicrobials.

Meanwhile, 1,2-decanediol is an established antimicrobial used in deodorant, anti-perspirant, anti-dandruff, anti-acne and other hygiene applications for oral or personal care.

However, the formulation and in particular 1,2-decanediol has the disadvantage of an unpleasant inherent smell.

Furthermore, it is known that microbes living on the human skin produce a variety of different compounds like formic acid, butyric acid, 3-hydroxy-3-methylhexanoic acid and 3-methyl-3-sulfanylhexane-1-ol from the sweat produced by the apocrine sweat glands. Several compounds produced by the microbes are responsible for an unpleasant odour e.g. butyric acid has a very intense and unpleasant odour.

In this contect, important microorganisms living on the skin or scalp of humans are microorganisms from the genus Staphylococcus, Corynebactrerium, Anaerococcus, Finegoldia, Moraxella, Porphyromonas, Fusobacterium, Aspergillus, Candida, Malassezia, Escherichia, Peptoniphilus; Streptococcus, Lactobacillus; Gardnerella, Fannyhessea, Epidermophyton, Trichophyton, Fusobacterium, Cutibacterium, in particular the microorganisms *Staphylococcus epidermidis; Staphylococcus hominis; Corynebacterium xerosis; Corynebacterium jeikeium; Anaerococcus octavius; Finegoldia magna; Moraxella osloensis; Moraxella atlantae; Porphyromonas gingivalis; Aspergillus brasiliensis; Candida albicans; Escherichia coli; Staphylococcus aureus; Peptoniphilus lacrimalis; Streptococcus agalactiae; Lactobacillus acidophilus; Gardnerella vaginalis; Fannyhessea vaginae (Atopobium vaginae); Epidermophyton floccosum; Trichophyton rubrum; Streptococcus mutans; Fusobacterium nucleatum.* Recently, it has been discovered that the microorganisms *Anaerococcus octavius* and *Corynebacterium jeikeium* are responsible for the production of compounds having an unpleasant smell from human sweat.

In addition, the pheromone-like compounds like the steroids androstenone and androsterone sulfate which are directly produced by the apocrine sweat glands also are responsible for an unpleasant malodour or body odour.

For an efficient malodour management the composition needs to reduce the the microbial activity on the skin. Additionally, the compound or composition must cover the unpleasant smell of different compounds produced by microbes or directly produced by the sweat glands.

In addition, in various cosmetic or pharmaceutical composition compounds have to be used as active substances or as additives which possess an unpleasant intrinsic odour. Therefore, there is a need for new substances which can modulate, modificate or suppress different fragrance notes.

In the context of modulation, modification or suppression of fragrance notes there is also an ongoing search for new compounds or compositions which can be used in insect repellent products or for masking insect alluring odours. It is known that 1-octen-3-ol attracts biting insects such as mosquitoes and that 1-octen-3-ol has an unpleasant smell. To provide an efficient insect repellent a compound or composition is needed which combats the malodour of 1-octen-3-ol. In many insect repellent products active agents are used which work by an intense insect repelling odour. In this case the compound or composition must not cover the insect repelling odour.

It is to be noted that the substances used in cosmetics and pharmaceuticals must fulfil certain requirements. The compounds must be toxicologically acceptable, readily tolerated by the skin, stable in conventional cosmetic and/or pharmaceutical formulations, inexpensive to prepare, easy to formulate and the compounds need to be active at different pH values. Furthermore, the substance must not have an unpleasant smell. In addition, the substances or compositions used in cosmetic or pharmaceutical products need to be compatibel with different media e.g. water or ethanol. In the search for new substances which are useful in cosmetic and pharmaceutical compositions there is no predictable connection between the chemical structure and other physico-chemical parameters relevant to the field of cosmetics and pharmaceuticals, i.e. the toxicological acceptability, the skin tolerability, the stability, solubility and formulation properties and the smell of a substance.

In summary, the cosmetic or pharmaceutical industry is permanently searching for new ingredients and compositions which can further improve the usefulness of cosmetic and pharmaceutical compositions or homecare products such as enhancing the antioxidative effect of an antioxidant in a cosmetic or pharmaceutical composition or homecare product, improving the sensory properties, i.e. skin sensation, of a cosmetic or pharmaceutical composition comprising a liquid lipophilic component, in sebum control, in malodour management, in modulating fragrance notes, in modification of fragrance notes, in suppression of fragrance notes, in topical applications, in coverage of the malodour of 1-octen-3-ol, in masking insect alluring odours and in repelling insects.

Therefore, it is an object of the present invention to provide multifunctional compositions and multifunctional cosmetic or pharmaceutical compositions which can be efficiently used for enhancing the antioxidative effect of an

antioxidant in a cosmetic or pharmaceutical composition or homecare product, improving the sensory properties, i.e. skin sensation, of a cosmetic or pharmaceutical composition comprising a liquid lipophilic component, in sebum control, or in malodour malodour management, or in the modulation of fragrance notes, or in the modification of fragrance notes, or in the suppression of fragrance notes, or in topical applications, or in the coverage of the malodour of 1-octen-3-ol, or in masking insect alluring odours, or in repelling insects.

**Summary of the invention**

In order to accomplish the above problem, the present invention provides in a first aspect a cosmetic or pharmaceutical composition or homecare product comprising or consisting of:

(a) at least one linear 1 ,2-alkanediol having a carbon chain of 5 to 13 carbon atoms; and
(b) at least one linear 2,3-alkanediol having a carbon chain of 5 to 13 carbon atoms; or

a cosmetic or pharmaceutical composition or homecare product comprising or consisting of:
(b) at least one linear 2,3-alkanediol having a carbon chain of 5 to 13 carbon atoms.

Furthermore, the present invention provides in a secod aspect a composition comprising or consisting of:

(a) at least one linear 1 ,2-alkanediol having a carbon chain of 5 to 13 carbon atoms; and
(b) at least one linear 2,3-alkanediol having a carbon chain of 5 to 13 carbon atoms; or

a composition comprising or consisting of:
(b) at least one linear 2,3-alkanediol having a carbon chain of 5 to 13 carbon atoms.

Moreover, in a further aspect the present invention relates to the use of the compositions according to the present invention for enhancing the antioxidative effect of an antioxidant in a cosmetic or pharmaceutical composition or homecare product, improving the sensory properties, i.e. skin sensation, of a cosmetic or pharmaceutical composition comprising a liquid lipophilic component, for sebum control, or for malodour management, or for modulating fragrance notes, or for modification of fragrance notes, or for suppression of fragrance notes, or for topical applications, or for coverage of the malodour of 1-octen-3-ol, or for masking insect alluring odours, or for repelling insects.

Finally, in a further aspect the present invention pertains to a cosmetic or pharmaceutical composition or homecare product comprising a composition according to the present invention, wherein the product is a deodorant and/or antiperspirant, an aerosol-based deo spray, deo pump spray, deo stick, deo roll on, deo cream, deo wipes, deo crystals, pickering emulsions, hydrodisperion gels, skin balms, shampoo, shower gel, foam bath, micellar water, facial cleansing solutions, cleansing wipes, intimate spray, intimate cream, intimate wash lotion, intimate wipes, aerosol-based foot spray, foot pump spray, foot bath, foot balm, soap, liquid washing, shower and bath preparation, bath product, bath capsule, bath oil, bath tablet, bath salt, bath soap, effervescent preparation, mouth wash concentrate, mouth wash ready to use, repellent, anti-insect applications, in particular insect repellent lotion, spray, solution or cream, mosquito repellents, human-smell-masking applications, perfume compositions and tooth paste.

The present invention is based on the recognition that the compositions according to the present invention comprising at least one linear 1 ,2-alkanediol having a carbon chain of 5 to 13 carbon atoms and/or at least one linear 2,3-alkanediol having a carbon chain of 5 to 13 carbon atoms are multifunctional compositions which provide an excellent antioxidation enhancing effect of an antioxidant in a cosmetic or pharmaceutical composition or homecare product, an excellent sensory properties improving effect of a cosmetic or pharmaceutical composition comprising a liquid lipophilic component, excellent antimicrobial effect, an excellent malodour coverage effect, excellent effects in modulating or modficating or suppressing fragrance notes, and an excellent sebum reduction effect which leads to a reduced or minimized sebum overproduction of the skin or scalp and/or reduces dandruff of the skin or scalp upon application of the composition of the present invention.

Therefore, the utilization of the compositions according to the present invention leads to an enhanced antioxidative effect of an antioxidant, especially in a cosmetic or pharmaceutical composition or homecare product.

Additionally, the utilization of the compostions according to the present invention results in an improvement of the sensory properties in terms of spreadability, fatty/greasy skin feeling and absorption (remaining residue on the skin upon application) of a cosmetic or pharmaceutical composition comprising a liquid lipophilic component.

Furthermore, the utilisation of the compositions according to the present invention leads to a reduced production of compounds having an unpleasant odour from sweat by microbes on the skin or scalp which is inhibited due to the excellent antimicrobial effect. Additionally, the malodour of compounds contained in sweat is efficiently reduced due to the coverage of unpleasant odours by the composition according to the present invention (malodour coverage). The compositions according to the present invention provide excellent effects for modulating, modificating or suppressing undesired fragrance notes. In particular, the malodour of 1-octen-3-ol can efficiently be covered and at the same time

the odour of insect repelling agents is not significantly hampered. Therefore, the compositions of the present invention can be used for providing efficient insect repellents and for masking insect alluring odours. Moreover, the compositions according to the present invention are compatible with different media such as water, ethanol or glycols.

Consequently, the compositions according to the present invention are highly efficient in enhancing the antioxidative effect of an antioxidant, in improving spreadability, fatty/greasy skin feeling and absorption of a liquid lipophilic component, in sebum control, in malodour management, in the modulation of fragrance notes, in the modification of fragrance notes, in the suppression of fragrance notes, in topical applications, in the coverage of the malodour of 1 -octen-3-ol, in masking insect alluring odours and in repelling insects.

Finally, the compositions or the cosmetic or pharmaceutical compositions or homecare products according to the present invention are toxicologically acceptable, readily tolerated by the skin, stable, inexpensive to prepare, easy to formulate, active at different pH values and do not possess an unpleasant smell.

**Figures**

Figure 1 is a diagram showing the synergistic antimicrobial effect of 1,2-heptanediol and 2,3-heptanediol.

Figures 2a and 2b are diagrams showing odour modification results for C7 and C8 alkanediols according to Example B.2

Figure 3 is a diagram showing odour modification results of compositions containing 1-octen-3-ol according to Example B.3.

Figure 4 is a diagram showing the impact of different 1,2-alkanediols and 2,3-alkanediols on the sebum production of sebaceous glands.

Figure 5 is a diagram showing the impact of 1,2-nonanediol at different concentrations on the sebum production of sebaceous glands.

Figures 6a and 6b are diagrams showing the ROS scores of different compositions comprising tocopherol and/or a 1,2-alkanediol or a 2,3-alkanediol according to the present invention in a lipophilic test system.

Figures 7a and 7b are diagrams showing the ROS scores of different compositions comprising tocopherol and/or a 1,2-alkanediol or a 2,3-alkanediol according to the present invention in an aqueous/alcoholic test system.

Figure 8 is a diagram showing the delta IP values of tocopherol, tocopherol and 1,2-heptanediol and tocopherol and a blend of 1,2-heptanediol and 2,3-heptanediol (95 : 5).

Figure 9 is a diagram showing the delta IP values of tocopherol and 1,2-heptanediol, tocopherol and 2,3-heptanediol and tocopherol and a blend of 1,2-heptanediol and 2,3-heptanediol (98 : 2).

Figure 10 is a diagram showing the delta IP values of tocopherol and 1,2-heptanediol, tocopherol and 2,3-heptanediol and tocopherol and a blend of 1,2-heptanediol and 2,3-heptanediol (99 : 1).

Figure 11 is a diagram showing the delta IP values of tocopherol and 1,2-hexanediol, tocopherol and 2,3-hexanediol and tocopherol and a blend of 1,2-hexanediol and 2,3-hexanediol (95 : 5).

Figure 12 is a diagram showing the delta IP values of tocopherol and 1,2-hexanediol, tocopherol and 2,3-hexanediol and tocopherol and a blend of 1,2-hexanediol and 2,3-hexanediol (50 : 50).

Figure 13 is a diagram showing the delta IP values of tocopherol and 1,2-octanediol, tocopherol and 2,3-octanediol and tocopherol and a blend of 1,2-octanediol and 2,3-octanediol (50 : 50).

Figure 14 is a diagram showing the delta IP values of tocopherol and 1,2-decanediol, tocopherol and 2,3-decanediol and tocopherol and a blend of 1,2-decanediol and 2,3-decanediol (95 : 5).

Figure 15 is a diagram showing the delta IP values of tocopherol and 1,2-decanediol, tocopherol and 2,3-decanediol

and tocopherol and a blend of 1,2-decanediol and 2,3-decanediol (50 : 50).

Figure 16 is a diagram showing the delta IP values of tocopherol and 1,2-heptanediol, tocopherol and 2,3-hexanediol and tocopherol and a blend of 1,2-heptanediol and 2,3-hexanediol (50 : 50).

Figure 17 is a diagram showing the delta IP values of tocopherol and 1,2-heptanediol, tocopherol and 2,3-octanediol and tocopherol and a blend of 1,2-heptanediol and 2,3-otanediol (95 : 5).

Figure 18 is a diagram showing the delta IP values of tocopherol and 1,2-nonanediol, tocopherol and 2,3-nonanediol and tocopherol and a blend of 1,2-nonanediol and 2,3-nonanediol (50 : 50).

Figure 19 is a diagram showing the delta IP values of Symdecanox HA and 1,2-heptanediol, Symdecanox HA and 2,3-heptanediol and Symdexanox HA and a blend of 1,2-heptanediol and 2,3-heptanediol (95 : 5).

Figure 20 is a diagram showing the delta IP values of Symdecanox HA and 1,2-octanediol, Symdecanox HA and 2,3-octanediol and Symdexanox HA and a blend of 1,2-octanediol and 2,3-octanediol (95 : 5).

Figure 21 is a diagram showing the delta IP values of Pentaerythrityl Tetra-dit-butyl Hydroxyhydrocinnamate and 1,2-heptanediol, Pentaerythrityl Tetra-di-t-butyl Hydroxyhydrocinnamate and 2,3-heptanediol and Pentaerythrityl Tetra-di-t-butyl Hydroxyhydrocinnamate and a blend of 1,2-heptanediol and 2,3-heptanediol (95 : 5).

Figure 22 is a diagram showing the delta IP values of Hydroxyacetophenone and 1,2-heptanediol, Hydroxyacetophenone and 2,3-heptanediol and hydroxyacetophenone.

Figure 23 is a diagram showing the delta IP values of Ascorbyl Palmitate and 1,2-heptanediol, Ascorbyl Palmitate and 2,3-heptanediol.

Figure 24 is a diagram showing the ROS scores of different compositions comprising Dihydroavenanthramide D and 1,2-heptanediol or 2,3-heptanediol or blend of 1,2-heptanediol and 2,3-heptanediol according to the present invention in an aqueous/alcoholic test system.

Figure 25 is a diagram showing the ROS scores of different compositions comprising Cannabidiol and 1,2-heptanediol or 2,3-heptanediol or a blend of 1,2-heptanediol and 2,3-heptanediol according to the present invention in an aqueous/alcoholic test system.

Figure 26a and 26b are diagrams showing the masking effect according to Example B.4 (Samples A, B, C, D).

The present invention relates to the following aspects:

1. A cosmetic or pharmaceutical composition, comprising or consisting of:

(a) at least one linear 1,2-alkanediol having a carbon chain of 5 to 13 carbon atoms; and
(b) at least one linear 2,3-alkanediol having a carbon chain of 5 to 13 carbon atoms;

or
a cosmetic or pharmaceutical composition, comprising or consisting of:

(a) at least one linear 2,3-alkanediol having a carbon chain of 5 to 13 carbon atoms.

2. A composition, comprising or consisting of:

(a) at least one linear 1,2-alkanediol having a carbon chain of 5 to 13 carbon atoms; and
(b) at least one linear 2,3-alkanediol having a carbon chain of 5 to 13 carbon atoms;

or
a composition, comprising or consisting of:
(b) at least one linear 2,3-alkanediol having a carbon chain of 5 to 13 carbon atoms.

3. The composition of aspect 1 or aspect 2, wherein

i.component (a) is 1,2-pentanediol and the component (b) is 2,3-pentanediol, or
ii.component (a) is 1,2-hexanediol and the component (b) is 2,3-hexanediol, or
iii.component (a) is 1,2-heptanediol and the component (b) is 2,3-heptanediol, or
iv.component (a) is 1,2-octanediol and the component (b) is 2,3-octanediol, or
v.component (a) is 1,2-nonanediol and the component (b) is 2,3-nonanediol, or
vi.component (a) is 1,2-decanediol and the component (b) is 2,3-decanediol, or
vii.component (a) is 1,2-undecanediol and the component (b) is 2,3-undecanediol, or
viii. component (a) is 1,2-dodecanediol and the component (b) is 2,3-dodecanediol, or
ix.component (a) is 1,2-tridecanediol and the component (b) is 2,3-tridecanediol.

4. The composition of aspect 1 or aspect 2, wherein

x.component (a) is 1,2-heptanediol and the component (b) is selected from the group consisting of 2,3-octanediol and 2,3-nonanediol, or
xi.component (a) is 1 ,2-ocatnediol and the component (b) is selected from the group consisting of 2,3-heptanediol and 2,3-nonanediol, or
xii.component (a) is 1,2-nonanediol and the component (b) is selected from the group consisting of 2,3-heptanediol and 2,3-octanediol.

5. The composition according to any one of aspects 1 to 4, wherein the ratio of the compounds (a) and (b) is in a range of 50 : 50 to 99.9 : 0.1, preferably wherein the ratio of the compounds (a) and (b) is in a range of 75 : 25 to 99 : 1, more preferred wherein the ratio of the compounds (a) and (b) is in a range of 80 : 20 to 98 : 2, still more preferred wherein the ratio of the compounds (a) and (b) is in a range of 90 : 10 to 95 : 5.

6. The composition according to any one of aspects 1 to 5, comprising the component (a) and the component (b) in an amount of 0.001 to 15.0 % by weight, particularly in an amount of 0.01 to 10.0 % by weight, more preferred in an amount of 0.1 to 5.0 % by weight, still more preferred in an amount of 0.3 to 3.0 % by weight, most preferred in an amount of 0.5 to 1.0 % by weight, based on the total weight of the composition.

7. The composition according to any one of aspects 1 to 6, further comprising:
(c) at least one or more insect repellent compounds, in particular an insect repellent compound selected from the group consisting of: 1-(1-Methylpropoxycarbonyl)-2-(2-hydroxyethyl)piperidine, N,N-Diethyl-meta-toluamide, p-Menthane-3,8-diol, ethyl butylacetylaminopropionate, 2-undecanone, as well as essential oils, such as citronella oil, lemongrass oil, lavender oil, neem oil and eucalyptus oil, in particular p-Menthane-3,8-diol, essential oils, such as citronella oil, lemongrass oil, lavender oil, neem oil and eucalyptus oil.

8. The composition according to aspect 7, comprising the compound (c) in an amount of 0.1 to 50.0 % by weight, particularly in an amount of 0.5 to 45.0 % by weight, more particularly in an amount of 1.0 to 40 % by weight, most particularly in an amount of 2.0 to 25.0 % by weight, based on the total weight of the composition.

9. The composition according to any one of aspects 1 to 8, further comprising:
(d) at least one cosmetically or pharmaceutically active substance and/or additive.

10. The composition according to aspect 9, wherein the at least one cosmetically or pharmaceutically active substance and/or additive is selected from the group consisting of antimicrobial compounds, cooling agents, agents against ageing of the skin, antioxidants, chelating agents, emulsifiers, preservatives, green and synthetic polymers, rheology additives, oils, fragrances or perfume oils, polyols and mixtures of two or more of the afore-mentioned substances.

11. The composition according to any one of aspects 2 to 10, wherein the composition is a cosmetic or pharmaceutical composition or homecare product.

12. Use of the composition according to any one of aspects 1 to 11

- for enhancing the antioxidative effect of an antioxidant, or
- for improving the sensory properties, i.e. skin sensation, of a liquid lipophilic component, for sebum control, or
- for malodour management, or for modulating fragrance notes, or

- for modification of fragrance notes, or
- for suppression of fragrance notes, or for topical applications, or
- for coverage of the malodour of 1-octen-3-ol, or for masking insect alluring odours, or
- for repelling insects.

13. Cosmetic product comprising a composition according to any one of aspects 1 to 11, wherein the product is selected from the group consisting of a deodorant and/or antiperspirant, an aerosol-based deo spray, deo pump spray, deo stick, deo roll on, deo cream, deo wipes, deo crystals, pickering emulsions, hydrodispersion gels, skin balms, shampoo, shower gel, foam bath, micellar water, facial cleansing solutions, cleansing wipes, intimate spray, intimate cream, intimate wash lotion, intimate wipes, aerosol-based foot spray, foot pump spray, foot bath, foot balm, soap, liquid washing, shower and bath preparation, bath product, bath capsule, bath oil, bath tablet, bath salt, bath soap, effervescent preparation, mouth wash concentrate, mouth wash ready to use, repellent, anti-insect application, in particular insect repellent lotion, spray, solution or cream, mosquito repellent, human-smell-masking application, perfume composition and tooth paste.

14. Pharmaceutical comprising a composition according to any one of aspects 1 to 11, for use in the prevention and treatment of a condition of the skin or mucosa.

15. Homecare product comprising a composition according to any one of aspects 1 to 11, wherein the product is selected from the group consisting of all-purpose cleaners, dishwashing detergents, hard floor and surface cleaners, glass cleaners, carpet cleaners, oven cleaners, laundry detergents, fabric softeners, laundry scent, scent lotions, car shampoo, rim block gel, car shampoo, furniture polish, all afore-mentioned goods also in encapsulated form or air fresheners.

## Detailed description of the invention

The present invention is specified in the appended claims. The invention itself, and its preferred variants, other objects and advantages, are however also apparent from the following detailed description in conjunction with the accompanying examples.

The present invention relates in a first aspect to a cosmetic or pharmaceutical composition or homecare product comprising or consisting of:

(a) at least one linear 1,2-alkanediol having a carbon chain of 5 to 13 carbon atoms; and
(b) at least one linear 2,3-alkanediol having a carbon chain of 5 to 13 carbon atoms.

In an alternative the present invention relates in a first aspect to a cosmetic or pharmaceutical composition or homecare product comprising or consisting of:
(b) at least one linear 2,3-alkanediol having a carbon chain of 5 to 13 carbon atoms.

Furthermore, the present invention relates in a second aspect to a composition comprising or consisting of:

(a) at least one linear 1,2-alkanediol having a carbon chain of 5 to 13 carbon atoms; and
(b) at least one linear 2,3-alkanediol having a carbon chain of 5 to 13 carbon atoms.

in an alternative the present invention relates in a second aspect to a composition product comprising or consisting of:
(b) at least one linear 2,3-alkanediol having a carbon chain of 5 to 13 carbon atoms.

Preferably, the composition comprising:

(a) at least one linear 1,2-alkanediol having a carbon chain of 5 to 13 carbon atoms; and/or
(b) at least one linear 2,3-alkanediol having a carbon chain of 5 to 13 carbon atoms; is a cosmetic or pharmaceutical composition or homecare product.

The term "comprising" means that the named components are essential, but other components may be added and is still embraced by the present invention.

The term "consisting of" as used according to the present invention means that the total amount of components (a) to (b) adds up to 100 % by weight, based on the total weight of the compoisiton or the cosmetic or pharmaceutical composition or homecare product, and signifies that the subject matter is closed-ended and can only include the limitations that are expressly recited.

Whenever reference is made to "comprising" it is intended to cover both meanings as alternatives, that is the meaning can be either "comprising" or "consisting of", unless the context dictates otherwise.

The term "at least one ..." means that the composition or cosmetic or pharmaceutical composition or homecare product according to the present invention can comprise either one or a mixture of two, three, four, five, six or even more different of the respective components following said term.

The term "optionally" means that the subsequently described compound may but need not to be present in the composition, and that the description includes variants, in which the compound is included or variants, in which the compound is absent.

Alkanediols are glycols, i.e. any of a class of organic compounds belonging to the alcohol family; in the molecule of a glycol, two hydroxyl (-OH) groups are attached to different carbon atoms of a carbon chain.

In the context of the present text, the terms "1,2-alkanediol" and "2,3-alkanediol" includes both the corresponding S-configured enantiomers and also the R-enantiomers as well as arbitrary mixtures of these S- and R-configured enantiomers, i.e. mixtures of racemates of the respective diols.

The compositions according to the first aspect or second aspect of the present invention comprises in a first alternative a component (a), i.e. at least one linear 1,2-alkanediol having a carbon cain of 5 to 13 carbon atoms, and a component (b), i.e. at least one linear 2,3-alkanediol having a carbon cain of 5 to 13 carbon atoms.

In a second alternative, the compositions according to the first aspect or second aspect of the present invention comprises only component (b), i.e. at least one linear 2,3-alkanediol having a carbon chain of 5 to 13 carbon atoms.

**Component (a)**

The component (a) in the compositions according to the first aspect or second aspect of the present invention is at least one linear 1,2-alkanediol having a carbon chain of 5 to 13 carbon atoms.

The term "at least one linear 1,2-alkanediol having a carbon chain of 5 to 13 carbon atoms" means that the compositions according to the present invention can comprise either one, two, three or even more different 1,2-alkanediols.

Preferably, the compositions according to the present comprise one or two different 1,2-alkanediols.

. The at least one linear 1,2-alkanediol having a carbon chain of 5 to 13 carbon atoms in the compositions according to the first and second aspect of the present invention are selected from the group consisting of 1,2-pentanediol, 1,2-hexanediol, 1,2-heptanediol, 1,2-octanediol, 1,2-nonanediol, 1,2-decanediol, 1,2-undecanediol, 1,2-dodecanediol, 1,2-tridecanediol, and mixtures thereof.

The component (a) belongs to the category of alkanediols and are straight chain alkanediols and are represented by the following formulae:

1,2-pentanediol

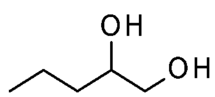

1,2-hexanediol

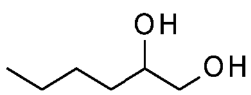

1,2-heptanediol

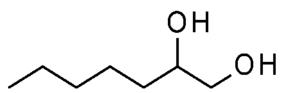

1,2-octanediol

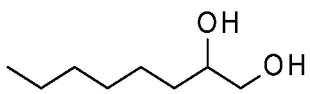

1,2-nonanediol

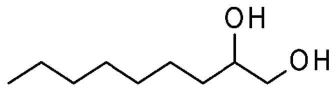

1,2-decanediol

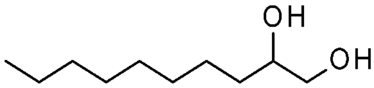

1,2-undecanediol

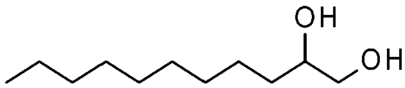

1,2-dodecanediol

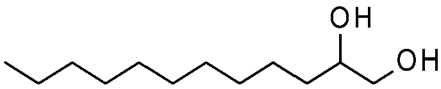

1,2-tridecanediol

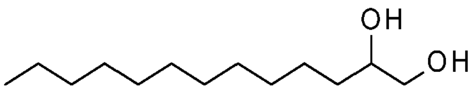

More preferably, the component (a) is 1,2-heptanediol or 1,2-octanediol.

Of the aforesaid linear 1,2-alkanediols the following 1,2-alkanediols are especially preferred: 1,2-pentanediol, 1,2-hexanediol, 1,2-heptanediol. Said alkanediols are liquid at a purity of 90 to 99 %.

Of the aforesaid liquid alkanediols 1,2-pentanediol, 1,2-hexanediol and 1,2-heptanediol or mixtures of said liquid alkanediols are particularly preferred. Said 1,2-alkanediols can be easier incorporated into semi-finished products or final products.

Preferably, the composition of the present invention comprises the component (a) in an amount of 0.001 to 15.0 % by weight, particularly in an amount of 0.01 to 10.0 % by weight, more preferred in an amount of 0.1 to 5.0 % by weight, still more preferred in an amount of 0.3 to 3.0 % by weight, most preferred in an amount of 0.5 to 1.0 % by weight, based on the total weight of the composition.

**Component (b)**

The component (b) in the compositions according to the first aspect or second aspect of the present invention is at least one linear 2,3-alkanediol having a carbon chain of 5 to 13 carbon atoms.

The term "at least one linear 2,3-alkanediol having a carbon chain of 5 to 13 carbon atoms" means that the compositions according to the present invention can comprise either one, two, three or even more different 2,3-alkanediols.

Preferably, the compositions according to the present comprise one or two different 2,3-alkanediols.

The at least one linear 2,3-alkanediol having a carbon chain of 5 to 13 carbon atoms in the compositions according to the first and second aspect of the present invention are selected from the group consisting of 2,3-pentanediol, 2,3-hexanediol, 2,3-heptanediol, 2,3-octanediol, 2,3-nonanediol, 2,3-decanediol, 2,3-undecanediol, 2,3-dodecanediol, 2,3-tridecanediol, and mixtures thereof.

The component (b) belongs to the category of alkanediols and are straight chain alkanediols and are represented by the following formulae:

2,3-pentanediol

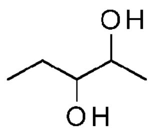

2,3-hexanediol

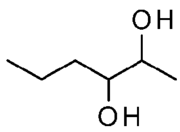

2,3-heptanediol

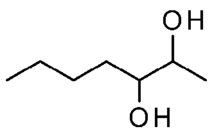

2,3-octanediol

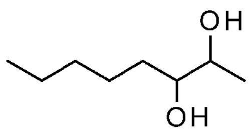

2,3-nonanediol

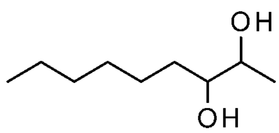

2,3-decanediol

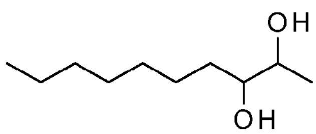

2,3-undecanediol

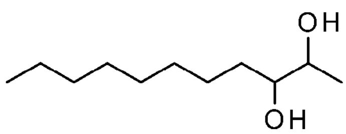

2,3-dodecanediol

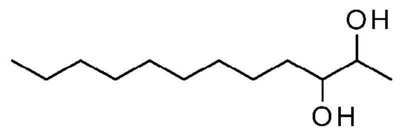

2,3-tridecanediol

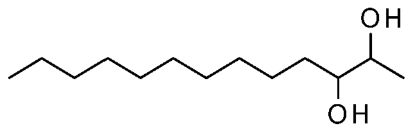

2,3-tetradecanediol

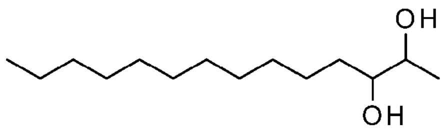

More preferably, the component (b) is 2,3-heptanediol or 2,3-octanediol.

Of the aforesaid linear 2,3-alkanediols the following 2,3-alkanediols are especially preferred: 2,3-pentanediol, 2,3-hexanediol, 2,3-heptanediol, 2,3-octanediol, 2,3-nonanediol, 2,3-decanediol, 2,3-undecanediol, 2,3-dodecanediol and 2,3-tridecanediol. Said alkanediols are liquid at a purity of 90 to 99 %.

Of the aforesaid liquid alkanediols 2,3-pentanediol, 2,3-hexanediol, 2,3-heptanediol, 2,3-octanediol, 2,3-nonanediol or mixtures of said liquid alkanediols are particularly preferred. Said 1,2-alkanediols can be easier incorporated into semi-finished products or final products.

Due to their sebum reducing effect, the 2,3-alkanediols are particualry preferred in the compositions according to the first aspect or second aspect of the present invention.

Preferably, the composition of the present invention comprises the component (b) in an amount of 0.001 to 15.0 % by weight, particularly in an amount of 0.01 to 10.0 % by weight, more preferred in an amount of 0.1 to 5.0 % by weight, still more preferred in an amount of 0.3 to 3.0 % by weight, most preferred in an amount of 0.5 to 1.0 % by weight, based on the total weight of the composition.

According the first aspect and second aspect of the present invention, the composition comprises a mixture comprising at least one linear 1,2-alkanediol having a carbon chain of 5 to 13 carbon atoms and at least one linear 2,3-alkanediol having a carbon chain of 5 to 14 carbon atoms wherein the number of the carbon atoms of the 1,2-alkanediol and 2,3-alkanediol is either same or different.

If both, the 1,2-alkanediol and 2,3-alkanediol have the same number of carbon atoms, such an alkanediol combination is herein also referred to as "homo alkanediol mixture" or "homo combination". For example, the linear 1,2-alkanediol and the linear 2,3-alkanediol have a carbon chain of 7 carbon atoms.

If the 1,2-alkanediol and 2,3-alkandeiol have a different number of carbon atoms, such an alkanediol combination is herein also referred to as "hetero alkanediol mixture" or "hetero combination". For example, the linear 1,2-alkanediol has a carbon chain of 7 carbon atoms and the linear 2,3-alkanediol has a carbon chain of 8 carbon atoms.

Hence, the compositions according to the first aspect or second aspect of the present invention may include one of the following combinations of component (a) and component (b):

- 1,2-pentanediol and 2,3-pentanediol; or
- 1,2-pentanediol and 2,3-hexanediol; or
- 1,2-pentanediol and 2,3-heptanediol; or
- 1,2-pentanediol and 2,3-octanediol; or
- 1,2-pentanediol and 2,3-nonanediol; or
- 1,2-pentanediol and 2,3-decanediol; or
- 1,2-pentanediol and 2,3-undecanediol; or
- 1,2-pentanediol and 2,3-dodecanediol; or
- 1,2-pentanediol and 2,3-tridecanediol; or
- 1,2-hexanediol and 2,3-pentanediol; or

- 1,2-hexanediol and 2,3-hexanediol; or
- 1,2-hexanediol and 2,3-heptanediol; or
- 1,2-hexanediol and 2,3-octanediol; or
- 1,2-hexanediol and 2,3-nonanediol; or
- 1,2-hexanediol and 2,3-decanediol; or
- 1,2-hexanediol and 2,3-undecanediol; or
- 1,2-hexanediol and 2,3-dodecanediol; or
- 1,2-hexanediol and 2,3-tridecanediol; or
- 1,2-heptanediol and 2,3-pentanediol; or
- 1,2-heptanediol and 2,3-hexanediol; or
- 1,2-heptanediol and 2,3-heptanediol; or
- 1,2-heptanediol and 2,3-octanediol; or
- 1,2-heptanediol and 2,3-nonanediol; or
- 1,2-heptanediol and 2,3-decanediol; or
- 1,2-heptanediol and 2,3-undecanediol; or
- 1,2-heptanediol and 2,3-dodecanediol; or
- 1,2-heptanediol and 2,3-tridecanediol; or
- 1,2-octanediol and 2,3-pentanediol; or
- 1,2-octanediol and 2,3-hexanediol; or
- 1,2-octanediol and 2,3-heptanediol; or
- 1,2-octanediol and 2,3-octanediol; or
- 1,2-octanediol and 2,3-nonanediol; or
- 1,2-octanediol and 2,3-decanediol; or
- 1,2-octanediol and 2,3-undecanediol; or
- 1,2-octanediol and 2,3-dodecanediol; or
- 1,2-octanediol and 2,3-tridecanediol; or
- 1,2-nonanediol and 2,3-pentanediol; or
- 1,2-nonanediol and 2,3-hexanediol; or
- 1,2-nonanediol and 2,3-heptanediol; or
- 1,2-nonanediol and 2,3-octanediol; or
- 1,2-nonanediol and 2,3-nonanediol; or
- 1,2-nonanediol and 2,3-decanediol; or
- 1,2-nonanediol and 2,3-undecanediol; or
- 1,2-nonanediol and 2,3-dodecanediol; or
- 1,2-nonanediol and 2,3-tridecanediol; or
- 1,2-decanediol and 2,3-pentanediol; or
- 1,2-decanediol and 2,3-hexanediol; or
- 1,2-decanediol and 2,3-heptanediol; or
- 1,2-decanediol and 2,3-octanediol; or
- 1,2-decanediol and 2,3-nonanediol; or
- 1,2-decanediol and 2,3-decanediol; or
- 1,2-decanediol and 2,3-undecanediol; or
- 1,2-decanediol and 2,3-dodecanediol; or
- 1,2-decanediol and 2,3-tridecanediol; or
- 1,2-undecanediol and 2,3-pentanediol; or
- 1,2-undecanediol and 2,3-hexanediol; or
- 1,2-undecanediol and 2,3-heptanediol; or
- 1,2-undecanediol and 2,3-octanediol; or
- 1,2-undecanediol and 2,3-nonanediol; or
- 1,2-undecanediol and 2,3-decanediol; or
- 1,2-undecanediol and 2,3-undecanediol; or
- 1,2-undecanediol and 2,3-dodecanediol; or
- 1,2-undecanediol and 2,3-tridecanediol; or
- 1,2-dodecanediol and 2,3-pentanediol; or
- 1,2-dodecanediol and 2,3-hexanediol; or
- 1,2-dodecanediol and 2,3-heptanediol; or
- 1,2-dodecanediol and 2,3-octanediol; or
- 1,2-dodecanediol and 2,3-nonanediol; or

- 1,2-dodecanediol and 2,3-decanediol; or
- 1,2-dodecanediol and 2,3-undecanediol; or
- 1,2-dodecanediol and 2,3-dodecanediol; or
- 1,2-dodecanediol and 2,3-tridecanediol; or
- 1,2-tridecanediol and 2,3-pentanediol; or
- 1,2-tridecanediol and 2,3-hexanediol; or
- 1,2-tridecanediol and 2,3-heptanediol; or
- 1,2-tridecanediol and 2,3-octanediol; or
- 1,2-tridecanediol and 2,3-nonanediol; or
- 1,2-tridecanediol and 2,3-decanediol; or
- 1,2-tridecanediol and 2,3-undecanediol; or
- 1,2-tridecanediol and 2,3-dodecanediol; or
- 1,2-tridecanediol and 2,3-tridecanediol; or

Due to their outstanding effects as described above, the composition according to the first aspect or second aspect of the present invention comprises preferably one of the following hetero alkanediol mixtures:

a mixture comprising 1,2-hexanediol and 2,3-octanediol; or
a mixture comprising 1,2-octanediol and 2,3-hexanediol; or
a mixture comprising 1,2-octanediol and 1,2-heptanediol.

Furthermore, due to their outstanding effects as described above, the composition according to the first aspect or second aspect of the present invention comprises preferably one of the following hetero alkanediol mixtures:

a mixture comprising 1,2-pentanediol and 2,3-hexanediol; or
a mixture comprising 1,2-pentanediol and 1,2-heptanediol; or
a mixture comprising 1,2-pentanediol and 2,3-heptanediol; or
a mixture comprising 1,2-pentanediol and 2,3-octanediol; or
a mixture comprising 1,2-pentanediol and 1,2-nonanediol; or
a mixture comprising 1,2-pentanediol and 2,3-nonanediol.

More preferred, the composition according to the first aspect or second aspect of the present invention comprises preferably one of the following hetero alkanediol mixtures:

a mixture comprising 1,2-heptanediol and 1,2-octanediol; or
a mixture comprising 1,2-heptanediol and 2,3-octanediol; or
a mixture comprising 1,2-heptanediol and 1,2-nonanediol; or
a mixture comprising 1,2-heptanediol and 2,3-nonanediol.

In said preferred hetero alkanediol mixtures the 1,2-alkanediol and the 2,3-alkanediol have a different number of carbon atoms.

In a particular preferred variant, the compositions according to the first or second aspect of the present invention includes an alkanediol mixture or a combination wherein component (a) is 1,2-pentanediol and component (b) is 2,3-pentanediol.

In a further preferred variant, the compositions according to the first or second aspect of the present invention includes an alkanediol mixture or a combination wherein component (a) is 1,2-hexanediol and component (b) is 2,3-hexanediol.

In a further preferred variant, the compositions according to the first or second aspect of the present invention includes an alkanediol mixture or a combination wherein component (a) is 1,2-octanediol and component (b) is 2,3-octanediol.

In a further preferred variant, the compositions according to the first or second aspect of the present invention includes an alkanediol mixture or a combination wherein component (a) is 1,2-nonanediol and component (b) is 2,3-nonaediol.

In a further preferred variant, the compositions according to the first or second aspect of the present invention includes an alkanediol mixture or a combination wherein component (a) is 1,2-decanediol and component (b) is 2,3-decanediol.

In a further preferred variant, the compositions according to the first or second aspect of the present invention includes an alkanediol mixture or a combination wherein component (a) is 1,2-undecanediol and component (b) is 2,3-undecanediol.

In a further preferred variant, the compositions according to the first or second aspect of the present invention includes an alkanediol mixture or a combination wherein component (a) is 1,2-dodecanediol and component (b) is 2,3-dodecanediol.

In a further preferred variant, the compositions according to the first or second aspect of the present invention includes an alkanediol mixture or a combination wherein component (a) is 1,2-tridecanediol and component (b) is 2,3-tridecanediol.

Particularly favorable is a combination including an alkanediol combination wherein component (a) is 1,2-heptanediol and component (b) is 2,3-heptanediol, or an alkanediol combination wherein component (a) is 1,2-octanediol and component (b) is 2,3-octanediol, or an alkanediol combination wherein component (a) is 1,2-nonanediol and component (b) is 2,3-nonanediol.

In said preferred homo alkanediol mixtures the 1,2-alkanediol and the 2,3-alkanediol have the same number of carbon atoms.

The above specified alkanediol compositions according to the first aspect or second aspect of the present invention surprisingly improve, even in a synergistic way, the antioxidative effect of an antioxidant as it is demonstrated by the following examples. Consequently, the compositions according to the present invention are highly suitable as ingredients in compositions for use in cosmetic or pharmaceutical compositions or homecare products which are susceptible for oxidation and which comprise an antioxidant, since they prevent oxidative degradation in the formulation and, thus, prolonge shelf life.

Additionally, said specified alkanediols compositions are capable to improve the skin sensation of liquid lipophilic components in a cosmetic or pharmaceutical composition as it is demonstrated by the following examples. Consequently, the compounds are highly suitable as ingredients in compositions for use in cosmetic or pharmaceutical compositions comprising a liquid lipophilic component which has an inherent poor spreadability, causes a fatty/greasy skin feeling or has only a poor absorption and leaves a residue on the skin or scalp upon application.

Moreover, the specified alkanediol compositions according to the present invention exhibits an excellent sebum reduction effect which leads to a reduced or minimized sebum overproduction of the skin or scalp and/or reduces dandruff of the skin or scalp upon application as it is demonstrated by the following examples. Therefore, the compositions of the present invention can be as ingredients in compositions of for sebum control.

The above specified alkanediol compositions according to the first aspect or second aspect of the present invention surprisingly possess a synergistic antimicrobial effect. Moreover, these compositions surprisingly exhibited a synergistic malodour coverage effect, and an excellent effect for masking insect alluring odours. In particular, the malodour of 1-octen-3-ol can efficiently be covered by these compositions. Therefore, these compositions can be used in malodour management applications, for topical applications, for coverage of the malodour of 1-octen-3-ol and for masking insect alluring odours. Furthermore, the strong first smell of insect repellents such as citriodiol can be reduced while the longterm efficacy of the insect repellent is not significantly hampered. Therefore, these compositions can be used in efficient and more pleasant insect repelling products. The single components (a) provide an excellent sebum reduction effect and it is believed that the combination of the components (a) and (b) leads to an improved sebum reduction effect, maybe even a synergistic sebum reduction effect. Finally, these compositions can be used for modulating, or modificating or suppression of fragrance notes of different compositions.

In the context of the present invention the term "malodour coverage" means that the unpleasant smell of one or more substances or a composition is entirely suppressed or reduced, but the coverage is not achieved by a beneficial intrinsic odour of an added compound. Generally, an unpleasant smell of a substance or a composition is improved by adding a compound exhibiting a malodour coverage effect, however the added compound does not improve the unpleasant smell of the substance or composition due to a beneficial intrinsic odour of the added comdpound. The added compound is an odour neutral molecule without a beneficial intrinsic odour. Therefore, in the context of the present invention the term "malodour coverage" does not relate to fragrance compounds which only cover an unpleasant smell due to a strong beneficial intrinsic odour.

In the context of the present invention, the term "malodour management" means that the formation of an unpleasant smell is entirely suppressed or inhibited and/or an already existing unpleasant smell is entirely suppressed or reduced. For example, the suppression or inhibition of the formation of an unpleasant smell may be achieved by the suppression or inhibition of the production of sweat and/or by the suppression or inhibition of microbial activity. The suppression or reduction of an already existing unpleasant smell is achieved by malodour coverage. Therefore, "malodour management" may be achieved by inhibiting malodour development and/or malodour coverage.

In the context of the present invention, the term "topical application" means that the application takes place on a surface, or on the skin or scalp of a human or mammalian.

In a still more preferred embodiment, the composition of the present invention is a composition,

wherein the component (a) is 1,2-heptanediol and the component (b) is selected from the group consisting of 2,3-octanediol and 2,3-nonanediol, or
wherein the component (a) is 1,2-ocatnediol and the component (b) is selected from the group consisting of 2,3-heptanediol and 2,3-nonanediol, or
wherein the component (a) is 1,2-nonanediol and the component (b) is selected from the group consisting of 2,3-heptanediol and 2,3-octanediol.

The compositions according to this preferred embodiment enhances the antioxidative effect of an antioxidant in a cosmetic or pharmaceutical composition or homecare product, improves the sensory properties, i.e. skin sensation, of a cosmetic or pharmaceutical composition comprising a liquid lipophilic component, or possess a synergistic antimicrobial effect. Moreover, these compositions exhibit an excellent malodour coverage effect maybe even a synergistic malodour coverage effect, and an excellent effect for masking insect alluring odours. The single components (a) provide an excellent sebum reduction effect and it is believed that the combination of the components (a) and (b) leads to an improved sebum reduction effect, maybe even a synergistic sebum reduction effect. Therefore, these compositions can be used in sebum control, in malodour management applications, in topical applications and in masking insect alluring odours. Finally, these compositions can be used for modulating, or modificating or suppression of fragrance notes of different compositions.

The compositions according to the frist aspect or second aspect of the present invention, comprises component (a) and component (b) in a ratio in a range from 99.5 : 0.5 to 0.5 : 99.5; the ratio of the compounds (a) and (b) is preferably from 99 : 1 to 1 : 99, more preferred the ratio of the compounds (a) and (b) is from 98:2 to 2:98.

In a preferred variant, the compositions according to the first aspect or second aspect of the present invention comprises the 1,2-alkanediol (component (a)) and the 2,3-alkanediol (component (b)) in a ratio in a range of 50 : 50 to 99.9 : 0.1, preferably in a ratio in a range of 75 : 25 to 99 : 1, more preferred in a ratio in a range of 80 : 20 to 98 : 2, still more preferred in a ratio in a range of 90 : 10 to 95 : 5.

In the cosmetic or pharmaceutical composition according to the first aspect of the present invention, 1,2-alkanediol and 2,3-alkanediol are comprised preferably in a ratio in a range of 98 : 2 to 99.9 : 0.1.

In the homecare products according to the first aspect of the present invention, 1,2-alkanediol and 2,3-alkanediol are comprised in a ratio in a range of 95 : 5 to 99.9 : 0.1.

More preferred, the 1,2-alkanediol (component (a)) and 2,3-alkanediol (component (b)) are comprised in the composition according to the first aspect or second aspect of the present invention preferred in a ratio in a range of ≥ 95 : ≤ 5, more preferred in a ratio of ≥ 96 : ≤ 4; still more preferred in a ratio of ≥ 97 : ≤ 3, and most preferred in a ratio of ≥ 98 : ≤ 2.

Most of all the composition according to the first aspect of the present invention comprises 1,2-alkanediol (component (a)) and 2,3-alkanediol (component (b)) in a ratio in a range of ≥ 95 : ≤ 5, including the ratios ≥ 95.5 : ≤ 4.5; ≥ 96 : ≤ 4; ≥ 96.5 : ≤ 3.5; ≥ 97 : ≤ 3; ≥ 97.5 : 2,5 and ≥ 98.0 : ≤ 2.0.

Even more preferred the composition according to the first aspect of the first aspect or second aspect of the present invention comprises the 1,2-alkanediol (component (a)) and 2,3-alkanediol (component (b)) in a ratio in a range of ≥ 98 : ≤ 2, including the ratios of ≥ 98.1 : ≤ 1.9; ≥ 98.2 : ≤ 1.8; ≥ 98.3 : ≤ 1.7; ≥ 98.4 : ≤ 1.6; ≥ 98.5 : ≤ 1.5; ≥ 98.6 : ≤ 1.4; ≥ 98.7 : ≤ 1.3; ≥ 98.8 : ≤ 1.2; ≥ 98.9 : ≤ 1.1; ≥ 99 : ≤ 1.0; ≥ 99.1 : ≤ 0.9; ≥ 99.2 : ≤ 0.8; ≥ 99.3 : ≤ 0.7; ≥ 99.4 : ≤ 0.6; ≥ 99.5 : ≤ 0.5; ≥ 99.6 : ≤ 0.4; ≥ 99.7 : ≤ 0.3; ≥ 99.8 : ≤ 0.2 and ≥ 99.9 : ≤ 0.1.

By using compositions, wherein the ratio of the component (a) and component (b) is as defined above, beneficial effects and maybe even synergistic effects such as antioxidative enhancing effect, improvement of skin sensation of liquid lipophilic components, sebum reduction effect, malodour coverage effect and antimicrobial effects can be achieved. Moreover, fragrance notes can be modulated, and/or modified and/or suppressed.

Preferably, the compositions of the present invention are compositions wherein the ratio of the component (a) and component (b) is from 95 : 5 to 5 : 95, or from 90 : 10 to 10 : 90, or from 80 : 20 to 20 : 80, or from 70 : 30 to 30 : 70, or from 60 : 40 to 40 : 60, or the ratio is 50 : 50. By using compositions wherein the ratio of the compounds (a) and (b) is as defined above, synergistic effects such as sebum reduction effect, malodour coverage effect and antimicrobial effects can be achieved. Moreover, fragrance notes can be modulated, and/or modified and/or suppressed. Therefore, the compositions are highly suitable for subum control, maldour management, topical applications and modulating fragrances notes, modificating fragrance notes and suppressing fragrance notes. Furthermore, with these compositions the malodour of 1-octen-3-ol can be efficiently covered. Therefore, the compositions are useful in the coverage of the malodour of 1-octen-3-ol and for masking insect alluring odours. In addition, with these compositions the strong first smell of insect repellents such as citriodiol can be reduced while the longterm efficacy of the insect repellent is not significantly hampered. Therefore, these compositions can be used in efficient and more pleasant insect repelling products.

Preferably, the composition of the present invention is a composition, wherein the ratio of the component (a) and component (b) is from 99.5 : 0.5 to 50 : 50, or from 99 : 1 to 50 : 50, or from 98 : 2 to 50 : 50, or from 95 : 5 to 50 : 50, or from 90 : 10 to 50 : 50, or from 80 : 20 to 50 : 50, or from 70 : 30 to 50 : 50, or from 60 : 40 to 50 : 50, most

preferably ratio of the compounds (a) and (b) is from 95 : 5 to 50 : 50.

Preferably, the composition of the present invention is a composition, wherein the ratio of the component (a) and component (b) is 99.5 : 0.5, or 99 : 1, or 98 : 2, or 95 : 5, or 90 : 10, or 80 : 20, or 70 : 30, or 60 : 40, or 50 : 50, or 40 : 60, or 30 : 70, or 20 : 80, or 10 : 90, or 5 : 95, or 2 : 98, or 1 : 99, or 0.5 : 99.5, more preferably the ratio of the compounds (a) and (b) is 99 : 1, or 98 : 2, or 95 : 5, or 90 : 10, or 80 : 20, or 70 : 30, or 60 : 40, or 50 : 50, even more preferably the ratio of the compounds (a) and (b) is 95 : 5, or 90 : 10, or 80 : 20, or 50 : 50.

In a more advantageous variant according to the first or second aspect of the present invention, in the compositions comprising a combination of a 1,2-alkanediol as component (a) and the corresponding 2,3-alkanediol as component (b) such as

1,2-pentanediol and 2,3-pentanediol, or
1,2-hexanediol and 2,3-hexanediol, or
1,2-heptanediol and 2,3-heptanediol, or
1,2-octanediol and 2,3-octanediol, or
1,2-nonanediol and 2,3-nonanediol, or
1,2-decanediol and 2,2-decanediol, or
1,2-undecanediol and 2,3-undecenaediol, or
1,2-dodecanediol and 2,3-dodecanediol, or
1,2-tridecanediol and 2,3-tridecanediol,
the 1,2-alkanediol (component (a)) and the 2,3-alkanediol (component (b)) are present in a ratio in a range of 50 : 50 to 99.9 : 0.1, preferably in a ratio in a range of 75 : 25 to 99 : 1, more preferred in a ratio in a range of 80 : 20 to 98 : 2, still more preferred in ratio in a range of 90 : 10 to 95 : 5.

Likewise, for said mixtures, the ratios of 1,2-alkanediol as component (a) : 2,3-alkanediol as component (b) or ranges of ratios as described above are also applicable.

By these mixing ratios the afore mentioned mixtures show improved or even synergistic effects as described above in direct comparison with the corresponding individual substances, 1,2-alkanediols or 2,3-alkanediols.

The component (a) (1,2-alkanediol) and the component (b) (2,3-alkanediol) according to the first aspect or second aspect of the present invention are present in the composition in an amount of 0.001 to 15.0 % by weight, based on the total weight of the composition. In a preferred variant, the composition comprises the component (a) (1,2-alkanediol) and component (b) (2,3-alkanediol) in an amount of 0.01 to 10.0 % by weight, based on the total weight of the composition In a more preferred variant, the component (a) (1,2-alkanediol) and component (b) are advantageously used in the composition in an amount of at 0.1 to 5.0 % by weight, based on the total weight of the composition. In a still more preferred variant, the component (a) (1,2-alkanediol) and the component (b) (2,3-alkanediol) are present in the composition in an amount of 0.3 to 3.0 % by weight, based on the total weight of the composition. Most preferred, the component (a) (1,2-alkanediol) and the component (b) (2,3-alkanediol) are advantageously used in the composition in an amount of 0.5 to 1.0 % by weight, based on the total weight of the composition.

In a particular preferred variant of the present invention, the composition according to the first aspect or second aspect comprises the 2,3-alkanediol in an amount of 0.001 to 15.0 % by weight, preferred in an amount of 0.01 to 10.0 % by weight, more preferred in an amount of 0.1 to 5.0 % by weight, still more preferred in an amount of 0.3 bis 3.0 % by weight, and most preferred in an amount of 0.5 to 1.0 % by weight, based on the total weight of the composition.

Even more preferred, the composition according to the first aspect or second aspect comprises the 2,3-alkanediol in an amount of 0.001 to 0.5 % weight, preferably in an amount of 0.005 to 0.1 % by weight and most preferably in an amount of 0.01 to 0.075 % by weight, based on the total weight of the composition.

In a second alternative, the compositions according to the first aspect or second aspect of the present invention comprises only component (b), i.e. at least one linear 2,3-alkanediol having a carbon chain of 5 to 13 carbon atoms. Hence, said composition according to the first aspect or second aspect of the present invention does not comprise a component (a) as defined herein.

In a preferred variant, the cosmetic or pharmaceutical composition or homecare product according to the first aspect of the present invention comprises only component (b), i.e. at least one linear 2,3-alkanediol having a carbon chain of 5 to 13 carbon atoms. Hence, the cosmetic or pharmaceutical composition or homecare product or composition according to the first aspect or second aspect of the present invention does not comprise a component (a) as defined herein.

The term "at least one linear 2,3-alkanediol having a carbon chain of 5 to 13 carbon atoms" means that the compositions according to the present invention can comprise either one, two, three or even more different 2,3-alkanediols.

Preferably, the compositions according to the present comprise one or two different 2,3-alkanediols.

The at least one linear 2,3-alkanediol having a carbon chain of 5 to 13 carbon atoms in the compositions

according to the first and second aspect of the present invention are selected from the group consisting of 2,3-pentanediol, 2,3-hexanediol, 2,3-heptanediol, 2,3-octanediol, 2,3-nonanediol, 2,3-decanediol, 2,3-undecanediol, 2,3-dodecanediol, 2,3-tridecanediol, and mixtures thereof.

Said specified 2,3-alkanediols improve the antioxidative effect of an antioxidant as it is demonstrated by the following examples. Consequently, the compounds are highly suitable as ingredients in compositions for use in cosmetic or pharmaceutical compositions or homecare products which are susceptible against oxidation and which comprise an antioxidant, since they prevent oxidative degradation in the formulation and, thus, prolonge shelf life.

Additionally, said specified 2,3-alkanediols are capable to improve the skin sensation of liquid lipophilic components in a cosmetic or pharmaceutical composition as it is demonstrated by the following examples. Consequently, the compounds are highly suitable as ingredients in compositions for use in cosmetic or pharmaceutical compositions comprising a liquid lipophilic component which has an inherent poor spreadability, causes a fatty/greasy skin feeling or has only a poor absorption and leaves a residue on the skin or scalp upon application.

Moreover, the 2,3-alkanediols exhibits an excellent sebum reduction effect which leads to a reduced or minimized sebum overproduction of the skin or scalp and/or reduces dandruff of the skin or scalp upon application of the composition of the present invention. Therefore, the compounds (a) and the compositions of the present invention can be used for sebum control.

The component (b), i.e. 2,3-heptanediol, 2,3-octanediol and 2,3-nonanediol exhibit an antimicrobial effect and a malodour coverage effect. Consequently, the compounds are highly suitable as ingredients in compositions for use in malodour management and in topical applications e.g. on surfaces, or on the skin or scalp of humans or mammalians.

Of the aforesaid linear 2,3-alkanediols the following 2,3-alkanediols are especially preferred: 2,3-pentanediol, 2,3-hexanediol, 2,3-heptanediol, 2,3-octanediol, 2,3-nonanediol, 2,3-decanediol, 2,3-undecanediol, 2,3-dodecanediol and 2,3-tridecanediol. Said alkanediols are liquid at a purity of 90 to 99 %.

Of the aforesaid liquid alkanediols 2,3-pentanediol, 2,3-hexanediol, 2,3-heptanediol, 2,3-octanediol, 2,3-nonanediol or mixtures of said liquid alkanediols are particularly preferred. Said 1,2-alkanediols can be easier incorporated into semi-finished products or final products.

Preferably, the component (b) is 2,3-heptanediol and 2,3-octanediol.

Due to their sebum reducing effect, the 2,3-alkanediols are particualry preferred in the compositions according to the first aspect or second aspect of the present invention.

Additionally, with the admixture of the corresponding liquid 2,3-alkanediol, even in small amounts, to a solid 1,2-alkanediol, the solid 1,2-alkanedioles, such as 1,2-octanediol, 1,2-nonanediol etc., can be solved, resulting in a liquid alkanediol mixture. The 2,3-alkanediol serves as solvent for the solid 1,2-alkanediols. Such liquid mixtures have the benefit that firstly the availability of the solid 1,2-alkanediol in the mixture is improved and secondly the incorporation of the solid 1,2-alkanediol in semi-finished products or final products is facilitated. This effect is particularly favorably for emulsions, in which lipophilic 1,2-alkanediols having a carbon chain of 8 or more carbon atoms, when used alone, tend to migrate into the oil phase or tend to precipitate or recrystallize.

With the solution of the solid 1,2-octanediol in the liquid 2,3-octanediol or of the solid 1,2-nonanediol in the liquid 2,3-nonanediol, the availability of the 1,2-octanediol or 1,2-nonanediol can be likewise improved in the end use.

Thus, with the afore-mentioned properties of the specified alkanediol mixtures including a 1,2-alkanediol and the respective 2,3-alkanediol the processability in formulations can be improved.

Hence, a good compromise can be achieved when the alkanediol mixture is combined in such a way as to maintain the above-described effects, while improving processability in formulations. The above specified 1,2-alkanediol and corresponding 2,3-alkanediol combinations solve this balancing problem over the 1,2-alkanediol substances or 2,3-alkanediol substances alone. This is well achieved by combinations such as comprising 1,2-pentanediol and 2,3-pentanediol but also for 1,2-hexanediol and 2,3-hexandiol. Also combinations such as comprising 1,2-heptanediol and 2,3-heptanediol, but also 1,2-octanediol and 2,3-octanediol show this effect. The same also counts for 1,2-nonanediol in combination with 2,3-nonanediol, but also for 1,2-decanediol in combination with 2,3-decanediol.

Furthermore, cosmetic or pharmaceutical composition, comprising at least one linear 2,3-alkanediol have the advantage of excellent formulation properties combined with good antimicrobial activity. Preferably such 2,3-alkandiols have a carbon chain of 5 to 13 carbon atoms. More preferably 6 to 11 carbon atoms, and even more preferably 8 to 10 carbon atoms. Longer chains have a stronger antimicrobial activity but this must be balanced with the formulability in cosmetic and pharmaceutical applications. In addition, a good skin feel is important and can be achieved by 2,3-alkanediols, inparticular those of chain length C8 and C9. This balance of formulability and antimicrobial properties is met with the above alkanediols, in particular those of chain length C8 to C10, thus C8, C9 and C10. However, also longer chain lengths of C11 and C12 are possible for 2,3-alkanediols which work better than the corresponding higher 1,2-alkanediols of the same length.

In a preferred variant of the second alternative of the composition according to the first aspect or second aspect of the present invention, the component (b) (2,3-alkanediol) is present in the composition in an amount of 0.001 to 15.0 % by weight, based on the total weight of the composition. In a preferred variant of the second alternative of the composition, the composition comprises the component (b) (2,3-alkanediol) in an amount of 0.01 to 10.0 % by weight, based on the total weight of the composition. In a more preferred variant, the component (b) is advantageously used in the composition in an amount of at 0.1 to 5.0 % by weight, based on the total weight of the composition. In a still more preferred variant, the component (b) (2,3-alkanediol) is present in the composition in an amount of 0.3 to 3.0 % by weight, based on the total weight of the composition. Most preferred, the component (b) (2,3-alkanediol) is advantageously used in the composition fo the second alternative in an amount of 0.5 to 1.0 % by weight, based on the total weight of the composition.

Even more preferred, the composition according to the first aspect or second aspect comprises the 2,3-alkanediol in an amount of 0.001 to 0.5 % weight, preferably in an amount of 0.005 to 0.1 % by weight and most preferably in an amount of 0.01 to 0.075 % by weight, based on the total weight of the composition.

**Component (c)**

In a preferred embodiment, the compositions of the present invention are compositions further comprising:

(c) at least one or more insect repellent compounds, in particular an insect repellent compound;
selected from the group consisting of: 1-(1-Methylpropoxycarbonyl)-2-(2-hydroxyethyl)piperidine, N,N-Diethyl-meta-toluamide, p-Menthane-3,8-diol, ethyl butylacetylaminopropionate, 2-undecanone, as well as essential oils, such as citronella oil, lemongrass oil, lavender oil, neem oil and eucalyptus oil, in particular p-Menthane-3,8-diol, essential oils, such as citronella oil, lemongrass oil, lavender oil, neem oil and eucalyptus oil.

Preferably, the compound (c) is at least one or more insect repellent compounds selected from the group consisting of 1-(1-Methylpropoxycarbonyl)-2-(2-hydroxyethyl)piperidine, N,N-Diethyl-meta-toluamide, p-Menthane-3,8-diol, ethyl butylacetylaminopropionate, 2-undecanone, as well as essential oils, such as citronella oil, lemongrass oil, lavender oil, neem oil and eucalyptus oil, in particular p-Menthane-3,8-diol, essential oils, such as citronella oil, lemongrass oil, lavender oil, neem oil and eucalyptus oil, more preferably selected from the group consisting of 1-(1-Methylpropoxycarbonyl)-2-(2-hydroxyethyl)piperidine, N,N-Diethyl-meta-toluamide, p-Menthane-3,8-diol, and ethyl butylacetylaminopropionate, most preferably the insect repellent compound is p-Menthane-3,8-diol (Citriodiol).

The compositions further comprising compound (c) are highly suitable for masking insect alluring odours and for repelling insects. The combination of the compounds (a) and (b) lead to the reduction of body odour and the masking of insect alluring odours like the odour of 1-octen-3-ol, and therefore biting insects are less attracted. In addition, the insect repelling compound (c) ensures that insects are repelled from the skin of the human or mammalian. These effects support each other and therefore provide an excellent insect repelling application can be provided. Moreover, by using compositions of the present invention the strong first smell of insect repellents such as citriodiol can be reduced while the longterm efficacy of the insect repellent is not significantly hampered. Consequently, insect repellent products can be provided which are very pleasant for the users.

Preferably, the compositions according to the present invention are compositions comprising the compound (c) in an amount of 0.1 to 50.0 % by weight, particularly in an amount of 0.5 to 45.0 % by weight, more particularly in an amount of 1.0 to 40 % by weight, most particularly in an amount of 2.0 to 25.0 % by weight, based on the total weight of the composition.

**Component (d)**

Preferably, the compositions according to the present invention are compositions further comprising:
(d) at least one cosmetically or pharmaceutically active substance and/or additive.

Within the context of the present invention, it is also possible - and in some cases advantageous - to combine the cosmetic or pharmaceutical composition according to the present invention with other active substances, adjuvants or additives.

The at least one cosmetically or pharmaceutically active substance and/or additive according to the present invention may be any cosmetically or pharmaceutically active substance and/or additive known in the art.

Preferably, the cosmetic or pharmaceutical composition according to the present invention comprises at least one cosmetically or pharmaceutically active substance and/or additive.

Optionally, other conventional cosmetically and/or pharmaceutically active substances, adjuvants or additives, as further described below, may be added as component (d), i.e. in order to obtain a ready-for-use composition or formulation.

The cosmetic or pharmaceutical composition according to the present invention can advantageously be combined with other cosmetically or pharmaceutically active agents and/or adjuvants and/or additives or auxiliaries, such as are customarily used in such compositions, such as for example abrasives, anti-acne agents, agents against ageing

of the skin, anti-cellulitis agents, anti-dandruff agents, anti-inflammatory agents, anti-microbial agents, irritation-preventing agents, irritation-inhibiting agents, antioxidants, astringents, odor absorbers, perspiration-inhibiting agents, antiseptic agents, anti-statics, binders, buffers, carrier materials, chelating agents, cell stimulants, cleansing agents, depilatory agents, surface-active substances, deodorizing agents, antiperspirants, softeners, emulsifiers, enzymes, enzyme inhibitors, essential oils, fibers, film-forming agents, fixatives, foam-forming agents, foam stabilizers, substances for preventing foaming, foam boosters, gelling agents, gel-forming agents, hair care agents, hair-setting agents, hair-straightening agents, moisture-donating agents, moisturizing substances, moisture-retaining substances, bleaching agents, strengthening agents, stain-removing agents, optically brightening agents, impregnating agents, dirt-repellent agents, dyes, friction-reducing agents, lubricants, moisturizing creams, ointments, opacifying agents, plasticizing agents, covering agents, polish, preservatives, gloss agents, green and synthetic polymers, powders, proteins, re-oiling agents, abrading agents, silicones, skin-soothing agents, skin-cleansing agents, skin care agents, skin-healing agents, skin-lightening agents, skin-protecting agents, skin-softening agents, hair promotion agents, cooling agents, skin-cooling agents, warming agents, skin-warming agents, stabilizers, surfactants, UV-absorbing agents, UV filters, primary sun protection factors, secondary sun protection factors, detergents, fabric conditioning agents, suspending agents, skin-tanning agents, actives modulating skin or hair pigmentation, matrix-metalloproteinase inhibitors, skin moisturizing agents, glycosaminoglycan stimulators, TRPV1 antagonists, desquamating agents, anti-cellulite agents or fat enhancing agents, hair growth activators or inhibitors, thickeners, rheology additives, vitamins, oils, waxes, pearlizing waxes, fats, phospholipids, saturated fatty acids, mono- or polyunsaturated fatty acids, $\alpha$-hydroxy acids, polyhydroxy fatty acids, liquefiers, dyestuffs, colour-protecting agents, pigments, anti-corrosives, fragrances or perfume oils, aromas, flavouring substances, odoriferous substances, polyols, electrolytes, organic solvents, and mixtures of two or more of the aforementioned substances, as further described below.

Preferably, the at least one cosmetically or pharmaceutically active substance and/or additive is selected from the group consisting of antimicrobial compounds, cooling agents, agents against ageing of the skin, antioxidants, chelating agents, emulsifiers, preservatives, green and synthetic polymers, rheology additives, oils, fragrances or perfume oils, polyols and mixtures of two or more of the afore-mentioned substances.

The cosmetically or pharmaceutically active agents and/or adjuvants and/or additives can in some instances provide one or more than one benefit or operate via more than one mode of action.

Since dermatological conditions or diseases are often associated with dry skin, scratched skin, skin lesions or even inflammation, the cosmetic or pharmaceutical composition according to the present invention advantageously contains preferably anti-inflammatories, antibacterial or antimycotic substances, substances having a reddening-alleviating or itch-alleviating action, lenitive substances, moisturisers and/or cooling agents, osmolytes, keratolytic substances, nurturing substances, anti-inflammatory, antibacterial or antimycotic substances, substances having a reddening-alleviating or itch-alleviating action, lenitive substances, antidandruff substances, or other active compounds such as solvents, fragrances antioxidants, preservatives, (metal) chelating agents, penetration enhancers, or mixtures of two or more of afore specified agents, as further described below.

**Anti-ageing actives:** The cosmetic or pharmaceutical composition according to the present invention preferably contains one or more anti-ageing actives. In the context of the invention, anti-ageing or biogenic agents are, for example antioxidants, matrix-metalloproteinase inhibitors (MMPI), skin moisturizing agents, glycosaminglycan stimulators, anti-inflammatory agents, TRPV1 antagonists and plant extracts.

**Antioxidants:** A preferred cosmetic or pharmaceutical composition according to the present invention comprises one or more antioxidants. Suitable antioxidants encompass amino acids (preferably glycine, histidine, tyrosine, tryptophan) and derivatives thereof, imidazoles (preferably urocanic acid) and derivatives thereof, peptides, preferably D,L-carnosine, D-carnosine, L-carnosine and derivatives thereof (preferably anserine), carnitine, creatine, matrikine peptides (preferably lysyl-threonyl-threonyl-lysyl-serine) and palmitoylated pentapeptides, carotenoids, carotenes (preferably alpha-carotene, beta-carotene, lycopene) and derivatives thereof, lipoic acid and derivatives thereof (preferably dihydrolipoic acid), aurothioglucose, propyl thiouracil and other thiols (preferably thioredoxine, glutathione, cysteine, cystine, cystamine and glycosyl, N-acetyl, methyl, ethyl, propyl, amyl, butyl and lauryl, palmitoyl, oleyl, gamma-linoleyl, cholesteryl, glyceryl and oligoglyceryl esters thereof) and salts thereof, dilauryl thiodipropionate, distearyl thiodipropionate, thiodipropionic acid and derivatives thereof (preferably esters, ethers, peptides, lipids, nucleotides, nucleosides and salts) and sulfoximine compounds (preferably buthionine sulfoximines, homocysteine sulfoximine, buthionine sulfones, penta-, hexa-, heptathionine sulfoximine) in very small tolerated doses (e.g. pmol to $\mu$mol/kg), also (metal) chelators (preferably alpha-hydroxy fatty acids, palmitic acid, phytic acid, lactoferrin, alpha-hydroxy acids (preferably citric acid, lactic acid, malic acid), humic acid, bile acid, bile extracts, tannins, bilirubin, biliverdin, EDTA, EGTA and derivatives thereof), unsaturated fatty acids and derivatives thereof (preferably gamma-linolenic acid, linoleic acid, oleic acid), folic acid and derivatives thereof, ubiquinone and derivatives thereof, ubiquinol and derivatives thereof, vitamin C and derivatives (preferably ascorbyl palmitate, Mg ascorbyl phosphate, ascorbyl acetate, ascorbyl glucoside), tocopherols and derivatives (preferably vitamin E acetate), vitamin A and derivatives (vitamin A palmitate) and coniferyl benzoate of benzoic resin, rutinic acid and derivatives thereof, flavonoids and glycosylated precursors thereof, in particular quercetin

and derivatives thereof, preferably alpha-glucosyl rutin, rosmarinic acid, carnosol, carnosolic acid, resveratrol, caffeic acid and derivatives thereof, sinapic acid and derivatives thereof, ferulic acid and derivatives thereof, curcuminoids, chlorogenic acid and derivatives thereof, retinoids, preferably retinyl palmitate, retinol or tretinoin, ursolic acid, levulinic acid, butyl hydroxytoluene, butyl hydroxyanisole, nordihydroguaiac acid, nordihydroguaiaretic acid, trihydroxybutyrophenone, uric acid and derivatives thereof, mannose and derivatives thereof, zinc and derivatives thereof (preferably ZnO, $ZnSO_4$), selenium and derivatives thereof (preferably selenium methionine), superoxide dismutase, stilbenes and derivatives thereof (preferably stilbene oxide, trans-stilbene oxide) and the derivatives (salts, esters, ethers, sugars, nucleotides, nucleosides, peptides and lipids) of these cited active ingredients which are suitable according to the invention or extracts or fractions of plants having an antioxidant effect, preferably green tea, rooibos, honeybush, grape, rosemary, sage, melissa, thyme, lavender, olive, oats, cocoa, ginkgo, ginseng, liquorice, honeysuckle, sophora, pueraria, pinus, citrus, Phyllanthus emblica or St. John's wort, grape seeds, wheat germ, Phyllanthus emblica, coenzymes, preferably coenzyme Q10, plastoquinone and menaquinone. Preferred antioxidants are selected from the group consisting of vitamin A and derivatives, vitamin C and derivatives, tocopherol and derivatives, preferably tocopheryl acetate, and ubiquinone. If vitamin E and/or derivatives thereof are used as the antioxidant(s), it is advantageous to choose their concentrations from the range from about 0.001 to about 10 % by weight, based on the total weight of the composition. If vitamin A or vitamin A derivatives or carotenes or derivatives thereof are used as the antioxidant(s), it is advantageous to choose their concentrations from the range from about 0.001 to about 10 % by weight based on the total weight of the composition.

**Matrix-Metalloproteinase inhibitors (MMPI):** A preferred cosmetic or pharmaceutical composition according to the present invention comprises one or more matrix-metalloproteinase inhibitors, especially those inhibiting matrix-metalloproteinases enzymatically cleaving collagen, selected from the group consisting of: ursolic acid, retinyl palmitate, propyl gallate, precocenes, 6-hydroxy-7-methoxy-2,2-dimethyl-1(2H)-benzopyran, 3,4-dihydro-6-hydroxy-7-methoxy-2,2-dimethyl-1(2H)-benzopyran, benzamidine hydrochloride, the cysteine proteinase inhibitors N-ethylmalemide and epsilon-amino-n-caproic acid of the serinprotease inhibitors: phenylmethylsufonylfluoride, collhibin (company Pentapharm; INCI: hydrolysed rice protein), oenotherol (company Soliance; INCI: propylene glycol, aqua, Oenothera biennis root extract, ellagic acid and ellagitannins, for example from pomegranate), phosphoramidone hinokitiol, EDTA, galardin, EquiStat (company Collaborative Group; apple fruit extract, soya seed extract, ursolic acid, soya isoflavones and soya proteins), sage extracts, MDI (company Atrium; INCI: glycosaminoglycans), fermiskin (company Silab/Mawi; INCI: water and lentinus edodes extract), actimp 1.9.3 (company Expanscience/Rahn; INCI: hydrolysed lupine protein), lipobelle soyaglycone (company Mibelle; INCI: alcohol, polysorbate 80, lecithin and soy isoflavones), extracts from green and black tea and further plant extracts, proteins or glycoproteins from soya, hydrolysed proteins from rice, pea or lupine, plant extracts which inhibit MMPs, preferably extracts from shitake mushrooms, extracts from the leaves of the Rosaceae family, sub-family Rosoideae, quite particularly extracts of blackberry leaf, as e.g. SymMatrix (company Symrise, INCI: Maltodextrin, Rubus Fruticosus (Blackberry) Leaf Extract). Preferred actives of are selected from the group consisting of retinyl palmitate, ursolic acid, extracts from the leaves of the Rosaceae family, sub-family Rosoideae, genistein and daidzein.

**Skin-moisturizing agents:** A preferred cosmetic or pharmaceutical composition according to the present invention comprises one or more skin-moisturizing agents. Preferred skin moisturizing agents are selected from the group consisting of alkane diols or alkane triols comprising 3 to 12 carbon atoms, preferably $C_3$-$C_{10}$-alkane diols and $C_3$-$C_{10}$-alkane triols. More preferably the skin moisturizing agents are selected from the group consisting of: glycerol, 1,2-propylene glycol, 1,2-butylene glycol, 1,3-butylene glycol, 1,2-pentanediol, 1,2-hexanediol, 1,2-octanediol and 1,2-decanediol.

**Glycosaminoglycan stimulators:** A preferred cosmetic or pharmaceutical composition according to the present invention comprises one or more substances stimulating the synthesis of glycosaminoglycans which are selected from the group consisting of hyaluronic acid and derivatives or salts, Subliskin (Sederma, INCI: Sinorhizobium Meliloti Ferment Filtrate, Cetyl Hydroxyethylcellulose, Lecithin), Hyalufix (BASF, INCI: Water, Butylene Glycol, Alpinia galanga leaf extract, Xanthan Gum, Caprylic/Capric Triglyceride), Stimulhyal (Soliance, INCI: Calcium ketogluconate), Syn-Glycan (DSM, INCI: Tetradecyl Aminobutyroylvalylaminobutyric Urea Trifluoroacetate, Glycerin, Magnesium chloride), Kalpariane (Biotech Marine), DC Upregulex (Distinctive Cosmetic Ingredients, INCI: Water, Butylene Glycol, Phospholipids, Hydrolyzed Sericin), glucosamine, N-acetyl glucosamine, retinoids, preferably retinol and vitamin A, Arctium lappa fruit extract, Eriobotrya japonica extract, Genkwanin, N-Methyl-L-serine, (-)-alpha-bisabolol or synthetic alpha-bisabolol such as e.g. Dragosantol and Dragosantol 100 from Symrise, oat glucan, Echinacea purpurea extract and soy protein hydrolysate. Preferred actives are selected from the group consisting of hyaluronic acid and derivatives or salts, retinol and derivatives, (-)-alpha-bisabolol or synthetic alpha-bisabolol such as e.g. Dragosantol and Dragosantol 100 from Symrise, oat glucan, Echinacea purpurea extract, Sinorhizobium Meliloti Ferment Filtrate, Calcium ketogluconate, Alpinia galanga leaf extract and tetradecyl aminobutyroylvalylaminobutyric urea trifluoroacetate.

**TRPV1 antagonists:** A preferred cosmetic or pharmaceutical composition according to the present invention comprises one or more TRPV1 antagonsits. Suitable compounds which reduce the hypersensitivity of skin nerves based

on their action as TRPV1 antagonists, encompass e.g. trans-4-tert-butyl cyclohexanol, or indirect modulators of TRPV1 by an activation of the μ-receptor, e.g. acetyl tetrapeptide-15, are preferred.

**Plant extracts:** A preferred cosmetic or pharmaceutical composition according to the present invention comprises one or more plant extracts. Plant extracts, special highly active plant extract fractions and also highly pure active substances isolated from plant extracts can also be used in the cosmetic or pharmaceutical composition according to the present invention. Extracts, fractions and active substances from camomile, *aloe vera, Commiphora* species, *Rubia* species, willows, willow-herb, ginger, marigold, arnica, Glycyrrhiza species, Echinacea species, Rubus species and pure substances such as *inter alia* bisabolol, apigenin, apigenin-7-glucoside, gingerols such as [6]-gingerol, paradols such as [6]-paradol, boswellic acid, phytosterols, glycyrrhizine, glabridin or licochalcone A are particularly preferred.

**Anti-inflammatory agents:** The cosmetic or pharmaceutical composition according to the present invention preferably also contains anti-inflammatory and/or redness and/or itch ameliorating ingredients, in particular steroidal substances of the corticosteroid type selected from the group consisting of hydrocortisone, dexamethasone, dexamethasone phosphate, methyl prednisolone or cortisone, are advantageously used as anti-inflammatory active ingredients or active ingredients to relieve reddening and itching, the list of which can be extended by the addition of other steroidal anti-inflammatories. Non-steroidal anti-inflammatories can also be used. More particularly:

(i) steroidal anti-inflammatory substances of the corticosteroid type, in particular hydrocortisone, hydrocortisone derivatives such as hydrocortisone 17-butyrate, dexamethasone, dexamethasone phosphate, methylprednisolone or cortisone,
(ii) non-steroidal anti-inflammatory substances, in particular oxicams such as piroxicam or tenoxicam, salicylates such as aspirin, disalcid, solprin or fendosal, acetic acid derivatives such as diclofenac, fenclofenac, indomethacin, sulindac, tolmetin or clindanac, fenamates such as mefenamic, meclofenamic, flufenamic or niflumic, propionic acid derivatives such as ibuprofen, naproxen or benoxaprofen, pyrazoles such as phenylbutazone, oxyphenylbutazone, febrazone or azapropazone,
(iii) natural or naturally occuring anti-inflammatory substances or substances that alleviate reddening and/or itching, in particular extracts or fractions from camomile, Aloe vera, Commiphora species, Rubia species, willow, willow-herb, oats, calendula, arnica, St John’s wort, honeysuckle, rosemary, Passiflora incarnata, witch hazel, ginger or Echinacea, or single active compounds thereof,
(iv) histamine receptor antagonists, serine protease inhibitors (e.g. of Soy extracts), TRPV1 antagonists (e.g. 4-t-Butylcyclohexanol), NK1 antagonists (e.g. Aprepitant, Hydroxyphenyl Propamidobenzoic Acid), cannabinoid receptor agonists (e.g. Palmitoyl Ethanolamine) and TRPV3 antagonists.

Examples which can be cited here are oxicams such as piroxicam or tenoxicam; salicylates such as aspirin, disalcid, solprin or fendosal; acetic acid derivatives such as diclofenac, fenclofenac, indomethacin, sulindac, tolmetin or clindanac; fenamates such as mefenamic, meclofenamic, flufenamic or niflumic; propionic acid derivatives such as ibuprofen, naproxen, benoxaprofen or pyrazoles such as phenylbutazone, oxyphenylbutazone, febrazone or azapropazone. Anthranilic acid derivatives are preferred anti-itch ingredients in a composition according to the present invention.

Also useful are natural or naturally occurring anti-inflammatory mixtures of substances or mixtures of substances that alleviate reddening and/or itching, in particular extracts or fractions from camomile, Aloe vera, Commiphora species, Rubia species, willow, willow-herb, oats, calendula, arnica, St John’s wort, honeysuckle, rosemary, Passiflora incarnata, witch hazel, ginger or Echinacea; preferably selected from the group consisting of extracts or fractions from camomile, Aloe vera, oats, calendula, arnica, honeysuckle, rosemary, witch hazel, ginger or Echinacea, and/or pure substances, preferably alpha-bisabolol, apigenin, apigenin-7-glucoside, gingerols, shogaols, gingerdiols, dehydrogingerdiones, paradols, natural or naturally occuring avenanthramides, preferably tranilast, avenanthramide A, avenanthramide B, avenanthramide C, non-natural or non-naturally occuring avenanthramides, preferably dihydroavenanthramide D, dihydroavenanthramide E, avenanthramide D, avenanthramide E, avenanthramide F, boswellic acid, phytosterols, glycyrrhizin, glabridin and licochalcone A; preferably selected from the group consisting of alpha-bisabolol, natural avenanthramides, non-natural avenanthramides, preferably dihydroavenanthramide D (as described in WO 2004 047833 A1), boswellic acid, phytosterols, glycyrrhizin, and licochalcone A, and/or allantoin, panthenol, lanolin, (pseudo-)ceramides [preferably Ceramide 2, hydroxypropyl bispalmitamide MEA, cetyloxypropyl glyceryl methoxypropyl myristamide, N-(1-hexadecanoyl)-4-hydroxy-L-proline (1-hexadecyl) ester, hydroxyethyl palmityl oxyhydroxypropyl palmitamide], glycosphingolipids, phytosterols, chitosan, mannose, lactose and β-glucans, in particular 1,3->1,4-ß-glucan from oats.

When bisabolol is used in the context of the present invention it can be of natural or synthetic origin, and is preferably "alpha-bisabolol". Preferably, the bisabolol used is synthetically prepared or natural (-)-alpha-bisabolol and/or synthetic mixed-isomer alpha-bisabolol. If natural (-)-alpha-bisabolol is used, this can also be employed as a constituent of an essential oil or of a plant extract or of a fraction thereof, for example as a constituent of (fractions of) oil or extracts of camomile or of Vanillosmopsis (in particular Vanillosmopsis erythropappa or Vanillosmopsis arborea). Synthetic alpha-bisabolol is obtainable, for example, under the name "Dragosantol" from Symrise.

In case ginger extract is used in the context of the present invention, preferably extracts of the fresh or dried ginger root are used which are prepared by extraction with methanol, ethanol, iso-propanol, acetone, ethyl acetate, carbon dioxide ($CO_2$), hexane, methylene chloride, chloroform or other solvents or solvent mixtures of comparable polarity. The extracts are characterized by the presence of active skin irritation-reducing amounts of constituents such as e.g. gingerols, shogaols, gingerdiols, dehydrogingerdiones and/or paradols.

**Physiological cooling agents:** The cosmetic or pharmaceutical composition according to the present invention can be particularly advantageously combined with one or more physiological cooling agent(s). The use of cooling agents can alleviate itching. Preferred individual cooling agents for use within the framework of the present invention are listed below. The person skilled in the art can add many other cooling agents to this list; the cooling agents listed can also be used in combination with one another: which are preferably selected here from the following list: menthol and menthol derivatives (for example L-menthol, D-menthol, racemic menthol, isomenthol, neoisomenthol, neomenthol) menthylethers (for example (l-menthoxy)-1,2-propanediol, (l-menthoxy)-2-methyl-1 ,2-propanediol, l-menthyl-methylether), menthone glyceryl acetal, menthone glyceryl ketal or mixtures of both, menthylesters (for example menthylformiate, menthylacetate, menthylisobutyrate, menthyhydroxyisobutyrat, menthyllactates, L-menthyl-L-lactate, L-menthyl-D-lactate, menthyl-(2-methoxy)acetate, menthyl-(2-methoxyethoxy)acetate, menthylpyroglutamate), menthylcarbonates (for example menthylpropyleneglycolcarbonate, menthylethyleneglycolcarbonate, menthylglycerolcarbonate or mixtures thereof), the semi-esters of menthols with a dicarboxylic acid or derivatives thereof (for example mono-menthylsuccinate, mono-menthylglutarate, mono-menthylmalonate, O-menthyl succinic acid ester-N,N-(dimethyl)amide, O-menthyl succinic acid ester amide), menthanecarboxylic acid amides (in this case preferably menthanecarboxylic acid-N-ethylamide [WS3] or $N^{\alpha}$-(menthanecarbonyl)glycinethylester [WS5], menthanecarboxylic acid-N-(4-cyanophenyl)amide or menthanecarboxylic acid-N-(4-cyanomethylphenyl)amide, menthanecarboxylic acid-N-(alkoxyalkyl)amides), menthone and menthone derivatives (for example L-menthone glycerol ketal), 2,3-dimethyl-2-(2-propyl)-butyric acid derivatives (for example 2,3-dimethyl-2-(2-propyl)-butyric acid-N-methylamide [WS23]), isopulegol or its esters (l-(-)-isopulegol, l-(-)-isopulegolacetate), menthane derivatives (for example p-menthane-3,8-diol), cubebol or synthetic or natural mixtures, containing cubebol, pyrrolidone derivatives of cycloalkyldione derivatives (for example 3-methyl-2(1-pyrrolidinyl)-2-cyclopentene-1-one) or tetrahydropyrimidine-2-one (for example iciline or related compounds, as described in WO 2004/026840), further carboxam ides (for example N-(2-(pyridin-2-yl)ethyl)-3-p-menthanecarboxamide or related compounds), (1R,2S,5R)-N-(4-Methoxyphenyl)-5-methyl-2-(1-isopropyl)cyclohexane-carboxamide [WS12], oxamates and [(1R,2S,5R)-2-isopropyl-5-methyl-cyclohexyl] 2-(ethylamino)-2-oxo-acetate (X Cool). Cooling agents which are preferred due to their particular synergistic effect are l-menthol, d-menthol, racemic menthol, menthone glycerol acetal (trade name: Frescolat® MGA), menthyl lactate (preferably l-menthyl lactate, in particular l-menthyl l-lactate (trade name: Frescolat® ML)), substituted menthyl-3-carboxamides (such as menthyl-3-carboxylic acid N-ethyl amide), 2-isopropyl-N-2,3-trimethyl butanamide, substituted cyclohexane carboxamides, 3-menthoxypropane-1,2-diol, 2-hydroxyethyl menthyl carbonate, 2-hydroxypropyl menthyl carbonate and isopulegol. Particularly preferred cooling agents are l-menthol, racemic menthol, menthone glycerol acetal (trade name: Frescolat® MGA), menthyl lactate (preferably l-menthyl lactate, in particular l-menthyl l-lactate (trade name: Frescolat® ML)), 3-menthoxypropane-1,2-diol, 2-hydroxyethyl menthyl carbonate and 2-hydroxypropyl menthyl carbonate. Very particularly preferred cooling agents are l-menthol, menthone glycerol acetal (trade name: Frescolat® MGA) and menthyl lactate (preferably l-menthyl lactate, in particular l-menthyl l-lactate (trade name: Frescolat® ML).

**Moisturising and/or moisture-retaining substances:** Itching occurs with particular intensity when the skin is dry. The use of skin-moisturising and/or moisture-retaining substances can significantly alleviate itching. The cosmetic or pharmaceutical composition according to the present invention can therefore advantageously also contain one or more of the following moisturising and/or moisture-retaining substances: sodium lactate, urea, urea derivatives, alcohols, glycerol, diols such as propylene glycol, hexylene glycol, collagen, elastin or hyaluronic acid, diacyl adipates, petrolatum, urocanic acid, lecithin, panthenol, phytantriol, lycopene, (pseudo-)ceramides, glycosphingolipids, cholesterol, phytosterols, chitosan, chondroitin sulphate, lanolin, lanolin esters, amino acids, alpha-hydroxy acids (such as citric acid, lactic acid, malic acid) and their derivatives, mono-, di- and oligosaccharides such as glucose, galactose, fructose, mannose, fructose and lactose, polysugars such as R-glucans, in particular 1,3-1,4-β-glucan from oats, alpha-hydroxy fatty acids, triterpene acids such as betulinic acid or ursolic acid, and algae extracts.

**Lenitive substances:** The cosmetic or pharmaceutical composition according to the present invention can also contain advantageously one or more lenitive substances, wherein any lenitive substances can be used which are suitable or customary in cosmetic or pharmaceutical applications such as alpha-bisabolol, azulene, guaiazulene, 18-beta-glycyrrhetinic acid, allantoin, Aloe vera juice or gel, extracts of Hamamelis virginiana (witch hazel), Echinacea species, Centella asiatica, chamomile, Arnica monatana, Glycyrrhiza species, algae, seaweed and Calendula officinalis, and vegetable oils such as sweet almond oil, baobab oil, olive oil and panthenol, Laureth-9, Trideceth-9 and 4-t-butylcyclohexanol.

**Antibacterial or antimycotic active substances:** Antibacterial or antimycotic active substances can also particularly advantageously be used in the cosmetic or pharmaceutical composition according to the present invention,

wherein any antibacterial or antimycotic active substances can be used which are suitable or customary in cosmetic or pharmaceutical, in particular dermatological applications. In addition to the large group of conventional antibiotics, other products which are advantageous here include those relevant to cosmetics such as in particular triclosan, climbazole, octoxyglycerin, Octopirox® (1-hydroxy-4-methyl-6-(2,4,4-trimethylpentyl)-2(1H)-pyridone 2-aminoethanol salt), chitosan, farnesol, glycerol monolaurate or combinations of said substances, which are used *inter alia* against underarm odour, foot odour or dandruff.

**Anti-microbial agents:** The cosmetic or pharmaceutical composition according to the present invention preferably contains one or more anti-microbioal agents. Suitable anti-microbial agents are, in principle, all substances effective against Gram-positive bacteria, such as, for example, 4- hydroxybenzoic acid and its salts and esters, N-(4-chlorophenyl)-N'-(3,4- dichlorophenyl)urea, 2,4,4'-trichloro-2'-hydroxydiphenyl ether (triclosan), 4-chloro-3,5-dimethyl-phenol, 2,2'-methylenebis(6-bromo-4-chlorophenol), 3-methyl-4-(1-methylethyl)phenol, 2-benzyl-4-chloro-phenol, 3-(4-chlorophenoxy)-1,2-propanediol, 3-iodo-2-propynyl butylcarbamate, chlorhexidine, 3,4,4'-trichlorocarbanilide (TTC), antibacterial fragrances, thymol, thyme oil, eugenol, oil of cloves, menthol, mint oil, farnesol, phenoxyethanol, glycerol monocaprate, glycerol monocaprylate, glycerol monolaurate (GML), diglycerol monocaprate (DMC), salicylic acid N-alkylamides, such as, for example, n-octylsalicylamide or n-decylsalicylamide.

**Desquamating agents:** The cosmetic or pharmaceutical composition according to the present invention preferably contains one or more desquamating agents. The expression "desquamating agent" is understood to mean any compound capable of acting:

- either directly on desquamation by promoting exfoliation, such as β-hydroxy acids, in particular salicylic acid and its derivatives (including 5-n-octanoylsalicylic acid); α-hydroxy acids, such as glycolic, citric, lactic, tartaric, malic or mandelic acids; urea; gentisic acid; oligofucoses; cinnamic acid; extract of Sophora japonica; resveratrol and some derivatives of jasmonic acid;
- or on the enzymes involved in the desquamation or the degradation of the corneodesmosomes, glycosidases, stratum corneum chymotryptic enzyme (SCCE) or other proteases (trypsin, chymotrypsin-like). There may be mentioned agents chelating inorganic salts: EDTA; N-acyl-N,N',N'-ethylenediaminetriacetic acid; aminosulphonic compounds and in particular (N-2-hydroxyethylpiperazine-N-2-ethane)sulphonic acid (HEPES); derivatives of 2-oxothiazolidine-4-carboxylic acid (procysteine); derivatives of alpha-amino acids of the glycine type (as described in EP-0 852 949, and sodium methylglycine diacetate marketed by BASF under the trade name TRILON M); honey; sugar derivatives such as O-octanoyl-6-D-maltose and N-acetylglucosamine; chestnut extracts such as those marketed by the company SILAB under the name Recoverine®, prickly pear extracts such as those marketed under the name Exfolactive® by the company SILAB, or Phytosphingosine SLC® (phytosphingosine grafted with a salicylic acid) marketed by the company Degussa.

Desquamating agents suitable for the invention may be chosen in particular from the group comprising sulphonic acids, calcium chelators, α-hydroxy acids such as glycolic, citric, lactic, tartaric, malic or mandelic acids; ascorbic acid and its derivatives such as ascorbyl glucoside and magnesium ascorbyl phosphate; nicotinamide; urea; (N-2-hydroxyethylpiperazine-N-2-ethane)sulphonic acid (HEPES), β-hydroxy acids such as salicylic acid and its derivatives, retinoids such as retinol and its esters, retinal, retinoic acid and its derivatives, chestnut or prickly pear extracts, in particular marketed by SILAB; reducing compounds such as cysteine or cysteine precursors.

Desquamating agents which can be used are also nicotinic acid and its esters and nicotinamide, also called vitamin B3 or vitamin PP, and ascorbic acid and its precursors.

**Anti-dandruff substances:** In addition, the cosmetic or pharmaceutical composition according to the present invention can also advantageously be used in combination with one or more anti-dandruff substances, including triclosan, climbazole, octoxyglycerin, Octopirox® (1-hydroxy-4-methyl-6-(2,4,4-trimethylpentyl)-2(1H)-pyridone 2-aminoethanol salt), chitosan, farnesol, glycerol monolaurate, Propanediol Monocaprylate or combinations of said substances, which are used inter alia against dandruff.

Further suitable anti-dandruff agents are Pirocton Olamin (1-hydroxy-4-methyl-6-(2,4,4-trimethylpentyl)-2-(1H)-pyridinone monoethanolamine salt), Baypival® (Climbazole), Ketoconazol® (4-acetyl-1-{4-[2-(2,4-dichlorophenyl) r-2-(1H-imidazol-1-ylmethyl)-1,3-dioxylan-c-4-ylmethoxyphenyl}-piperazine, ketoconazole, elubiol, selenium disulfide, colloidal sulfur, sulfur polyethylene glycol sorbitan monooleate, sulfur ricinol polyethoxylate, sulfur tar distillate, salicylic acid (or in combination with hexachlorophene), undecylenic acid, monoethanolamide sulfosuccinate Na salt, Lamepon® UD (protein/undecylenic acid condensate), zinc pyrithione, aluminium pyrithione and magnesium pyrithione/dipyrithione magnesium sulfate.

**(Metal) chelating agents:** A combination with one or more (metal) chelating agents can also be advantageous used in the cosmetic or pharmaceutical composition according to the present invention, wherein any metal chelating agents can be used which are suitable or customary in cosmetic or pharmaceutical applications. Preferred (metal) chelating agents include α-hydroxy fatty acids, phytic acid, lactoferrin, α-hydroxy acids, such as *inter alia* gluconic acid,

glyceric acid, glycolic acid, isocitric acid, citric acid, lactic acid, malic acid, mandelic acid, tartaric acid, as well as humic acids, bile acids, bile extracts, bilirubin, biliverdin or EDTA, EGTA and their derivatives. The use of one or more chelating agent(s) improves the stability of the composition according to the present invention.

**Emulsifiers:** In addition, the cosmetic or pharmaceutical composition according to the present invention can also advantageously contain one or more emulsifiers, including for example:

- products of the addition of 2 to 30 mol ethylene oxide and/or 0 to 5 mol propylene oxide onto linear $C_{8-22}$ fatty alcohols, onto $C_{12-22}$ fatty acids and onto alkyl phenols containing 8 to 15 carbon atoms in the alkyl group;
- $C_{12/18}$ fatty acid monoesters and diesters of addition products of 1 to 30 mol ethylene oxide onto glycerol;
- glycerol mono- and diesters and sorbitan mono- and diesters of saturated and unsaturated fatty acids containing 6 to 22 carbon atoms and ethylene oxide addition products thereof;
- addition products of 15 to 60 mol ethylene oxide onto castor oil and/or hydrogenated castor oil;
- polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
- addition products of 2 to 15 mol ethylene oxide onto castor oil and/or hydrogenated castor oil;
- partial esters based on linear, branched, unsaturated or saturated $C_{6/22}$ fatty acids, ricinoleic acid and 12-hydroxystearic acid and glycerol, polyglycerol, pentaerythritol, dipentaerythritol, sugar alcohols (for example sorbitol), alkyl glucosides (for example methyl glucoside, butyl glucoside, lauryl glucoside) and polyglucosides (for example cellulose);
- mono-, di and trialkyl phosphates and mono-, di- and/or tri-PEG-alkyl phosphates and salts thereof;
- wool wax alcohols;
- polysiloxane/polyalkyl polyether copolymers and corresponding derivatives;
- mixed esters of pentaerythritol, fatty acids, citric acid and fatty alcohol and/or mixed esters of $C_{6-22}$ fatty acids, methyl glucose and polyols, preferably glycerol or polyglycerol;
- polyalkylene glycols; and
- glycerol carbonate.

The addition products of ethylene oxide and/or propylene oxide onto fatty alcohols, fatty acids, alkylphenols, glycerol mono- and diesters and sorbitan mono- and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. $C_{12/18}$ fatty acid monoesters and diesters of addition products of ethylene oxide onto glycerol are known as lipid layer enhancers for cosmetic formulations. The preferred emulsifiers are described in more detail as follows:

Partial glycerides: Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride and technical mixtures thereof which may still contain small quantities of triglyceride from the production process. Addition products of 1 to 30 and preferably 5 to 10 mol ethylene oxide onto the partial glycerides mentioned are also suitable.

Sorbitan esters: Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 and preferably 5 to 10 mol ethylene oxide onto the sorbitan esters mentioned are also suitable.

Polyglycerol esters: Typical examples of suitable polyglycerol esters are Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) and Polyglyceryl Polyricinoleate (Admul® WOL 1403), Polyglyceryl Dimerate Isostearate, and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.

Anionic emulsifiers: Typical anionic emulsifiers are aliphatic C12 to C 22 fatty acids, such as palmitic acid, stearic acid or behenic acid for example, and C12 to C22 dicarboxylic acids, such as azelaic acid or sebacic acid for example.

Amphoteric emulsifiers: Other suitable emulsifiers are amphoteric or zwitterionic surfactants. Zwitterionic surfactants are surface-active compounds which contain at least one quaternary ammonium group and at least one carboxylate and one sulfonate group in the molecule. Particularly suitable zwitterionic surfactants are the so-called betaines, such as the N-alkyl-N,N-dimethyl ammonium glycinates, for example cocoalkyl dimethyl ammonium glycinate, N-acylaminopropyl-N,N-dimethyl ammonium glycinates, for example cocoacylaminopropyl dimethyl ammonium glycinate, and 2-alkyl-3-carboxymethyl-3-hydroxyethyl imidazolines containing 8 to 18 carbon atoms in the alkyl or acyl group and cococoacylaminoethyl hydroxyethyl carboxymethyl glycinate. The fatty acid amide derivative known under the CTFA name of *Cocamidopropyl Betaine* is particularly preferred. Ampholytic surfactants are also suitable emulsifiers. Ampholytic surfactants are surface-active compounds which, in addition to a $C_{8/18}$ alkyl or acyl group, contain at least one free amino group and at least one -COOH- or -SOsH- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-coco-alkylaminopropionate, cocoacylaminoethyl aminopropionate and $C_{12/18}$ acyl sarcosine.

**Surfactants:** For the preparation of solid surfactant compositions, the cosmetic or pharmaceutical composition according to the present invention preferably includes one or more surfactant(s) which is/are commonly used therefor. Suitable surfactants include Sodium Laureth sulfate, Cocamidopropyl Betaine, Sodium Stearate, Sodium Lauryl Sulfate, Glycol distearate, Coco-Glucoside, Sodium Coco-Sulfate, Sodium Cocoate, Coco-Betaine, Cocamide MEA, Lauryl Glucoside, PEG-7 Glyceryl Cocoate, Decyl Glucoside, Potassium Lauryl Sulfate, Sodium Palmate, Sodium Palm Kernelate, Sodium Cocoyl Glutamate, Disodium Laureth Sulfosuccinate, Sodium lauroyl sarcosinate, Sodium Lauroyl Sarcosinate, Sodium Cocoyl Isethionate, Sodium cocoamphoacetate, Sodium Methyl Cocoyl Taurate, Caprylyl/Capryl Glucoside, Disodium Lauryl Sulfosuccinate, Sodium Lauryl Sulfosuccinate, PPG-5-ceteth-20, Polyglyceryl-4 Laurate, Sodium Lauryl Sulfoacetate, Sodium Lauryl Sulfoacetate, Sodium Myristoyl Glutamate, Alpha Olefin Sulfonate, Diethylhexyl Sodium Sulfosuccinate, Ammonium Cocoyl Isethionate, Sodium Oleoyl Sarcosinate, Ammonium Cocoyl Sarcosinate, or mixtures of one or more of the aforesaid surfactants.

Preferred surfactants for the preparation of solid surfactant compositions according to the present invention are selected from the group consisting of Sodium Stearate, Sodium Lauryl Sulfate, Coco-Glucoside, Sodium Coco-Sulfate, Sodium Cocoate, Coco-Betaine, Lauryl Glucoside, Decyl Glucoside, Potassium Lauryl Sulfate, Sodium Palmate, Sodium Palm Kernelate, Sodium Cocoyl Glutamate, Disodium Laureth sulfosuccinate, Sodium lauroyl sarcosinate, Sodium Lauroyl Sarcosinate, Sodium Cocoyl Isethionate, Sodium Cocoamphoacetate, Sodium Methyl Cocoy Taurate, Caprylyl/Capryl glucoside, Disodium Lauryl Sulfosuccinate, and mixtures of two or more of the aforesaid surfactants.

**Preservatives:** For preservative purposes, the cosmetic or pharmaceutical composition according to the present invention preferably includes one or more preservatives which are suitable or customary in cosmetic or pharmaceutical composition. Suitable and advantageously preservatives are, for example, phenoxyethanol, formaldehyde solution, parabens, pentanediol or sorbic acid.

**Green and synthetic polymers:** The cosmetic or pharmaceutical composition according to the present invention preferably includes one or more green or synthetic polymers. Suitable cationic polymers are, for example, cationic cellulose derivatives such as, for example, the quaternized hydroxyethyl cellulose obtainable from Amerchol under the name of Polymer JR 400®, cationic starch, copolymers of diallyl ammonium salts and acrylamides, quaternized vinyl pyrrolidone/vinyl imidazole polymers such as, for example, Luviquat® (BASF), condensation products of polyglycols and amines, quaternized collagen polypeptides such as, for example, Lauryldimonium Hydroxypropyl Hydrolyzed Collagen (Lamequat® L, Grünau), quaternized wheat polypeptides, polyethyleneimine, cationic silicone polymers such as, for example, amodimethicone, copolymers of adipic acid and dimethylaminohydroxypropyl diethylenetriamine (Cartaretine®, Sandoz), copolymers of acrylic acid with dimethyl diallyl ammonium chloride (Merquat® 550, Chemviron), polyaminopolyamides and crosslinked water-soluble polymers thereof, cationic chitin derivatives such as, for example, quaternized chitosan, optionally in microcrystalline distribution, condensation products of dihaloalkyls, for example dibromobutane, with bis-dialkylamines, for example bis-dimethylamino-1,3-propane, cationic guar gum such as, for example, Jaguar®CBS, Jaguar®C-17, Jaguar®C-16 of Celanese, quaternized ammonium salt polymers such as, for example, Mirapol®A-15, Mirapol® AD-1, Mirapol® AZ-1 of Miranol and the various polyquaternium types (for example 6, 7, 32 or 37) which can be found in the market under the tradenames Rheocare® CC or Ultragel® 300. Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl

methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized cellulose ethers and silicones. In a preferred variant, the cosmetic or pharmaceutical composition according to the present invention preferably includes an uncrosslinked or polyol-crosslinked polyacrylic acid as polymer component.

**Thickening agents and/or rheology additives:** The cosmetic or pharmaceutical composition according to the present invention preferably includes one or more thickening agents and/or rheology additives. Suitable thickeners are polymeric thickeners, such as Aerosil® types (hydrophilic silicas), polysaccharides, more especially xanthan gum, guar-guar, agar-agar, alginates and tyloses, carboxymethyl cellulose and hydroxyethyl cellulose, also relatively high molecular weight polyethylene glycol monoesters and diesters of fatty acids, polyacrylates (for example Carbopols® [Goodrich] or Synthalens® [Sigma]), polyacrylamides, polyvinyl alcohol and polyvinyl pyrrolidone, surfactants such as, for example, ethoxylated fatty acid glycerides, esters of fatty acids with polyols, for example pentaerythritol or trimethylol propane, narrow-range fatty alcohol ethoxylates and electrolytes, such as sodium chloride and ammonium chloride.

**Perfume oils and/or fragrances:** The cosmetic or pharmaceutical composition according to the present invention preferably includes one or more perfume oils and/or fragrances. Suitable perfume oils are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, beta-isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

**Anti-cellulite agent:** The cosmetic or pharmaceutical composition according to the present invention preferably includes one or more anti-cellulite agents. Anti-cellulite agents and lipolytic agents are preferably selected from the group consisting of beta-adrenergic receptor agonists such as synephrine and its derivatives, and cyclohexyl carbamates. Agents enhancing or boosting the activity of anti-cellulite agents, in particular agents which stimulate and/or depolarise C nerve fibres, are preferably selected from the group consisting of capsaicin and derivatives thereof, vanillyl-nonylamid and derivatives thereof, L-carnitine, coenzym A, isoflavonoides, soy extracts, ananas extract and conjugated linoleic acid.

**Fat enhancing agents:** The cosmetic or pharmaceutical composition according to the present invention preferably includes one or more fat enhancing agents and/or adipogenic agents as well as agents enhancing or boosting the activity of fat enhancing agents. A fat enhancing agent is for example hydroxymethoxyphenyl propylmethylmethoxybenzofuran (trade name: Sym3D®).

**Oil bodies:** The cosmetic or pharmaceutical composition according to the present invention preferably includes one or more oil bodies. Suitable oil bodies, which form constituents of the O/W emulsions, are, for example, Guerbet alcohols based on fatty alcohols having 6 to 18, preferably 8 to 10, carbon atoms, esters of linear C5 - C22 fatty acids with linear or branched C5 - C22 fatty alcohols or esters of branched C1 - C13 carboxylic acids with linear or branched C6 - C22 fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate,

erucyl isostearate, erucyl oleate, erucyl behenate and erucyl erucate. Also suitable are esters of linear C6 - C22 fatty acids with branched alcohols, in particular 2-ethylhexanol, esters of C18 - C38 alkylhydroxy carboxylic acids with linear or branched C6 - C22 fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C6 - C10 fatty acids, liquid mono-/di-/triglyceride mixtures based on C6 - C18 fatty acids, esters of C6 - C22 fatty alcohols and/or Guerbet alcohols with aromatic carboxylic acids, in particular benzoic acid, esters of C2 - C12 dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched CC - C22 fatty alcohol carbonates, such as, for example, Dicaprylyl Carbonate (Cetiol® CC), Guerbet carbonates, based on fatty alcohols having 6 to 18, preferably 8 to 10, carbon atoms, esters of benzoic acid with linear and/or branched $C_6$-$C_{22}$-alcohols (e.g. Finsolv® TN), linear or branched, symmetrical or asymmetrical dialkyl ethers having 6 to 22 carbon atoms per alkyl group, such as, for example, dicaprylyl ether (Cetiol® OE), ring-opening products of epoxidized fatty acid esters with polyols, silicone oils (cyclomethicones, silicone methicone grades, etc.) and/or aliphatic or naphthenic hydrocarbons, such as, for example, squalane, squalene or dialkylcyclohexanes.

**Superfatting agents and/or consistency factors:** The cosmetic or pharmaceutical composition according to the present invention preferably includes one or more superfatting agents and/or consistency factors. Superfatting agents may be selected from such substances as, for example, lanolin and lecithin and also polyethoxylated or acylated lanolin and lecithin derivatives, polyol fatty acid esters, monoglycerides and fatty acid alkanolamides, the fatty acid alkanolamides also serving as foam stabilizers. The consistency factors mainly used are fatty alcohols or hydroxyfatty alcohols containing 12 to 22 and preferably 16 to 18 carbon atoms and also partial glycerides, fatty acids or hydroxyfatty acids. A combination of these substances with alkyl oligoglucosides and/or fatty acid N-methyl glucamides of the same chain length and/or polyglycerol poly-12-hydroxystearates is preferably used.

**Pearlizing waxes:** The cosmetic or pharmaceutical composition according to the present invention preferably includes one or more pearlizing waxes. Suitable pearlising waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxysubstituted carboxylic acids with fatty alcohols containing 6 to 22 carbon atoms, especially long-chain esters of tartaric acid; fatty compounds, such as for example fatty alcohols, fatty ketones, fatty aldehydes, fatty ethers and fatty carbonates which contain in all at least 24 carbon atoms, especially laurone and distearylether; fatty acids, such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups, and mixtures thereof.

**Silicones:** In order to impart a silky, spreadable, and luxurious texture and to make skin look and feel smoother, and additionally to improve processability (antifoaming) the cosmetic or pharmaceutical composition according to the present invention preferably includes one or more silicones or silicone deratives. Suitable silicones can be chosen from the group consisting of Acefylline Methylsilanol Mannuronate, Acetylmethionyl Methylsilanol Elastinate Acrylates/Behenyl, Acrylate/Dimethicone Methacrylate Copolymer, Acrylates/Behenyl Methacrylate/Dimethicone Methacrylate Copolymer, Acrylates/Bis-Hydroxypropyl Dimethicone Crosspolymer, Acrylates/Dimethicone Copolymer, Acrylates/Dimethicone Methacrylate/Ethylhexyl Acrylate Copolymer, Acrylates/Dimethiconol Acrylate Copolymer, Acrylates/Ethylhexyl Acrylate/Dimethicone Methacrylate Copolymer, Acrylates/Octylacrylamide/Diphenyl Amodimethicone Copolymer, Acrylates/Polytrimethylsiloxymethacrylate Copolymer, Acrylates/Propyl Trimethicone Methacrylate Copolymer, Acrylates/Stearyl Acrylate/Dimethicone Methacrylate Copolymer, Acrylates/Tridecyl Acrylate/Triethoxysilylpropyl Methacrylate/Dimethicone Methacrylate Copolymer, Acrylates/Trifluoropropylmethacrylate/Polytrimethyl Siloxymethacrylate Copolymer, Amino Bispropyl Dimethicone, Aminoethylaminopropyl Dimethicone, Aminopropyl Dimethicone, Aminopropyl Phenyl Trimethicone, Aminopropyl Triethoxysilane, Ammonium Dimethicone PEG-7 Sulfate, Amodimethicone, Amodimethicone Hydroxystearate, Amodimethicone/Silsesquioxane Copolymer, Ascorbyl Carboxydecyl Trisiloxane, Ascorbyl Methylsilanol Pectinate, Behenoxy Dimethicone, Behentrimonium Dimethicone PEG-8 Phthalate, Behenyl Dimethicone, Bisamino PEG/PPG-41/3 Aminoethyl PG-Propyl Dimethicone, Bis-Aminopropyl/Ethoxy Aminopropyl Dimethicone, Bis(Butylbenzoate) Diaminotriazine Aminopropyltrisiloxane, Bis-Butyldimethicone Polyglyceryl-3, Bis-Butyloxyamodimethicone/PEG-60 Copolymer, Bis(C13-15 Alkoxy) Hydroxybutamidoamodimethicone, Bis(C13-15 Alkoxy) PG- Amodimethicone, Bis-(C1-8 Alkyl Lauroyl Lysine Decylcarboxamide) Dimethicone, Bis-Cetyl Cetyl Dimethicone, Bis-Cetyl/PEG-8 Cetyl PEG-8 Dimethicone, Bis-Diphenylethyl Disiloxane, Bis-Ethyl Ethyl Methicone, Bis-Gluconamidoethylaminopropyl Dimethicone, Bis-Hydrogen Dimethicone, Bis- Hydroxyethoxypropyl Dimethicone Bis-Hydroxylauryl, Dimethicone/IPDI Copolymer, Bis- Hydroxy/Methoxy Amodimethicone, Bis-Hydroxypropyl Dimethicone Behenate, Bis-Hydroxypropyl Dimethicone/SMDI Copolymer, Bis-Isobutyl PEG-14/Amodimethicone Copolymer, Bis-Isobutyl PEG-15/Amodimethicone Copolymer, Bis-Isobutyl PEG/PPG-20/35/Amodimethicone Copolymer, Bis- Isobutyl PEG/PPG-10/7/Dimethicone Copolymer, Bis-Isobutyl PEG-24/PPG-7/Dimethicone Copolymer, Bis-PEG-1 Dimethicone, Bis-PEG-4 Dimethicone, Bis-PEG-8 Dimethicone, Bis-PEG-12 Dimethicone,Bis-PEG-20 Dimethicone, Bis-PEG-12 Dimethicone Beeswax, Bis-PEG-12 Dimethicone Candelillate, Bis-PEG-15 Dimethicone/IPDI Copolymer, Bis-PEG-15 Methyl Ether Dimethicone, Bis- PEG-18 Methyl Ether Dimethyl Silane, Bis-PEG/PPG-14/14 Dimethicone, Bis-PEG/PPG-15/5 Dimethicone, Bis-PEG/PPG-18/6 Dimethicone, Bis-PEG/PPG-20/20 Dimethicone, Bis-PEG/PPG- 16/16 PEG/PPG-16/16 Dimethicone, Bis-PEG/PPG-20/5 PEG/PPG-20/5 Dimethicone, Bisphenylhexamethicone, Bis-Phenylpropyl Dimethicone, Bispolyethylene Dimethicone, Bis- (Polyglyceryl-3 Oxyphenylpropyl) Dimethicone, Bis-(Polyglyceryl-7 Oxyphenylpropyl) Dimethicone, Bis-PPG-15 Dimethicone/IPDI Copolymer, Bis(PPG-7 Undeceneth-21) Dimethicone, Bis-Stearyl Dimethicone, Bis-Trimethoxysilylethyl Tetramethyldisiloxyethyl Dimethicone, Bis-Vinyldimethicone, Bis-Vinyl Dimethicone/Dimethicone Copolymer, Borage Seed Oil PEG-7 Dimethicone Esters, Butyl Acrylate/C6-14 Perfluoroalkylethyl Acrylate/Mercaptopropyl Dimethicone Copolymer, Butyl Acrylate/Hydroxypropyl Dimethicone Acrylate Copolymer, Butyl Dimethicone Acrylate/Cyclohexylmethacrylate/Ethylhexyl Acrylate Copolymer, Butyldimethicone Methacrylate/Methyl Methacrylate Crosspolymer, t-Butyl Dimethyl Silyl Grape Seed Extract, Butyl Polydimethylsiloxyl Ethylene/Propylene/Vinylnorbornene Copolymer, C6-8 Alkyl C3-6 Alkyl Glucoside Dimethicone, C20-24 Alkyl Dimethicone,C24-28 Alkyl Dimethicone, C26-28 Alkyl Dimethicone, C30-45 Alkyl Dimethicone, C30-60 Alkyl Dimethicone, C32 Alkyl Dimethicone, C30-45 Alkyl Dimethicone/Polycyclohexene Oxide Crosspolymer, C26-28 Alkyldimethylsilyl Polypropylsilsesquioxane, C30-45 Alkyldimethylsilyl Polypropylsilsesquioxane, C20-24 Alkyl Methicone, C24-28 Alkyl Methicone, C26-28 Alkyl Methicone, C30-45 Alkyl Methicone, C20-28 Alkyl Perfluorodecylethoxy Dimethicone, C26-54 Alkyl Tetradecyl Dimethicone, Capryl Dimethicone, Caprylyl Dimethicone Ethoxy Glucoside, Caprylyl Methicone, Caprylyl Trimethicone, Carboxydecyl Trisiloxane, Castor Oil Bis-Hydroxypropyl Dimethicone Esters Cerotyl Dimethicone, Cetearyl Dimethicone Crosspolymer, Cetearyl Dimethicone/Vinyl Dimethicone Crosspolymer, Cetearyl Methicone, Cetrimonium Carboxydecyl PEG-8 Dimethicone, Cetrimonium Dimethicone PEG-7 Phthalate, Cetyl Behenyl Dimethicone, Cetyl Dimethicone, Cetyl Dimethicone/Bis-Vinyldimethicone Crosspolymer, Cetyl Hexacosyl Dimethicone, Cetyloxy Dimethicone, Cetyl PEG-8 Dimethicone, Cetyl PEG/PPG-15/15 Butyl Ether Dimethicone, Cetyl PEG/PPG-7/3 Dimethicone, Cetyl PEG/PPG-10/1 Dimethicone, Cetyl Triethylmonium Dimethicone PEG-8 Phthalate, Cetyl Triethylmonium Dimethicone PEG-8 Succinate, Copper Acetyl Tyrosinate Methylsilanol, Copper PCA Methylsilanol, C4-14 Perfluoroalkylethoxy Dimethicone, Cycloethoxymethicone, Cycloheptasiloxane, Cyclohexasiloxane, Cyclomethicone, Cyclopentasiloxane, Cyclophenylmethicone, Cyclotetrasiloxane,mCyclovinylmethicone, Cystine Bis-PG-Propyl Silanetriol, DEA PG-Propyl PEG/PPG-18/21 Dimethicone, Diisostearoyl Trimethylolpropane Siloxy Silicate, Dilauroyl Trimethylolpropane Siloxy Silicate, Dilinoleamidopropyl Dimethylamine Dimethicone PEG-7 Phosphate, Dimethicone, Dimethicone Crosspolymer, Dimethicone Crosspolymer-3, Dimethicone/Divinyldimethicone/Silsesquioxane Crosspolymer, Dimethicone Ethoxy Glucoside, Dimethicone Hydroxypropyl Trimonium Chloride, Dimethicone/Mercaptopropyl Methicone Copolymer, Dimethicone PEG-15 Acetate Dimethicone PEG-8 Adipate, Dimethicone PEG-7 Avocadoate, Dimethicone PEG-8 Avocadoate, Dimethicone PEG-8 Beeswax, Dimethicone PEG-8 Benzoate, Dimethicone PEG-8 Borageate, Dimethicone PEG-7 Cocoate, Dimethicone/PEG-10 Crosspolymer, Dimethicone/PEG-10/15 Crosspolymer, Dimethicone/PEG-15 Crosspolymer, Dimethicone PEG-7 Isostearate, Dimethicone PEG-8 Isostearate, Dimethicone PEG-7 Lactate, Dimethicone PEG-8 Lanolate, Dimethicone PEG-8 Laurate, Dimethicone PEG-8 Meadowfoamate, Dimethicone PEG-7 Octyldodecyl Citrate, Dimethicone PEG-7 Olivate, Dimethicone PEG-8 Olivate, Dimethicone PEG-7 Phosphate, Dimethicone PEG-8 Phosphate, Dimethicone PEG-10 Phosphate, Dimethicone PEG-7 Phthalate, Dimethicone PEG-8 Phthalate, Dimethicone PEG-8 Polyacrylate, Dimethicone PEG/PPG- 20/23 Benzoate, Dimethicone PEG/PPG-7/4 Phosphate, Dimethicone PEG/PPG-12/4 Phosphate, Dimethicone PEG-7 Succinate, Dimethicone PEG-8 Succinate, Dimethicone PEG-7 Sulfate, Dimethicone PEG-7 Undecylenate, Dimethicone PG-Diethylmonium Chloride, Dimethicone/Phenyl Vinyl Dimethicone Crosspolymer, Dimethicone/Polyglycerin-3 Crosspolymer, Dimethicone/PPG-20 Crosspolymer, Dimethicone Propylethylenediamine Behenate, Dimethicone Propyl PG-Betaine, Dimethicone/Silsesquioxane Copolymer, Dimethicone Silylate, Dimethicone/Vinyldimethicone Crosspolymer, Dimethicone/Vinyltrimethylsiloxysilicate Crosspolymer, Dimethiconol, Dimethiconol Arginine, Dimethiconol Beeswax, Dimethiconol Behenate, Dimethiconol Borageate, Dimethiconol Candelillate, Dimethiconol Carnaubate, Dimethiconol Cysteine, Dimethiconol Dhupa Butterate, Dimethiconol Fluoroalcohol Dilinoleic Acid, Dimethiconol Hydroxystearate, Dimethiconol Illipe Butterate, Dimethiconol/IPDI Copolymer, Dimethiconol Isostearate, Dimethiconol Kokum Butterate, Dimethiconol Lactate, Dimethiconol Meadowfoamate, Dimethiconol Methionine, Dimethiconol/Methylsilanol/Silicate Crosspolymer, Dimethiconol Mohwa Butterate, Dimethiconol Panthenol, Dimethiconol Sal Butterate, Dimethiconol/Silica Crosspolymer, Dimethiconol/Silsesquioxane Copolymer, Dimethiconol Stearate, Dimethiconol/Stearyl, Methicone/Phenyl Trimethicone Copolymer, Dimethoxysilyl Ethylenediaminopropyl Dimethicone, Dimethylaminopropylamido PCA Dimethicone, Dimethyl Oxobenzo Dioxasilane, Dimethylsilanol Hyaluronate, Dioleyl Tocopheryl Methylsilanol, Diphenyl Amodimethicone, Diphenyl Dimethicone, Diphenyl Dimethicone Crosspolymer Diphenyl Dimethicone?/inyl Diphenyl Dimethicone/Silsesquioxane Crosspolymer, Diphenylethyl Benzyloxy Dilsiloxane, Diphenylisopropyl Dimethicone, Diphenylsiloxy Phenyl/Propyl Trimethicone, Diphenylsiloxy Phenyl Trimethicone Disiloxane, Disodium Amodimethicone Disuccinamide, Disodium PEG-12 Dimethicone Sulfosuccinate, Disodium PEG-8 Lauryl Dimethicone Sulfosuccinate, Divinyldimethicone/Dimethicone Copolymer, Divinyldimethicone/Dimethicone Crosspolymer, Drometrizole Trisiloxane, Ethylhexyl Acrylate/VP/Dimethicone Methacrylate Copolymer, Ethyl Methicone, Ethyl Trisiloxane, Fluoro C2-8 Alkyldimethicone, Gluconamidopropyl Aminopropyl Dimethicone, 4-(2-Beta-Glucopyranosiloxy) Propoxy-2-Hydroxybenzophenone, Glyceryl Undecyl Dimethicone, Glycidoxy Dimethicone, Hexadecyl Methicone, Hexyl Dimethicone, Hexyl Methicone, Hexyltrimethoxysilane, Hydrogen Dimethicone, Hydrogen Dimethicone/Octyl Silsesquioxane Copolymer, Hydrolyzed Collagen PG-Propyl Dimethiconol, Hydrolyzed Collagen PG-Propyl Methylsilanediol, Hydrolyzed Collagen PG-Propyl Silanetriol, Hydrolyzed Keratin PG-Propyl Methylsilanediol, Hydrolyzed Sesame Protein PG-Propyl Methylsilanediol, Hydrolyzed Silk PG-Propyl Methylsilanediol, Hydrolyzed Silk PG-Propyl Methylsilanediol Crosspolymer, Hydrolyzed Soy Protein/Dimethicone PEG-7 Acetate, Hydrolyzed Soy Protein PG-Propyl Methylsilanediol, Hydrolyzed Vegetable Protein PG-Propyl Silanetriol, Hydrolyzed Wheat Protein/Cystine Bis-PG-Propyl Silanetriol Copolymer, Hydrolyzed Wheat Protein/Dimethicone PEG-7 Acetate, Hydrolyzed Wheat Protein/Dimethicone PEG-7 Phosphate Copolymer, Hydrolyzed Wheat Protein PG-Propyl Methylsilanediol, Hydrolyzed Wheat Protein PG-Propyl Silanetriol, Hydroxyethyl Acetomonium PG-Dimethicone, Hydroxypropyldimethicone, Hydroxypropyl Dimethicone Behenate, Hydroxypropyl Dimethicone Isostearate, Hydroxypropyl Dimethicone Stearate, Isobutylmethacrylate/Bis-Hydroxypropyl Dimethicone Acrylate Copolymer, Isobutylmethacrylate/Trifluoroethylmethacrylate/Bis-Hydroxypropyl Dimethicone Acrylate Copolymer, Isopentyl Trimethoxycinnamate Trisiloxane, Isopolyglyceryl-3 Dimethicone, Isopolyglyceryl-3 Dimethiconol, Isopropyl Titanium Triisostearate/Triethoxysilylethyl, Polydimethylsiloxyethyl Dimethicone Crosspolymer, Isostearyl Carboxydecyl PEG-8 Dimethicone, Lactoyl Methylsilanol Elastinate, Lauryl Dimethicone, Lauryl Dimethicone PEG-15 Crosspolymer, Lauryl Dimethicone PEG-10 Phosphate, Lauryl Dimethicone/Polyglycerin-3 Crosspolymer, Lauryl Methicone, Lauryl PEG-8 Dimethicone, Lauryl PEG-10 Methyl Ether Dimethicone, Lauryl PEG-9 Polydimethylsiloxyethyl Dimethicone, Lauryl PEG/PPG-18/18 Methicone, Lauryl Phenylisopropyl Methicone, Lauryl Phenylpropyl Methicone, Lauryl Polydimethylsiloxyethyl Dimethicone/Bis-Vinyldimethicone Crosspolymer, Lauryl Polyglyceryl-3 Polydimethylsiloxyethyl Dimethicone, Lauryl Trimethicone, Linoleamidopropyl PG-Dimonium Chloride Phosphate Dimethicone, Methacryloyl Propyltrimethoxysilane, Methicone, Methoxy Amodimethicone/Silsesquioxane Copolymer, Methoxycinnamidopropyl Polysilsesquioxane, Methoxycinnamoylpropyl Silsesquioxane Silicate, Methoxy PEG-13 Ethyl Polysilsesquioxane, Methoxy PEG/PPG-7/3 Aminopropyl Dimethicone, Methoxy PEG/PPG-25/4 Dimethicone, Methoxy PEG-10 Propyltrimethoxysilane, Methyleugenyl PEG-8 Dimethicone, Methylpolysiloxane Emulsion, Methylsilanol Acetylmethionate, Methylsilanol Acetyltyrosine, Methylsilanol Ascorbate, Methylsilanol Carboxymethyl Theophylline, Methylsilanol Carboxymethyl Theophylline Alginate, Methylsilanol Elastinate, Methylsilanol Glycyrrhizinate, Methylsilanol Hydroxyproline, Methylsilanol Hydroxyproline Aspartate, Methylsilanol Mannuronate, Methylsilanol PCA, Methylsilanol PEG-7 Glyceryl Cocoate, Methylsilanol/Silicate Crosspolymer, Methylsilanol Spirulinate, Methylsilanol Tri-PEG-8 Glyceryl Cocoate, Methyl Trimethicone, Methyltrimethoxysilane, Myristylamidopropyl Dimethylamine Dimethicone PEG-7 Phosphate, Myristyl Methicone, Myristyl Trisiloxane, Nylon-611/Dimethicone Copolymer, PCA Dimethicone, PEG-7 Amodimethicone, PEG-8 Amodimethicone, PEG-8 Cetyl Dimethicone, PEG-3 Dimethicone, PEG-6 Dimethicone, PEG-7 Dimethicone, PEG-8 Dimethicone, PEG-9 Dimethicone, PEG-10 Dimethicone, PEG-12 Dimethicone, PEG-14 Dimethicone, PEG-17 Dimethicone, PEG-10 Dimethicone Crosspolymer, PEG-12 Dimethicone Crosspolymer, PEG-8 Dimethicone Dimer Dilinoleate, PEG-8 Dimethicone/Dimer Dilinoleic Acid Copolymer, PEG-10 Dimethicone/Vinyl Dimethicone Crosspolymer, PEG-8 Distearmonium Chloride PG-Dimethicone, PEG-10/Lauryl Dimethicone Crosspolymer, PEG- 15/Lauryl Dimethicone Crosspolymer, PEG-15/Lauryl Polydimethylsiloxyethyl Dimethicone Crosspolymer, PEG-8 Methicone, PEG-6 Methicone Acetate, PEG-6 Methyl Ether Dimethicone, PEG- 7 Methyl Ether Dimethicone, PEG-8 Methyl Ether Dimethicone, PEG-9 Methyl Ether Dimethicone, PEG-10 Methyl Ether Dimethicone, PEG-11 Methyl Ether Dimethicone, PEG-32 Methyl Ether Dimethicone, PEG-8 Methyl Ether Triethoxysilane, PEG-10 Nonafluorohexyl Dimethicone Copolymer, PEG-4 PEG-12 Dimethicone, PEG-8 PG-Coco-Glucoside Dimethicone, PEG-9 Polydimethylsiloxyethyl Dimethicone, PEG/PPG-20/22 Butyl Ether Dimethicone, PEG/PPG-22/22 Butyl Ether Dimethicone, PEG/PPG-23/23 Butyl Ether Dimethicone, PEG/PPG-24/18 Butyl Ether Dimethicone, PEG/PPG-27/9 Butyl Ether Dimethicone, PEG/PPG-3/10 Dimethicone, PEG/PPG-4/12 Dimethicone, PEG/PPG-6/4 Dimethicone, PEG/PPG-6/11 Dimethicone, PEG/PPG-8/14 Dimethicone, PEG/PPG-8/26 Dimethicone, PEG/PPG-10/2 Dimethicone, PEG/PPG-12/16 Dimethicone, PEG/PPG- 12/18 Dimethicone, PEG/PPG-14/4 Dimethicone, PEG/PPG-15/5 Dimethicone, PEG/PPG-15/15 Dimethicone, PEG/PPG-16/2 Dimethicone, PEG/PPG-16/8 Dimethicone, PEG/PPG-17/18 Dimethicone, PEG/PPG-18/6 Dimethicone, PEG/PPG-18/12 Dimethicone, PEG/PPG-18/18 Dimethicone, PEG/PPG-19/19 Dimethicone, PEG/PPG-20/6 Dimethicone, PEG/PPG-20/15 Dimethicone, PEG/PPG-20/20 Dimethicone, PEG/PPG-20/23 Dimethicone, PEG/PPG-20/29 Dimethicone, PEG/PPG-22/23 Dimethicone, PEG/PPG-22/24 Dimethicone, PEG/PPG-23/6 Dimethicone, PEG/PPG-25/25 Dimethicone, PEG/PPG-27/27 Dimethicone, PEG/PPG-30/10 Dimethicone, PEG/PPG-25/25 Dimethicone/Acrylates Copolymer, PEG/PPG-20/22 Methyl Ether Dimethicone, PEG/PPG-24/24 Methyl Ether Glycidoxy Dimethicone, PEG/PPG-10/3 Oleyl Ether Dimethicone, PEG/PPG-5/3 Trisiloxane, PEG-4 Trifluoropropyl Dimethicone Copolymer, PEG-8 Trifluoropropyl Dimethicone Copolymer, PEG-10 Trifluoropropyl Dimethicone Copolymer, PEG-8 Trisiloxane, Perfluorocaprylyl riethoxysilylethyl Methicone, Perfluorononyl Dimethicone, Perfluorononyl Dimethicone/Methicone/Amodimethicone Crosspolymer, Perfluorononylethyl Carboxydecyl Behenyl Dimethicone, Perfluorononylethyl Carboxydecyl Hexacosyl Dimethicone, Perfluorononylethyl Carboxydecyl Lauryl/Behenyl Dimethicone, Perfluorononylethyl Carboxydecyl Lauryl Dimethicone, Perfluorononylethyl Carboxydecyl PEG-8 Dimethicone, Perfluorononylethyl Carboxydecyl PEG-10 Dimethicone, Perfluorononylethyl Dimethicone/Methicone Copolymer, Perfluorononylethyl PEG-8 Dimethicone, Perfluorononylethyl Stearyl Dimethicone, Perfluorooctylethyl/Diphenyl Dimethicone Copolymer, Perfluorooctylethyl Triethoxysilane, Perfluorooctylethyl Trimethoxysilane, Perfluorooctylethyl Trisiloxane, Perfluorooctyl Triethoxysilane, PG-Amodimethicone, Phenethyl Dimethicone, Phenethyl Disiloxane, Phenyl Dimethicone, Phenylisopropyl Dimethicone, Phenyl Methicone, Phenyl Methiconol, Phenylpropyldimethylsiloxysilicate, Phenylpropyl Ethyl Methicone, Phenyl Propyl Trimethicone, Phenyl Propyl Trimethicone/Diphenylmethicone, Phenyl Trimethicone, Platinum Divinyldisiloxane, Polyacrylate-6, Polydiethylsiloxane, Polydimethylsiloxyethyl Dimethicone/Bis-Vinyldimethicone Crosspolymer, Polydimethylsiloxyethyl Dimethicone/Methicone Copolymer, Polydimethylsiloxy PEG/PPG-24/19 Butyl Ether Silsesquioxane, Polydimethylsiloxy PPG- 13 Butyl Ether Silsesquioxane, Polyglyceryl-3 Disiloxane Dimethicone, Polyglyceryl-3/Lauryl Polydimethylsiloxyethyl Dimethicone Crosspolymer, Polyglyceryl-3 Polydimethylsiloxyethyl Dimethicone, Poly(Glycol Adipate)/Bis-Hydroxyethoxypropyl Dimethicone Copolymer, Polymethylsilsesquioxane, Polymethylsilsesquioxane/Trimethylsiloxysilicate, Polyphenylsilsesquioxane, Polypropylsilsesquioxane, Polysilicone-1, Polysilicone-2, Polysilicone-3, Polysilicone-4, Polysilicone-5, Polysilicone-6, Polysilicone-7, Polysilicone-8, Polysilicone-9, Polysilicone-10, Polysilicone-11, Polysilicone-12, Polysilicone-13, Polysilicone-14, Polysilicone-15, Polysilicone-16, Polysilicone-17, Polysilicone-18, Polysilicone-19, Polysilicone-20, Polysilicone-21, Polysilicone-18 Cetyl Phosphate, Polysilicone-1 Crosspolymer, Polysilicone-18 Stearate, Polyurethane-10, Potassium Dimethicone PEG-7 Panthenyl Phosphate, Potassium Dimethicone PEG- 7 Phosphate, PPG-12 Butyl Ether Dimethicone, PPG-2 Dimethicone, PPG-12 Dimethicone, PPG-27 Dimethicone, PPG-4 Oleth-10 Dimethicone, Propoxytetramethyl Piperidinyl Dimethicone, Propyl Trimethicone, Quaternium-80, Retinoxytrimethylsilane, Silanediol Salicylate, Silanetriol, Silanetriol Arginate, Silanetriol Glutamate, Silanetriol Lysinate, Silanetriol Melaninate, Silanetriol Trehalose Ether, Silica, Silica Dimethicone Silylate, Silica Dimethyl Silylate, Silica Silylate, Silicon Carbide, Silicone Quaternium-1, Silicone Quaternium-2, Silicone Quaternium-2 Panthenol Succinate, Silicone Quaternium-3, Silicone Quaternium-4, Silicone Quaternium-5, Silicone Quaternium-6, Silicone Quaternium-7, Silicone Quaternium-8, Silicone Quaternium-9, Silicone Quaternium-10, Silicone Quaternium-11, Silicone Quaternium-12, Silicone Quaternium-15, SiliconeQuaternium-16, Silicone Quaternium-16/Glycidoxy Dimethicone Crosspolymer, Silicone Quaternium-17, Silicone Quaternium- 18, Silicone Quaternium-19, Silicone Quaternium-20, Silicone Quaternium-21, Silicone Quaternium- 22, Silicone Quaternium-24, Silicone Quaternium-25, Siloxanetriol Alginate, Siloxanetriol Phytate, Simethicone, Sodium Carboxydecyl PEG-8 Dimethicone, Sodium Dimethicone PEG-7 Acetyl Methyltaurate, Sodium Hyaluronate Dimethylsilanol, Sodium Lactate Methylsilanol, Sodium Mannuronate Methylsilanol, Sodium PCA Methylsilanol, Sodium PG-Propyldimethicone Thiosulfate Copolymer, Sodium PG-Propyl Thiosulfate Dimethicone, Sodium Propoxyhydroxypropyl Thiosulfate Silica, Sorbityl Silanediol, Soy Triethoxysilylpropyldimonium Chloride, Stearalkonium Dimethicone PEG-8 Phthalate, Stearamidopropyl Dimethicone, Steardimonium Hydroxypropyl Panthenyl PEG-7 Dimethicone Phosphate Chloride, Steardimonium Hydroxypropyl PEG-7 Dimethicone Phosphate Chloride, Stearoxy Dimethicone, Stearoxymethicone/Dimethicone Copolymer, Stearoxytrimethylsilane, Stearyl Aminopropyl Methicone, Stearyl Dimethicone, Stearyl/Lauryl Methacrylate Crosspolymer, Stearyl Methicone, Stearyl Triethoxysilanek, Stearyl Trimethicone, Styrene/Acrylates/Dimethicone Acrylate Crosspolymer, Styrene/Acrylates/Dimethicone Copolymer, TEA-Dimethicone PEG-7 Phosphate, Tetrabutoxypropyl Trisiloxane, Tetramethyl Hexaphenyl Tetrasiloxane, Tetramethyl Tetraphenyl Trisiloxane, Tocopheryloxypropyl Trisiloxane, Trideceth-9 PG-Amodimethicone, Triethoxycaprylylsilane, Triethoxysilylethyl Dimethicone/Methicone Copolymer, Triethoxysilylethyl Polydimethylsiloxyethyl Dimethicone, Triethoxysilylethyl Polydimethylsiloxyethyl Hexyl Dimethicone, Triethoxysilylpropylcarbamoyl Ethoxypropyl Butyl Dimethicone, Trifluoromethyl C1-4 Alkyl Dimethicone, Trifluoropropyl Cyclopentasiloxane, Trifluoropropyl Cyclotetrasiloxane, Trifluoropropyl Dimethicone, Trifluoropropyl Dimethicone/PEG-10 Crosspolymer, Trifluoropropyl Dimethicone/Trifluoropropyl Divinyldimethicone Crosspolymer, Trifluoropropyl Dimethicone/Vinyl Trifluoropropyl, Dimethicone/Silsesquioxane Crosspolymer, Trifluoropropyl Dimethiconol, Trifluoropropyldimethyl/trimethylsiloxysilicate, Trifluoropropyl Methicone, Trimethoxycaprylylsilane, Trimethoxysilyl Dimethicone, Trimethyl Pentaphenyl Trisiloxane, Trimethylsiloxyamodimethicone, Trimethylsiloxyphenyl Dimethicone, Trimethylsiloxysilicate, Trimethylsiloxysilicate/Dimethicone Crosspolymer, Trimethylsiloxysilicate/Dimethiconol Crosspolymer, Trimethylsiloxysilylcarbamoyl Pullulan, Trimethylsilyl Hydrolyzed Conchiolin Protein PG-Propyl Methylsilanediol Crosspolymer, Trimethylsilyl Hydrolyzed Silk PG-Propyl Methylsilanediol Crosspolymer, Trimethylsilyl Hydrolyzed Wheat Protein PG-Propyl Methylsilanediol Crosspolymer, Trimethylsilyl Pullulan, Trimethylsilyl Trimethylsiloxy Glycolate, Trimethylsilyl Trimethylsiloxy Lactate, Trimethylsilyl Trimethylsiloxy Salicylate, Triphenyl Trimethicone, Trisiloxane, Tris-Tributoxysiloxymethylsilane, Undecylcrylene Dimethicone, Vinyl Dimethicone, Vinyl Dimethicone/Lauryl Dimethicone Crosspolymer, Vinyl Dimethicone/Methicone Silsesquioxane Crosspolymer, Vinyldimethyl/Trimethylsiloxysilicate Stearyl Dimethicone Crosspolymer, VP/Dimethiconylacrylate/Polycarbamyl/Polyglycol Ester, Zinc Carboxydecyl Trisiloxane and Zinc Dimethicone PEG-8 Succinate, and mixtures thereof.

More preferably the silicones to be contained in the cosmetic or pharmaceutical composition according to the invention are Dimethicone, Cyclomethicone, Cyclopentasiloxane, Cyclotetrasiloxane, Phenyl Trimethicone, and Cyclohexasiloxane.

**Waxes and/or stabilizers:** Besides natural oils used, one or more waxes may also be present in the cosmetic or pharmaceutical composition according to the present invention, more especially natural waxes such as, for example, candelilla wax, carnauba wax, Japan wax, espartograss wax, cork wax, guaruma wax, rice oil wax, sugar cane wax,

ouricury wax, montan wax, beeswax, shellac wax, spermaceti, lanolin (wool wax), uropygial fat, ceresine, ozocerite (earth wax), petrolatum, paraffin waxes and microwaxes; chemically modified waxes (hard waxes) such as, for example, montan ester waxes, sasol waxes, hydrogenated jojoba waxes and synthetic waxes such as, for example, polyalkylene waxes and polyethylene glycol waxes. Metal salts of fatty acids such as, for example, magnesium, aluminium and/or zinc stearate or ricinoleate may be used as stabilizers.

**Primary sun protection factors:** In addition to the above disclosed liquid UV filters, the cosmetic or pharmaceutical composition according to the present invention preferably includes one or more further primary sun protection factors in order to optimize the sun protection factor (SPF) of the formulation, i.e. to obtain a high SPF of 5 to 50, or even 5 to 60, and to cover a broad UVA and UVB range. Primary sun protection factors in the context of the invention are, for example, organic substances (light filters) which are liquid or crystalline at room temperature and which are capable of absorbing ultraviolet radiation and of releasing the energy absorbed in the form of longer-wave radiation, for example heat.

The cosmetic or pharmaceutical composition according to the invention advantageously contains at least one UV-A filter and/or at least one UV-B filter and/or a broadband filter and/or at least one inorganic pigment. Compositions according to the invention preferably contain at least one UV-B filter or a broadband filter, more particularly preferably at least one UV-A filter and at least one UV-B filter. Preferred cosmetic compositions, preferably topical compositions according to the present invention comprise one, two, three or more sun protection factors selected from the group consisting of 4-aminobenzoic acid and derivatives, salicylic acid derivatives, benzophenone derivatives, dibenzoylmethane derivatives, diphenyl acrylates, 3-imidazol-4-yl acrylic acid and esters thereof, benzofuran derivatives, benzylidene malonate derivatives, polymeric UV absorbers containing one or more organosilicon radicals, cinnamic acid derivatives, camphor derivatives, trianilino-s-triazine derivatives, 2-hydroxyphenylbenzotriazole derivatives, phenylbenzimidazole sulfonic acid derivatives and salts thereof, anthranilic acid menthyl esters, benzotriazole derivatives and indole derivatives.

In addition, a combination with active ingredients which penetrate into the skin and protect the skin cells from inside against sunlight-induced damage and reduce the level of cutaneous matrix metalloproteases is advantageously. Preferred respective ingredients are so called arylhydrocarbon receptor antagonists. Preferred is 2-benzylidene-5,6-dimethoxy-3,3-dimethylindan-1-one.

The UV filters cited below which can be used within the context of the present invention are preferred but naturally are not limiting.

UV filters which are preferably used are selected from the group consisting of

- p-aminobenzoic acid
- p-aminobenzoic acid ethyl ester (25 mol) ethoxylated (INCI name: PEG-25 PABA)
- p-dimethylaminobenzoic acid-2-ethylhexyl ester
- p-aminobenzoic acid ethyl ester (2 mol) N-propoxylated
- p-aminobenzoic acid glycerol ester
- salicylic acid homomenthyl ester (homosalates) (Neo Heliopan®HMS)
- salicylic acid-2-ethylhexyl ester (Neo Heliopan®OS)
- triethanolamine salicylate
- 4-isopropyl benzyl salicylate
- anthranilic acid menthyl ester (Neo Heliopan®MA)
- diisopropyl cinnamic acid ethyl ester
- p-methoxycinnamic acid-2-ethylhexyl ester (Neo Heliopan®AV)
- diisopropyl cinnamic acid methyl ester
- p-methoxycinnamic acid isoamyl ester (Neo Heliopan®E 1000)
- p-methoxycinnamic acid diethanolamine salt
- p-methoxycinnamic acid isopropyl ester
- 2-phenylbenzimidazole sulfonic acid and salts (Neo Heliopan®Hydro)
- 3-(4'-trimethylammonium) benzylidene bornan-2-one methyl sulfate
- beta-imidazole-4(5)-acrylic acid (urocanic acid)
- 3-(4'-sulfo)benzylidene bornan-2-one and salts
- 3-(4'-methyl benzylidene)-D,L-camphor (Neo Heliopan®MBC)
- 3-benzylidene-D,L-camphor
- N-[(2 and 4)-[2-(oxoborn-3-ylidene) methyl]benzyl] acrylamide polymer
- 4,4'-[(6-[4-(1,1-dimethyl)aminocarbonyl) phenylamino]-1,3,5-triazine-2,4-diyl)diimino]-bis-(benzoic acid-2-ethylhexyl ester) (Uvasorb®HEB)
- benzylidene malonate polysiloxane (Parsol®SLX)
- glyceryl ethylhexanoate dimethoxycinnamate

- dipropylene glycol salicylate
- tris(2-ethylhexyl)-4,4',4"-(1,3,5-triazine-2,4,6-triyltriimino)tribenzoate (= 2,4,6-trianilino-(p-carbo-2'-ethylhexyl-1'-oxy)-1 ,3,5-triazine) (Uvinul®T150).

In a preferred variant the cosmetic or pharmaceutical composition according to the present invention comprises a combination with one or more broadband filters which are selected from the group consisting of

- 2-ethylhexyl-2-cyano-3,3-diphenyl acrylate (Neo Heliopan®303)
- ethyl-2-cyano-3,3'-diphenyl acrylate
- 2-hydroxy-4-methoxybenzophenone (Neo Heliopan®BB)
- 2-hydroxy-4-methoxybenzophenone-5-sulfonic acid
- dihydroxy-4-methoxybenzophenone
- 2,4-dihydroxybenzophenone
- tetrahydroxybenzophenone
- 2,2'-dihydroxy-4,4'-dimethoxybenzophenone
- 2-hydroxy-4-n-octoxybenzophenone
- 2-hydroxy-4-methoxy-4'-methyl benzophenone
- sodium hydroxymethoxybenzophenone sulfonate
- disodium-2,2'-dihydroxy-4,4'-dimethoxy-5,5'-disulfobenzophenone
- phenol, 2-(2H-benzotriazol-2-yl)-4-methyl-6-(2-methyl-3(1,3,3,3-tetramethyl-1-(trimethylsilyl)oxy)disiloxyanyl) propyl) (MexorylOXL)
- 2,2'-methylene bis-(6-(2H-benzotriazol-2-yl)-4-1,1,3,3-tetramethylbutyl) phenol) (Tinosorb®M)
- 2,4-bis-[4-(2-ethylhexyloxy)-2-hydroxyphenyl]-1,3,5-triazine
- 2,4-bis-[{(4-(2-ethylhexyloxy)-2-hydroxy}phenyl]-6-(4-methoxyphenyl)-1,3,5-triazine (Tinosorb®S)
- 2,4-bis-[{(4-(3-sulfonato)-2-hydroxypropyloxy)-2-hydroxy}phenyl]-6-(4-methoxyphenyl)-1,3,5-triazine sodium salt
- 2,4-bis-[{(3-(2-propyloxy)-2-hydroxypropyloxy)-2-hydroxy}phenyl]-6-(4-methoxyphenyl)-1,3,5-triazine
- 2,4-bis-[t4-(2-ethylhexyloxy)-2-hydroxylphenyl]-6-[4-(2-methoxyethyl carbonyl) phenylamino]-1,3,5-triazine
- 2,4-bis-[t4-(3-(2-propyloxy)-2-hydroxypropyloxy)-2-hydroxylphenyl]-6-[4-(2-ethylcarboxyl) phenylamino]-1,3,5-triazine
- 2.4-bis-[{4-(2-ethylhexyloxy)-2-hydroxy}phenyl]-6-(1-methylpyrrol-2-yl)-1,3,5-triazine
- 2,4-bis-[{4-tris-(trimethylsiloxysilylpropyloxy)-2-hydroxy}phenyl]-6-(4-methoxyphenyl)-1,3,5-triazine
- 2,4-bis-[{4-(2"-methylpropenyloxy)-2-hydroxy}phenyl]-6-(4-methoxyphenyl)-1,3,5-triazine
- 2,4-bis-[f4-(1',1',1',3',5',5',5'-heptamethylsiloxy-2"-methylpropyloxy)-2-hydroxy}phenyl]-6-(4-methoxyphenyl)-1,3,5-triazine.

In a preferred variant the cosmetic or pharmaceutical composition according to the present invention comprises a combination with one or more UV-A filters which are selected from the group consisting of

- 4-isopropyl dibenzoyl methane
- terephthalylidene dibornane sulfonic acid and salts (Mexoryl®SX)
- 4-t-butyl-4'-methoxydibenzoyl methane (avobenzone) / (Neo Heliopan®357)
- phenylene bis-benzimidazyl tetrasulfonic acid disodium salt (Neo Heliopan®AP)
- 2,2'-(1,4-phenylene)-bis-(1H-benzimidazole-4,6-disulfonic acid), monosodium salt
- 2-(4-diethylamino-2-hydroxybenzoyl) benzoic acid hexyl ester (Uvinul® A Plus)
- indanylidene compounds.

In a more preferred variant, the cosmetic or pharmaceutical composition according to the present invention comprises a combination with one or more UV filters which are selected from the group consisting of

- p-aminobenzoic acid
- 3-(4'-trimethylammonium) benzylidene bornan-2-one methyl sulfate
- salicylic acid homomenthyl ester (Neo Heliopan®HMS)
- 2-hydroxy-4-methoxybenzophenone (Neo Heliopan®BB)
- 2-phenylbenzimidazole sulfonic acid (Neo Heliopan®Hydro)
- terephthalylidene dibornane sulfonic acid and salts (Mexoryl®SX)
- 4-tert-butyl-4'-methoxydibenzoyl methane (Neo Heliopan®357)
- 3-(4'-sulfo)benzylidene bornan-2-one and salts
- 2-ethylhexyl-2-cyano-3,3-diphenyl acrylate (Neo Heliopan®303)

- N-[(2 and 4)-[2-(oxoborn-3-ylidene) methyl]benzyl] acrylamide polymer
- p-methoxycinnamic acid-2-ethylhexyl ester (Neo Heliopan®AV)
- p-aminobenzoic acid ethyl ester (25 mol) ethoxylated (INCI name: PEG-25 PABA)
- p-methoxycinnamic acid isoamyl ester (Neo Heliopan®E1000)
- 2,4,6-trianilino-(p-carbo-2'-ethylhexyl-1'-oxy)-1,3,5-triazine (Uvinul®T150)
- phenol, 2-(2H-benzotriazol-2-yl)-4-methyl-6-(2-methyl-3(1,3,3,3-tetramethyl-1-(trimethylsilyl)oxy)disiloxyanyl) propyl) (Mexoryl®XL)
- 4,4'-[(6-[4-(1,1-dimethyl)aminocarbonyl) phenylamino]-1,3,5-triazine-2,4-diyl)diimino]-bis-(benzoic acid-2-ethylhexyl ester) (Uvasorb HEB)
- 3-(4'-methyl benzylidene)-D,L-camphor (Neo Heliopan®MBC)
- 3-benzylidene camphor
- salicylic acid-2-ethylhexyl ester (Neo Heliopan®OS)
- 4-dimethylaminobenzoic acid-2-ethylhexyl ester (Padimate O)
- hydroxy-4-methoxybenzophenone-5-sulfonic acid and Na salt
- 2,2'-methylene bis-(6-(2H-benzotriazol-2-yl)-4-1,1,3,3-tetramethylbutyl) phenol) (Tinosorb®M)
- phenylene bis-benzimidazyl tetrasulfonic acid disodium salt (Neo Heliopan®AP)
- 2,4-bis-[{(4-(2-ethylhexyloxy)-2-hydroxy}phenyl]-6-(4-methoxyphenyl)-1,3,5-triazine (Tinosorb®S)
- benzylidene malonate polysiloxane (Parsol®SLX)
- menthyl anthranilate (Neo Heliopan®MA)
- 2-(4-diethylamino-2-hydroxybenzoyl) benzoic acid hexyl ester (Uvinul® A Plus)
- indanylidene compounds.

In a further preferred variant the cosmetic or pharmaceutical composition according to the invention contains a total amount of sunscreen agents, i.e. in particular UV filters and/or inorganic pigments (UV filtering pigments) so that the composition according to the invention has a light protection factor of greater than or equal to 5. Such compositions according to the invention are particularly suitable for protecting the skin and hair.

**Secondary sun protection factors:** In addition to the above disclosed liquid UV filters and besides the groups of primary sun protection factors mentioned above, secondary sun protection factors of the antioxidant type may also be advantageously used in the cosmetic or pharmaceutical composition according to the present invention. Secondary sun protection factors of the antioxidant type interrupt the photochemical reaction chain which is initiated when UV rays penetrate into the skin. Typical examples are amino acids (for example glycine, histidine, tyrosine, tryptophane) and derivatives thereof, imidazoles (for example urocanic acid) and derivatives thereof, peptides, such as D,L-carnosine, D-carnosine, L-carnosine and derivatives thereof (for example anserine), carotinoids, carotenes (for example alpha-carotene, beta-carotene, lycopene), phytoene, phytofluene and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, alpha-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and heptathionine sulfoximine) in very small compatible dosages, also (metal) chelators (for example alpha-hydroxyfatty acids, palmitic acid, phytic acid, lactoferrine), alpha-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, glycosyl rutin, ferulic acid, furfurylidene glucitol, carnosine, butyl hydroxytoluene, butyl hydroxyanisole, nordihydroguaiac resin acid, nordihydroguaiaretic acid, trihydroxybutyrophenone, uric acid and derivatives thereof, mannose and derivatives thereof, superoxide dismutase, titanium dioxide (for example dispersions in ethanol), zinc and derivatives thereof (for example ZnO, $ZnSO_4$), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

Advantageous inorganic secondary light protection pigments are finely dispersed metal oxides and metal salts. The total quantity of inorganic pigments, in particular hydrophobic inorganic micro-pigments in the finished cosmetic preparation according to the present invention is advantageously from 0.1 to 30 % by weight, preferably 0.5 to 10.0 % by weight, in each case based on the total weight of the preparation.

Also preferred are particulate UV filters or inorganic pigments, which can optionally be hydrophobed, can be used, such as the oxides of titanium ($TiO_2$), zinc (ZnO), iron ($Fe_2O_3$), zirconium ($ZrO_2$), silicon ($SiO_2$), manganese (e.g.

MnO), aluminium ($Al_2O_3$), cerium (e.g. $Ce_2O_3$) and/or mixtures thereof.

**Actives modulating skin and/or hair pigmentation:** The cosmetic or pharmaceutical composition according to the present invention may include active ingredients for skin and/or hair lightening. Preferred active ingredients for skin and/or hair lightening are selected from the group consisting of kojic acid (5-hydroxy-2-hydroxymethyl-4-pyranone), kojic acid derivatives, preferably kojic acid dipalmitate, arbutin, ascorbic acid, ascorbic acid derivatives, preferably magnesium ascorbyl phosphate, hydroquinone, hydroquinone derivatives, resorcinol, resorcinol derivatives, preferably 4-alkylresorcinols and 4-(1-phenylethyl)1,3-dihydroxybenzene (phenylethyl resorcinol), cyclohexylcarbamates, sulfur-containing molecules, preferably glutathione or cysteine, alpha-hydroxy acids (preferably citric acid, lactic acid, malic acid), salts and esters thereof, N-acetyl tyrosine and derivatives, undecenoyl phenylalanine, gluconic acid, chromone derivatives, preferably aloesin, flavonoids, 1-aminoethyl phosphinic acid, thiourea derivatives, ellagic acid, nicotinamide (niacinamide), zinc salts, preferably zinc chloride or zinc gluconate, thujaplicin and derivatives, triterpenes, preferably maslinic acid, sterols, preferably ergosterol, benzofuranones, preferably senkyunolide, vinyl guiacol, ethyl guiacol, dionic acids, preferably octodecene dionic acid and/or azelaic acid, inhibitors of nitrogen oxide synthesis, preferably L-nitroarginine and derivatives thereof, 2,7-dinitroindazole or thiocitrulline, metal chelators (preferably alpha-hydroxy fatty acids, phytic acid, humic acid, bile acid, bile extracts, EDTA, EGTA and derivatives thereof), retinoids, soy milk and extract, serine protease inhibitors or lipoic acid or other synthetic or natural active ingredients for skin and hair lightening, the latter preferably used in the form of an extract from plants, preferably bearberry extract, rice extract, papaya extract, turmeric extract, mulberry extract, bengkoang extract, nutgrass extract, liquorice root extract or constituents concentrated or isolated therefrom, preferably glabridin or licochalcone A, artocarpus extract, extract of rumex and ramulus species, extracts of pine species (pinus), extracts of vitis species or stilbene derivatives isolated or concentrated therefrom, saxifrage extract, scutelleria extract, grape extract and/or microalgae extract, in particular Tetraselmis suecica Extract.

Preferred skin lighteners are kojic acid and phenylethyl resorcinol as tyrosinase inhibitors, beta- and alpha-arbutin, hydroquinone, nicotinamide, dioic acid, Mg ascorbyl phosphate and vitamin C and its derivatives, mulberry extract, Bengkoang extract, papaya extract, turmeric extract, nutgrass extract, licorice extract (containing glycyrrhizin), alpha-hydroxy-acids, 4-alkylresorcinols, 4-hydroxyanisole. These skin lighteners are preferred due to their very good activity, in particular in combination with sclareolide according to the present invention. In addition, said preferred skin lighteners are readily available.

Advantageous skin and hair tanning active ingredients in this respect are substrates or substrate analogues of tyrosinase such as L-tyrosine, N-acetyl tyrosine, L-DOPA or L-dihydroxyphenylalanine, xanthine alkaloids such as caffeine, theobromine and theophyl-line and derivatives thereof, proopiomelanocortin peptides such as ACTH, alpha-MSH, peptide analogues thereof and other substances which bind to the melanocortin receptor, peptides such as Val-Gly-Val-Ala-Pro-Gly, Lys-Ile-Gly-Arg-Lys or Leu-Ile-Gly-Lys, purines, pyrimidines, folic acid, copper salts such as copper gluconate, chloride or pyrrolidonate, 1,3,4-oxadiazole-2-thiols such as 5-pyrazin-2-yl-1,3,4-oxadiazole-2-thiol, curcumin, zinc diglycinate (Zn(Gly)2), manganese(II) bicarbonate complexes ("pseudocat-alases"), tetrasubstituted cyclohexene derivatives, isoprenoids, melanin derivatives such as Melasyn-100 and MelanZe, diacyl glycerols, aliphatic or cyclic diols, psoralens, prostaglandins and analogues thereof, activators of adenylate cyclase and compounds which activate the transfer of melanosomes to keratinocytes such as serine proteases or agonists of the PAR-2 receptor, extracts of plants and plant parts of the chrysanthemum species, sanguisorba species, walnut extracts, urucum extracts, rhubarb extracts, microalgae extracts, in particular Isochrysis galbana, trehalose, erythrulose and dihydroxyacetone. Flavonoids which bring about skin and hair tinting or browning (e.g. quercetin, rhamnetin, kaempferol, fisetin, genistein, daidzein, chrysin and apigenin, epicatechin, diosmin and diosmetin, morin, quercitrin, naringenin, hesperidin, phloridzin and phloretin) can also be used.

**Hair growth activators or inhibitors:** Cosmetic or pharmaceutical compositions according to the present invention may also comprise one or more hair growth activators, i.e. agents to stimulate hair growth. Hair growth activators are preferably selected from the group consisting of pyrimidine derivatives such as 2,4-diaminopyrimidine-3-oxide (Aminexil), 2,4-diamino-6-piperidinopyrimidine-3-oxide (Minoxidil) and derivatives thereof, 6-amino-1,2-dihydro-1-hydroxy-2-imino-4-piperidinopyrimidine and its derivatives, xanthine alkaloids such as caffeine, theobromine and theophylline and derivatives thereof, quercetin and derivatives, dihydroquercetin (taxifolin) and derivatives, potassium channel openers, antiandrogenic agents, synthetic or natural 5-reductase inhibitors, nicotinic acid esters such as tocopheryl nicotinate, benzyl nicotinate and C1-C6 alkyl nicotinate, proteins such as for example the tripeptide Lys-Pro-Val, diphencypren, hormones, finasteride, dutasteride, flutamide, bicalutamide, pregnane derivatives, progesterone and its derivatives, cyproterone acetate, spironolactone and other diuretics, calcineurin inhibitors such as FK506 (Tacrolimus, Fujimycin) and its derivatives, Cyclosporin A and derivatives thereof, zinc and zinc salts, polyphenols, procyanidins, proanthocyanidins, phytosterols such as for example beta-sitosterol, biotin, eugenol, (±)-beta-citronellol, panthenol, glycogen for example from mussels, extracts from microorganisms, algae, plants and plant parts of for example the genera dandelion (Leontodon or Taraxacum), Orthosiphon, Vitex, Coffea, Paullinia, Theobroma, Asiasarum, Cucurbita or Styphnolobium, Serenoa repens (saw palmetto), Sophora flavescens, Pygeum africanum, Panicum miliaceum, Cimicifuga racemosa, Glycine max, Eugenia caryophyllata, Cotinus coggygria, Hibiscus rosa-sinensis, Camellia sinensis, Ilex paraguariensis,

Isochrysis galbana, licorice, grape, apple, barley or hops or/nd hydrolysates from rice or wheat.

Alternatively, the cosmetic or pharmaceutical composition according to the present invention may include one or more hair growth inhibitors (as described above), i.e. agents to reduce or prevent hair growth. Hair growth inhibitors are preferably selected from the group consisting of activin, activin derivatives or activin agonists, ornithine decarboxylase inhibitors such as alpha-difluoromethylornithine or pentacyclic triterpenes like for example ursolic acid, betulin, betulinic acid, oleanolic acid and derivatives thereof, 5alpha-reductase inhibitors, androgen receptor antagonists, S-adenosylmethionine decarboxylase inhibitors, gamma-glutamyl transpeptidase inhibitors, transglutaminase inhibitors, soybean-derived serine protease inhibitors, extracts from microorganisms, algae, different microalgae or plants and plant parts of for example the families Leguminosae, Solanaceae, Graminae, Asclepiadaceae or Cucurbitaceae, the genera Chondrus, Gloiopeltis, Ceramium, Durvillea, Glycine max, Sanguisorba officinalis, Calendula officinalis, Hamamelis virginiana, Arnica montana, Salix alba, Hypericum perforatum or Gymnema sylvestre.

**Enzyme inhibitors:** The cosmetic or pharmaceutical composition according to the present invention may comprise one or more enzyme inhibitors. Suitable enzyme inhibitors are, for example, esterase inhibitors. These are preferably trialkyl citrates, such as trimethyl citrate, tripropyl citrate, triisopropyl citrate, tributyl citrate and, in particular, triethyl citrate (Hydagen CAT). The substances inhibit enzyme activity, thereby reducing the formation of odour. Other substances which are suitable esterase inhibitors are sterol sulfates or phosphates, such as, for example, lanosterol, cholesterol, campesterol, stigmasterol and sitosterol sulfate or phosphate, dicarboxylic acids and esters thereof, such as, for example, glutaric acid, monoethyl glutarate, diethyl glutarate, adipic acid, monoethyl adipate, diethyl adipate, malonic acid and diethyl malonate, hydroxycarboxylic acids and esters thereof, such as, for example, citric acid, malic acid, tartaric acid or diethyl tartrate, and zinc glycinate.

**Odour absorbers and/or antiperspirant active agents:** The cosmetic or pharmaceutical composition according to the present invention may include one or more odour absorbers and/or antiperspirant active agents (antiperspirants). Suitable odour absorbers are substances which are able to absorb and largely retain odour-forming compounds. They lower the partial pressure of the individual components, thus also reducing their rate of diffusion. It is important that perfumes must remain unimpaired in this process. Odour absorbers are not effective against bacteria. They comprise, for example, as main constituent, a complex zinc salt of ricinoleic acid or specific, largely odour-neutral fragrances which are known to the person skilled in the art as "fixatives", such as, for example, extracts of labdanum or styrax or certain abietic acid derivatives. The odour masking agents are fragrances or perfume oils, which, in addition to their function as odour masking agents, give the deodorants their respective fragrance note. Perfume oils which may be mentioned are, for example, mixtures of natural and synthetic fragrances. Natural fragrances are extracts from flowers, stems and leaves, fruits, fruit peels, roots, woods, herbs and grasses, needles and branches, and resins and balsams. Also suitable are animal products, such as, for example, civet and castoreum. Typical synthetic fragrance compounds are products of the ester, ether, aldehyde, ketone, alcohol, and hydrocarbon type. Fragrance compounds of the ester type are, for example, benzyl acetate, p-tert-butylcyclohexyl acetate, linalyl acetate, phenylethyl acetate, linalyl benzoate, benzyl formate, allyl cyclohexylpropionate, styrallyl propionate and benzyl salicylate. The ethers include, for example, benzyl ethyl ether, and the aldehydes include, for example, the linear alkanals having 8 to 18 carbon atoms, citral, citronellal, citronellyloxyacetaldehyde, cyclamen aldehyde, hydroxycitronellal, lilial and bourgeonal, the ketones include, for example, the ionones and methyl cedryl ketone, the alcohols include anethole, citronellol, eugenol, isoeugenol, geraniol, linalool, phenylethyl alcohol and terpineol, and the hydrocarbons include mainly the terpenes and balsams. Preference is, however, given to using mixtures of different fragrances which together produce a pleasing fragrance note. Essential oils of relatively low volatility, which are mostly used as aroma components, are also suitable as perfume oils, e.g. sage oil, camomile oil, oil of cloves, melissa oil, mint oil, cinnamon leaf oil, linden flower oil, juniperberry oil, vetiver oil, olibanum oil, galbanum oil, labdanum oil and lavandin oil. Preference is given to using bergamot oil, dihydromyrcenol, lilial, lyral, citronellol, phenylethyl alcohol, $\alpha$-hexylcinnamaldehyde, geraniol, benzylacetone, cyclamen aldehyde, linalool, boisambrene forte, ambroxan, indole, hedione, sandelice, lemon oil, mandarin oil, orange oil, allyl amyl glycolate, cyclovertal, lavandin oil, clary sage oil, $\beta$-damascone, geranium oil bourbon, cyclohexyl salicylate, Vertofix coeur, iso-E-super, Fixolide NP, evernyl, iraldein gamma, phenylacetic acid, geranyl acetate, benzyl acetate, rose oxide, romilat, irotyl and floramat alone or in mixtures.

Suitable astringent antiperspirant active ingredients are primarily salts of aluminium, zirconium or of zinc. Such suitable antihydrotic active ingredients are, for example, aluminium chloride, aluminium chlorohydrate, aluminium dichlorohydrate, aluminium sesquichlorohydrate and complex compounds thereof, e.g. with 1,2-propylene glycol, aluminium hydroxyallantoinate, aluminium chloride tartrate, aluminium zirconium trichlorohydrate, aluminium zirconium tetrachlorohydrate, aluminium zirconium pentachlorohydrate and complex compounds thereof, e.g. with amino acids, such as glycine.

**Film formers:** The cosmetic or pharmaceutical composition according to the present invention may include one or more film formers. Standard film formers are preferably chitosan, microcrystalline chitosan, quaternized chitosan, polyvinyl pyrrolidone, vinyl pyrrolidone/vinyl acetate copolymers, polymers of the acrylic acid series, quaternary cellulose derivatives, collagen, hyaluronic acid and salts thereof and similar compounds.

**Carriers and hydrotropes:** The cosmetic or pharmaceutical composition according to the present invention may comprise a carrier or a mixture of different carriers. Preferred cosmetics carrier materials are solid or liquid at 25°C and 1013 mbar (including highly viscous substances) as for example glycerol, 1,2-propylene glycol, 1,2-butylene glycol, 1,3-propylene glycol, 1,3-butylene glycol, ethanol, water, and mixtures of two or more of said liquid carrier materials with water. Optionally, these preparations according to the invention may be produced using preservatives or solubilizers. Other preferred liquid carrier substances, which may be a component of a preparation according to the invention are selected from the group consisting of oils such as vegetable oil, neutral oil and mineral oil.

Preferred solid carrier materials, which may be a component of the cosmetic or pharmaceutical composition according to the invention are hydrocolloids, such as starches, degraded starches, chemically or physically modified starches, dextrins, (powdery) maltodextrins (preferably with a dextrose equivalent value of 5 to 25, preferably of 10 - 20), lactose, silicon dioxide, glucose, modified celluloses, gum arabic, ghatti gum, traganth, karaya, carrageenan, pullulan, curdlan, xanthan gum, gellan gum, guar flour, carob bean flour, alginates, agar, pectin, inulin, and mixtures of two or more of these solids, in particular maltodextrins (preferably with a dextrose equivalent value of 15 - 20), lactose, silicon dioxide and/or glucose.

In addition, hydrotropes, for example ethanol, isopropyl alcohol or polyols, may be used to improve flow behaviour. Suitable polyols preferably contain 2 to 15 carbon atoms and at least two hydroxyl groups. The polyols may contain other functional groups, more especially amino groups, or may be modified with nitrogen. Typical examples are

- glycerol;
- alkylene glycols such as, for example, ethylene glycol, diethylene glycol, propylene glycol, butylene glycol, hexylene glycol and polyethylene glycols with an average molecular weight of 100 to 1000 Dalton;
- technical oligoglycerol mixtures with a degree of self-condensation of 1.5 to 10, such as for example technical diglycerol mixtures with a diglycerol content of 40 to 50% by weight;
- methylol compounds such as, in particular, trimethylol ethane, trimethylol propane, trimethylol butane, pentaerythritol and dipentaerythritol;
- lower alkyl glucosides, particularly those containing 1 to 8 carbon atoms in the alkyl group, for example methyl and butyl glucoside;
- sugar alcohols containing 5 to 12 carbon atoms, for example sorbitol or mannitol,
- sugars containing 5 to 12 carbon atoms, for example glucose or sucrose;
- amino sugars, for example glucamine;
- dialcoholamines, such as diethanolamine or 2-aminopropane-1,3-diol.

Dyes: The cosmetic or pharmaceutical composition according to the present invention may comprise one or more dyes. Suitable dyes are any of the substances suitable and approved for cosmetic purposes. Examples include cochineal red A (CI. 16255), patent blue V (CI. 42051), indigotin (CI. 73015), chlorophyllin (CI. 75810), quinoline yellow (CI. 47005), titanium dioxide (CI. 77891), indanthrene blue RS (CI. 69800) and madder lake (CI. 58000). Luminol may also be present as a luminescent dye. Advantageous coloured pigments are for example titanium dioxide, mica, iron oxides (e.g. $Fe_2O_8$ $Fe_3O_4$, FeO(OH)) and/or tin oxide. Advantageous dyes are for example carmine, Berlin blue, chromium oxide green, ultramarine blue and/or manganese violet.

In addition to the above-described substances, further ingredients commonly used in the cosmetic or pharmaceutical industry, which are suitable or customary in the compositions of the present invention, can be used.

Preferably, the cosmetic or pharmaceutical composition as disclosed herein is an aqueous or aqueous/alcoholic, preferably aqueous/ethanolic, or aqueous/glycolic based solution. The aqueous/alcoholic or aqueous/glycolic based solution comprises an aliphatic alcohol or a glycol in an amount of 0.1 to ≤ 50 % by weight, based on the total weight of the solution. The aliphatic alcohol is preferably selected from the group consisting of ethanol, isopropanol, n-propanol. The glycol is preferably selected from the group consisting of glycerin, propylene glycol, butylene glycol or dipropylene glycol. Preferably, the overall water content in the final composition of such compositions can be ≥ 60 %, more preferably ≥ 70 %, even more preferably ≥ 80 %, and even more preferably ≥ 90 %. New applications such as those including solutions for water wipes have high water content. In a particular variant, the inventive compositions can be used for such wet wipe applications. They may then most preferably contain ≥ 95 % water, or even ≥ 98 % water.

The higher the water content in such a formulation, the lower the solubility of the liquid lipophilic components.

Such aqueous or aqueous/alcoholic or aqueous/glycolic based solutions include for example deo/antiperspirant preparations, after shave, cleansing preparations, anti-acne preparations, or wet wipe solutions.

In one preferred variant, the cosmetic or pharmaceutical composition according to the present invention is an emulsion as defined herein, advantageously an oil-in-water (O/W) emulsion comprising an oily phase dispersed in an aqueous phase in the presence of an O/W emulsifier or is a water free formulation or a solid surfactant composition.

These formulations or compositions are prepared according to usual and known methods.

Preferably, the cosmetic composition according to the present invention is a preparation for cosmetic use and/or

non-therapeutic use in personal care, preferably for skin protection, skin care, scalp protection, scalp care, hair care, nail care, in particular for the prevention and/or treatment of skin conditions, intolerant or sensitive skin, skin irritation, skin reddening, rosacea, wheals, pruritus (itching), skin aging, wrinkle formation, loss of skin volume, loss of skin elasticity, pigment spots, pigment abnormalities, skin dryness, flaking, greasiness, hypopigmentation and/or hyperpigmentation of the skin; or is a functional preparation or a preparation for animal care.

Examples of personal care are preferably anti-ageing preparations, skin care emulsions, body oils, body lotions, cleansing lotions, face or body balms , after shave balms, after sun balms, deo emulsions, cationic emulsions, body gels, treatment creams, skin protection ointments, moisturizing gels, face and/or body moisturizers, light protective preparations (sunscreens), micellar water, hair spray, colour protection hair care products, skin lightening product, anti-dark spot preparations, etc.

The present invention provides in a further aspect a cosmetic product comprising a composition according to the present invention, wherein the product is a deodorant and/or antiperspirant, an aerosol-based deo spray, deo pump spray, deo stick, deo roll on, deo cream, deo wipes, deo crystals, pickering emulsions, hydrodisperion gels, skin balms, shampoo, shower gel, foam bath, micellar water, facial cleansing solutions, cleansing wipes, intimate spray, intimate cream, intimate wash lotion, intimate wipes, aerosol-based foot spray, foot pump spray, foot bath, foot balm, soap, liquid washing, shower and bath preparation, bath product, bath capsule, bath oil, bath tablet, bath salt, bath soap, effervescent preparation, mouth wash concentrate, mouth wash ready to use, repellent, anti-insect applications, in particular insect repellent lotion, spray, solution or cream, mosquito repellents, human-smell-masking applications, perfume compositions and tooth paste.

Alternatively, the present invention provides a pharmaceutical preparation comprising a composition according to the present invention.

The pharmaceutical, in particular dermatological, composition according to the present invention is a preparation for the prevention and treatment of a condition of the skin or mucosa.

Preferably, the pharmaceutical composition according to the first and second aspect of the present invention is used in the prevention and/or treatment of of dysfunctions of human hair, skin and/or nails, in particular dermatological or keratological diseases, wherein the dermatological or keratological diseases are selected from the group consisting of atopic dermatitis (neurodermitis), psoriasis, acneiform exanthema, sebostasis, xerosis, eczema, hyper seborrhea and hypo seborrhea, dermatitis, rosacea, erythema, wheals, pruritus (itching), inflammation, irritation, fibrosis, *lichen planus, pityriasis rosea*, *pityriasis versicolor,* autoimmune bullous diseases, urticaria, angioedema, allergic skin reactions, wound healing, tissue regeneration and in the treatment of inflammatory diseases.

In order to be used, the cosmetic or pharmaceutical, in particular dermatological composition according to the first aspect or second aspect of the present invention is applied to the skin, hair, scalp and/or nails in an adequate amount in such manner as is customary with cosmetics and dermatological products.

In a further alternative, the present invention provides a homecare product comprising a composition according to the present invention. The homecare products according to the present invention are predominantly detergent formulations, usually liquids, powders, sprays, granules or tablets, used to remove dirt, including dust, stains, bad smells and clutter on surfaces or other types of housecleaning or laundry detergent that is added for cleaning laundry or liquid soap. Typical homecare products include all-purpose cleaners, dishwashing detergents, hard floor and surface cleaners, glass cleaners, carpet cleaners, oven cleaners, laundry detergents, fabric softeners, laundry scent, scent lotions, car shampoo, rim block gel, car shampoo, furniture polish, all afore-mentioned goods also in encapsulated form or air fresheners.

The present invention provides in a further aspect for the use of the composition according to the first aspect or second aspect of the present invention for enhancing the antioxidative effect of an antioxidant in a cosmetic or pharmaceutical composition or homecare product, for improving the sensory properties, i.e. skin sensation, of a cosmetic or pharmaceutical composition comprising a liquid lipophilic component, for sebum control, or for malodour management, or for modulating fragrance notes, or for modification of fragrance notes, or for suppression of fragrance notes, or for topical applications, or for coverage of the malodour of 1-octen-3-ol, or for masking insect alluring odours, or for repelling insects.

Still further, the present invention provides for the use of a composition according to the first aspect or second aspect of the the present invention as defined above for producing a cosmetic or pharmaceutical product or homecare product according to the present invention.

The present invention shall now be described in detail with reference to the following examples, which are merely illustrative of the present invention, such that the content of the present invention is not limited by or to the following examples.

## Examples

### A) Antimicrobial activity: Minimum Inhibitory Concentration (MIC)

Methods

The MIC test is a test on growth inhibition. Estimation of Minimum Inhibitory Concentration (MIC) is executed in 96 well plates. Through the comparison of bacterial growth with positive and negative controls via optical density (OD), different concentrations of given test substances are evaluated.

Bacteria are cultivated under adjusted conditions based on information of the German Collection of Microorganisms and Cell Cultures (DSMZ) and stored in 50% Glycerol prior to use. Afterwards, the following method was applied: Adding growth medium (according to microorganism) to each well of the microplate. Adding the test-substances in different concentrations, positive controls (water controls only with medium) and negative controls (benchmarks according to organism). Adding the microorganism in a concentration of $10^6$ CFU / ml (CFU = colony forming units). Incubation of the microplates at appropriate conditions.

According to resulting growth, substance concentrations are labelled either as inhibiting or not and MIC is defined as the lowest concentration where a complete growth inhibition is visible. Experiments at concentration limits (highest concentration labelled as growth and lowest concentration labelled as inhibiting) are performed at least twice.

### Results

**Example A.1: Antimicrobial properties of alkanediols against microorganisms naturally related to the human skin**

**Table 1:** Antimicrobial properties of 1,2-heptanediol, 1,2-octanediol, 1,2-nonanediol, 2,3-heptanediol, 2,3-octanediol and 2,3-nonanediol against microorganisms naturally related to the human skin: Minimum Inhibitory Concentration (MIC) in ppm.

| | Staphylo -coccus aureus | Candi da albica ns | Esche richia coli | Staphylo coccus hominis | Coryneb acterium jeikeium | Propionibacteriu m acnes |
|---|---|---|---|---|---|---|
| **1,2-Heptanediol** | 10000 | 5000 | 5000 | 10000 | 7500 | > 5000 |
| **1,2-Octanediol** | 2500 | 2500 | 5000 | 5000 | 5000 | |
| **1,2-Nonanediol** | 1000 | 1000 | 500 | 1000 | 2500 | 1000 |
| **2,3-Heptanediol** | 20000 | >5000 | >5000 | >5000 | >5000 | >5000 |
| **2,3-Octanediol** | 5000 | 5000 | 2500 | >5000 | 5000 | >5000 |
| **2,3-Nonanediol** | 2500 | 2500 | 1000 | 5000 | 2500 | 2500 |

Minimum Inhibitory Concentration (MIC) are given in ppm. "> 5000" indicates that 5000 ppm was tested and no growth inhibition found, higher values were not investigates.

As can be seen from these results, all the tested alkanediols exhibited an excellent antimicrobial effect for the tested microorganisms naturally related to the human skin. The best antimicrobial effect was achieved by 1,2-nonanediol. In addition, 2,3-nonanediol exhibited a remarkably strong antimicrobial effect. It was only recently discovered that the microorganism Corynebacterium jeikeium is one microorganism which is responsible for producing compounds having an unpleasant smell from human sweat. The inhibition of the microbial activity of Corynebacterium jeikeium is difficult. It has been surprisingly found, that 1,2-heptanediol, 1,2-nonanediol and 2,3-nonanediol exhibited an antimicrobial effect for Corynebacterium jeikeium. Especially, the tested nonanediols showed a remarkably strong antimicrobial effect for Corynebacterium jeikeium. Therefore, all the tested alkanediols are especially useful for malodour management, for

topical applications, and for masking insect alluring odours, since insects are attracted for example by the smell of compounds produced by microbes from sweat. Particularly useful are 1,2-nonanediol and 2,3-nonanediol.

**Table 2:** Antimicrobial properties of 2,3-heptanediol, 2,3-octanediol, 2,3-nonanediol and 2,3-undecanediol in comparison to 1,2-decanediol against microorganisms naturally related to the human skin: Minimum Inhibitory Concentration (MIC) in ppm

| | Staphylococcus aureus | Candida albicans | Escherichia coli | Staphylococcus hominis | Corynebacterium jeikeium | Propionibacterium acnes |
|---|---|---|---|---|---|---|
| **1,2-Decanediol** | 250 | 250 | 250 | 500 | 2500 | 500 |
| **2,3-Heptanediol** | 20000 | > 5000 | >5000 | > 5000 | > 5000 | > 5000 |
| **2,3-Octanediol** | 5000 | 5000 | 2500 | > 5000 | 5000 | > 5000 |
| **2,3-Nonanediol** | 2500 | 2500 | 1000 | 5000 | 2500 | 2500 |
| **2,3-Undecanediol** | 250 | 250 | 500 | 250 | 250 | 250 |

Minimum Inhibitory Concentration (MIC) are given in ppm. "> 5000" indicates that 5000 ppm was tested and no growth inhibition found, higher values were not investigated.

As it is obvious from Table 2, 2,3-undecanediol has the lowest MIC values against all tested microorganisms. This means that less amounts of 2,3-undecanediol are required for inhibiting microorganisms' growth. In addition, 2,3-undecanediol has similar MIC values as 1,2-decanediol, which on the other hand, however, has an unpleasant inherent smell. The antimicrobial performance of 2,3-undecanediol against *C. jeikeium* is even better than that of 1,2-decanediol.

**Example A.2: Synergistic activity of combinations including a 1,2-alkanediol and a 2,3-alkanediol against *Staphylococcus aureus***

The synergistic activity of mixtures including a 1,2-alkanediol and a 2,3-alkanediol was determined by measuring the Minimum Inhibitory Concentrations (MIC) according to the method as described above in methods section.

To verify that the tested combinations act synergistically against microorganisms of the natural microbiota of mammalian skin, corresponding experiments were conducted with *Staphylococcus aureus,* a well-known pathogen on the skin. The synergistic activities of different alkanediol mixtures including a 1,2-alkanediol and a 2,3-alkanediol in ratios as specified in the following Table 3 were determined by measuring the MIC of the mixture as indicated in methods section.

In order to calculate the synergy index with MIC values, the Kull equation (I) was used:

$$SI = (MIC_{mixture} \times P_A) / MIC_A + (MIC_{mixture} \times P_B) / MIC_B \qquad (I)$$

wherein

| | |
|---|---|
| SI | is the Synergy Index according to Kull |
| $MIC_A$ | is the MIC value for Component A |
| $MIC_B$ | is the MIC value for Component B |
| $MIC_{mixture}$ | is the MIC value for the mixture of Components A and B |
| $P_A$ | is the proportion of the Component A in the mixture |
| $P_B$ | is the proportion of the Component B in the mixture |

**Table 3:** MIC values and synergy index of different alkanediol mixtures including a 1,2-alkanediol and a 2,3-alkanediol as antimicrobial component against *Aspergillus brasiliensis*

| Compo -nent A | Component B | Portion Component A | Portion Compon ent B | MIC Compone nt A in ppm | MIC Compone nt B in ppm | MIC Mixture in ppm | Synerg y Index |
|---|---|---|---|---|---|---|---|
| 1,2-hexanediol | 2,3-hexan-diol | 0.9 | 0.1 | 7500 | 20000 | 7500 | 0.94 |
| 1,2-heptanediol | 2,3-heptan e-diol | | | 5000 | 7500 | 2500 | 0.49 |
| 1,2-octanediol | 2,3-octane -diol | | | 2500 | 2500 | 500 | 0.20 |
| 1,2-nonanediol | 2,3-nonan e-diol | | | 500 | 1000 | 250 | 0.48 |

For all tested 1,2-alkanediol and 2,3-alkanediol mixtures according to the present invention, synergistic effects regarding the antimicrobial efficacy were found with SI values below 1.

In addition, a synergistic antimicrobial efficacy could be observed for an alkanediol mixture including 1,2-heptanediol and 2,3-heptanediol (90 : 10) against *Pseudomonas aeruginosa.* The Synergy Index for said alkanediol mixture was 0.50.

**Example A.3: Further antimicrobial properties of 1,2-nonanediol**

**Table 4:** Antimicrobial properties of 1,2-nonanediol: Minimum Inhibitory Concentration (MIC) in ppm

| Organism | MIC (ppm) |
|---|---|
| *Aspergillus niger* | 2500 |
| *Corynebacterium xerosis* | 2500 |
| *Staphylococcus epidermidis* | 1000 |
| *Anaerococcus octavius* | 1000 |

In addition, as can be seen from the results of Table 4, 1,2-nonanediol exhibited an excellent antimicrobial effect for further tested microorganisms naturally related to the human skin. It was only recently discovered that the microorganism *Anaerococcus octavius* is one microorganism which is responsible for producing compounds having an unpleasant smell from human sweat. The inhibition of the microbial activity of *Anaerococcus octavius* is difficult. It has been surprisingly found, that 1,2-nonanediol exhibited an antimicrobial effect for *Anaerococcus octavius.* Consequently, 1,2-nonanediol is especially useful for malodour management, topical applications and masking insect alluring odours.

**Example A.4: Synergistic antimicrobial efficacy of 1,2-alkanediol and 2,3-alkanediol mixtures and 2,3-alkanediols**

The following preservation efficacy tests (PET) were performed to show the antimicrobial efficacy of compositions according to the present invention and in particular to demonstrate the boosting, i.e. synergistic antimicrobial efficacy of said compositions.

The preservation efficacy test (PET) was performed in accordance with European Pharmacopoeia § 5.1.3: The test is a reference European Pharmacopoeia method, that is to be used to evaluate the preservation system of a cosmetic or pharmaceutical prepration. The test consists of challenging the preparation under test, with prescribed inoculums of suitable microorganisms. The preservation porperties of the preparation are adequate if, in the conditions of the test, there is a significant fall or no increase, as appropriate, in the number of microorganisms in the inoculated preparations after the time prescribed.

The test microorganisms which were used, are the following:

Pseudomonas aeruginosa (PA) (bacterium);

Staphylococcus aureus (SA) (bacterium);
Escherichia coli (EC) (bacterium);
Candida albicans (CA) (yeast); and
Aspergillus brasiliensis (AB) (fungus).

**Table 5:** Results for the synergistic antimicrobial efficacy of 1,2-heptanediol and 2,3-heptanediol against bacteria, yeast and fungi/mould in o/w emulsion

| Substance | | Inoculum | 2 days | 7 days | 14 days | 28 days |
|---|---|---|---|---|---|---|
| 0.4% 1,2-heptanediol | PA | 5.300.000 | 1 | 1 | 1 | 1 |
| | SA | 5.800.000 | 15.000 | 2.600 | 1 | 1 |
| | EC | 5.000.000 | 23.000 | 4.100 | 1 | 1 |
| | CA | 1.400.000 | 71.000 | 2.800 | 1 | 1 |
| | AB | 1.100.000 | 1.000.000 | 180.000 | 150.000 | 1 |
| 0.4% 2,3-heptanediol | PA | 3.800.000 | 1 | 1 | 1 | 1 |
| | SA | 1.900.000 | 1.000.000 | 12.000 | 1 | 1 |
| | EC | 4.300.000 | 320.000 | 2.400 | 1 | 1 |
| | CA | 1.500.000 | 640.000 | 350.000 | 130.000 | 11.000 |
| | AB | 2.500.000 | 790.000 | 930.000 | 500.000 | 390.000 |
| 0.4% Heptanediol Blend (95% 1,2-heptanediol, 5% 2,3-heptanediol) | PA | 3.800.000 | 1 | 1 | 1 | 1 |
| | SA | 1.900.000 | 3.000 | 1 | 1 | 1 |
| | EC | 4.300.000 | 140 | 1 | 1 | 1 |
| | CA | 1.500.000 | 20.000 | 160 | 1 | 1 |
| | AB | 2.500.000 | 820.000 | 480.000 | 430.000 | 330.000 |

The synergistic antimicrobial efficacy of 1,2-heptanediol and 2,3-heptanediol against yeast is visualized in Figure 1.

The synergistic antimicrobial effect of 1,2-heptanediol and 2,3-heptanediol as shown in Table 5 demonstrates that compositions according to the present invention comprising at least one 1 ,2-alkanediol and at least one 2,3-alkanediol as defined above are useful in efficient malodour management products, in products for topical applications and in products for masking insect alluring odours.

**Table 6:** Results for the synergistic antimicrobial efficacy of 1,2-heptanediol, 2,3-heptanediol, 2,3-octanediol and 2,3-nonanediol in shampoo

| Substance | | Inoculum | 2 days | 7 days | 14 days | 28 days |
|---|---|---|---|---|---|---|
| 0,1 % 1,2-heptanediol | PA | 4.700.000 | 2.500 | 10 | 1 | 1 |
| | SA | 3.200.000 | 10 | 10 | 1 | 1 |
| | EC | 4.600.000 | 2.100.000 | 1.400.000 | 200.000 | 1 |
| | CA | 3.000.000 | 220.000 | 62.000 | 8.100 | 1 |
| | AB | 2.500.000 | 240.000 | 220.000 | 150.000 | 19.000 |
| 0,1% 2,3-heptandiol | PA | 4.900.000 | 230.000 | 730.000 | 3.500.000 | 7.000.000 |
| | SA | 5.900.000 | 27.000 | 490 | 1 | 1 |
| | EC | 3.300.000 | 1.100.000 | 1.500.000 | 1.600.000 | 1.400.000 |
| | CA | 1.800.000 | 110.000 | 20.000 | 100 | 1 |
| | AB | 3.700.000 | 1.600.000 | 970.000 | 110.000 | 120.000 |

(continued)

| Substance | | Inoculum | 2 days | 7 days | 14 days | 28 days |
|---|---|---|---|---|---|---|
| 0,4% 2,3-heptandiol | PA | 4.900.000 | 2.900 | 1 | 1 | 1 |
| | SA | 5.900.000 | 3.500 | 1 | 1 | 1 |
| | EC | 3.300.000 | 1.200.000 | 100.000 | 75.000 | 380.000 |
| | CA | 1.800.000 | 81 | 1 | 1 | 1 |
| | AB | 3.700.000 | 2.200.000 | 350.000 | 130.000 | 140.000 |
| 0,1% 2,3-octanediol | PA | 4.900.000 | 280.000 | 3.700.000 | 13.000.000 | 8.100.000 |
| | SA | 5.900.000 | 2.000 | 1 | 1 | 1 |
| | EC | 3.300.000 | 2.600.000 | 3.800.000 | 1.500.000 | 1.300.000 |
| | CA | 1.800.000 | 200.000 | 19.000 | 390 | 1 |
| | AB | 3.700.000 | 2.300.000 | 1.300.000 | 250.000 | 250.000 |
| 0,4% 2,3-octanediol | PA | 4.900.000 | 21.000 | 1 | 1 | 1 |
| | SA | 5.900.000 | 4.000 | 1 | 1 | 1 |
| | EC | 3.300.000 | 1.100.000 | 1.900.000 | 2.100.000 | 600.000 |
| | CA | 1.800.000 | 44.000 | 17.000 | 510 | 1 |
| | AB | 3.700.000 | 1.100.000 | 1.100.000 | 2.100.000 | 410.000 |
| 0,1% 2,3-nonanediol | PS | 4.900.000 | 160.000 | 2.000.000 | 10.000.000 | 8.800.000 |
| | SA | 5.900.000 | 31.000 | 200 | 1 | 1 |
| | EC | 3.300.000 | 2.300.000 | 4.200.000 | 1.800.000 | 1.100.000 |
| | CA | 1.800.000 | 110.000 | 29.000 | 870 | 1 |
| | AB | 3.700.000 | 1.800.000 | 2.000.000 | 680.000 | 530.000 |
| 0,4% 2,3-nonanediol | PA | 4.900.000 | 17.000 | 1 | 1 | 1 |
| | SA | 5.900.000 | 1.800 | 1 | 1 | 1 |
| | EC | 3.300.000 | 3.200.000 | 4.200.000 | 1.300.000 | 1.400.000 |
| | CA | 1.800.000 | 68.000 | 14.000 | 690 | 1 |
| | AB | 3.700.000 | 2.100.000 | 1.800.000 | 1.200.000 | 1.100.000 |

From the above Table 6 it is obvious that 2,3-heptanediol, 2,3-octanediol and 2,3-nonanediol also have an antimicrobial efficacy against different bacteria (represented by *Pseudomonas aeruginosa* (PA), *Staphylococcus aureus* (SA), *Escherichia coli* (EC), yeast (represented by *Candida albicans* (CA) and fungi (represented by *Aspergillus brasiliensis* (AB)) when used in a shampoo formulation.

**Table 7:** Results for the synergistic antimicrobial efficacy of 1,2-nonanediol and 2,3-nonanediol against yeast and fungi/mold in o/w emulsion

| Substance | | Inoculum | 2 days | 7 days | 14 days | 28 days |
|---|---|---|---|---|---|---|
| 0.1% 1,2-Nonanediol | PA | 3.300.000 | 1 | 1 | 1 | 1 |
| | SA | 3.600.000 | 2.300 | 1 | 1 | 1 |
| | EC | 4.300.000 | 6.700 | 1 | 1 | 1 |
| | CA | 1.800.000 | 1.600.000 | 120.000 | 890 | 1 |
| | AB | 4.000.000 | 2.300.000 | 1.300.000 | 1.200.000 | 900.000 |

(continued)

| Substance | | Inoculum | 2 days | 7 days | 14 days | 28 days |
|---|---|---|---|---|---|---|
| 0.1% 2,3-Nonanediol | PA | 3.300.000 | 1 | 1 | 1 | 1 |
| | SA | 3.600.000 | 2.200.000 | 66.000 | 1 | 1 |
| | EC | 4.300.000 | 810.000 | 27.000 | 1.100 | 1 |
| | CA | 1.800.000 | 2.000.000 | 2.500.000 | 1.900.000 | 970.000 |
| | AB | 4.000.000 | 1.200.000 | 1.100.000 | 890.000 | 1.100.000 |
| 0.1% Blend (1,2-C9 + 2,3-C9 95:5) | PA | 3.300.000 | 1 | 1 | 1 | 1 |
| | SA | 3.600.000 | 11.000 | 1 | 1 | 1 |
| | EC | 4.300.000 | 15.000 | 1 | 1 | 1 |
| | CA | 1.800.000 | 1.100.000 | 110.000 | 29.000 | 1 |
| | AB | 4.000.000 | 2.500.000 | 1.900.000 | 840.000 | 740.000 |

From the above Table 7 it is obvious that a blend of 1 ,2-nononanediol and 2,3-nonanediol also has an antimicrobial efficacy against different yeast (represented by *Candida albicans* (CA) and fungi (represented by *Aspergillus brasiliensis* (AB)).

**Table 8:** Results for the synergistic antimicrobial efficacy of 1,2-octanediol and 2,3-octanediol (in wet wipes) against yeast in wet wipes solution

| Substance | | Inoculum | 2 days | 7 days | 14 days | 28 days |
|---|---|---|---|---|---|---|
| 0.1% 1,2-octanediol | EC | 4.000.000 | 1.300.000 | 3.500.000 | 2.100.000 | 2.300 |
| | PA | 3.900.000 | 1.300.000 | 2.500.000 | 750.000 | 350.000 |
| | SA | 6.900.000 | 425 | 50 | 50 | 50 |
| | CA | 4.000.000 | 4.700.000 | 3.900.000 | 5.800.000 | 9.300.000 |
| | AB | 4.700.000 | 22.000.000 | 8.500.000 | 11.000.000 | 12.000.000 |
| 0.1% 2,3-octanediol | EC | 4.000.000 | 1.300.000 | 2.600.000 | 850.000 | 1.600 |
| | PA | 3.900.000 | 1.300.000 | 630.000 | 310.000 | 27.000 |
| | SA | 6.900.000 | 50 | 50 | 50 | 50 |
| | CA | 4.000.000 | 5.000.000 | 2.400.000 | 6.900.000 | 5.500.000 |
| | AB | 4.700.000 | 12.000.000 | 8.500.000 | 9.300.000 | 12.000.000 |
| 0.1% Blend 1,2-octanediol and 2,3-octanediol (90:10) | EC | 4.000.000 | 1.300.000 | 3.400.000 | 1.000.000 | 2.500 |
| | PA | 3.900.000 | 1.300.000 | 400.000 | 800.000 | 50 |
| | SA | 6.900.000 | 12.000 | 50 | 50 | 50 |
| | CA | 4.000.000 | 3.800.000 | 2.000.000 | 230.000 | 750 |
| | AB | 4.700.000 | 8.300.000 | 13.000.000 | 10.000.000 | 12.000.000 |

From the above Table 8 it is obvious that a blend of 1,2-nononanediol and 2,3-nonanediol also has an antimicrobial efficacy against yeast (represented by *Candida albicans* (CA).

**Table 9:** Results of synergistic antimicrobial efficacy of 1,2-octanediol and 2,3-octanediol against fungi in o/w emulsion

| Substance | | Inoculum | 2 days | 7 days | 14 days | 28 days |
|---|---|---|---|---|---|---|
| 0.2% 1,2-octanediol | PA | 3.300.000 | 1 | 1 | 1 | 1 |
| | SA | 3.600.000 | 1 | 1 | 1 | 1 |
| | EC | 4.300.000 | 1 | 1 | 1 | 1 |
| | CA | 1.800.000 | 950.000 | 15.000 | 36 | 1 |
| | AB | 4.000.000 | 2.400.000 | 2.700.000 | 2.100.000 | 1.200.000 |
| 0.2% 2,3-octanediol | PA | 3.300.000 | 1 | 1 | 1 | 1 |
| | SA | 3.600.000 | 1.300.000 | 84.000 | 1 | 1 |
| | EC | 4.300.000 | 48.000 | 290 | 1 | 1 |
| | CA | 1.800.000 | 2.000.000 | 1.800.000 | 1.900.000 | 390.000 |
| | AB | 4.000.000 | 1.700.000 | 2.000.000 | 2.100.000 | 2.000.000 |
| | PA | 3.300.000 | 1 | 1 | 1 | 1 |
| 0.2% Blend (1,2-octanediol and 2,3-octanediol ,95:5) | SA | 3.600.000 | 1 | 1 | 1 | 1 |
| | EC | 4.300.000 | 1 | 1 | 1 | 1 |
| | CA | 1.800.000 | 950.000 | 11.000 | 310 | 1 |
| | AB | 4.000.000 | 2.200.000 | 790.000 | 910.000 | 1.300.000 |

From the above Table 9 it is obvious that a blend of 1,2-octanediol and 2,3-octanediol also has an antimicrobial efficacy against fungi (represented by *Aspergillus brasiliensis* (AB)).

**Table 10:** Results of synergistic antimicrobial efficacy of 1,2-heptanediol and 2,3-heptanediol against fungi in o/w emulsion

| Substance | | Inoculum | 2 days | 7 days | 14 days | 28 days |
|---|---|---|---|---|---|---|
| 0.2% 1,2-heptanediol | PA | 3.300.000 | 1 | 1 | 1 | 1 |
| | SA | 3.600.000 | 95.000 | 1 | 1 | 1 |
| | EC | 4.300.000 | 38.000 | 73 | 1 | 1 |
| | CA | 1.800.000 | 1.300.000 | 1.600.000 | 1.200.000 | 620.000 |
| | AB | 4.000.000 | 2.200.000 | 2.000.000 | 2.500.000 | 2.100.000 |
| 0.2% 2,3-heptanediol | PA | 3.300.000 | 1 | 1 | 1 | 1 |
| | SA | 3.600.000 | 1.300.000 | 790.000 | 55 | 1 |
| | EC | 4.300.000 | 170.000 | 1.600 | 82 | 1 |
| | CA | 1.800.000 | 1.900.000 | 2.200.000 | 2.100.000 | 370.000 |
| | AB | 4.000.000 | 1.600.000 | 1.600.000 | 3.000.000 | 2.000.000 |
| 0.2% Blend (1,2-heptanediol and 2,3-heptanediol , 70:30) | PA | 3.300.000 | 1 | 1 | 1 | 1 |
| | SA | 3.600.000 | 300.000 | 1 | 1 | 1 |
| | EC | 4.300.000 | 75.000 | 54 | 1 | 1 |
| | CA | 1.800.000 | 1.800.000 | 2.000.000 | 1.800.000 | 450.000 |
| | AB | 4.000.000 | 2.900.000 | 1.300.000 | 2.100.000 | 1.700.000 |

From the above Table 10 it is obvious that a blend of 1,2-heptanediol and 2,3-heptanediol also has an antimicrobial efficacy against fungi (represented by *Aspergillus brasiliensis* (AB)).

## B) Malodour coverage

### Example B.1: Malodour coverage of sweat by 1,2-heptanediol

#### Method

Malodour coverage was tested in an established setup with a trained panel (n=8-9). For this sensory test a defined amount of the material was applied on a filter paper together with a defined amount of sweat malodour. For evaluation the panelists were asked to rate the individual samples on a scale from 0 to 10 (0: odorless; 10: strongest imaginable).

A significant malodour reduction was found for 1,2-alkanediols on the tested example 1,2-heptanediol after 6 h. The start malodour value was reduced from 5.6 to 4.8 after 6 h.

**Table 11:** Malodour coverage effect of 1,2-heptanediol

| | Fresh | 6 h |
|---|---|---|
| 1,2-heptanediol | 5.7 | 4.8 |

1,2-heptanediol does not possess a recognizable intrinsic odour. Therefore, the malodour coverage effect of 1,2-heptanediol is not based on an odour which overrules the malodour of sweat. The reason or the mechanism of the malodour coverage effect of the alkanediols according to the present invention is still unknown. However, the malodour coverage effect is remarkably high. Consequently, the alkanediols of the present invention, in particular 1,2-heptanediol, are useful for efficient malodour management, topical applications, and in products for masking insect alluring odours.

**Table 12:** Results of the malodour coverage experiments for the test compositions based on heptanediol (C7) after 6 h and 24 h (Ranking: 1 = highest malodour to 6 = lowest malodour)

| | 6 h | 24 h |
|---|---|---|
| 0.5 % 1,2-C7 | 2.3 | 3.4 |
| 0.5 % 2,3-C7 | 3.2 | 3.5 |
| Mixture of 1,2-C7 and 2,3-C7 in a ratio of 95 : 5 | 3.4 | 4 |
| Mixture of 1,2-C7 and 2,3-C7 in a ratio of 7 : 3 | 3.4 | 4 |

Table 12 shows the results of the malodour coverage experiments for samples being based on heptanediols (C7): The above results show that a combination of 1,2-heptanediol with 2,3-heptanediol in different ratios allows for a pronounced improvement in odour based on an efficient malodour coverage compared to the use of 1,2-heptanediol of 2,3-heptanediol alone. A combination of the isomeric heptanediols results in a synergistic efficiency which is superior to the effects of the single substances. A further combination of said isomeric mixtures with additional cooling agents results in an even more increased synergy. Thereby, already small portions of the 2,3-compound are sufficient in order to achieve a pronounced advantageous effect.

**Table 13:** Results of the malodour coverage experiments for the test compositions based on octanediol (C8) after 6 h and 24 h (Ranking: 1 = highest malodour to 6 = lowest malodour)

| | 24 h |
|---|---|
| 0.5 % 1,2-C8 | 2.8 |
| 0.5 % 2,3-C8 | 3.2 |
| Mixture of 1,2-C8 and 2,3-C8 in a ratio of 95 : 5 | 3.6 |
| Mixture of 1,2-C8 and 2,3-C8 in a ratio of 1 : 1 | 3.7 |
| Mixture of 1,2-C8 and 2,3-C8 in a ratio of 7 : 3 | 4.2 |

As can be seen from the results above the pure alkanediol compounds exhibit an odour with a rate of 2.8 and 3.2 after 24 h. Consequently, corresponding mixtures of both compounds should exhibit an expected odour with a rate

ranging between 2.8 and 3.2 measured after 24 h. However, actually the mixtures of both compounds in different ratios exhibit an odour with a rate ranging between 3.6 and 4.2 after 24 h indicating an advantageous synergy between the isomeric octanediol compounds. Therefore, the mixtures of both isomeric forms, i.e. compositions comprising an 1,2-alkanediol and an 2,3-alkanediol according to the present invention, show a synergistic malodour coverage effect after 24 h. Thereby, already small portions of the 2,3-compound are sufficient in order to achieve a pronounced advantageous effect.

**Table 14:** Results of the malodour coverage experiments for the test compositions based on combinations of heptanediols (C7) and octanediols (C8) after 6 h and 24 h (Ranking: 1 = highest malodour to 6 = lowest malodour).

| | **6 h** | **24 h** |
|---|---|---|
| 0.5 % 1,2-C7 | 3.5 | 3.1 |
| 0.5 % 2,3-C8 | 2.3 | 3.4 |
| Mixture of 1,2-C7 and 2,3-C8 in a ratio of 95 : 5 | 3.8 | 3.8 |
| Mixture of 1,2-C7 and 2,3-C8 in a ratio of 1 : 1 | 3.5 | 4.1 |
| Mixture of 1,2-C7 and 2,3-C8 in a ratio of 7 : 3 | 4.5 | 3.4 |

As can be seen from the results above the pure alkanediol compounds differing in their chain lengths exhibit an odour with a rate of 3.1 and 3.4 after 24 h. Consequently, corresponding mixtures of both compounds should exhibit an expected odour with a rate ranging between 3.2 and 3.4 measured after 24 h. However, actually the mixtures of both compounds in different ratios exhibit an odour with a rate ranging between 3.4 and 4.1 after 24 h indicating an advantageous synergy between the 1,2-heptanediol and the 2,3-octanediol having different chain lengths. Therefore, the mixtures of said different alkanediols in different ratios, i.e. compositions comprising an 1,2-alkanediol and an 2,3-alkanediol differing in their chain lengths (hetero combination) according to the present invention, show a synergistic malodour coverage effect after 24 h. Thereby, already small portions of the 2,3-compound are sufficient in order to achieve a pronounced advantageous effect and to efficiently cover unpleasant malodour.

**Example B.2: Malodour corerage effect on fragrance ingredients**

**Method**

Testing conditions:
The fragrance ingredients (1 % w/w) in aqueous/ethanolic solution (7 : 3) were sprayed on paper stripes and compared with solutions containing alkanediols after 10 min and 25 min, resepctively.

Tested substances:

**Table 15:** Tested alkanediols

| Alkanediol | Abbreviation |
|---|---|
| 1,2-Heptanediol | 1,2-C7 |
| 2,3-Heptanediol | 2,3-C7 |
| 1,2-Octanediol | 1,2-C8 |
| 2,3-Octanediol | 2,3-C8 |

**Table 16:** Tested fragrance blend:

| **Name** | **Structure** | **w/w%** |
|---|---|---|
| Aldehyd C12 NMA | 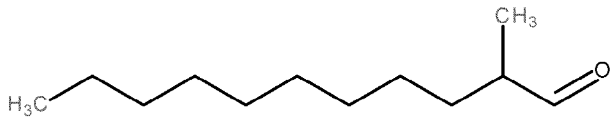 | 25.0 |
| Globalide | 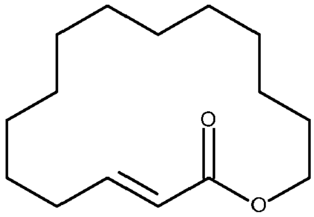 | 25.0 |
| Dihydromyrcenol | 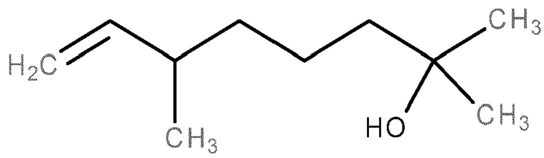 | 25.0 |
| Citronellylacetat | 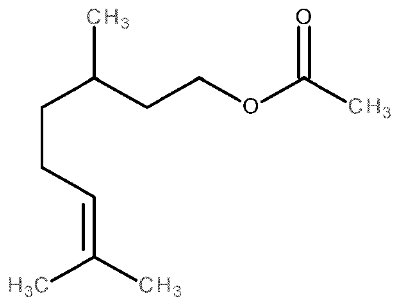 | 25.0 |

Preparation of the fragrance blend ("FRA-blend"):

The fragrance ingredients specified above in Table 13 were mixed by stirring at ambient temperature with a magnetic stirrer for 5 min at 350 rpm.

**Table 17:** Tested ethanolic/aqueous solution (heptanediols and octanediols)

| **Substance** | **w/w%** | | | | | | |
|---|---|---|---|---|---|---|---|
| | Samples 1 and 5 | Sample 2 | Sample 3 | Sample 4 | Sample 6 | Sample 7 | Sample 8 |
| | (placebo) | heptanediol | | | octanediol | | |
| Ethanol pa. | 69.0 | 67.0 | 67.0 | 67.0 | 67.0 | 67.0 | 67.0 |
| Water, dist. | 30.0 | 30.0 | 30.0 | 30.0 | 30.0 | 30.0 | 30.0 |
| FRA-Blend | 1.0 | 1.0 | 1.0 | 1.0 | 1.0 | 1.0 | 1.0 |
| 1,2-C7 | - | 2.0 | - | 1.0 | - | - | - |
| 2,3-C7 | - | - | 2.0 | 1.0 | - | - | - |
| 1,2-C8 | - | - | - | - | 2.0 | - | 1.0 |
| 2,3-C8 | - | - | - | - | - | 2.0 | 1.0 |

Preparation of the fragrance-solutions:

The FRA blend specified above was pre-solved in the alkanediol mixture according to Table 14 and ethanol

was added. The solution was vortexed for 15 seconds. Subsequently, water was added and the resulting solution was vortexed again for further 15 seconds.

For evaluating the sensorial behavior of alkanediols in combination with different odorous compounds, the odor compounds were blended with the alkanediol mixtures and dissolved in a EtOH/water solution and assessed by untrained panelists in comparison to the corresponding samples which were prepared without the addition of alkanediol (placebo). The odor intensity was accorded a corresponding value between 1 and 5 (ranking: 1 = weak odor; 5 = strong odor/high odor intensity)

**Table 18:** Evaluation after 10 minutes (alkanediol: heptanediol)

| **Panelist** | **Sample 1** (placebo) | **Sample 2** (1,2-C7) | **Sample 3** (2,3-C7) | **Sample 4** (1,2-C7 and 2,3-C7 |
|---|---|---|---|---|
| P1 | 5 | 2 | 3 | 2 |
| P2 | 5 | 3 | 2 | 1 |
| P3 | 5 | 2 | 3 | 3 |
| P4 | 5 | 3 | 4 | 3 |
| P5 | 5 | 2 | 3 | 2 |
| Mean value Ø | 5.0 | 2.4 | 3.0 | 2.2 |

**Table 19:** Evaluation after 25 minutes (alkanediol: heptandiol)

| **Panelist** | **Sample 1** (placebo) | **Sample 2** (1,2-C7) | **Sample 3** (2,3-C7) | **Sample 4** (1,2-C7 and 2,3-C7) |
|---|---|---|---|---|
| P1 | 4 | 2 | 2 | 1 |
| P2 | 5 | 3 | 2 | 1 |
| P3 | 5 | 3 | 2 | 2 |
| P4 | 5 | 2 | 4 | 2 |
| P5 | 4 | 2 | 2 | 1 |
| Mean value Ø | 4.6 | 2.4 | 2.4 | 1.4 |

The results of the evaluation are additionally shown in Figure 2a.

**Table 20:** Evaluation after 10 minutes (alkanediol: octanediol)

| **Panelist** | **Sample 5** (placebo) | **Sample 6** (1,2-C8) | **Sample 7** (2,3-C8) | **Sample 8** (1,2-C8 and 2,3-C8) |
|---|---|---|---|---|
| P1 | 5 | 3 | 3 | 2 |
| P2 | 5 | 4 | 4 | 3 |
| P3 | 5 | 5 | 4 | 3 |
| P4 | 5 | 2 | 3 | 3 |
| P5 | 5 | 3 | 3 | 2 |
| Mean value Ø | 5.0 | 3.4 | 3.4 | 2.6 |

**Table 21:** Evaluation after 25 minutes (alkanediol: octanediol)

| Panelist | **Sample 5** (placebo) | **Sample 6** (1,2-C8) | **Sample 7** (2,3-C8) | **Sample 8** (1,2-C8 and 2,3-C8) |
|---|---|---|---|---|
| P1 | 4 | 3 | 2 | 2 |
| P2 | 4 | 2 | 3 | 2 |
| P3 | 4 | 3 | 3 | 2 |
| P4 | 5 | 2 | 3 | 2 |

(continued)

| Panelist | **Sample 5** (placebo) | **Sample 6** (1,2-C8) | **Sample 7** (2,3-C8) | **Sample 8** (1,2-C8 and 2,3-C8) |
|---|---|---|---|---|
| P5 | 4 | 3 | 3 | 2 |
| Mean value Ø | 4.2 | 2.6 | 2.8 | 2.0 |

The results of the evaluation are additionally shown in Figure 2b.

As can be seen from the data of tables 18 to 21 and Figures 2a and 2b there is a synergistic malodour coverage effect for the samples 4 and 8 comprising compositions according to the present invention comprising a mixture of 1,2-alkanediols and 2,3-alkanediols. A clear reduction of the fragrance intensity was observed after 10 min and 25 min, respectively, by incorporating 1,2-alkanediols, 2,3-alkanediols as well as mixtures of the corresponding 1,2-alkanediols and 2,3-alkanediols. In addition, all panelists identified a reduction of a stingy / sharp notes. Especially, Aldehyd C12 NMA has a strong stingy / sharp note, which is usually hard to suppress or to cover. Therefore, the efficient suppression or coverage of the undesired fragrance note of Aldehyd C12 NMA by incorporating 1,2-alkanediols, 2,3-alkanediols as well as mixtures of the corresponding 1,2-alkanediols and 2,3-alkanediols is remarkable. Efficient improvements were found for the use of 1,2-alkanediols or 2,3-alkanediols as such. The particularly remarkable coverage of the aforementioned unpleasant fragrance note was especially efficient for compositions comprising 1,2-heptanediols in combination with 2,3-heptanediol and 1,2-octanediol in combination with 2,3-octanediol. No other effect was found out of pure ethanol as fragrance vehicle, therefore the compositions achieve an excellent malodour coverage effect in different media or media compositions. As can be seen from these results the compositions according to the present invention can be efficiently used for malodour reduction and for body odour control. In particular, compositions comprising 1,2-heptanediol, 2,3-heptanediol, 1,2-octanediol and/or 2,3-octanediol provide excellent effects for malodour reduction and for body odour control.

**Example B.3: Malodour coverage effect on malodour and insect alluring compounds**

**Method**

Testing conditions:

The ingredients (1 % w/w) in an aqueous/ethanolic solution (7 : 3) were sprayed on paper stripes and compared with solutions containing alkanediols directly and after 5 min, respectively.

Tested substances:

**Table 22:** Tested alkanediols

| Alkanediol | Abbreviation |
|---|---|
| 1,2-Heptanediol | 1,2-C7 |
| 2,3-Heptanediol | 2,3-C7 |
| 1,2-Octanediol | 1,2-C8 |
| 2,3-Octanediol | 2,3-C8 |

**Table 23:** Malodour/insect alluring compound which is a typical compound found in human sweat

| Name | Structure |
|---|---|
| 1-Octen-3-ol | 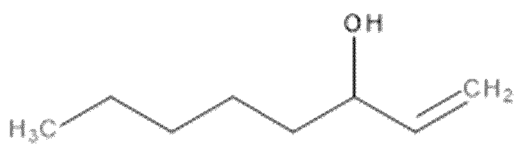 |

**Table 24:** Tested ethanolic/aqueous solution

| Substance | w/w% | | |
|---|---|---|---|
| | **Sample 1** (placebo) | **Sample 2 (heptanediol** | **Sample 3 (octanediol)** |
| Ethanol pa | 69.0 | 67.0 | 67.0 |
| Water, dist. | 30.0 | 30.0 | 30.0 |
| 1-Octen-3-ol | 1.0 | 1.0 | 1.0 |
| 1,2-Heptanediol | - | 1.0 | - |
| 2,3-Heptanedol | - | 1.0 | - |
| 1,2-Octanediol | - | - | 1.0 |
| 2,3-Octanedol | - | - | 1.0 |

Preparation of the fragrance-solutions:

The 1-octen-3ol was pre-solved in the alkanediol mixtures as specified in Table 21, ethanol added and the solutions vortexed for 15 seconds. Subsequently, water was added and the resulting solutions vortexed again for further 15 seconds.

**Table 25:** Evaluation after 0 minutes (1-octen-3-ol)

| **Panelist** | **Sample 1** | **Sample 2** | **Sample 3** |
|---|---|---|---|
| P1 | 5 | 1 | 2 |
| P2 | 5 | 4 | 2 |
| P3 | 4 | 3 | 2 |
| P4 | 5 | 3 | 2 |
| P5 | 5 | 4 | 2 |
| P6 | 4 | 3 | 3 |
| Mean value Ø | 4.7 | 3.0 | 2.2 |

**Table 26:** Evaluation after 5 minutes (1-octen-3-ol)

| **Panelist** | **Sample 1** | **Sample 2** | **Sample 3** |
|---|---|---|---|
| P1 | 4 | 1 | 2 |
| P2 | 4 | 2 | 1 |
| P3 | 3 | 2 | 2 |
| P4 | 4 | 2 | 1 |
| P5 | 4 | 3 | 2 |
| P6 | 4 | 3 | 2 |
| Mean value Ø | 3.8 | 2.2 | 1.7 |

The results of the evaluation are additionally shown in Figure 3.

A clear reduction of compound intensity was observed directly and after 5 min, respectively, by adding the alkanediol mixtures specified in Table 24. All panelists (P1, P2, P3, P4, P5, P6) identified a significant reduction of the unpleasant earthy/rotten note. Therefore, a strong impact on the spot was observed for the reduction of the malodour of 1-octen-3-ol. The excellent suppression of the malodour of 1-octen-3-ol achieved by the compositions according to the present invention demonstrates that the compositions of the present invention are useful for efficiently masking insect

alluring odours and sweat odours. No other effect was found out of pure ethanol as compound vehicle, therefore, the compositions achieve an excellent malodour coverage effect and effect for masking insect alluring odours in different media or media compositions. These results demonstrate that the compositions according to the present invention can be used for pronounced malodour reduction, for body odour control and for masking insect alluring odours.

**Example B.4: Boosting/masking effect of alkanediols on fragrance ingredients**

Testing conditions:

Paper stripes were dunked in the fragrance solution (1 % w/w; aqueous/ethanolic (7 : 3); see Example 5) and compared with solutions containing alkanediols after 10 and 30 minutes, respectively.

For evaluating the sensorial behaviour of the alkanediols in combination with different odorous compounds, the odour compounds were blended with the alkanediol (mixture) and analysed according to the method described in Example B.2 in comparison to the corresponding samples which were prepared without the addition of alkanediol(s) (placebo sample).

The test conditions as well as the FRA-Blend correspond to those used in Example B.2.

Preparation of the fragrance-solutions:

The FRA-Blend specified above (according to Table 13) was pre-solved in the alkanediol (mixture) according to Table 24 and ethanol was added. The solution was stirred for 3 min with a magnetic stirrer. Subsequently, water was added, and the resulting solution stirred again for further 3 min.

For evaluating the sensorial behaviour of alkanediols in combination with different odorous compounds, the odour compounds were blended with the alkanediol (mixture) and dissolved in a EtOH/water-solution and assessed by untrained panelists (P1, P2, P3, P4, P5, P6, P7) in comparison to the corresponding samples which were prepared without the addition of alkanediol(s) (placebo sample). The odour intensity was accorded a corresponding value between 1 and 5 (Ranking: 1 = weak odour; 5 = strong odour/high odour intensity).

**Table 27:** Tested ethanolic / aqueous solutions

| **Substance** | **Sample (w/w%)** | | | |
|---|---|---|---|---|
| | **A** (placebo) | **B** | **C** | **D** |
| Ethanol pa. | 69.0 | 67.0 | 67.0 | 67.0 |
| Water, dist. | 30.0 | 30.0 | 30.0 | 30.0 |
| FRA-Blend | 1.0 | 1.0 | 1.0 | 1.0 |
| 1,2-Heptanediol (1,2-C7) | - | **2.0** | - | **1.0** |
| 2,3-Octanediol (2,3-C8) | - | - | **2.0** | **1.0** |

The odour intensity was accorded a corresponding value between 1 and 5 (Ranking: 1 = weak odour; 5 = strong odour/high odour intensity).

**Table 28:** Evaluation after 10 and 30 minutes, respectively.

| **Panelist** | **Samples after 10 min** | | | | **Samples after 30 min** | | | |
|---|---|---|---|---|---|---|---|---|
| | Placebo | 1,2-C7 | 2,3-C8 | 1,2-C7 and 2,3-C8 (1:1) | Placebo | 1,2-C7 | 2,3-C8 | 1,2-C7 and 2,3-C8 (1:1) |
| | **A** | **B** | **C** | **D** | **A** | **B** | **C** | **D** |
| | | | | | | | | |
| P1 | 5 | 2 | 4 | 3 | 5 | 4 | 3 | 4 |
| P2 | 5 | 2 | 3 | 3 | 5 | 2 | 3 | 2 |
| P3 | 5 | 3 | 4 | 3 | 5 | 3 | 4 | 3 |
| P4 | 5 | 2 | 3 | 2 | 4 | 2 | 3 | 2 |

(continued)

| Panelist | Samples after 10 min | | | | Samples after 30 min | | | |
|---|---|---|---|---|---|---|---|---|
| | Placebo | 1,2-C7 | 2,3-C8 | 1,2-C7 and 2,3-C8 (1:1) | Placebo | 1,2-C7 | 2,3-C8 | 1,2-C7 and 2,3-C8 (1:1) |
| | **A** | **B** | **C** | **D** | **A** | **B** | **C** | **D** |
| | | | | | | | | |
| P5 | 5 | 3 | 3 | 4 | 4 | 2 | 2 | 3 |
| P6 | 5 | 3 | 3 | 2 | 4 | 2 | 3 | 2 |
| P7 | 5 | 2 | 3 | 5 | 5 | 2 | 3 | 4 |
| Mean value Ø | 5.0 | 2.4 | 3.3 | 3.1 | 4.6 | 2.4 | 3.1 | 2.6 |

**Table 29:** Evaluation after 10 and 30 minutes, respectively - sorted results based on masking effect

| **10 min** | | | | | | | |
|---|---|---|---|---|---|---|---|
| Sample | Placebo **(A)** | > | 2,3-C8 **(C)** | > | Blend of 1,2-C7 and 2,3-C8 (1 : 1) **(D)** | > | 1,2-C7 **(B)** |
| Fragrance intensity | 5.0 | | 3.3 | | 3.1 | | 2.4 |
| **30 min** | | | | | | | |
| Sample | Placebo **(A)** | > | 2,3-C8 **(C)** | > | Blend of 1,2-C7 and 2,3-C8 (1 : 1) **(D)** | > | 1,2-C7 **(B)** |
| Fragrance intensity | 4.6 | | 3.1 | | 2.6 | | 2.4 |

The results of the evaluation are additionally shown in Figures 26a and 26b, respectively.

Based on the above results it can be concluded that both the alkanediols as such as well as mixtures of the 1,2-alkanediols with 2,3-alkanediols efficiently mask the fragrance blend. Thereby, 1,2-heptanediol masks the fragrance the most, followed by the mixture of the two diols differing in their chain lengths (hetero combination). 2,3-Octandiol alone shows the lowest masking effect in the present trial.

**C) Sebum Control**

**Example C.1: Sebum control**

Additional tests were performed with human sebaceous glands in order to verify the modulatory activity of different 1 ,2-alkanediols and 2,3-alkanediols on sebum secretion. Several microbial species on the skin are lipid dependent and feed on the human sebum (e.g. Corynebacterium, Propionibacterium). Accordingly, sebum reduction can indirectly support the reduction of microorganisms on the skin and in this way also indirectly support e.g. dandruff or acne reduction.

For these experiments sebaceous glands had been micro-dissected and cultured with medium. The medium without any supplement served as untreated control. Medium with 5 μM Capsaicin served as positive control. Additionally, culture medium was supplemented with different concentrations of alkanediols. After six days the sebaceous glands were collected and lipids and proteins quantified. The total lipid amount being representative of sebum production was obtained by normalizing the quantified lipids upon the quantified proteins (i.e. mg of lipids/mg of proteins).

The results are shown in Figure 4.

As shown in the Figure 4, all tested 1,2-alkandediols showed sebum reduction compared to an untreated control. This demonstrates the sebum reduction activity of 1,2-alkanediols.

Even a stronger sebum reducing effect is achieved for 2,3-alkanediols, in particular 2,3-heptanediol, 2,3-nonanediol and 2,3-undecanediol: 1,2-heptanediol shows a sebum reduction of 22 % whereas 2,3-heptanediol shows a

sebum reduction of 30 %; 1 ,2-nonanediol reduces sebum secretion by 28 % whereas the 2,3-nonanediol reduces sebum secretion by 41 %. The best sebum reduction was observed for 1,2-undecanediol with 44 %, whereas the 2,3-undecanediol leads to a sebum reduction of more than 50 %. Hence, the 2,3-alkanediol substances have a distinguished effect on sebum reduction compared to the corresponding 1,2-alkanediol substances. Nevertheless, no linear correlation between molecule length and activity was found. The best performance of the 1,2-alkanediols had 1,2-undecanediol and the best performance of the 2,3-alkanediols had 2,3-nonanediol and 2,3-undecanediol.

All results shown in this figure resulted from a single experimental run with human sebaceous glands derived from the same human donor.

For 1,2-nonanediol additionally the concentration dependency was investigated and experimentally confirmed.

The results are shown in Figure 5.

All results shown in this figure resulted from a single experimental run with human sebaceous glands derived from the same human donor being different from the experimental results shown above.

Based on these results, it is assumed that 1,2-alkanediols, especially 1,2-undecanediol and 2,3-alkanediols, especially 2,3-nonanediol and 2,3-undecanediol, have microbiota balancing or reducing properties indirectly via sebum reduction.

Consequently, the compositions of the present invention can be used for sebum control, for malodour management and for topical applications.

**D) Antioxidative enhancing effect**

To evaluate the influence of 1,2-alkanediols and/or of 2,3-alkanediols on the antioxidative capacity of antioxidants, two different tests were performed:

(1) Oxipres treatment to simulate product protection; and
(2) Evaluation of reactive oxygen species (ROS) for skin protection.

The following tests were performed with sunflower oil +/- antioxidant and +/alkanediols. Sunflower oil was used because it is primarily composed of less stable polyunsaturated and monounsaturated fatty acids. Treating it with heat and oxygen triggers and accelerates oxidation easily.

**Example D.1: Oxipres test to simulate product protection**

An Oxipres test was performed in order to evaluate the antioxidative capacity of different test samples as described below.

Test samples:

| | |
|---|---|
| Sample 1: | Sunflower oil without additives |
| Sample 2: | Sunflower oil plus 0.1 % tocopherol |
| Sample 3: | Sunflower oil plus 0.1 % tocopherol plus 0.5 % 1,2-heptanediol |
| Sample 4: | Sunflower oil plus 0.1 % tocopherol plus 0.5 % of an alkanediol blend of 95 % 1,2-heptanediol plus 0.5 % 2,3 heptanediol |
| Sample 5: | Sunflower oil plus 0.5 % 1,2-heptanediol |
| Sample 6: | Sunflower oil plus 0.5 % 2,3-heptanediol |
| Sample 7: | Sunflower oil plus 0.5 % 1,2-nonanediol |
| Sample 8: | Sunflower oil plus 0.1 % tocopherol plus 0.5 % 1,2-nonanediol |
| Sample 9: | Sunflower oil plus 0.1 % 1,2-decanediol |
| Sample 10: | Sunflower oil plus 0.1% tocopherol plus 0.1 % 1,2-decanediol |
| Sample 11: | Sunflower oil plus 0.5 % 2,3-octanediol |
| Sample 12: | Sunflower oil plus 0.1 % tocopherol plus 0.5 % 2,3-octanediol |
| Sample 13: | Sunflower oil without additives |
| Sample 14: | Sunflower oil plus 0.1% tocopherol |
| Sample 15: | Sunflower oil plus 0.1% tocopherol plus 0.5% 1,2-heptanediol |
| Sample 16: | Sunflower oil plus 0.1% tocopherol plus 0.5% 2,3-heptanediol |

(continued)

| | |
|---|---|
| Sample 17: | Sunflower oil plus 0.1% tocopherol plus 0.5% blend of 1,2-heptanediol and 2,3-heptanediol; ratio 98:2 |
| Sample 18: | Sunflower oil plus 0.1% tocopherol plus 0.5% blend of 1,2-heptanediol and 2,3-heptanediol; ratio 99:1 |
| Sample 19: | Sunflower oil without additives |
| Sample 20: | Sunflower oil plus 0.1% tocopherol |
| Sample 21: | Sunflower oil plus 0.1% tocopherol plus 0.5% 1,2-hexanediol |
| Sample 22: | Sunflower oil plus 0.1% tocopherol plus 0.5% 2,3-hexanediol |
| Sample 23: | Sunflower oil plus 0.1% tocopherol plus 0.5% blend of 1,2-hexanediol and 2,3-hexanediol; ratio 95:5 |
| Sample 24: | Sunflower oil plus 0.1% tocopherol plus 0.5% blend of 1,2-hexanediol and 2,3-hexanediol; ratio 50:50 |
| Sample 25: | Sunflower oil plus 0.1% tocopherol plus 0.5% 1,2-octanediol |
| Sample 26: | Sunflower oil plus 0.1% tocopherol plus 0.5% 2,3-octanediol |
| Sample 27: | Sunflower oil plus 0.1% tocopherol plus 0.5% blend of 1,2-octanediol and 2,3-octanediol; ratio 50:50 |
| Sample 28: | Sunflower oil plus 0.1% tocopherol plus 0.5% 1,2-decanediol |
| Sample 29: | Sunflower oil plus 0.1% tocopherol plus 0.5% 2,3-decanediol |
| Sample 30: | Sunflower oil plus 0.1% tocopherol plus 0.5% blend of 1,2-decanediol and 2,3-decanediol; ratio 95:5 |
| Sample 31: | Sunflower oil plus 0.1% tocopherol plus 0.5% blend of 1,2-decanediol and 2,3-decanediol; ratio 50:50 |
| Sample 32: | Sunflower oil without additives |
| Sample 33: | Sunflower oil plus 0.1% tocopherol |
| Sample 34: | Sunflower oil plus 0.1% tocopherol plus 0.5% 1,2-heptanediol |
| Sample 35: | Sunflower oil plus 0.1% tocopherol plus 0.5% 2,3-hexanediol |
| Sample 36: | Sunflower oil plus 0.1% tocopherol plus 0.5% blend of 1,2-heptanediol and 2,3-hexanediol; ratio 50:50 |
| Sample 37: | Sunflower oil plus 0.1% tocopherol plus 0.5% 2,3-octanediol |
| Sample 38: | Sunflower oil plus 0.1% tocopherol plus 0.5% blend of 1,2-heptanediol and 2,3-octanediol; ratio 95:5 |
| Sample 39: | Sunflower oil without additives |
| Sample 40: | Sunflower oil plus 0.1% tocopherol |
| Sample 41: | Sunflower oil plus 0.1% tocopherol plus 0.5% 1,2-nonanediol |
| Sample 42: | Sunflower oil plus 0.1% tocopherol plus 0.5% 2,3-nonanediol |
| Sample 43: | Sunflower oil plus 0.1% tocopherol plus 0.5% blend of 1,2-nonanediol and 2,3-nonanediol; ratio 50:50 |
| Sample 44: | Sunflower oil without additives |
| Sample 45: | Sunflower oil plus 2.0% Symdecanox HA* |
| Sample 46: | Sunflower oil plus 2.0% Symdecanox HA plus 0.5% 1,2-heptanediol |
| Sample 47: | Sunflower oil plus 2.0% Symdecanox HA plus 0.5% 1,2-heptanediol |
| Sample 48: | Sunflower oil plus 2.0% Symdecanox HA plus 0.5% blend of 1,2-heptanediol and 2,3- heptanediol; ratio 95:5 |
| Sample 49: | Sunflower oil plus 2.0% Symdecanox HA plus 0.5% 1,2-octanediol |
| Sample 50: | Sunflower oil plus 2.0% Symdecanox HA plus 0.5% 2,3-octanediol |
| Sample 51: | Sunflower oil plus 2.0% Symdecanox HA plus 0.5% blend of 1,2-octanediol and 2,3-octanediol; ratio 95:5 |
| Sample 52: | Sunflower oil without additives |
| Sample 53: | Sunflower oil plus 0.1% Pentaerythrityl Tetra-di-t-butyl Hydroxyhydrocinnamate (Tinogard Sample TT) |
| Sample 54: | Sunflower oil plus 0.1% Pentaerythrityl Tetra-di-t-butyl |

(continued)

| | |
|---|---|
| | Hydroxyhydrocinnamate (Tinogard TT) plus 0.5% 1,2-heptandiol |
| Sample 55: | Sunflower oil plus 0.1% Pentaerythrityl Tetra-di-t-butyl Hydroxyhydrocinnamate (Tinogard TT) plus 0.5% 2,3-heptanediol |
| Sample 56: | Sunflower oil plus 0.1% Pentaerythrityl Tetra-di-t-butyl Hydroxyhydrocinnamate (Tinogard TT) plus 0.5% blend of 1,2-octanediol and 2,3-octanediol; ratio 95:5 |
| Sample 57: | Sunflower oil without additives |
| Sample 58: | Sunflower oil plus 0.5 % Hydroxyacetophenone |
| Sample 59: | Sunflower oil plus 0.5 % Hydroxyacetophenone plus 0.5% 1,2-heptanediol |
| Sample 60: | Sunflower oil plus 0.5 % Hydroxyacetophenone plus 0.5% 2,3-heptanediol |
| Sample 61: | Sunflower oil without additives |
| Sample 62: | Sunflower oil plus 0.05% Ascorbyl Palmitate |
| Sample 63: | Sunflower oil plus 0.05% Ascorbyl Palmitate plus 0.5% 1,2-heptanediol |
| Sample 64: | Sunflower oil plus 0.05% Ascorbyl Palmitate plus 0.5% 2,3-heptanediol |

* INCI Symdecanox HA: Caprylic Capric Triglyceride, Hydroxymethoxyphenyl Decanonenone

Test procedure: For the evaluation of the antioxidative capacity of the above-described samples, an Oxipres test was conducted. The Oxipres method is bases on oxygen consumption at high temperatures and pressures and allows the determination of oxidative resistance (shelf life) of e.g. oils. The test runs under elevated pressure and temperature, where the process is accelerated. In the Oxipres test the sample is placed inside a hermetically closed iron vessel that is subjected to high oxygen pressures and temperatures of 90 to 120 °C. The above-described samples were treated in Oxipres device for 48 hours at 80 °C and 5 bar pressure and determination of induction period. The consumption of oxygen results in a pressure drop in the vessel during the test. Higher decrease of pressure indicates more consumption of oxygen and higher oxidation of the test product. Oils with a high degree of unsaturation are most susceptible to autoxidation.

The induction period is the period during which a fat or oil shows stability against oxidation because of its content of antioxidants, either naturally or added. In the test, the antioxidants are oxidized preferentially before the oxidizable fat or oil is oxidized. Thus, the antioxidants protect the fat or oil against oxidation. After this there is a sudden and large consumption of oxygen and the fat becomes rancid. This can be defined by the induction period (IP) in hours. A lower IP correlates with a faster oxidation.

Equipment: The tests were performed with an Oxipres device ex Mikrolab. The instrument is a modification of the bomb method (ASTM D941), which is based on oxidation with oxygen.

Before and after Oxipres treatment additionally an odor evaluation of the samples was performed and the acid value of the samples before and after Oxipres treatment was determined. The principle of acid value determination: Neutralization of the free acids by titration with ethanolic or aqueous solution of potassium hydroxide. It shows the number of milligram (mg) KOH required in order to neutralize the free acids in 1 g test substance. The titration according to the IFU respectively § 64 LFGB (formerly § 35 LMBG) is carried out potentiometrically with potassium hydroxide solution to a pH value of 8.1.

The following table gives an overview of the samples, the antioxidants, the alkanediols and the resulting induction period (IP) value.

**Table 30: Overview Antioxidants, alkanediols and Induction Period (IP)**

| Sample, No. | Antioxidant | Alkanediol | IP |
|---|---|---|---|
| **1** | **No** | **no** | **22.6** |
| 2 | 0.1% tocopherol | no | 29.5 |
| 3 | 0.1% tocopherol | 0.5% 1,2-heptanediol | 31.2 |
| 4 | 0.1% tocopherol | 0.5% (blend of 1,2-heptanediol and 2,3-heptanediol; ratio 95:5) | 31.7 |
| 5 | No | 0.5% 1,2-heptanediol | 23.6 |
| 6 | No | 0.5% 2,3-heptanediol | 24.3 |

(continued)

| Sample, No. | Antioxidant | Alkanediol | IP |
|---|---|---|---|
| 7 | No | 0.5% 1,2-nonanediol | 22.7 |
| 8 | 0.1% tocopherol | 0.5% 1,2-nonanediol | 32.4 |
| 9 | No | 0.5% 1,2-decanediol | 25 |
| 10 | 0.1% tocopherol | 0.1% 1,2-decanediol | 30 |
| 11 | No | 0.5% 2,3-decanediol | 19.8 |
| 12 | 0.1% tocopherol | 0.5% 2,3-octanediol | 32.3 |
| | | | |
| **13** | **no** | **no** | **25.4** |
| 14 | 0.1% tocopherol | no | 31.6 |
| 15 | 0.1% tocopherol | 0.5% 1,2-heptanediol | 31.0 |
| 16 | 0.1% tocopherol | 0.5% 2,3-heptanediol | 30.9 |
| 17 | 0.1% tocopherol | 0.5% (blend of 1,2-heptanediol and 2,3-heptanediol; ratio 98:2) | 32.0 |
| 18 | 0.1% tocopherol | 0.5% (blend of 1,2-heptanediol and 2,3-heptanediol; ratio 99:1) | 32.0 |
| 19 | no | no | 24.5 |
| 20 | 0.1% tocopherol | no | 31.2 |
| 21 | 0.1% tocopherol | 0.5% 1,2-hexanediol | 30.3 |
| 22 | 0.1% tocopherol | 0.5% 2,3-hexanediol | 31.6 |
| 23 | 0,1% tocopherol | 0.5% (blend of 1,2-hexanediol and 2,3-hexanediol; ratio 95:5) | 31.2 |
| 24 | 0.1% tocopherol | 0.5% (blend of 1,2-hexanediol and 2,3-hexanediol; ratio 50:50) | 32.8 |
| 25 | 0.1% tocopherol | 0.5% 1,2-octanediol | 32.2 |
| 26 | 0.1% tocopherol | 0.5% 2,3-octanediol | 31.7 |
| 27 | 0.1% tocopherol | 0.5% (blend of 1,2-octanediol and 2,3-octanediol; ratio 50:50) | 32.9 |
| 28 | 0.1% tocopherol | 0.5% 1,2-decanediol | 31.7 |
| 29 | 0.1% tocopherol | 0.5% 2,3-decanediol | 31.8 |
| 30 | 0.1% tocopherol | 0.5% (blend of 1,2-decanediol and 2,3-decanediol; ratio 95:5) | 31.9 |
| 31 | 0.1% tocopherol | 0.5% (blend of 1,2-decanediol and 2,3-decanediol; ratio 50:50) | 32.4 |
| | | | |
| **32** | **no** | **no** | **25.9** |

(continued)

| Sample, No. | Antioxidant | Alkanediol | IP |
|---|---|---|---|
| 33 | 0.1% tocopherol | no | 31.6 |
| 34 | 0.1% tocopherol | 0.5% 1,2-heptanediol | 32.3 |
| 35 | 0.1% tocopherol | 0.5% 2,3-hexanediol | 32.9 |
| 36 | 0.1% tocopherol | 0.5% (blend of 1,2-heptanediol and 2,3-hexanediol; ratio 50:50) | 33.2 |
| 37 | 0.1% tocopherol | 0.5% 2,3-octanediol | 31.2 |
| 38 | 0.1% tocopherol | 0.5% (blend of 1,2-heptanediol and 2,3-octanediol; ratio 95:5) | 32.2 |
| | | | |
| **39** | **no** | **no** | **16.2** |
| 40 | 0.1% tocopherol | no | 23.5 |
| 41 | 0.1% tocopherol | 0.5% 1,2-nonanediol | 24.1 |
| 42 | 0.1% tocopherol | 0.5% 2,3-nonanediol | 25.4 |
| 43 | 0.1% tocopherol | 0.5% (blend of 1,2-nonanediol and 2,3-nonanediol; ratio 50: 50) | 25.7 |
| | | | |
| **44** | **no** | **no** | **23.1** |
| 45 | 2% Symdecanox HA | no | 24.4 |
| 46 | 2% Symdecanox HA | 0.5% 1,2-heptanediol | 24.4 |
| 47 | 2% Symdecanox HA | 0.5% 1,2-heptanediol | 24.7 |
| 48 | 2% Symdecanox HA | 0.5% (blend of 1,2-heptanediol and 2,3-heptanediol; ratio 95:5) | 25.3 |
| 49 | 2% Symdecanox HA | 0.5% 1,2-octanediol | 23.9 |
| 50 | 2% Symdecanox HA | 0.5% 2,3-octanediol | 24.0 |
| 51 | 2% Symdecanox HA | 0.5% (blend of 1,2-octanediol and 2,3-octanediol; ratio 95:5) | 25.1 |
| | | | |
| **52** | **no** | **No** | **21.9** |
| 53 | 0.01% Pentaerythrityl Tetra-di-t-butyl Hydroxyhydrocinnamate (Tinoqard TT) | No | 25.8 |
| 54 | 0.01% Pentaerythrityl Tetra-di-t-butyl Hydroxyhydrocinnamate (Tinoqard TT) | 0.5% 1,2-heptandiol | 27.7 |
| 55 | 0.01% Pentaerythrityl Tetra-di-t-butyl Hydroxyhydrocinnamate (Tinogard TT) | 0.5%2,3-heptanediol | 27.6 |
| 56 | 0.01% Pentaerythrityl Tetra-di-t-butyl Hydroxyhydrocinnamate (Tinogard TT) | 0.5% (blend of 1,2-octanediol and 2,3-octanediol; ratio 95:5) | 28.1 |
| | | | |
| **57** | **no** | **no** | **22.9** |

(continued)

| Sample, No. | Antioxidant | Alkanediol | IP |
|---|---|---|---|
| 58 | 0.5% Hydroxyacetophenone | no | 23.2 |
| 59 | 0.5 % Hydroxyacetophenone | 0.5% 1,2-heptanediol | 24.4 |
| 60 | 0.5 % Hydroxyacetophenone | 0.5% 2,3-heptanediol | 24.3 |
| 61 | no | no | 23.5 |
| 62 | 0.05% Ascorbyl Palmitate | no | 25.5 |
| 63 | 0.05% Ascorbyl Palmitate | 0.5% 1,2-heptanediol | 26.7 |
| 64 | 0.05% Ascorbyl Palmitate | 0.5% 2,3-heptanediol | 26.6 |

**Table 31:** Results of induction period (IP) and acid value after Oxipres treatment

| Sample No. | Induction period (h) | Acid value (mg KOH/g) start | Acid value (mg KOH/g) after treatment |
|---|---|---|---|
| 1 | 22.6 | 0.2 | 8.5 |
| 2 | 29.5 | 0.2 | 6.1 |
| 3 | 31.2 | 0.1 | 5.1 |
| 4 | 31.7 | 0.1 | 5.2 |
| 5 | 23.6 | 0.2 | 8.2 |
| 6 | 24.3 | 0.1 | 8.7 |
| 7 | 22.7 | 0 | 7.5 |
| 8 | 32.4 | 0 | 5.6 |
| 9 | 25.0 | 0 | 8.6 |
| 10 | 30.0 | 0 | 6.6 |
| 11 | 19.8 | 0 | 8.0 |
| 12 | 32.3 | 0 | 3.9 |

The results in Table 31 clearly show that sample 3 (sunflower oil plus 0.1 % tocopherol plus 0.5 % 1,2-heptanediol), sample 4 (sunflower oil plus 0.1 % tocopherol plus 0.5 % of an alkanediol blend of 95 % 1,2-heptanediol and 0.5 % 2,3 heptanediol), sample 8 (sunflower oil plus 0.1 % tocopherol plus 0.5 % 1,2-nonanediol), sample 10 (sunflower oil plus 0.1% tocopherol plus 0.1 % 1,2-decanediol) and sample 12 (sunflower oil plus 0.1 % tocopherol plus 0.5 % 2,3-octanediol), i.e., samples containing an antioxidant in combination with an 1,2- alkanediol and/or an 2,3-alkanediol or a mixture thereof have prolonged induction periods (indicating longer shelf life). This means that the samples are more stable against oxidative degradation than the comparative samples. The samples with 1,2-alkanediol or 2,3-alkanediol alone have no antioxidative effect.

The above advantageous results are confirmed by a lower acid value. The acid value is defined as the number of milligrams of potassium hydroxide required to neutralize the free fatty acids present in one gram of fat. It is a relative measure of rancidity as free fatty acids are normally formed during decomposition of triglycerides. Hence, the acid value correlates with the rancidity degree in an oil. In comparison, comparative sample 1 (sunflower oil without antioxidant or alkanediol, comparative sample 2 (sunflower oil with tocopherol) and comparative samples 5 and 6 (sunflower oil with alkanediols without tocopherol) had a shorter induction period (IP) (indicating shorter shelf life), which means that oxidation degradation starts earlier in comparison to the samples containing an antioxidant plus an alkanediol. The comparative samples showed also higher acid vales.

In addition, for the above samples delta values of IP points were calculated versus the corresponding IP points of sunflower oil without additives as indicated in Table 28. The results of the ROS tests are summarized in the following tables:

**Table 32:**

| Sample No. | Heptanediol | IP value (h) | delta IP versus sunflower oil without additive |
|---|---|---|---|
| 1 | sunflower oil without additive | **22.6** | |
| 2 | plus 0.1% tocopherol | 29.5 | 6.9 |
| 3 | plus 0.1% tocopherol + 0.5% 1,2-heptanediol | 31.2 | 8.6 |
| 4 | plus 0.1% tocopherol plus 0.5% blend of 1,2-heptanediol and 2,3-heptanediol (ratio 95:5) | 31.7 | 9.1 |

The results are shown in Figure 8. As can be seen from Figure 8, both sample 3 and sample 4 have prolonged induction periods (indicating longer shelf life). This means that these samples are more stable against oxidative degradation than the comparative samples.

**Table 33:**

| Sample No. | Heptanediol | IP value (h) | delta IP vs sunflower oil wo additive |
|---|---|---|---|
| 13 | sunflower oil without additive | **25.4** | |
| 14 | plus 0.1% tocopherol | 31.6 | 6.2 |
| 15 | plus 0.1% tocopherol plus 0.5% 1,2-heptanediol | 31 | 5.6 |
| 16 | plus 0.1% tocopherol plus 0.5% 2,3-heptanediol | 30.9 | 5.5 |
| 17 | plus 0.1% tocopherol plus 0.5% blend of 1,2- and 2,3-heptanediol (ratio 98:2) | 32 | 6.6 |

The results are shown in Figure 9. As can be seen from Figure 9, a blend of 1,2-heptanediol and 2,3-heptanediol (98 : 2) results in prolonged induction periods (indicating longer shelf life). This means that the sample is more stable against oxidative degradation than the comparative samples.

**Table 34:**

| Sample No. | Heptanediol | IP value (h) | delta IP versus sunflower oil without additive |
|---|---|---|---|
| 13 | sunflower oil without additive | **25.4** | |
| 14 | plus 0.1% tocopherol | 31.6 | 6.2 |
| 15 | plus 0.1% tocopherol plus 0.5% 1,2-heptanediol | 31 | 5.6 |
| 16 | plus 0.1% tocopherol plus 0.5% 2,3-heptanediol | 30.9 | 5.5 |
| 18 | plus 0.1% tocopherol plus 0.5% blend of 1,2- and 2,3-heptanediol (ratio 99:1) | 32 | 6.6 |

The results are shown in Figure 10. As can be seen from Figure 10, a blend of 1,2-heptanediol and 2,3-heptanediol (99 : 1) results in prolonged induction periods (indicating longer shelf life). This means that the sample is more stable against oxidative degradation than the comparative samples.

**Table 35:**

| Sample No. | Hexanediol | IP value (h) | delta IP versus sunflower oil without additive |
|---|---|---|---|
| 19 | sunflower oil without additive | **24.5** | |
| 20 | plus 0.1% tocopherol | 31.2 | 6.7 |
| 21 | plus 0.1% tocopherol plus 0.5% 1,2-hexanediol | 30.3 | 5.8 |

(continued)

| Sample No. | Hexanediol | IP value (h) | delta IP versus sunflower oil without additive |
|---|---|---|---|
| 22 | plus 0.1% tocopherol plus 0.5% 2,3-hexanediol | 31.6 | 7.1 |
| 23 | plus 0.1% tocopherol plus 0.5% blend of 1,2- and 2,3-hexanediol (ratio 95:5) | 31.2 | 6.7 |

The results are shown in Figure 11. As can be seen from Figure 11, 2,3-hexanediol results in prolonged induction periods (indicating longer shelf life). This means that the sample is more stable against oxidative degradation than the comparative sample.

**Table 36:**

| Sample No. | Hexanediol | IP value (h) | delta IP versus sunflower oil without additive |
|---|---|---|---|
| 19 | sunflower oil without additive | **24.5** | |
| 20 | plus 0.1% tocopherol | 31.2 | 6.7 |
| 21 | plus 0.1% tocopherol plus 0.5% 1,2-hexanediol | 30.3 | 5.8 |
| 22 | plus 0.1% tocopherol plus 0.5% 2,3-hexanediol | 31.6 | 7.1 |
| 24 | plus 0.1% tocopherol plus 0.5% blend of 1,2- and 2,3-hexanediol (ratio 50:50) | 32.8 | 8.3 |

The results are shown in Figure 12. As can be seen from Figure 12, both sample 22 and sample 24 results in prolonged induction periods (indicating longer shelf life). This means that the sample is more stable against oxidative degradation than the comparative samples.

**Table 37:**

| Sample No. | Octanediol | IP value (h) | delta IP versus sunflower oil without additive |
|---|---|---|---|
| 19 | sunflower oil without additive | **24.5** | |
| 20 | plus 0.1% tocopherol | 31.2 | 6.7 |
| 25 | plus 0.1% tocopherol plus 0.5% 1,2-octanediol | 32.2 | 7.7 |
| 26 | plus 0.1% tocopherol plus 0.5% 2,3-octanediol | 31.7 | 7.2 |
| 27 | plus 0.1% tocopherol plus 0.5% blend of 1,2-octanediol and 2,3-octanediol (ratio 50:50) | 32.9 | 8.4 |

The results are shown in Figure 13. As can be seen from Figure 13, a blend of 1,2-octanediol and 2,3-octanediol (50 : 50) results in prolonged induction periods (indicating longer shelf life). This means that the sample is more stable against oxidative degradation than the comparative samples.

**Table 38:**

| Sample No. | Decanediol | IP value (h) | delta IP versus sunflower oil without additive |
|---|---|---|---|
| 19 | sunflower oil without additive | **24.5** | |
| 20 | plus 0.1% tocopherol | 31.2 | 6.7 |
| 28 | plus 0.1% tocopherol plus 0.5% 1,2-decanediol | 31.7 | 7.2 |
| 29 | plus 0.1% tocopherol plus 0.5% 2,3-decanediol | 31.8 | 7.3 |

(continued)

| Sample No. | Decanediol | IP value (h) | delta IP versus sunflower oil without additive |
|---|---|---|---|
| 30 | plus 0.1% tocopherol plus 0.5% blend of 1,2- decanediol and 2,3-decanediol (ratio 95:5) | 31.9 | 7.4 |

The results are shown in Figure 14. As can be seen from Figure 14, a blend of 1,2-decanediol and 2,3-decanediol (95 : 5) results in prolonged induction periods (indicating longer shelf life). This means that the sample is more stable against oxidative degradation than the comparative samples.

**Table 39:**

| Sample No. | Decanediol | IP value (h) | delta IP versus sunflower oil without additive |
|---|---|---|---|
| 19 | sunflower oil without additive | 24.5 | |
| 20 | plus 0.1% tocopherol | 31.2 | 6.7 |
| 28 | plus 0.1% tocopherol plus 0.5% 1,2-decanediol | 31.7 | 7.2 |
| 29 | plus 0.1% tocopherol plus 0.5% 2,3-decanediol | 31.8 | 7.3 |
| 31 | plus 0.1% tocopherol plus 0.5% blend of 1,2-decanediol and 2,3-decanediol (ratio 50:50) | 32.4 | 7.9 |

The results are shown in Figure 15. As can be seen from Figure 15, a blend of 1,2-decanediol and 2,3-decanediol (50 : 50) results in prolonged induction periods (indicating longer shelf life). This means that the sample is more stable against oxidative degradation than the comparative samples. The induction period is even higher compared to a blend of 1,2-decanediol and 2,3-decanediol (95 : 5).

**Table 40:**

| Sample No. | Heptanediol / Hexanediol | IP value (h) | delta IP versus sunflower oil without additive |
|---|---|---|---|
| 32 | sunflower oil without additive | **25.9** | |
| 33 | plus 0.1% tocopherol | 31.6 | 5.7 |
| 34 | plus 0.1% tocopherol + 0.5% 1,2-heptanediol | 32.3 | 6.9 |
| 35 | plus 0.1% tocopherol plus 0.5% 2,3-hexanediol | 32.9 | 7.5 |
| 36 | plus 0.1% tocopherol plus 0.5% blend of 1,2-heptanediol and 2,3-hexanediol (ratio 50:50) | 33.2 | 7.8 |

The results are shown in Figure 16. As can be seen from Figure 16, a blend of 1,2-heptanediol and 2,3-hexanediol (50 : 50) results in prolonged induction periods (indicating longer shelf life). This means that the sample is more stable against oxidative degradation than the comparative samples.

**Table 41:**

| Sample No. | Heptanediol / Octanediol | | delta IP versus sunflower oil without additive |
|---|---|---|---|
| 32 | sunflower oil without additive | **25.9** | |
| 33 | plus 0.1% tocopherol | 31.2 | 5.3 |
| 34 | plus 0.1% tocopherol plus 0.5% 1,2-heptanediol | 32.3 | 6.4 |
| 37 | plus 0.1% tocopherol plus 0.5% 2,3-octanediol | 31.2 | 5.3 |

(continued)

| Sample No. | Heptanediol / Octanediol | | delta IP versus sunflower oil without additive |
|---|---|---|---|
| 38 | plus 0.1% tocopherol plus 0.5% blend of 1,2-heptanediol and 2,3-octanediol (ratio 95:5) | 32.2 | 6.3 |

The results are shown in Figure 17.

**Table 42:**

| Sample No. | Nonanediol | IP value (h) | delta IP versus sunflower oil without additive |
|---|---|---|---|
| 39 | sunflower oil without additive | **16.2** | |
| 40 | plus 0.1% tocopherol | 23.5 | 7.3 |
| 41 | plus 0.1% tocopherol + 0.5% 1,2-nonanediol | 24.1 | 7.9 |
| 42 | plus 0.1% tocopherol + 0.5% 2,3-nonanediol | 25.4 | 9.2 |
| 43 | plus 0.1% tocopherol plus 0.5% blend of 1,2-nonanediol and 2,3-nonanediol (ratio 50:50) | 25.7 | 9.5 |

The results are shown in Figure 18. As can be seen from Figure 18, sample 41 and sample results in prolonged induction periods (indicating longer shelf life). However, a blend of 1,2-nonanediol and 2,3-nonanediol considerably prolonged the induction period. This means that the samples are more stable against oxidative degradation than the comparative samples.

**Table 43:**

| Sample No. | Heptanediol | IP value (h) | delta IP versus sunflower oil without additive |
|---|---|---|---|
| 44 | sunflower oil without additive | **23.1** | |
| 45 | plus 2.0% Symdecanox HA | 24.4 | 1.3 |
| 46 | plus 2.0% Symdecanox HA plus 0.5% 1,2-heptanediol | 24.4 | 1.3 |
| 47 | plus 2.0% Symdecanox HA plus 0.5% 2,3-heptanediol | 24.7 | 1.6 |
| 48 | plus 2.0% Symdecanox HA plus 0.5% blend of 1,2-heptanediol and 2,3-heptanediol (ratio 95:5) | 25.3 | 2.2 |

The results are shown in Figure 19. As can be seen from Figure 19, sample 47 results in prolonged induction periods (indicating longer shelf life). However, a blend of 1,2-heptanediol and 2,3-heptanediol (95 : 5) considerably prolonged the induction period. This means that the samples are more stable against oxidative degradation than the comparative samples.

**Table 44:**

| Sample No. | Octanediol | IP value (h) | delta IP versus sunflower oil without additive |
|---|---|---|---|
| 44 | sunflower oil without additive | **23.1** | |
| 45 | plus 2.0% Symdecanox HA | 24.4 | 1.3 |
| 49 | plus 2.0% Symdecanox HA plus 0.5% 1,2-octanediol | 23.9 | 0.8 |
| 50 | plus 2.0% Symdecanox HA plus 0.5% 2,3-octanediol | 24 | 0.9 |

(continued)

| Sample No. | Octanediol | IP value (h) | delta IP versus sunflower oil without additive |
|---|---|---|---|
| 51 | plus 2.0% Symdecanox HA plus 0.5% blend of 1,2-octanediol and 2,3-octanediol (ratio 95:5) | 25.1 | 2 |
| INCI Symdecanox HA: Caprylic/Capric Triglyceride, Hydroxymethyoxyphenyl decanone | | | |

The results are shown in Figure 20. As can be seen from Figure 20, sample 51 results in prolonged induction periods (indicating longer shelf life). This means that the samples are more stable against oxidative degradation than the comparative samples.

**Table 45:**

| Sample No. | Heptanediol | IP value (h) | delta IP versus sunflower oil without additive |
|---|---|---|---|
| 52 | sunflower oil without additive | **21.9** | |
| 53 | plus 0.01% Pentaerythrityl Tetra-di-t-butyl Hydroxyhydrocinnamate | 25.8 | 3.9 |
| 54 | plus 0.01% Pentaerythrityl Tetra-di-t-butyl Hydroxyhydrocinnamate plus 0.5% 1,2-heptanediol | 27.7 | 5.8 |
| 55 | plus 0.01% Pentaerythrityl Tetra-di-t-butyl Hydroxyhydrocinnamate plus 0.5% 2,3-heptanediol | 27.6 | 5.7 |
| 56 | plus 0.01% Pentaerythrityl Tetra-di-t-butyl Hydroxyhydrocinnamate plus 0.5% blend of 1,2-heptanediol and 2,3-heptanediol (ratio 95:5) | 28.1 | 6.2 |

The results are shown in Figure 21. As can be seen from Figure 21, both sample 54 and sample 55 results in prolonged induction periods (indicating longer shelf life). However, a blend of 1,2-heptanediol and 2,3-heptanediol (95 : 5) considerably prolonged the induction period. This means that the samples are more stable against oxidative degradation than the comparative samples.

**Table 46:**

| Sample No. | Heptanediol | IP value (h) | delta IP versus sunflower oil without additive |
|---|---|---|---|
| 57 | sunflower oil without additive | **22.9** | |
| 58 | plus 0.5% Hydroxyacetophenone | 23.2 | 0.3 |
| 59 | plus 0.5% Hydroxyacetophenone plus 0.5% 1,2-heptanediol | 24.4 | 1.5 |
| 60 | plus 0.5% Hydroxyacetophenone plus 0.5% 2,3-heptanediol | 24.3 | 1.4 |

The results are shown in Figure 22. As can be seen from Figure 22, both 1,2-heptanediol and 2,3-heptanediol in combination with Hydroxyacetophenone result in remarkably prolonged induction periods (indicating longer shelf life). This means that the samples are more stable against oxidative degradation than the comparative samples.

Table **47:**

| Sample No. | Heptanediol | IP value (h) | delta IP versus sunflower oil without additive |
|---|---|---|---|
| 61 | sunflower oil without additive | **23.5** | |
| 62 | plus 0.05% Ascorbyl Palmitate | 25.5 | 2.0 |
| 63 | plus 0.05% Ascorbyl Palmitate plus 0.5% 1,2-heptanediol | 26.7 | 3.2 |
| 64 | plus 0.05% Ascorbyl Palmitate plus 0.5% 2,3-heptanediol | 26.6 | 3.1 |

The results are shown in Figure 23. As can be seen from Figure 23, both 1,2-heptanediol and 2,3-heptanediol in combination with Ascorbyl Palmitate result in remarkably prolonged induction periods (indicating longer shelf life). This means that the samples are more stable against oxidative degradation than the comparative samples.

As it is demonstrated by the above example, the induction period of the samples can be extended with an antioxidant (tocopherol) and can be even more, i.e., synergistically, improved with a combination of an antioxidant in combination with an alkanediol. Hence, the oxidation process of sample including an oil can be decelerated with the concurrent use of an antioxidant and an alkanediol.

**Example D.2: Odor evaluation of the samples 1 to 12 of Example D.1**

A sensory evaluation of the samples of Example D.1 after Oxipres treatment was performed with 12 untrained panelists. The panelists evaluated the rancid odor of each sample on a scale from 1 to 5 (5 = strong rancid odor; 1 = no rancid odor).

Test samples:

| | |
|---|---|
| Sample 1: | Sunflower oil without additives |
| Sample 2: | Sunflower oil plus 0.1 % tocopherol |
| Sample 3: | Sunflower oil plus 0.1 % tocopherol and 0.5 % 1,2-heptanediol |
| Sample 4: | Sunflower oil plus 0.1 % tocopherol and 0.5 % of an alkanediol blend of 95 % 1,2-heptanediol and 0.5 % 2,3-heptanediol |
| Sample 5: | Sunflower oil plus 0.5 % 1,2-heptanediol |
| Sample 6: | Sunflower oil plus 0.5 % 2,3-heptanediol |
| Sample 7: | Sunflower oil plus 0.5 % 1,2-nonanediol |
| Sample 8: | Sunflower oil plus 0.1 % tocopherol and 0.5 % 1,2-nonanediol |
| Sample 9: | Sunflower oil plus 0.1 % 1,2-decanediol |
| Sample 10: | Sunflower oil plus 0.1% tocopherol and 0.1 % 1,2-decanediol |
| Sample 11: | Sunflower oil plus 0.5 % 2,3-octanediol |
| Sample 12: | Sunflower oil plus 0.1 % tocopherol and 0.5 % 2,3-octanediol |

The results of the sensory evaluation of samples 1 to 12 are summarized in Table 46.

**Table 48:** Results of sensory evaluation

| Panelists | Sample 1 | Sample 2 | Sample 3 | Sample 4 | Sample 5 | Sample 6 | Sample 7 | Sample 8 | Sample 9 | Sample 10 | Sample 11 | Sample 12 |
|---|---|---|---|---|---|---|---|---|---|---|---|---|
| **A** | 5 | 4 | 3 | 2 | 5 | 5 | 5 | 3 | 5 | 2 | 5 | 2 |
| **B** | 5 | 4 | 2 | 3 | 4 | 4 | 4 | 4 | 4 | 3 | 4 | 2 |
| **C** | 5 | 4 | 2 | 2 | 4 | 5 | 3 | 3 | 3 | 2 | 3 | 1 |
| **D** | 4 | 2 | 2 | 2 | 5 | 5 | 4 | 2 | 4 | 3 | 4 | 3 |

(continued)

| Panelists | Sample 1 | Sample 2 | Sample 3 | Sample 4 | Sample 5 | Sample 6 | Sample 7 | Sample 8 | Sample 9 | Sample 10 | Sample 11 | Sample 12 |
|---|---|---|---|---|---|---|---|---|---|---|---|---|
| E | 5 | 4 | 4 | 3 | 4 | 4 | 5 | 4 | 3 | 3 | 4 | 3 |
| mean value | 4.8 | 3.6 | 2.6 | 2.4 | 4.4 | 4.6 | 4.2 | 3.2 | 3.8 | 2.6 | 4.0 | 2.2 |

The values in Table 48 clearly show that sample 3 (sunflower oil plus 0.1 % tocopherol plus 0.5 % 1,2-heptanediol), sample 4 (sunflower oil plus 0.1 % tocopherol plus 0.5 % of an alkanediol blend of 95 % 1,2-heptanediol and 0.5 % 2,3 heptanediol), sample 8 (sunflower oil plus 0.1 % tocopherol plus 0.5 % 1,2-nonanediol), sample 10 (sunflower oil plus 0.1% tocopherol plus 0.1 % 1,2-decanediol) and sample 12 (sunflower oil plus 0.1 % tocopherol plus 0.5 % 2,3-octanediol), i.e. samples containing an antioxidant in combination with 1,2-heptanediol and/or 2,3-heptanediol have a better odor, i.e. the samples showed less rancid odor than the comparative examples. Comparative sample 1 (sunflower oil without antioxidant or alkanediol, comparative sample 2 (sunflower oil with tocopherol) and comparative samples 5 and 6 (sunflower oil with alkanediols without tocopherol) had a stronger rancid odor.

As it is demonstrated by the above tests, oxidative degradation can be reduced or minimized with an antioxidant (tocopherol) and can be even more reduced, i.e., boosted, with the addition of an antioxidant in combination with an alkanediol.

A distinguished efficacy for antioxidative boosting (prolonged induction time, less rancidity and reduced acid value) could be shown for 1,2-heptanediol as well as for 1,2-nonediol, 1,2-decanediol and 2,3-octanediol.

The above results allow the conclusion that the samples comprising an antioxidant in combination with an alkanediol have an antioxidative effect which is clearly above the one provided with an antioxidant alone.

**Example D.3: Antioxidant potential on ex vivo skin biopsies (lipophilic or aqueous/alcoholic (ethanolic) test samples)**

A dichlorofluorescein test (DCF) assay was performed in order to determine the amount of reactive oxygen species (ROS) on ex vivo skin treated with different test samples in a lipophilic or an aqueous/alcoholic system as described below.

DCF assay - assay principle: Ex vivo skin was incubated with 2',7'-dichlorodihydrofluorescein diacetate at 37 °C, 5 % $CO_2$. After PBS washing, samples were exposed or not to cumene hydroperoxide. Immediately after exposure, ex vivo skin samples were frozen in liquid nitrogen and 5 $\mu$m cryostat sections were made and fixed with acetone to allow the visualisation of fluorescence generated by ROS in cells of the reconstructed skins. The resulting fluorescence was measured at EX/EM 504/524 nm. Green fluorescence was quantified in ex vivo skin using Imaged software. The depth of immunostaining was measured as follows: green DCFH-DE positive cells were automatically detected using Histolab software and the distance between dermal epidermal junction and the deepest positive cells were measured in each condition. Means were compared using a student's test. Two means were considered statistically different when $p < 0.005$.

The image acquisition was performed by using Olympus BX51 microscope and Olympus DP70 camera. For each skin sample two skin sections have been taken and the related fluorescent images acquired and analyzed. Thus, for each test condition, 12 images have been acquired and analyzed (i.e. 12 data). The analysis of fluorescence has been performed within the dermis area. For each image the upper dermis has been analyzed by evaluating the fluorescence through modified Image-J application (NIH, USA). The analyzed area is selected from the upper part by following the perimeter of the basal lamina, to the deep dermis, by carefully avoiding the risk of including irregularities and agglomerates, such as blood vessels, sebaceous glands, hair follicles. The obtained value has been normalized upon the dimension of the selected area.

In addition, as positive control an AOX mix is always co-tested, i.e., as bench mark for the ROS analysis. The AOX mix is a mixture of the following antioxidants: 15 % vitamin C, 1 % vitamin E, 0.5 % ferulic acid in a EtOH/$H_2O$ (50 : 50) mixture.

ROS score method description: The pigmentation score is based on the following process steps:

(1) Analysis of the image based on pixel grey intensities;
(2) Selection of an informative area excluding the pixel area not covered from tissue;
(3) Transformation of pixel grey intensities in degrees of L*;
(4) Normalization of the obtained values on the ratio between the selected area and the area of the slide.

An increased level of ROS (reactive oxygen species) led to an increased amount of fluorescence.

The chemical principle of the dichlorofluorescein (DCF) assay is depicted below:

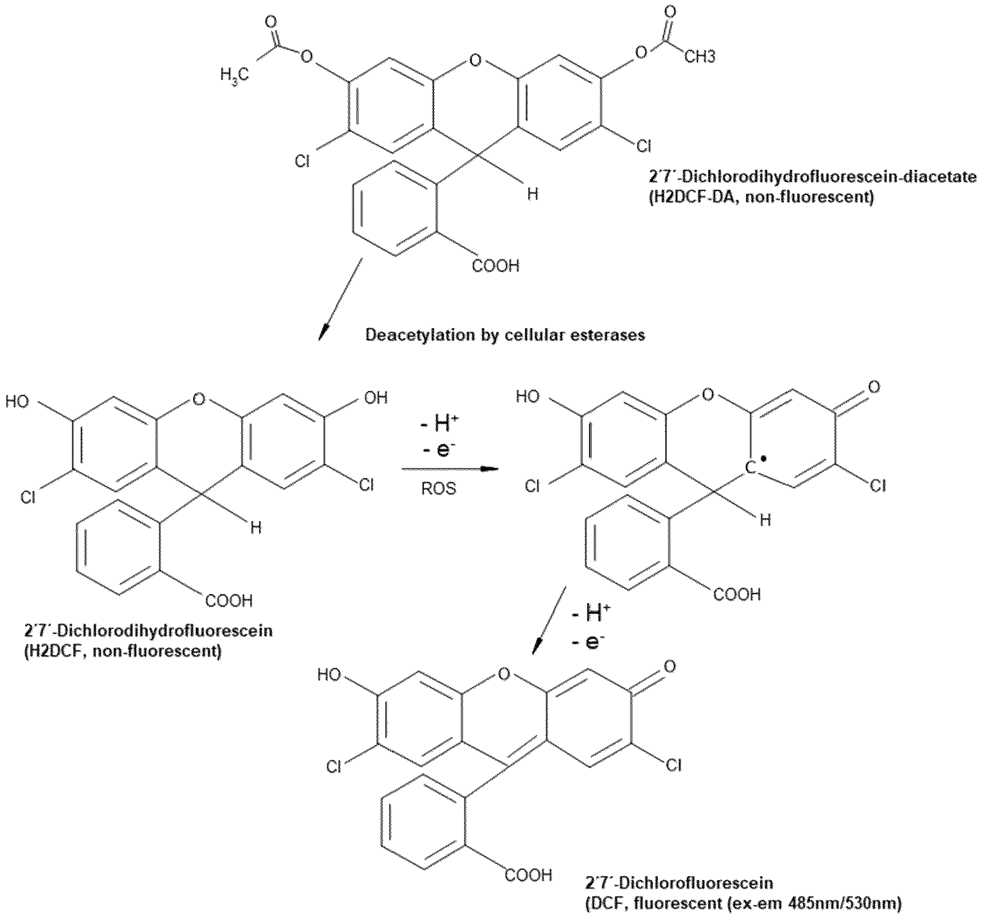

2'7'-Dichlorodihydrofluorescein-diacetate (H2DCF-DA, non-fluorescent)

2'7'-Dichlorodihydrofluorescein (H2DCF, non-fluorescent)

2'7'-Dichlorofluorescein (DCF, fluorescent (ex-em 485nm/530nm)

The chemical equation illustrates the conversion of 2',7'-dichlorodihydrofluorescein diacetate (H2DCFDA) by esterases in the intracellular environment and its subsequent two-step-oxidation in the presence of radical species (mainly ROS).

**Example D.3.1: DCF-Assay in biopsy antioxidation test: 1,2-alkanediols or 2,3-alkanediols plus/minus tocopherol for lipophilic test samples**

The dichlorofluorescein test (DCF) as described before was performed with different test samples in a lipophilic test system as described below:

Test samples:

| | |
|---|---|
| Sample A: | Vehicle: sunflower oil without additives |
| Sample B: | Sunflower oil plus 0.1 % tocopherol |
| Sample C: | Sunflower oil plus 0.1 % tocopherol plus 0.3 % 1,2-pentanediol |
| Sample D: | Sunflower oil plus 0.1 % tocopherol plus 0.3 % 1,2-hexanediol |
| Sample E: | Sunflower oil plus 0.1 % tocopherol plus 0.3 % 1,2-heptanediol |
| Sample F: | Sunflower oil plus 0.1 % tocopherol plus 0.3 % 2,3-heptanediol |
| Sample K: | Sunflower oil plus 0.5 % 1,2-pentanediol |
| Sample L: | Sunflower oil plus 0.5 % 1,2-hexanediol |
| Sample M: | Sunflower oil plus 0.5 % 1,2-heptanediol |

(continued)

Sample O: Sunflower oil plus 0.5 % 2,3-heptanediol
Sample P: Sunflower oil plus 0.5 % 1,2-nonanediol

The ROS scores of the above specified samples are summarized in Table 49.

**Table 49:** Results ROS score

| Test | No cumene | Cumene hydroperoxide 0.5 M | | | | | | | |
|---|---|---|---|---|---|---|---|---|---|
| | Untreated | Untreated cumene | AOX mix | A sunflower oil | B | C | D | E | F |
| 1 | 0.000 | 4.438 | 0.001 | 0.001 | 1.320 | 0.008 | 0.001 | 0.000 | 0.003 |
| 2 | 0.001 | 5.004 | 0.283 | 1.319 | 0.871 | 0.862 | 0.001 | 0.003 | 0.001 |
| 3 | 0.004 | 2.456 | 0.002 | 0.967 | 0.814 | 0.331 | 0.002 | 0.081 | 0.000 |
| 4 | 0.013 | 4.701 | 0.055 | 2.109 | 1.206 | 0.505 | 0.001 | 0.106 | 0.099 |
| 5 | 0.001 | 2.241 | 0.002 | 1.969 | 1.336 | 0.035 | 0.437 | 0.006 | 0.593 |
| 6 | 0.007 | 2.039 | 0.459 | 2.570 | 0.417 | 0.500 | 0.001 | 1.159 | 0.167 |
| 7 | 0.002 | 1.926 | 0.002 | 1.481 | 0.896 | 0.002 | 0.008 | 0.616 | 0.019 |
| 8 | 0.000 | 0.926 | 0.010 | 2.020 | 0.433 | 0.911 | 0.130 | 0.019 | 0.046 |
| 9 | 0.001 | 3.068 | 0.003 | 2.027 | 1.522 | 0.130 | 0.001 | 0.295 | 0.001 |
| 10 | 0.000 | 2.644 | 0.001 | 1.233 | 0.823 | 0.062 | 0.002 | 0.000 | 0.013 |
| 11 | 0.001 | 3.488 | 0.175 | 1.624 | 0.395 | 0.582 | 0.006 | 0.113 | 0.000 |
| 12 | 0.253 | 1.708 | 0.000 | 2.497 | 0.783 | 0.001 | 0.002 | 0.003 | 0.003 |
| Mean Score | 0.02 | 2.89 | 0.08 | 1.65 | 0.90 | 0.33 | 0.05 | 0.20 | 0.08 |
| St. Dev | 0.07 | 1.28 | 0.15 | 0.72 | 0.38 | 0.34 | 0.13 | 0.35 | 0.17 |
| | | | | | | | | | |
| D percent vs untreated cumene | | | -97% | -43% | -69% | -89% | -98% | -93% | -97% |
| D percent vs sample A cumene (sunflower oil) | | | | | -45% | -80% | -97% | -88% | -95% |
| D percent vs sample B cumene (sunflower oil plus plus 0.1 % tocopherol) | | | | | | **-64%** | **-95%** | **-78%** | **-91%** |

**Table 49: Results ROS score (cont.)**

| Test | No cumene | Cumene hydroperoxide 0.5 M | | | | | | | |
|---|---|---|---|---|---|---|---|---|---|
| | Untreated | Untreated cumene | AOX mix | A sunflower oil | K | L | M | O | P |
| 1 | 0.000 | 0.302 | 0.001 | 0.301 | 0.560 | 0.033 | 0.533 | 0.306 | 0.695 |
| 2 | 0.000 | 0.342 | 0.000 | 0.141 | 0.071 | 0.001 | 0.314 | 0.204 | 0.895 |
| 3 | 0.035 | 0.056 | 0.001 | 0.451 | 1.173 | 0.272 | 0.401 | 0.112 | 1.079 |
| 4 | 0.003 | 0.005 | 0.001 | 0.119 | 0.426 | 0.336 | 0.185 | 0.038 | 0.802 |
| 5 | 0.005 | 0.930 | 0.000 | 0.589 | 0.085 | 0.091 | 0.747 | 0.001 | 0.271 |
| 6 | 0.000 | 0.772 | 0.000 | 0.495 | 0.112 | 0.008 | 0.515 | 0.111 | 0.972 |

(continued)

| Test | No cumen e | Cumene hydroperoxide 0.5 M | | | | | | | |
|---|---|---|---|---|---|---|---|---|---|
| | Untrea ted | Untreat ed cumene | AOX mix | A sunflow er oil | K | L | M | O | P |
| 7 | 0.005 | 0.134 | 0.001 | 0.256 | 0.323 | 0.161 | 0.225 | 0.103 | 1.171 |
| 8 | 0.003 | 0.000 | 0.001 | 0.034 | 0.182 | 0.261 | 0.013 | 0.443 | 0.728 |
| 9 | 0.002 | 0.039 | 0.000 | 0.579 | 0.308 | 0.485 | 0.335 | 0.399 | 1.220 |
| 10 | 0.001 | 0.001 | 0.001 | 0.477 | 0.193 | 0.281 | 0.872 | 0.395 | 0.523 |
| 11 | 0.005 | 0.003 | 0.000 | 0.014 | 0.382 | 0.143 | 0.002 | 0.047 | 0.532 |
| 12 | 0.000 | 0.001 | 0.000 | 0.656 | 0.579 | 0.200 | 0.001 | 0.593 | 0.153 |
| Mean Score | 0.00 | 0.22 | 0.00 | 0.34 | 0.37 | 0.19 | 0.35 | 0.23 | 0.75 |
| St. Dev | 0.01 | 0.32 | 0.00 | 0.23 | 0.31 | 0.15 | 0.29 | 0.19 | 0.34 |
| SEM | 0.00 | 0.09 | 0.00 | 0.07 | 0.09 | 0.04 | 0.08 | 0.06 | 0.10 |
| | | | | | | | | | |
| D percent vs untreated cumene | | | -100% | 59% | 70% | -12% | 60% | 6% | 250% |
| D percent vs A cumene | | | | | 7% | -45% | 1% | -33% | 120% |

The ROS score results from Table 49 are visualized in Figures 6a and 6b.

The results in Table 49 and Figures 6a and 6b clearly demonstrate that the addition of 1,2-alkanediols and/or 2,3-alkanediols to a sample (C, D, E or F) including sunflower oil plus an antioxidant (tocopherol) leads to more than 60 % reduced ROS scores versus a sample of sunflower oil with only tocopherol and without the addition of a 1,2 alkanediol or a 2,3-alkanediol. The samples K, L, M, O and P including only a 1,2-alkanediol or a 2,3-alkanediol without an antioxidant do not have an antioxidative efficacy.

From the above it can be concluded, that the addition of an 1,2-alkanediol or an 2,3-alkanediol to a composition comprising an antioxidant results in a considerably reduced ROS scores. In other words: the combination of an antioxidant plus 1,2-alkanediol and/or 2,3-alkanediol shows an improved ROS scavenging efficacy.

**Example D.3.2: DCF-Assay in biopsy antioxidation test: 1,2-alkanediols or 2,3-alkanediols plus/minus tocopherol for aqueous/alcoholic test samples**

The dichlorofluorescein test (DCF) as described before was performed with different test samples in an aqueous/alcoholic (ethanolic) test system as described below:

Test samples:

Sample 1: Vehicle (EtOH/$H_2O$)
Sample 2: Vehicle plus 0.1 % tocopherol
Sample 3: Vehicle plus 0.1 % tocopherol plus 0.5 % 1,2-heptanediol
Sample 4: Vehicle plus 0.1 % tocopherol plus 0.5 % 2,3-heptanediol
Sample 5: Vehicle plus 0.1 % tocopherol plus alkanediol blend (0.5 % 1,2-heptanediol and 0.5 % 2,3-heptanediol)
Sample 6: Vehicle plus 0.1 % tocopherol plus 0.5 % 1,2-octanediol
Sample 7: Vehicle plus 0.1 % tocopherol plus 0.5 % 2,3-octanediol
Sample 8: Vehicle plus 0.1 % tocopherol plus alkanediol blend (0.5 % 1,2-octanediol and 0.5 % 2,3-octanediol)
Sample 10: Vehicle plus 0.1 % tocopherol plus 0.5 % 1,2-nonanediol
Sample 11: Vehicle plus 0.1 % tocopherol plus 0.5 % 1,2-undecanediol

(continued)

| | |
|---|---|
| Sample 12: | Vehicle plus 0.1 % tocopherol plus 0.5 % 2,3-undecanediol |
| Sample 13: | Vehicle plus 0.5 % 1,2-heptanediol |
| Sample 14: | Vehicle plus 0.5 % 2,3-heptanediol |
| Sample 15: | Vehicle plus 0.5 % 1,2-octanediol |
| Sample 16: | Vehicle plus 0.5 % 2,3-octanediol |
| Sample 17: | Vehicle plus 0.5 % 1,2-nonanediol |
| Sample 18: | Vehicle plus 0.5 % 1,2-undecanediol |
| Sample 19: | Vehicle plus 0.5 % 2,3-undecanediol |

The ROS scores are summarized in Table 50.

**Table 50:** Results ROS score

| Test | No cumene | Cumene Hydroperoxide 0.5 M | | | | | | | | | | | | |
|---|---|---|---|---|---|---|---|---|---|---|---|---|---|---|
| | **Untreat ed** | **Untreat ed cumene** | **AOX mix** | **1** | **2** | **3** | **4** | **5** | **6** | **7** | **8** | **10** | **11** | **12** |
| 1 | 0.003 | 0.356 | 0.001 | 0.552 | 0.001 | 0.001 | 0.001 | 0.001 | 0.000 | 0.000 | 0.035 | 0.000 | 0.001 | 0.004 |
| 2 | 0.001 | 0.617 | 0.017 | 0.795 | 0.000 | 0.001 | 0.022 | 0.000 | 0.001 | 0.000 | 0.001 | 0.022 | 0.012 | 0.029 |
| 3 | 0.001 | 0.421 | 0.002 | 0.196 | 0.001 | 0.001 | 0.002 | 0.002 | 0.000 | 0.003 | 0.038 | 0.001 | 0.000 | 0.000 |
| 4 | 0.080 | 0.769 | 0.003 | 0.880 | 0.038 | 0.029 | 0.000 | 0.000 | 0.074 | 0.014 | 0.001 | 0.000 | 0.000 | 0.001 |
| 5 | 0.001 | 0.999 | 0.000 | 0.528 | 0.180 | 0.001 | 0.001 | 0.001 | 0.000 | 0.001 | 0.000 | 0.001 | 0.001 | 0.002 |
| 6 | 0.000 | 0.584 | 0.149 | 0.067 | 0.679 | 0.001 | 0.009 | 0.001 | 0.001 | 0.000 | 0.002 | 0.001 | 0.109 | 0.000 |
| 7 | 0.370 | 1.005 | 0.036 | 0.288 | 0.001 | 0.002 | 0.000 | 0.001 | 0.016 | 0.010 | 0.002 | 0.001 | 0.000 | 0.043 |
| 8 | 0.009 | 0.642 | 0.001 | 0.441 | 0.018 | 0.000 | 0.001 | 0.001 | 0.001 | 0.001 | 0.001 | 0.002 | 0.015 | 0.008 |
| 9 | 0.000 | 0.417 | 0.001 | 0.546 | 0.011 | 0.000 | 0.001 | 0.001 | 0.000 | 0.001 | 0.028 | 0.093 | 0.000 | 0.002 |
| 10 | 0.003 | 0.861 | 0.001 | 0.623 | 0.192 | 0.001 | 0.006 | 0.000 | 0.001 | 0.018 | 0.025 | 0.001 | 0.000 | 0.054 |
| 11 | 0.018 | 0.349 | 0.001 | 0.547 | 0.437 | 0.001 | 0.000 | 0.001 | 0.019 | 0.001 | 0.130 | 0.001 | 0.000 | 0.000 |
| 12 | 0.000 | 1.327 | 0.001 | 0.043 | 0.170 | 0.000 | 0.001 | 0.002 | 0.001 | 0.007 | 0.002 | 0.000 | 0.002 | 0.051 |
| Mean Score | 0.04 | 0.70 | 0.02 | 0.46 | 0.14 | 0.00 | 0.00 | 0.00 | 0.01 | 0.00 | 0.02 | 0.01 | 0.01 | 0.02 |
| St. Dev | 0.11 | 0.31 | 0.04 | 0.26 | 0.21 | 0.01 | 0.01 | 0.00 | 0.02 | 0.01 | 0.04 | 0.03 | 0.03 | 0.02 |
| SEM | 0.03 | 0.09 | 0.01 | 0.08 | 0.06 | 0.00 | 0.00 | 0.00 | 0.01 | 0.00 | 0.01 | 0.01 | 0.01 | 0.01 |
| D percent vs untreated Cumene | | | -97% | -34% | -79% | -100 % | -99% | -100 % | -99% | -99% | -97% | -99% | -98% | -98% |
| D percent vs 1 cumene (vehicle (EtOH/$H_2O$)) | | | | | -69% | -99% | -99% | 100 % | -98% | -99% | -95% | -98% | -97% | -96% |
| D percent vs 2 cumene (vehicle plus 0.1 % tocopherol) | | | | | | -98% | -97% | -99% | -93% | -97% | -85% | -93% | -92% | -89% |

**Table 50: Results ROS score (cont.)**

| Test | No cumen e | Cumene Hydroperoxide 0.5 M | | | | | | | | | | |
|---|---|---|---|---|---|---|---|---|---|---|---|---|
| | Untrea ted | Untreat ed cumene | AOX mix | 1 | 2 | 13 | 14 | 15 | 16 | 17 | 18 | 19 |
| 1 | 0.003 | 0.356 | 0.001 | 0.552 | 0.001 | 0.779 | 0.645 | 0.595 | 0.438 | 1.160 | 0.186 | 0.655 |
| 2 | 0.001 | 0.617 | 0.017 | 0.795 | 0.000 | 0.319 | 0.646 | 1.058 | 0.432 | 0.284 | 0.043 | 0.447 |
| 3 | 0.001 | 0.421 | 0.002 | 0.196 | 0.001 | 0.479 | 0.469 | 0.461 | 0.270 | 0.287 | 0.169 | 0.394 |
| 4 | 0.080 | 0.769 | 0.003 | 0..88 0 | 0.038 | 0.042 | 0.313 | 0.410 | 0.502 | 0.431 | 0.756 | 0.365 |
| 5 | 0.001 | 0.999 | 0.000 | 0.528 | 0.180 | 0.438 | 0.992 | 0.334 | 0.326 | 0.539 | 0.720 | 0.876 |
| 6 | 0.000 | 0.584 | 0.149 | 0.067 | 0.679 | 0.089 | 0.574 | 0.664 | 0.323 | 0.429 | 0.724 | 1.300 |
| 7 | 0.370 | 1.005 | 0.036 | 0.288 | 0.001 | 0.379 | 0.521 | 0.454 | 0.157 | 0.364 | 0.292 | 0.290 |
| 8 | 0.009 | 0.642 | 0.001 | 0.441 | 0.018 | 0.527 | 0.238 | 0.816 | 0.379 | 0.412 | 0.001 | 0.775 |
| 9 | 0.000 | 0.417 | 0.001 | 0.546 | 0.011 | 0.086 | 0.211 | 0.627 | 0.442 | 0.145 | 0.842 | 1.268 |
| 10 | 0.003 | 0.861 | 0.001 | 0.623 | 0.192 | 0.663 | 0.158 | 0.551 | 0.245 | 0.022 | 0.001 | 0.529 |
| 11 | 0.018 | 0.349 | 0.001 | 0.547 | 0.437 | 0.444 | 0.513 | 0.217 | 0.175 | 0.523 | 0.558 | 0.435 |
| 12 | 0.000 | 1.327 | 0.001 | 0.043 | 0.170 | 1.199 | 0.195 | 0.365 | 0.357 | 0.121 | 0.402 | 0.205 |
| Mean Score | 0.04 | 0.70 | 0.02 | 0.46 | 0.14 | 0.45 | 0.46 | 0.55 | 0.34 | 0.39 | 0.39 | 0.63 |
| St. Dev | 0.11 | 0.31 | 0.04 | 0.26 | 0.21 | 0.33 | 0.25 | 0.23 | 0.11 | 0.29 | 0.32 | 0.36 |
| SEM | 0.03 | 0.09 | 0.01 | 0.08 | 0.06 | 0.09 | 0.07 | 0.07 | 0.03 | 0.08 | 0.09 | 0.10 |
| | | | | | | | | | | | | |
| D percent vs untreated cumene | | | -97% | -34% | -79% | -35% | -34% | -21% | -52% | -43% | -44% | -10% |
| D percent vs 1 cumene (vehicle ($EtOH/H_2O$)) | | | | | -69% | -1% | -1% | 19% | -26% | -14% | -15% | 37% |
| D percent vs 2 cumene (vehicle plus 0.1 % tocopherol) | | | | | | 215% | 217% | 279% | 134% | 173% | 172% | 336% |

The ROS score results from Table 50 are visualized in Figures 7a and 7b.

The results in Table 50 and Figures 7a and 7b clearly demonstrate that the addition of 1,2-alkanediols and/or 2,3-alkanediols to a sample including an aqueous alcoholic vehicle plus an antioxidant (tocopherol) leads to more than 80 % reduced ROS scores versus a sample of an aqueous/alcoholic vehicle with only tocopherol and without the addition of a 1,2 alkanediol or a 2,3-alkanediol. The samples including only the vehicle and a 1,2-alkanediol or a 2,3-alkanediol do not have an antioxidative efficacy.

From the above it can be concluded, that the addition of an 1,2-alkanediol and/or an 2,3-alkanediol to a composition comprising an antioxidant results in a considerably reduced ROS scores. In other words: the combination of an antioxidant plus 1,2-alkanediol and/or 2,3-alkanediol shows an improved ROS scavenging efficacy.

**Example D.3.3: DCF-Assay in biopsy antioxidation test: 1,2-alkanediols or 2,3-alkanediols plus/minus Dihydroavenanthramide D for aqueous/alcoholic test samples**

The dichlorofluorescein test (DCF) as described before was performed with different test samples in an aqueous/alcoholic (ethanolic) test system as described below:

Test samples:

Sample 1: Vehicle (EtOH/$H_2O$)
Sample 2: Vehicle plus 50ppm Dihydroavenanthramide D
Sample 3: Vehicle plus 50ppm Dihydroavenanthramide D plus 0.5% 1,2-heptanediol
Sample 4: Vehicle plus 50ppm Dihydroavenanthramide D plus 0.5% 2,3-heptanediol
Sample 5: Vehicle plus 50ppm Dihydroavenanthramid D plus 0.5% alkanediol blend of 1,2-heptanediol and 2,3-heptanediol (ratio 95:5)

The ROS scores are summarized in Table 50.

**Table 51:** Results ROS score

| | No Cumene | Cumene Hydroperoxide 0.5M | | | | | | |
|---|---|---|---|---|---|---|---|---|
| | UNTREATE D | UNTREATE D | AOX mix | 1 | 2 | 3 | 4 | 5 |
| 1 | | 2.397 | | 0.894 | 1.552 | 0.748 | 0.116 | 0.694 |
| 2 | 0.132 | 3.447 | 0.021 | 3.201 | 2.083 | 0.709 | 0.414 | 0.322 |
| 3 | 0.120 | 2.359 | 0.305 | 2.012 | 1.367 | 0.288 | 0.919 | 0.289 |
| 4 | 0.163 | 2.648 | 0.067 | 1.044 | 2.338 | 0.874 | 0.552 | 0.802 |
| 5 | 0.061 | 4.630 | 0.167 | 2.820 | 1.245 | 0.071 | 0.274 | 0.409 |
| 6 | 0.110 | 0.680 | 0.263 | 3.397 | 1.929 | 0.079 | 1.685 | 0.230 |
| 7 | 0.008 | 2.754 | 0.227 | 2.535 | outlier | 1.060 | 1.009 | outlier |
| 8 | 0.186 | 1.355 | 0.330 | 0.931 | 1.230 | 0.718 | 0.230 | 0.921 |
| 9 | 0.088 | 0.994 | 0.212 | 0.828 | 2.420 | 1.073 | 0.344 | 1.693 |
| 10 | 0.159 | 1.161 | 0.314 | 1.230 | 1.706 | 1.580 | 0.414 | 0.661 |
| 11 | 0.057 | 1.431 | 0.021 | 1.316 | 1.466 | 1.375 | 1.357 | 0.481 |
| **Mean Score** | **0.11** | **2.17** | **0.19** | **1.84** | **1.73** | **0.78** | **0.66** | **0.65** |
| St. Dev | 0.06 | 1.19 | 0.12 | 0.99 | 0.44 | 0.49 | 0.51 | 0.43 |
| SEM | 0.02 | 0.36 | 0.04 | 0.30 | 0.14 | 0.15 | 0.15 | 0.14 |
| | | | | | | | | |
| D percent vs Untr No Cumene | | 1900% | 78% | 1594% | 1499% | 619% | 513% | 500% |

(continued)

| | No Cumene | Cumene Hydroperoxide 0.5M | | | | | | |
|---|---|---|---|---|---|---|---|---|
| | UNTREATE D | UNTREATE D | AOX mix | 1 | 2 | 3 | 4 | 5 |
| D percent vs Untr Cumene | | | -91% | -15% | -20% | -64% | -69% | -70% |
| D percent vs -A Cumene | | | | | -6% | -58% | -64% | -65% |

The ROS score results from Table 51 are visualized in Figure 24.

The results in Table 51 and Figure 24 clearly demonstrate that the addition of 1,2-heptanediol or 2,3-heptanediol or an alkanediol mixture including 1,2-heptanediol and 2,3-heptanediol to a sample including an aqueous alcoholic vehicle plus an antioxidant (Dihydroavenanthramide D) leads to more than 60 % reduced ROS scores versus a sample of an aqueous/alcoholic vehicle with only Dihydroavenanthramide and without the addition of 1,2-heptanediol or 2,3-heptanediol. The samples including only the vehicle and 1,2-heptanediol or 2,3-heptanediol do not have an antioxidative efficacy.

From the above it can be concluded, that the addition of an 1,2-alkanediol and/or an 2,3-alkanediol to a composition comprising an antioxidant results in a considerably reduced ROS scores. In other words: the combination of an antioxidant plus 1,2-alkanediol and/or 2,3-alkanediol shows an improved ROS scavenging efficacy.

**Example D.3.4: DCF-Assay in biopsy antioxidation test: 1,2-alkanediols or 2,3-alkanediols plus/minus Cannabidiol for aqueous/alcoholic test samples**

The dichlorofluorescein test (DCF) as described before was performed with different test samples in an aqueous/alcoholic (ethanolic) test system as described below:

Test samples:

Sample 1: Vehicle (EtOH/$H_2O$)
Sample 2: Vehicle plus 5ppm Cannabidiol
Sample 3: Vehicle plus 5ppm Cannabidiol plus 0.5% 1,2-heptanediol
Sample 4 Vehicle plus 5ppm Cannabidiol plus 0.5% 2,3-heptanediol
Sample 5: Vehicle plus 5ppm Cannabidiol plus 0.5% alkanediol blend of 1,2-heptanediol and 2,3-heptanediol (ratio 95:5)

The ROS scores are summarized in Table 52.

**Table 52:** Results ROS score

| | No Cumene | Cumene Hydroperoxide 0.5M | | | | | | |
|---|---|---|---|---|---|---|---|---|
| | UNTREATE D | UNTREATE D | AOX mix | A | B | C | D | E |
| 1 | 0.003 | 1.800 | 0.069 | 1.602 | 0.560 | 0.002 | 0.003 | 0.047 |
| 2 | 0.006 | 3.127 | 0.498 | 2.370 | 0.362 | 0.054 | 0.410 | 0.017 |
| 3 | 0.104 | 0.903 | 0.008 | 3.935 | 0.621 | 0.016 | 0.075 | 0.147 |
| 4 | 0.028 | 1.980 | 0.001 | 1.927 | 0.200 | 0.114 | 0.082 | 0.018 |
| 5 | 0.111 | 1.903 | 0.402 | 2.072 | 1.230 | 0.673 | outlier | 0.028 |
| 6 | 0.030 | 0.719 | 0.007 | 2.770 | 1.096 | 0.182 | 0.027 | 0.414 |
| 7 | 0.005 | 1.705 | 0.007 | 1.016 | 0.010 | 0.587 | 0.006 | 0.028 |
| 8 | outlier | 3.104 | 0.157 | 2.499 | 1.025 | 0.127 | 0.188 | 0.164 |
| 9 | 0.060 | 1.330 | 0.196 | 0.932 | 0.304 | 0.080 | 0.350 | 0.065 |
| 10 | 0.007 | 2.331 | 0.234 | 0.952 | 0.005 | 0.586 | 0.313 | 0.484 |

(continued)

| | No Cumene | Cumene Hydroperoxide 0.5M | | | | | | |
|---|---|---|---|---|---|---|---|---|
| | UNTREATE D | UNTREATE D | AOX mix | A | B | C | D | E |
| 11 | 0.144 | 2.578 | 0.011 | 2.034 | 0.014 | 0.098 | 0.562 | 0.016 |
| **Mean Score** | **0.05** | **1.95** | **0.14** | **2.01** | **0.49** | **0.23** | **0.20** | **0.13** |
| St. Dev | 0.05 | 0.80 | 0.17 | 0.90 | 0.45 | 0.25 | 0.20 | 0.17 |
| SEM | 0.02 | 0.24 | 0.05 | 0.27 | 0.14 | 0.08 | 0.06 | 0.05 |
| D percent vs Untr No Cumene | | 3810% | 190% | 3924% | 887% | 359% | 304% | 160% |
| D percent vs Untr Cumene | | | -93% | 3% | -75% | -88% | -90% | -93% |
| D percent vs -1 Cumene | | | | | -75% | -89% | -90% | -94% |

The ROS score results from Table 52 are visualized in Figure 25.

The results in Table 52 and Figure 25 clearly demonstrate that the addition of 1,2-heptanediol or 2,3-heptanediol or an alkanediol mixture including 1,2-heptanediol and/or 2,3-heptanediol to a sample including an aqueous alcoholic vehicle plus an antioxidant (Cannabidiol) leads to more than 70 % reduced ROS scores versus a sample of an aqueous/alcoholic vehicle with only Cannabidiol and without the addition of 1,2-heptanediol or 2,3-heptanediol or . The samples including only the vehicle and 1,2-heptanediol or 2,3-heptanediol do not have an antioxidative efficacy.

From the above it can be concluded, that the addition of an 1,2-alkanediol and/or an 2,3-alkanediol to a composition comprising an antioxidant results in a considerably reduced ROS scores. In other words: the combination of an antioxidant plus 1,2-alkanediol and/or 2,3-alkanediol shows an improved ROS scavenging efficacy.

**E) Improvement of sensory properties of topical formulations comprising a liquid lipophilic components**

For evaluating the sensory behaviour, liquid lipophilic components specified in the following table were blended with 1,2-heptanediol or 2,3-heptanediol or a mixture of 1 ,2-heptanediol and 2,3-heptanediol or 1 ,2-octanediol or 2,3-octanediol or a mixture of 1,2-octanediol and 2,3-octanediol (formulation) in a ratio of 85 : 15 and assessed by untrained panellists in comparison to the liquid lipophilic component without addition of the specified alkanediols (placebo).

The test procedure was carried as follows: For evaluating the sensory behaviour of 1,2-alkanediols and/or 2,3-alkanediols in combination with different liquid lipophilic components, oil components specified in the following table were blended with a 1,2-alkanediol or a 2,3-alkanediol or a mixture including a 1,2-alkanediol and a 2,3-alkanediol in a ratio as specified in Table 53 and assessed by untrained panellists in comparison to the liquid lipophilic component without addition of the 1,2-alkanediol or 2,3-alkanediol or the mixture including a 1,2-alkanediol and 2,3-alkanediol (placebo).

Test procedure:

- Hands were pre-washed with liquid soap by following a defined procedure;
- 30 µl of corresponding sample was applied on the backside of each hand (randomized);
- After 30 s the spreading behaviour of the respective formulation on skin was evaluated versus the respective oil component without 1 ,2-heptanediol on a scale from 1 to 5 (1 = low spreading; 5 = high spreading);
- The samples were distributed with the fingertips of middle and forefinger with 2 x 5 cycles;
- After 2 minutes, the fatty/greasy skin feel was evaluated on a scale from 1 to 5 (1 = low fatty/greasy skin feel; 5 = high fatty/greasy skin feel);
- Furthermore the applicants were asked to assess the absorption, i.e. the impression of amount of remaining residue on the skin on a scale from 1 to 5 (1 = low absorption; 5 = high absorption).

**Table 53:**

| | Liquid lipophilic component | alkanediol | Spreading | | | Fatty/greasy skin feel | | | Absorption/ remaining residue | | |
|---|---|---|---|---|---|---|---|---|---|---|---|
| | | | withou t | wit h | Delt a *) | withou t | wit h | Delt a *) | withou t | Wit h | Delt a *) |
| Plant Oil | Argan Oil | 1,2-heptanediol | 2 | 4.2 | 2.2 | 4 | 2.4 | 1.6 | 3.8 | 2.8 | 1 |
| Plant Oil | Argan Oil | 2,3-heptanediol | 2 | 3 | 1 | 4 | 2.6 | 1.4 | 3.8 | 2.4 | 1.4 |
| Plant Oil | Argan Oil | 1,2-heptanediol/ 2,3-heptanediol (95:5) | 2 | 3.8 | 1.8 | 4 | 2.6 | 1.4 | 3.8 | 2.4 | 1.4 |
| | | | | | | | | | | | |
| Plant Oil | Argan Oil | 1,2-octanediol | 2 | 3.8 | 1.8 | 4 | 2.6 | 1.4 | 3.8 | 2.6 | 1.2 |
| Plant Oil | Argan Oil | 2,3-octanediol | 2 | 3.4 | 1.4 | 4 | 2.4 | 1.6 | 3.8 | 2.2 | 1.6 |
| Plant Oil | Argan Oil | 1,2-octanediol/ 2,3-octanediol (95:5) | 2 | 4.2 | 2.2 | 4 | 2.2 | 1.8 | 3.8 | 2 | 1.8 |
| | | | | | | | | | | | |
| Plant Oil | Avocado Oil | 1,2-heptanediol | 2.2 | 4.4 | 2.2 | 3.8 | 2.6 | 1.2 | 3.8 | 2.6 | 1.2 |
| Plant Oil | Avocado Oil | 2,3-heptanediol | 2.2 | 3.8 | 1.6 | 3.8 | 2 | 1.8 | 3.8 | 2.4 | 1.4 |
| Plant Oil | Avocado Oil | 1,2-heptanediol/ 2,3-heptanediol (95:5) | 2.2 | 3.6 | 1.4 | 3.8 | 2.2 | 1.6 | 3.8 | 2 | 1.8 |
| | | | | | | | | | | | |
| Plant Oil | Avocado Oil | 1,2-octanediol | 2 | 4 | 2 | 3.8 | 3 | 0.8 | 3.8 | 2.6 | 1.2 |
| Plant Oil | Avocado Oil | 2,3-octanediol | 2 | 3.2 | 1.2 | 3.8 | 2.8 | 1 | 3.8 | 2.4 | 1.4 |
| Plant Oil | Avocado Oil | 1,2-octanediol/ 2,3-octanediol (95:5) | 2 | 3.8 | 1.8 | 3.8 | 2.2 | 1.6 | 3.8 | 2.2 | 1.6 |

(continued)

| | Liquid lipophilic component | alkanediol | Spreading | | | Fatty/greasy skin feel | | | Absorption/ remaining residue | | |
|---|---|---|---|---|---|---|---|---|---|---|---|
| | | | withou t | wit h | Delt a *) | withou t | wit h | Delt a *) | withou t | Wit h | Delt a *) |
| | | | | | | | | | | | |
| Plant Oil | Butyrospermum Parkii (Shea) Butter | 1,2-heptanediol | 2 | 4 | 2 | 4 | 2 | 2 | 4.2 | 2.7 | 1.5 |
| | | | | | | | | | | | |
| Plant Oil | Cacao butter | 1,2-heptanediol | 1.8 | 3.8 | 2 | 4.2 | 3 | 1.2 | 3.6 | 3.6 | 0 |
| Plant Oil | Cacao butter | 2,3-heptanediol | 1.8 | 3 | 1.2 | 4.2 | 3.4 | 0.8 | 3.6 | 3.2 | 0.4 |
| Plant Oil | Cacao butter | 1,2-heptanediol/ 2,3-heptanediol (95:5) | 1.8 | 4 | 2.2 | 4.2 | 3 | 1.2 | 3.6 | 3 | 0.6 |
| | | | | | | | | | | | |
| Plant Oil | Cacao butter | 1,2-octanediol | 1.8 | 3.8 | 2 | 4.2 | 3.2 | 1 | 3.6 | 3 | 0.6 |
| Plant Oil | Cacao butter | 2,3-octanediol | 1.8 | 3 | 1.2 | 4.2 | 3 | 1.2 | 3.6 | 2.8 | 0.8 |
| Plant Oil | Cacao butter | 1,2-octanediol/ 2,3-octanediol (95:5) | 1.8 | 2.6 | 0.8 | 4.2 | 3.2 | 1 | 3 | 2.6 | 0.4 |
| | | | | | | | | | | | |
| Plant Oil | Caprylic Capric Triglyceride (neutral oil) | 1,2-heptanediol | 1.9 | 3.4 | 1.5 | 3.2 | 2.9 | 0.3 | 3.3 | 2.7 | 0.6 |
| Plant Oil | Caprylic Capric Triglyceride (neutral oil) | 2,3-heptanediol | 1.9 | 3.6 | 1.7 | 3.2 | 3.4 | -0.2 | 3.3 | 3 | 0.3 |
| Plant Oil | Caprylic Capric Triglyceride (neutral oil) | 1,2-heptanediol/ 2,3-heptanediol (95:5) | 1.9 | 3.8 | 1.9 | 3.2 | 2.6 | 0.6 | 3.3 | 2.8 | 0.5 |
| | | | | | | | | | | | |

(continued)

| | Liquid lipophilic component | alkanediol | Spreading | | | Fatty/greasy skin feel | | | Absorption/ remaining residue | | |
|---|---|---|---|---|---|---|---|---|---|---|---|
| | | | withou t | wit h | Delt a *) | withou t | wit h | Delt a *) | withou t | Wit h | Delt a *) |
| Plant Oil | Castor Oil | 1,2-heptanediol | 1.2 | 2.2 | 1 | 4.2 | 2.8 | 1.4 | 4.4 | 4 | 0.4 |
| Plant Oil | Castor Oil | 2,3-heptanediol | 1.2 | 1.6 | 0.4 | 4.2 | 3.6 | 0.6 | 4.4 | 3.8 | 0.6 |
| Plant Oil | Castor Oil | 1,2-heptanediol/ 2,3-heptanediol (95:5) | 1.2 | 1.8 | 0.6 | 4.2 | 3.4 | 0.8 | 4.4 | 3.4 | 1 |
| | | | | | | | | | | | |
| Plant Oil | Castor Oil | 1,2-octanediol | 1.2 | 1.6 | 0.4 | 4.2 | 3.6 | 0.6 | 4.4 | 3.4 | 1 |
| Plant Oil | Castor Oil | 2,3-octanediol | 1.2 | 1.8 | 0.6 | 4.2 | 2.8 | 1.4 | 4.4 | 3 | 1.4 |
| Plant Oil | Castor Oil | 1,2-octanediol/ 2,3-octanediol (95:5) | 1.2 | 2 | 0.8 | 4.2 | 3.4 | 0.8 | 4.4 | 3.2 | 1.2 |
| | | | | | | | | | | | |
| Plant oil | Chlorella oil (biotech) | 1,2-heptanediol | 1,3 | 4 | 2,7 | 3 | 2,7 | 0,3 | 3,2 | 3 | 0,2 |
| | | | | | | | | | | | |
| Plant Oil | Coconut Oil | 1,2-heptanediol | 2.4 | 4.2 | 1.8 | 3.2 | 1.8 | 1.4 | 3.4 | 2 | 1.4 |
| Plant Oil | Coconut Oil | 2,3-heptanediol | 2.4 | 3.4 | 1 | 3.2 | 2.4 | 0.8 | 3.4 | 2.4 | 1 |
| Plant Oil | Coconut Oil | 1,2-heptanediol/ 2,3-heptanediol (95:5) | 2.4 | 4.2 | 1.8 | 3.2 | 3 | 0.2 | 3.4 | 2.4 | 1 |
| | | | | | | | | | | | |
| Plant Oil | Coconut Oil | 1,2-octanediol | 2.4 | 4.4 | 2 | 3.6 | 2.6 | 1 | 3.8 | 3 | 0.8 |

(continued)

| | Liquid lipophilic component | alkanediol | Spreading | | | Fatty/greasy skin feel | | | Absorption/ remaining residue | | |
|---|---|---|---|---|---|---|---|---|---|---|---|
| | | | withou t | wit h | Delt a *) | withou t | wit h | Delt a *) | withou t | Wit h | Delt a *) |
| Plant Oil | Coconut Oil | 2,3-octanediol | 2.4 | 4.2 | 1.8 | 3.6 | 2.2 | 1.4 | 3.8 | 2.6 | 1.2 |
| Plant Oil | Coconut Oil | 1,2-octanediol/ 2,3-octanediol (95:5) | 2.4 | 4 | 1.6 | 3.6 | 2.8 | 0.8 | 3.8 | 3 | 0.8 |
| | | | | | | | | | | | |
| Plant Oil | Grapeseed Oil | 1,2-heptanediol | 2 | 3.8 | 1.8 | 4.2 | 2.2 | 2 | 2.6 | 2.8 | 0.2 |
| Plant Oil | Grapeseed Oil | 2,3-heptanediol | 2 | 3.6 | 1.6 | 4.2 | 2.6 | 1.6 | 3 | 2.2 | 0.8 |
| Plant Oil | Grapeseed Oil | 1,2-heptanediol/ 2,3-heptanediol (95:5) | 2 | 3.8 | 1.8 | 4.2 | 2.2 | 2 | 2.6 | 1.6 | 1 |
| | | | | | | | | | | | |
| Plant Oil | Grapeseed Oil | 1,2-octanediol | 2 | 4 | 2 | 4.2 | 2.8 | 1.4 | 2.6 | 1.6 | 1 |
| Plant Oil | Grapeseed Oil | 2,3-octanediol | 2 | 2.8 | 0.8 | 4.2 | 2.8 | 1.4 | 2.6 | 2.4 | 0.2 |
| Plant Oil | Grapeseed Oil | 1,2-octanediol/ 2,3-octanediol (95:5) | 2 | 3.8 | 1.8 | 4.2 | 2.2 | 2 | 2.3 | 1.4 | 0.9 |
| | | | | | | | | | | | |
| Plant Oil | Helianthus Annuus (Sunflower) Seed Oil | 1,2-heptanediol | 1.2 | 4.2 | 3 | 3.8 | 2.3 | 1.5 | 3.3 | 2 | 1.3 |
| Plant Oil | Helianthus Annuus (Sunflower) Seed Oil | 2,3-heptanediol | 1.6 | 3.8 | 2.2 | 3.6 | 2.6 | 1 | 3.6 | 2.6 | 1 |
| Plant Oil | Helianthus Annuus (Sunflower) Seed Oil | 1,2-heptanediol/ 2,3-heptanediol (95:5) | 1.6 | 4.4 | 2.8 | 3.6 | 2.4 | 1.2 | 3.6 | 2 | 1.6 |
| | | | | | | | | | | | |

(continued)

| | Liquid lipophilic component | alkanediol | Spreading | | | Fatty/greasy skin feel | | | Absorption/ remaining residue | | |
|---|---|---|---|---|---|---|---|---|---|---|---|
| | | | withou t | wit h | Delt a *) | withou t | wit h | Delt a *) | withou t | Wit h | Delt a *) |
| Plant Oil | Helianthus Annuus (Sunflower) Seed Oil | 1,2-octanediol | 1.4 | 4.2 | 2.8 | 3.8 | 2 | 1.8 | 3.8 | 2 | 1.8 |
| Plant Oil | Helianthus Annuus (Sunflower) Seed Oil | 2,3-octanediol | 1.4 | 3.4 | 2 | 3.8 | 3 | 0.8 | 3.8 | 3.4 | 0.4 |
| Plant Oil | Helianthus Annuus (Sunflower) Seed Oil | 1,2-octanediol/ 2,3-octanediol (95:5) | 1.4 | 4.4 | 3 | 3.8 | 2.4 | 1.4 | 3.6 | 2.6 | 1 |
| Plant Oil | Helianthus Annuus (Sunflower) Seed Oil | 1,2-heptanediol/ 1,2-octanediol (1:1) | 1.2 | 5 | 3.8 | 3.8 | 2.8 | 1 | 3.3 | 2.2 | 1.1 |
| Plant Oil | Helianthus Annuus (Sunflower) Seed Oil | 1,2-heptanediol/ 1,2-octanediol (95:5) | 1.2 | 4.2 | 3 | 3.8 | 2.8 | 1 | 3.3 | 2.4 | 0.9 |
| | | | | | | | | | | | |
| Plant Oil | Olive Oil | 1,2-heptanediol | 2.2 | 4 | 1.8 | 3.8 | 2 | 1.8 | 4 | 2.4 | 1.6 |
| Plant Oil | Olive Oil | 2,3-heptanediol | 2.2 | 4 | 1.8 | 3.8 | 2.2 | 1.6 | 4 | 2 | 2 |
| Plant Oil | Olive Oil | 1,2-heptanediol/ 2,3-heptanediol (95:5) | 2.2 | 3.8 | 1.6 | 3.8 | 1.8 | 2 | 4 | 2 | 2 |
| | | | | | | | | | | | |
| Plant Oil | Olive Oil | 1,2-octanediol | 2.2 | 4.2 | 2 | 3.8 | 2.6 | 1.2 | 4 | 2.6 | 1.4 |
| Plant Oil | Olive Oil | 2,3-octanediol | 2.4 | 3.2 | 0.8 | 3.8 | 2.4 | 1.4 | 4 | 2.6 | 1.4 |
| Plant Oil | Olive Oil | 1,2-octanediol/ 2,3-octanediol (95:5) | 2.2 | 4.4 | 2.2 | 3.8 | 2.2 | 1.6 | 4 | 2.4 | 1.6 |
| | | | | | | | | | | | |

(continued)

| | Liquid lipophilic component | alkanediol | Spreading | | | Fatty/greasy skin feel | | | Absorption/ remaining residue | | |
|---|---|---|---|---|---|---|---|---|---|---|---|
| | | | withou t | wit h | Delt a *) | withou t | wit h | Delt a *) | withou t | Wit h | Delt a *) |
| Plant Oil | Prunus Amygdalus Dulcis (Sweet Almond) Oil | 1,2-heptanediol | 1.3 | 3 | 1.7 | 3.3 | 2.5 | 0.8 | 3.7 | 2.2 | 1.5 |
| | | | | | | | | | | | |
| Plant Oil | Simmondsia Chinensis (Jojoba) Seed Oil | 1,2-heptanediol | 1.5 | 4 | 2.5 | 3.8 | 2.8 | 1 | 3.7 | 2.5 | 1.2 |
| | | | | | | | | | | | |
| Natura l oil | Microalgae oil | 1,2-heptanediol | 2.2 | 3.8 | 1.6 | 3.5 | 2.7 | 0.8 | 3.7 | 2.5 | 1.2 |
| | | | | | | | | | | | |
| | | | | | | | | | | | |
| Fatty alcoho l | Hexyldecanol (Eutanol G) | 1,2-heptanediol | 2.5 | 3.5 | 1 | 3 | 3 | 0 | 2.5 | 2.7 | 0.2 |
| | | | | | | | | | | | |
| Fatty ester | Cetearyl Nonanoate (SymMollient S) | 1,2-heptanediol | 1.7 | 4.2 | 2.5 | 3.2 | 2.5 | 0.7 | 3.2 | 2 | 1.2 |
| Fatty ester | Propanediol Dicaprylate/Dicaprat e (Symollient PDCC) | 1,2-heptanediol | 1.6 | 2.9 | 1.3 | 2.7 | 2.5 | 0.2 | 2.5 | 2.4 | 0.1 |
| | | | | | | | | | | | |
| UV filter | Ethylhexyl Methoxycinnamate (Neo Heliopan AV) | 1,2-heptanediol | 1.5 | 3.7 | 2.2 | 3.7 | 3.2 | 0.5 | 3.5 | 3 | 0.5 |
| UV filter | Ethylhexyl Salicylate (Neo Heliopan OS) | 1,2-heptanediol | 1.8 | 4.3 | 2.5 | 3.7 | 1.8 | 1.9 | 3.5 | 2.2 | 1.3 |
| UV filter | Homosalate (Neo Heliopan HMS) | 1,2-heptanediol | 2 | 2.8 | 0.8 | 2.7 | 3.2 | 0.5 | 2.8 | 2.5 | 0.3 |
| UV filter | Isoamyl p-Methoxycinnamate (Neo Heliopan E 1000) | 1,2-heptanediol | 1.5 | 2.7 | 1.2 | 4 | 2.7 | 1.3 | 4.2 | 2.5 | 1.7 |

(continued)

| | Liquid lipophilic component | alkanediol | Spreading | | | Fatty/greasy skin feel | | | Absorption/ remaining residue | | |
|---|---|---|---|---|---|---|---|---|---|---|---|
| | | | withou t | wit h | Delt a *) | withou t | wit h | Delt a *) | withou t | Wit h | Delt a *) |
| UV filter | Octocrylene (Neo Heliopan 303) | 1,2-heptanediol | 1 | 2.7 | 1.7 | 1.8 | 3.3 | 1.5 | 4 | 2.7 | 1.3 |
| | | | | | | | | | | | |
| Plant Oil | Caprylic Capric Triglyceride (CCT) (neutral oil) | 1,2-hexanediol | 1.8 | 3.6 | 1.8 | 4.6 | 3.2 | 1.4 | 4.6 | 2.8 | 1.8 |
| Plant Oil | Caprylic Capric Triglyceride (CCT) (neutral oil) | 2,3-hexanediol | 1.8 | 3.2 | 1.4 | 4.6 | 3.2 | 1.4 | 4.6 | 2.8 | 1.8 |
| Plant Oil | Caprylic Capric Triglyceride (CCT) (neutral oil) | 1,2-hexanediol/2,3-hexanediol (95:5) | 1.8 | 3.6 | 1.8 | 4.6 | 3.0 | 1.6 | 4.6 | 2.6 | 2.0 |
| | | | | | | | | | | | |
| Fatty ester | Cetearyl Nonanoate | 1,2-heptanediol | 2.2 | 4 | 1.8 | 4.8 | 3.4 | 1.4 | 4.8 | 3.2 | 1.6 |
| Fatty ester | Cetearyl Nonanoate | 2,3-heptanediol | 2.2 | 3.6 | 1.4 | 4.8 | 3.6 | 1.2 | 4.8 | 3.8 | 1.0 |
| | | | | | | | | | | | |
| | | | | | | | | | | | |
| Fatty ester | Octocrylene (Neo Heliopan 303) | 1,2-heptanediol | 1.0 | 3.5 | 2.5 | 4.0 | 3.5 | 0.5 | 4.0 | 3.5 | 0.5 |
| Fatty ester | Octocrylene (Neo Heliopan 303) | 2,3-heptanediol | 1.0 | 3.5 | 2.5 | 4.0 | 3.0 | 1.0 | 4.0 | 3.5 | 0.5 |
| Fatty ester | Octocrylene (Neo Heliopan 303) | 1,2-heptanediol/2,3 -heptanediol (95:5) | 1.0 | 4.0 | 3.0 | 4.0 | 3.0 | 1.0 | 4.0 | 2.5 | 1.5 |
| | | | | | | | | | | | |
| Fatty ester | Octocrylene (Neo Heliopan 303) | 1,2-octanediol | 1.0 | 3.5 | 2.5 | 4.0 | 3.5 | 0.5 | 4.0 | 3.5 | 0.5 |

(continued)

| | Liquid lipophilic component | alkanediol | Spreading | | | Fatty/greasy skin feel | | | Absorption/remaining residue | | |
|---|---|---|---|---|---|---|---|---|---|---|---|
| | | | withou t | wit h | Delt a *) | withou t | wit h | Delt a *) | withou t | Wit h | Delt a *) |
| Fatty ester | Octocrylene (Neo Heliopan 303) | 2,3-octanediol | 1.0 | 3.5 | 2.5 | 4 | 3.0 | 1.0 | 4.0 | 2.5 | 1.5 |
| Fatty ester | Octocrylene (Neo Heliopan 303) | 1,2-octanediol/2,3- | 1.0 | 4.5 | 3.5 | 4.0 | 2.5 | 1.5 | 4.0 | 2.5 | 1.5 |
| | | octanediol (95:5) | | | | | | | | | |

*) Higher delta values indicate better spreading properties, less fatty/greasy skin feel and less remaining residue on skin/ better absorption.

It was found that the use of 2,3-heptanediol or a mixture of 1,2-heptanediol and 2,3-heptanediol or 2,3-octanediol or a mixture of 1 ,2-octanediol and 2,3-octanediol improves the spreading behavior and/or significantly reduces the fatty/greasy skin feel of oily ingredients. In parallel, the absorption (less remaining residue) was improved too.

In particular liquid lipophilic components which have a poor spreading by nature, which initial spreading, i.e. without the addition of an alkanediol component, was scored with < 2, show a remarkably improvement in spreading both the tested single alkanediol compound and the tested alkanediol mixture. A mixture of 1,2-heptanediol and 2,3-heptanediol performed best.

**F) Formulation Examples**

The formulation examples below relate to the following applications:

- Deodorant Spray
- Deodorant Spray with AcH
- Deodorant Roll-on
- Cool Relief Deo Plus
- Sport Fit Deo
- Antiperspirant! deodorant roll-on
- Deodorant formulation in the form of a roll-on-gel
- Clear deo anti-perspirant roll-on
- Deodorant Aerosol
- Deodorant Stick
- Antiperspirant Stick
- Intimate Sensi-Gel
- Mild feminine wash
- Mosquito & tick repellent Spray
- Insect Repellent Spray, aqueous / ethanolic
- Insect Repellent solution for wipes
- Anti-Sweat Sunscreen Lotion with repellent
- Eau de Toilette/ Eau de parfume
- Syndet antimicrobial soap bar
- Syndet soap bar
- Ice Crystal Soap
- Antimicrobial toilet soap bar

The perfume oils PO1, PO2, PO3, PO4, or PO5 from the below examples were worked separately in each case into the formulations presented.

**Table 54:** Composition of perfume oil 1 (PO1, amounts in ‰ b.w.)

| Ingredients | Amount |
|---|---|
| ALDEHYDE C14 SO-CALLED | 2 |
| ALLYL AMYL GLYCOLATE 10% DPG | 5 |
| ANISIC ALDEHYDE PURE | 5 |
| APPLE OLIFFAC TYPE | 10 |
| BENZYLACETATE | 50 |
| BERGAMOT IDENTOIL® COLOURLESS | 15 |
| CANTHOXAL | 5 |
| CETALOX 10% IPM | 3 |
| CITRONELLOL 950 | 40 |
| DAMASCENONE TOTAL 1% DPG | 5 |
| DAMASCONE ALPHA 10% DPG | 5 |
| DAMASCONE DELTA 10% DPG | 2 |

(continued)

| Ingredients | Amount |
|---|---|
| DIMETHYL BENZYL CARBINYL BUTYRATE | 2 |
| DIPROPYLENE GLYCOL | 178 |
| EBANOL | 2 |
| ETHYL DECADIENOATE TRANS CIS-2,4 10% IPM | 2 |
| FLOROSA | 5 |
| FRAMBINON® 10% DPG | 7 |
| GALAXOLIDE 50% IN IPM | 100 |
| GALBEX TYPE BASE | 1 |
| GERANYL ACETATE PURE | 2 |
| HEDIONE | 30 |
| HELIOTROPIN | 10 |
| HEXENYL ACETATE CIS-3 10% DPG | 1 |
| HEXENYL SALICYLATE CIS-3 | 5 |
| HEXYL CINNAMIC ALDEHYDE ALPHA | 70 |
| HEXYL SALICYLATE | 50 |
| HYDROXY CITRONELLAL | 10 |
| ISO E SUPER | 15 |
| ISORALDEINE 70 | 20 |
| LEAFOVERT® | 1 |
| LILIAL | 60 |
| LINALOOL | 60 |
| LINALYL ACETATE | 20 |
| LYRAL | 7 |
| MANZANATE | 2 |
| PHENOXANOL | 7 |
| PHENYLETHYL ALCOHOL | 120 |
| SANDAL MYSORE CORE | 2 |
| SANDRANOL® | 7 |
| STYRALYL ACETATE | 3 |
| TAGETES RCO 10% TEC | 2 |
| TERPINEOL PURE | 20 |
| TETRAHYDROGERANIOL 10% DPG | 5 |
| TONALIDE | 7 |
| VERTOCITRAL 10% DPG | 5 |
| VERTOFIX | 15 |
| Total | 1000 |

**Table 55:** Composition of perfume oil 2 (PO2, amounts in ‰ b.w.)

| Ingredients | Amount |
|---|---|
| Acetophenone, 10% in DPG | 10 |
| n-Undecanal | 5 |
| Aldehyde C14, so-called (peach aldehyde) | 15 |
| Allylamyl glycolate, 10% in DPG | 20 |
| Amyl salicylate | 25 |
| Benzyl acetate | 60 |
| Citronellol | 80 |
| d-Limonene | 50 |
| Decenol trans-9 | 15 |
| Dihydromyrcenol | 50 |
| Dimethylbenzylcarbinyl acetate | 30 |
| Diphenyloxide | 5 |
| Eucalyptol | 10 |
| Geraniol | 40 |
| Nerol | 20 |
| Geranium oil | 15 |
| Hexenol cis-3, 10% in DPG | 5 |
| Hexenyl salicylate cis-3 | 20 |
| Indole, 10% in DPG | 10 |
| Alpha-ionone | 15 |
| Beta-ionone | 5 |
| Lilial® (2-methyl-3-(4-tert-butylphenyl)propanal) | 60 |
| Linalool | 40 |
| Methylphenyl acetate | 10 |
| Phenylethyl alcohol | 275 |
| Styrolyl acetate | 20 |
| Terpineol | 30 |
| Tetrahydrolinalool | 50 |
| Cinnamyl alcohol | 10 |
| Total: | 1000 |

**Table 56:** Composition of perfume oil 3 (PO3, amounts in ‰ b.w.)

| Ingredients | Amount |
|---|---|
| Benzyl acetate | 60 |
| Citronellyl acetate | 60 |
| Cyclamenaldehyde (2-methyl-3-(4-isopropylphenyl)propanal | 20 |
| Dipropylene glycol (DPG) | 60 |

(continued)

| Ingredients | Amount |
|---|---|
| Ethyllinalool | 40 |
| Florol (2-isobutyl-4-methyltetrahydro-2*H*-pyran-4-ol) | 30 |
| Globanone® [(E/Z)-8-cyclohexadecen-1-one] | 180 |
| Hedione® (methyldihydrojasmonate) | 140 |
| Hexenyl salicylate, cis-3 | 10 |
| Vertocitral (2,4-dimethyl-3-cyclohexenecarboxaldehyde) | 5 |
| Hydratropaldehyde, 10% in DPG | 5 |
| Isodamascone (1-(2,4,4-trimethyl-2-cyclohexen-1-yl)-2-buten- | 5 |
| Isomuscone (cyclohexadecanone) | 40 |
| Jacinthaflor (2-methyl-4-phenyl-1,3-dioxolane) | 10 |
| Cis-jasmone, 10% in DPG | 20 |
| Linalool | 50 |
| Linalyl acetate | 30 |
| Methyl benzoate, 10% in DPG | 25 |
| para-Methyl cresol, 10% in DPG | 10 |
| Nerol | 20 |
| Phenylpropylaldehyde | 5 |
| 2-Phenylethyl alcohol | 82 |
| Tetrahydrogeraniol | 13 |
| 2,2-Dimethyl-3-cyclohexyl-1-propanol | 80 |
| Total: | 1000 |

**Table 57:** Composition of perfume oil 4 (PO4, amounts in ‰ b.w.)

| Ingredients | Amount |
|---|---|
| AMBRETTOLIDE (MACRO) | 10 |
| AMBROXIDE 10% in IPM | 10 |
| BENZYL ACETATE | 20 |
| BENZYL SALICYLATE | 15 |
| BERGAMOT OIL. bergapten-free | 60 |
| CALONE® 1951 10% in DPG | 15 |
| COUMARIN | 5 |
| CYCLOGALBANATE® 10% in DPG | 10 |
| ALPHA -DAMASCONE 1% in DPG | 20 |
| DIHYDROMYRCENOL | 10 |
| ETHYL LINALOOL | 75 |
| ETHYL LINALYLACETATE | 50 |
| ETHYL MALTOL 1% in DEP | 10 |

(continued)

| Ingredients | Amount |
|---|---|
| ETHYLENE BRASSYLATE (MACRO) | 80 |
| FLOROSA | 40 |
| GERANYLACETATE | 10 |
| HEDIONE® HC/30 | 35 |
| HEDIONE® | 210 |
| HELIONAL® | 15 |
| HELVETOLIDE® (ALICYC) | 30 |
| HEXENYLSALICYLATE CIS-3 | 20 |
| ISO E SUPER® | 40 |
| LEAFOVERT® 10% in DEP | 10 |
| LILIAL® | 80 |
| LYRAL® | 20 |
| MANDARIN OIL | 10 |
| STYRALYL ACETATE | 5 |
| SYMROSE® | 15 |
| VANILLIN 10% in DEP | 20 |
| DIPROPYLENE GLYCOL (DPG) | 50 |
| TOTAL | 1000 |

**Table 58:** Composition of perfume oil 5 (PO5, amounts in ‰ b.w.)

| Ingredients | Amount |
|---|---|
| AMAROCITE® | 10 |
| AMBROCENIDE® 10% in DPG | 5 |
| AMBROXIDE | 15 |
| AURELIONE® (7/8-Cyclohexadecenone) (MACRO) | 70 |
| BERGAMOT OIL. bergapten-free | 90 |
| CALONE® 1951 10% in DPG | 20 |
| CARAWAY OIL | 10 |
| CITRAL | 20 |
| COUMARIN | 10 |
| ALPHA-DAMASCONE 1% in DPG | 15 |
| DIHYDROMYRCENOL | 70 |
| ESTRAGON OIL | 10 |
| ETHYL LINALOOL | 100 |
| ETHYL LINALYLACETATE | 90 |
| EUGENOL | 10 |

(continued)

| Ingredients | Amount |
|---|---|
| EVERNYL® | 5 |
| FRUCTATE® | 5 |
| GERANIUM OIL | 5 |
| HEDIONE® HC/30 | 100 |
| HELIONAL® | 10 |
| INDOLE 10% in DPG | 5 |
| ISO E SUPER® | 100 |
| KEPHALIS® | 5 |
| LAVENDER OIL | 40 |
| CITRUS OIL | 80 |
| LILIAL® | 30 |
| MANDARIN OIL | 20 |
| MUSCENONE (MACRO) | 5 |
| SANDRANOL® | 10 |
| VANILLIN 10% in DPG | 5 |
| DIPROPYLENE GLYCOL | 30 |
| TOTAL | 1000 |

Cosmetic formulations (compositions) - amounts are indicated as % by weight for all formulations.

**Table 59:** Deodorant Spray

| Ingredients | EU INCI | Amount |
|---|---|---|
| Aqua/Water | Aqua | ad 100 |
| Volarest™ FL | Acrylates/Beheneth-25 | 1.2 |
| Dracorin® GOC | Glyceryl oleate citrate; Caprylic/Capric triglyceride | 2.0 |
| SymDeo® Plus | Lauryl alcohol, Phenoxyethanol; 2-Benzylheptanol; Decylene Glycol | 0.5 |
| SymOcide® PS | Phenoxyethanol; Decylene Glycol; 1,2-Hexanediol | 0.8 |
| Dow Corning® 245 Fluid | Cyclomethicone | 5.0 |
| Isopropyl Myristate | Isopropyl myristate | 2.0 |
| Farnesol | Farnesol | 0.25 |
| Ethylhexylglycerin | Ethylhexylglycerin | 0.2 |
| SymDeo® B125 | 2-Methyl 5-Cycloheylpentanol | 0.15 |
| Tego® Cosmo P 813MB | Polyglyceryl-3 Caprylate | 0.25 |
| Perfume oil PO1, PO2, PO3, PO4, or PO5 | Fragrance | 0.8 |
| Frescolat® ML nat | Menthyl Lactate | 0.2 |
| Menthol | Menthol | 0.1 |

(continued)

| Ingredients | EU INCI | Amount |
|---|---|---|
| Sodium Hydroxide, 10% aqueous | Aqua; Sodium hydroxide | 0.6 |
| 1,2-heptanediol | 1,2-heptanediol | 0.5 |
| 2,3-heptanediol | 2,3-heptanediol | 0.02 |
| 1,2-nonanediol | 1,2-nonanediol | 0.2 |
| 2,3-nonandiol | 2,3-nonanediol | 0.01 |

**Table 60:** Deodorant Spray with AcH

| Ingredients | EU INCI | Amount |
|---|---|---|
| Triethylcitrate | Triethylcitrate | 3.0 |
| Menthol | Menthol | 0.1 |
| Solubilizer® | PEG-40 Hydrogenated Castor Oil, Trideceth-9, Propylene Glycol, Aqua | 1.0 |
| Ethanol 96% | Ethanol | 30.0 |
| 1,2-nonanediol | 1,2-nonanediol | 0.5 |
| 2,3-nonanediol | 2,3-nonanediol | 0.05 |
| 2,3-octanediol | 2,3-octanediol | 0.1 |
| Ethylhexylglycerin | Ethylhexylglycerin | 0.3 |
| SymDeo® B125 | 2-Methyl 5-Cycloheylpentanol | 0.25 |
| SymClariol® | Decylene Glycol | 0.25 |
| Frescolat® ML | Menthyl Lactate | 0.6 |
| Tego® Cosmo P 813MB | Polyglyceryl-3 Caprylate | 0.5 |
| Locron® L (50%) | Aqua, Aluminum Chlorohydrate | 25.0 |
| Perfume oil PO1, PO2, PO3, PO4, or PO5 | Parfum | 0.5 |
| Water, demin. | Aqua | ad 100 |

**Table 61:** Deodorant Roll-on

| Ingredients | EU INCI | Amount |
|---|---|---|
| Aqua/ Water | Aqua | ad 100 |
| Hydrolite® CG | Caprylyl Glycol | 0.3 |
| 1,2-heptanediol | 1,2-heptanediol | 1.0 |
| 2,3-heptanediol | 2,3-heptanediol | 0.05 |
| 1,2-nonanediol | 1,2-nonanediol | 0.1 |
| 2,3-nonandiol | 2,3-nonanediol | 0.05 |
| Menthol | Menthol | 0.1 |
| Frescolat® ML | Menthyl Lactate | 1.0 |
| SymSave® H | Hydroxyacetophenone | 0.5 |

(continued)

| Ingredients | EU INCI | Amount |
|---|---|---|
| EDTA NA2 | Disodium EDTA | 0.1 |
| Dracorin® GOC | Glyceryl oleate citrate; Caprylic/Capric triglyceride | 4.0 |
| Dragoxat® 89 | Ethylhexyl isononanoate | 5.0 |
| SymMollient® S | Cetearyl Nonanoate | 1.0 |
| Carbopol® Ultrez 20 Polymer | Acrylates/C10-30 Alkyl acrylate crosspolymer | 0.2 |
| Pemulen™ TR-2 Polymeric Emulsifier | Acrylates/C10-30 Alkyl acrylate crosspolymer | 0.2 |
| Farnesol | Farnesol | 0.3 |
| SymGuard® CD | Phenylpropanol, o-Cymen-5-ol, Decylene Glycol | 0.3 |
| Perfume oil PO1, PO2, PO3, PO4, or PO5 | Parfum | 0.5 |
| Sodium Hydroxide, 50% solution | Aqua; Sodium hydroxide | 0.2 |

**Table 62:** Cool Relief Deo Plus

| Ingredients | INCI | Amount |
|---|---|---|
| Aqua / Water | Aqua | ad 100 |
| Veegum® HS Granules | Magnesium Aluminium Silicate | 1.0 |
| Glycerin | Glycerin | 5.0 |
| EDTA NA2 | Disodium EDTA | 0.1 |
| Dracorin® 100 SEP | Glyceryl Stearate, PEG-100 Stearate | 6.0 |
| Lanette 16 | Cetyl alcohol | 2.0 |
| Eumulgin® B2 | Ceteareth-20 | 6.0 |
| Eutanol® G | Octyldodecanol | 8.0 |
| Dow Cornng FZ-3196 Volatile | Caprylyl Methicone | 4.0 |
| Tween 20 | Polysorbate20 | 0.3 |
| SymDeo® B125 | 2-Methyl 5-Cyclohexylpentanol | 0.3 |
| Dragosantol® 100 | Bisabolol | 0.05 |
| Aqua/Water | Aqua | 15.0 |
| Locron® P | Aluminium Chlorohydrate | 15.0 |
| 1,2-nonanediol / 2,3-nonanediol (95:5 w/w%) | 1,2-nonanediol/ 2,3-nonanediol | 1.0 |
| SymOcide® PS | Phenoxyethanol, Decylene Glycol, 1,2-Hexanediol | 1.0 |
| Frescolat® ML | Menthyl Lactate | 0.5 |
| Vitacel® CS020 | Cellulose | 1.0 |
| Extrapone® Rose Quartz GW | Aqua, Glycerin, Xanthan Gum, Quartz Powder | 1.0 |
| Perfume oil PO1, PO2, PO3, PO4, or PO5 | Parfum | 1.0 |
| Sodium hydroxide 10% solution | Aqua, Sodium hydroxide | 0.5 |

**Table 63:** Sport Fit Deo

| Ingredients | INCI | Amount |
|---|---|---|
| SymLite® G8 | Glyceryl caprylate | 0.15 |
| Menthol | Menthol | 0.05 |
| Ethanol | Alcohol denat | ad 100 |
| SymClariol® | Decylene Glycol | 0.25 |
| 1,2-heptanediol, 2,3-heptanediol (95:5 w/w%) | 1,2-heptanediol, 2, 3 Heptanediol | 0.5 |
| SymDeo® B125 | 2-Methyl 5-Cyclohexylpentanol | 0.25 |
| Frescolat® ML | Menthyl Lactate | 0.5 |
| Extrapone® Ginseng | Propylene Glycol, Aqua, Panax ginseng root extract, Lactic acid | 0.1 |
| Extrapone® Peppermint | Aqua, Propylene Glycol, Mentha Piperita Leaf extract | 0.1 |
| Ethylhexyglycerin | Ethylhexylglycerin | 0.2 |
| Perfume oil PO1, PO2, PO3, PO4, or PO5 | Parfum | 1.0 |

**Table 64:** Antiperspirant/deodorant roll-on

| Ingredients | INCI | Amount |
|---|---|---|
| SymLite® G8 | Glyceryl caprylate | 0.15 |
| Dragosantol® 100 | Bisabolol | 0.1 |
| 1,2-heptanediol | 1,2-heptanediol | 0.1 |
| 2,3-heptanediol | 2,3-heptanediol | 0.005 |
| 1,2-nonanediol | 1,2-nonanediol | 0.15 |
| 2,3-nonandiol | 2,3-nonanediol | 0.01 |
| Ethanol 96 % | Ethanol | 30.0 |
| Farnesol | Farnesol | 0.5 |
| Perfume oil PO1, PO2, PO3, PO4, or PO5 | Parfum | 1.5 |
| Frescolat®ML cryst. | Menthyl Lactate | 0.2 |
| Irgasan® DP 300 | Triclosan | 0.3 |
| Natrosol® 250 HHR | Hydroxyethyl-cellulose | 0.3 |
| Solubilizer 611674 | PEG-40 Hydrogenated Castor Oil, Trideceth-9, Water (Aqua) | 2.0 |
| SymDeo® B125 | 2-Methyl 5-Cyclohexylpentanol | 0.5 |
| Water (demineralized) | Water (Aqua) | ad 100 |
| Zirkonal L 450 | Aluminium Zirconium Pentachloro-hydrate (40 % aqueous solution) | 37.0 |

**Table 65:** Deodorant formulation in the form of a roll-on gel

| Ingredients | INCI | Amount |
|---|---|---|
| 1,3-butylene glycol | 1,3-butylene glycol | 2.0 |

(continued)

| Ingredients | INCI | Amount |
|---|---|---|
| Cremophor® CO 40 Surfactant | PEG-40-hydrogenated castor oil | 2.0 |
| Hydroxyethylcellulose | Hydroxyethylcellulose | 0.5 |
| Perfume oil PO1, PO2, PO3, PO4, or PO5 | Parfum | 0.3 |
| 1,3-propanediol | 1,3 Propanediol | 0.5 |
| SymGuard® CD | 3-Phenylpropanol, o-cymen-3-ol, Decylene glycol | 0.4 |
| Ethylhexylglycerin | Ethylhexylglycerin | 0.1 |
| 1,2-heptanediol | 1,2-heptanediol | 0.5 |
| 1,2-nonanediol, 2,3-nonanediol (95:5 w/w%) | 1,2-nonanediol, 2,3-nonanediol | 0.5 |
| SymLite® G8 | Glyceryl Caprylate | 0.15 |
| Frescolat®ML cryst. | Menthyl Lactate | 0.4 |
| Water | Aqua | ad 100 |

**Table 66:** Clear deo anti-perspirant roll-on

| Ingredients | INCI | Amount |
|---|---|---|
| Methocel™ E4M Premium | Hydroxypropyl Methylcellulose | 0.5 |
| Water | Water (Aqua) | ad 100 |
| Neo-PCL Water Soluble N | Trideceth-9, PEG-5 Ethylhexanoate, Water (Aqua) | 1.0 |
| Solubilizer | PEG-40 Hydrogenated Castor Oil, Trideceth-9, Propylene Glycol, Water (Aqua) | 3.0 |
| Deolite | Dimethyl Phenylpropanol, Pentylene Glycol | 0.5 |
| Locron® LW | Aluminium Chlorohydrate | 25.0 |
| Aloe Vera Gel Concentrate 10/1 | Aloe Barbadensis Leaf Juice | 1.0 |
| 1,2-Propylene Glycol 99 P GC | Propylene Glycol | 4.0 |
| Ethanol 96 % | Alcohol Denat. | 30.0 |
| Frescolat® ML | Menthyl Lactate | 1.5 |
| Perfume oil PO1, PO2, PO3, PO4, or PO5 | Parfum | 1.0 |
| 1,2-heptanediol, 2,3-heptanediol (w/w% 95:5) | 1,2-heptanediol, 2,3-heptanediol | 1.0 |

**Table 67:** Deodorant Aerosol

| Ingredients | EU INCI | Amount |
|---|---|---|
| Belsil® DM 0.65 | Disiloxane | ad 100 |
| Isoadipate | Diisopropyl adipate | 5.0 |
| Tegosoft® TN | C12-15 Alkyl benzoate | 10.0 |
| Vitamin E Acetate | Tocopheryl acetate | 0.5 |
| Farnesol | Farnesol | 0.3 |

(continued)

| Ingredients | EU INCI | Amount |
|---|---|---|
| Ethylhexylglycerin | Ethylhexylglycerin | 0.2 |
| 2,3-Heptanediol | 2,3-heptanediol | 0.1 |
| 1,2-nonanediol | 1,2-nonanediol | 0.2 |
| SymDeo® MPP | Dimethyl Phenylbutanol | 0.2 |
| Frescolat® ML nat | Menthyl Lactate | 0.25 |
| Perfume oil PO1, PO2, PO3, PO4, or PO5 | Parfum | 0.6 |

**Table 68:** Deodorant stick

| Ingredients | INCI | Amount |
|---|---|---|
| Sodium stearate | Sodium stearate | 8.0 |
| PPG-3 Myristyl ether | PPG-3 Myristyl ether | 70.0 |
| 1,2-propylene glycol | 1,2-propylene glycol | 10.0 |
| 1,1-dimethyl-3-phenylpropanol | 1,1-dimethyl-3-phenylpropanol | 0.2 |
| 2-butyloctanoic acid | 2-butyloctanoic acid | 0.2 |
| Perfume oil PO1, PO2, PO3, PO4, or PO5 | Parfume | 0.6 |
| Water | Aqua | ad 100 |
| Menthol | Menthol | 0.01 |
| SymDeo® Plus | Lauryl Alcohol, Phenoxyethanol, 2-Benzylheptanol, Decylene Glycol | 0.5 |
| 3-((-)-Menthoxy)propane-1,2-diol | 3-((-)-Menthoxy)propane-1,2-diol | 0.1 |
| N-Ethyl-p-menthane-3-carboxamide (Cooling Agent WS-3) | Ethyl Menthane Carboxamide | 0.3 |
| SymLite® G8 | Glyceryl Caprylate | 0.15 |
| 1,2-heptanediol | 1,2-heptanediol | 0.50 |

**Table 69:** Antiperspirant stick

| Ingredients | INCI | Amount |
|---|---|---|
| PCL-Liquid® 100 | Cetearyl ethylhexanonate | ad 100 |
| Silicone Fluid 345 | Cyclomethicone | 10.0 |
| CRODACOL™ C90 | Cetyl Alcohol | 8.0 |
| SYNCROWAX™ HGLC | C18-36 Triglyceride | 8.0 |
| CRODAMOL™ PTC | Pentaerythritol Tetracaprylate/Caprate | 5.0 |
| SYNCROWAX™ HRC | Tribehenin | 4.0 |
| Brij™ O5 | Oleth-5 | 1.0 |
| Titanium Dioxide | Titanium Dioxide | 1.0 |
| Menthol | Menthol | 0.05 |
| Frescolat® ML Nat | Menthyl Lactate | 0.75 |

(continued)

| Ingredients | INCI | Amount |
|---|---|---|
| Rezal 36GP | Aluminium Tetrachlorohydrex GLY | 20.0 |
| Dry-Flo® C | Aluminium Starch Octenyl Succinate | 22.5 |
| N-Ethyl-p-menthane-3-carboxamide (Cooling Agent WS-3) | Ethyl Menthane Carboxamide | 0.5 |
| Frescolat® MGA | Menthone Glycerin Acetal | 0.6 |
| Preservative | Phenoxyethanol | 0.8 |
| Perfume oil PO1, PO2, PO3, PO4, or PO5 | Parfume | 0.6 |
| 1,2-heptanediol, 2,3-heptanediol (95:5 w/w%) | 1,2-heptanediol, 2,3-heptanediol | 0.5 |
| 2,3-nonanediol | 2,3-nonanediol | 0.05 |

**Table 70:** Intimate Sensi-Gel

| Ingredients | EU INCI | Amount |
|---|---|---|
| Aqua/Water | Aqua | ad 100 |
| Sodium Benzoate | Sodium benzoate | 0.3 |
| Keltrol® CG-T | Xanthan Gum | 0.2 |
| Cyamopsis Tetragonolobus Gum | Cyamopsis Tetragonolobus Gum | 0.1 |
| Glycerin | Glycerin | 4.0 |
| Tego® Betain F50 | Cocamidopropyl betaine | 14.0 |
| Solubilizer® | PEG-40 Hydrogenated castor oil, Trideceth-9, Propylene Glycol, Aqua | 3.0 |
| 1,2-heptanediol | 1,2-heptanediol | 0.5 |
| Hydrolite® 6 | 1,2 Hexanediol | 1.0 |
| SymGuard® CD | Phenylpropanol, o-cymen-5-ol, Decylene Glycol | 0.3 |
| SymRelief® 100 | Bisabolol, Zingiber officinale root extract | 0.1 |
| Perfume oil PO1, PO2, PO3, PO4, or PO5 | Parfum | 0.2 |
| Frescolat® X-cool | Menthyl ethylamido oxalate | 0.4 |
| Frescolat® Plus | Menthol, Menthyl Lactate | 0.3 |
| Plantacare 2000 UP | Decyl Glucoside | 2.0 |
| Lactic acid 90% Nat. | Lactic Acid, Aqua | 0.3 |

**Table 71:** Mild feminine wash

| Ingredients | EU INCI | Amount |
|---|---|---|
| Aqua/Water | Aqua | ad 100 |
| SymSave® H | Hydroxyacetophenone | 0.5 |
| Keltrol CG-T | Xanthan Gum | 0.5 |
| Glycerin | Glycerin | 2.0 |
| Propylene Glycol | Propylene Glycol | 3.0 |

(continued)

| Ingredients | EU INCI | Amount |
|---|---|---|
| 1,2-heptanediol | 1,2-heptanediol | 0.5 |
| 2,3-Heptanediol | 2,3-Heptanediol | 0.03 |
| Tego Betain F50 | Cocamidopropyl betaine | 14.0 |
| SymGuard® CD | Phenylpropanol, o-cymen-5-ol, Decylene | 0.3 |
| Frescolat® ML nat | Menthyl Lactate | 0.2 |
| Plantacare® 2000 UP | Decyl Glucoside | 2.0 |
| Extrapone® Witch Hazel GW | Glycerin, Aqua, Hamamelis Virginiana Barkl-Leaf/Twig Extract | 1.0 |
| Solubilizer | PEG-40 Hydrogenated castor oil, Trideceth-9, Propylene Glycol, Aqua | 1.5 |
| Perfume oil PO1, PO2, PO3, | Parfum | 0.2 |
| SymCalmin® | Pentylene Glycol, Butylene Blycol, Hydroxyphenyl propamidobenzoic acid | 1.0 |
| Lactic acid 90% Nat. | Lactic Acid, Aqua | 0.32 |

**Table 72:** Mosquito & tick repellent Spray

| Tradename | INCI | w/w% |
|---|---|---|
| Ethanol | Ethanol | ad 100 |
| DEET | Diethyl Toluamide | 20.0 |
| Citriodiol | Cyclized Eucalyptus Citriodora | 3.0 |
| Perfume Oil PO1, PO2, PO3, PO4, | Fragrance | 0.2 |
| 1,2-heptanediol | 1,2-heptanediol | 1.5 |
| 2,3-heptanediol | 2,3-heptanediol | 0.02 |
| 1,2-octanediol | Caprylyl Glycol | 0.2 |
| 2,3-octanediol | 2.3-octanediol | 0.01 |

**Table 73:** Insect Repellent Spray, aqueous / ethanolic

| Tradename | INCI | w/w% |
|---|---|---|
| Ethanol | Ethanol | 60.0 |
| Water | Water (Aqua) | 20.0 |
| Perfume Oil PO1, PO2, PO3, PO4, or PO5 | Fragrance | 0.3 |
| Icaridin | Hydroxyethyl Isobutyl Piperidine Carboxylate | 20.0 |
| Eucalyptus Oil | Eucalyptus essential Oil | 0.3 |
| SymSol PF-3 | Water (Aqua). Sodium Lauryl Sulfoacetate. Pentylene Glycol. Sodium Oleoyl Sarcosinate. Sodium Chloride. Sodium | 1.5 |
| 1,2-heptanediol | 1.2-heptanediol | 1.0 |
| 2,3-heptanediol | 2.3-heptanediol | 0.01 |
| 1,2-octanediol | Caprylyl Glycol | 0.3 |

(continued)

| Tradename | INCI | w/w% |
|---|---|---|
| 2,3-octanediol | 2.3-octanediol | 0.05 |

**Table 74:** Insect Repellent Solution for Wipes

| Tradename | INCI | w/w% |
|---|---|---|
| Arlamol E | PPG-15 Stearylether | 3.0 |
| Polyethylenglycol 400 | PEG-8 | 5.0 |
| Ethanol | Ethanol | 35.0 |
| Glycerin | Glycerin | 4.0 |
| IR3535 | Etyhl Butylacetylaminopropionate | 20.0 |
| Citronellol | Citronellol | 0.5 |
| Perfume Oil PO1, PO2, PO3, PO4, | Fragrance | 0.3 |
| Solubilizer | Trideceth-9 PEG 40, Hydrogenated Castor Oil | 2.5 |
| Water | Water (Aqua) | ad 100 |
| 1,2-heptanediol | 1,2-heptanediol | 1.0 |
| 2,3-heptanediol | 2,3-heptanediol | 0.01 |
| 1,2-octanediol | Caprylyl Glycol | 0.3 |
| 2,3-octanediol | 2,3-octanediol | 0.05 |

**Table 75:** Anti-Sweat Sunscreen Lotion with repellent

| Raw material | INCI | Amoun |
|---|---|---|
| Aqua/Water | Aqua | ad 100 |
| 1,2-heptanediol | 1,2-heptanediol | 1.0 |
| 2,3-heptanediol | 2,3-heptanediol | 0.01 |
| 1,2-octanediol | Caprylyl Glycol | 0.3 |
| 2,3-octanediol | 2,3-octanediol | 0.05 |
| Citric Acid 30% Sol, | Aqua, Citric acid | 0.6 |
| Aristoflex® Velvet | Polyacrylate crosspolymer 11 | 0.6 |
| Glycerin 99,5 % | Glycerin, Aqua | 2.0 |
| SymSol® PF-3 | Aqua, Pentylene glycol, Sodium lauryl sulfoacetate, Sodium oleoyl sarcosinate, Sodium | 1.0 |
| SymSave® H | Hydroxyacetophenone | 0.3 |
| Neo Heliopan® 357 | Butyl methoxydibenzoylmethane | 3.0 |
| Neo Heliopan® 303 | Octocrylene | 10.0 |
| Neo Heliopan® OS | Ethylhexyl salicylate | 5.0 |
| Neo Heliopan® HMS | Homosalate | 10.0 |
| Neo Heliopan® E1000 | Isoamyl p-methoxycinnamate | 2.0 |
| Neo Heliopan® BMT | Bis-ethylhexyloxyphenol methoxyphenyl triazine | 3.0 |
| DEET | Diethyl Toluamide | 5.0 |
| Cpoconut Oil | Coconut Oil | 2.0 |
| Dragoxat® 89 | Ethylhexyl isononanoate | 6.0 |
| SymMollient® S | Cetearyl nonanoate | 1.0 |
| EDTA® BD | Disodium edta | 0.1 |
| SymDecanox™ HA | Caprylic/capric triglyceride Hydroxymethoxyphenyl decanone | 1.0 |
| Frescolat® Plus | Menthol, Menthyl lactate | 0.3 |
| Citriodiol | Cyclized Eucalyptus Citriodora Leaf/Twig Oil | 3.0 |
| N-Ethyl-p-menthane-3-carboxamide (Cooling Agent WS-3) | Ethyl Menthane Carboxamide | 0.2 |
| SymDeo® Plus | Lauryl alcohol, Phenoxyethanol, 2-benzylheptanol, Decylene glycol | 1.0 |
| Hydroviton® PLUS 2290 | Aqua, Pentylene glycol, Glycerin, Fructose, Urea, Citric acid, Sodium hydroxide, Maltose, Sodium pca, Sodium chloride, Sodium lactate, Trehalose, Allantoin, Sodium hyaluronate, Glucose | 4.0 |
| Sensocel® 10 | Cellulose | 2.0 |
| Perfume Oil PO1, PO2, PO3, PO4, or PO5 | Fragrance | 0.5 |

**Table 76:** Eau de Parfum/Eau de Toilette

| | | Eau de Parfum | Eau de Toilette |
|---|---|---|---|
| Raw material | INCI | Amount | Amount |
| Perfume Oil PO1, PO2, PO3, PO4, or | Fragrance | 20.0 | 10.0 |
| Ethanol | Ethanol | ad 100 | ad 100 |
| Water | Water (Aqua) | 10.0 | 20.0 |
| Solubilizer | Trideceth-9 PEG 40, Hydrogenated Castor Oil | 1.0 | 2.0 |
| 1,2-heptanediol | 1.2-heptanediol | 1.0 | 2.0 |
| 2,3-heptanediol | 2.3-heptanediol | 0.01 | 0.05 |
| 1,2-octanediol | Caprylyl Glycol | 0.3 | 0.1 |
| 2,3-octanediol | 2.3-octanediol | 0.05 | 0.05 |

**Table 77:** Syndet antimicrobial soap bar

| Ingredients | INCI | Amount |
|---|---|---|
| Zetesap 813 A | Disodium Lauryl Sulfosuccinate, Sodium Lauryl Sulfate, Corn Starch, Cetearyl Alcohol, Paraffin, Titanium Dioxide | ad 100 |
| Amphotensid GB 2009 | Disodium Cocoamphodiacetate | 6.0 |
| Allantoin | Allantoin | 1.0 |
| Perfume oil PO1; PO2; PO3; PO4 or PO5 | Fragrance | 1.0 |
| SymOcide C | o-cymen-5-ol | 0.1 |
| 1,2-heptanediol/2,3-heptanediol blend (95:5 w/w%) | 1,2-heptanediol/2,3-heptanediol blend (95:5 w/w%) | 5.0 |

**Table 78:** Syndet soap bar

| Ingredients | INCI | Amount |
|---|---|---|
| Fenopon AC-78 | Sodium Cocoyl Isethionate | 20.0 |
| Natrium laurylsulfoacetate | Sodium Lauryl Sulfoacetate | 16.0 |
| Paraffin | Paraffin | 19.0 |
| Wax. microcrystalline | Microcrystalline Wax | 1.0 |
| Corn Starch | Corn Starch | 8.0 |
| Coconut acid | Coconut acid | 2.0 |
| Lauric acid diethanol amide | Lauramide DEA | 2.0 |
| Dextrin | Dextrin | 21.0 |
| Lactic acid. 88% | Lactic Acid | 1.0 |
| SymGuard CD | 3-Phenylpropanol. o-cymen-5-ol. Decylene glycol | 0.3 |

(continued)

| Ingredients | INCI | Amount |
|---|---|---|
| Thymol | Thymol | 0.05 |
| Water | Water | ad 100 |
| Perfume oil PO1; PO2; PO3; PO4 or PO5 | Fragrance | 1.0 |
| 1,2-heptanediol/2,3-heptanediol blend (95:5 w/w%) | 1,2-heptanediol/2,3-heptanediol blend (95:5 w/w%) | 3.0 |

**Table 79:** Antimicrobial toilet soap bar

| Ingredients | Amount |
|---|---|
| Sodium soap from tallow | 60.0 |
| Sodium soap from palm oil | 27.0 |
| Glycerol | 2.0 |
| Sodium Chloride | 0.5 |
| 1-Hydroxyethane-1.1-diphosphonic acid. tetrasodium salt | 0.3 |
| Alpha-Tocopherol | 0.1 |
| Pigment Yellow 1 | 0.02 |
| Water | ad 100 |
| Perfume oil PO1; PO2; PO3; PO4 or PO5 | 3.0 |
| Farnesol | 0.2 |
| 2.3-heptanediol | 1.0 |
| 2.3-octanediol | 1.0 |

**Table 80:** Ice Crystal Soap

| Raw material | INCI | Amount |
|---|---|---|
| Crystal SLEA/SLS Free | Aqua, Glycerin, Sorbitol, Sodium stearate, Sodium Laurate, propylene Glycol, Sodium Oleate, Sodium Myristate, Sodium chloride, Glyceryl Laurate, Cocaidopropyl Betaine, Cocos Nucifera oil, Sodium Thiosulfate, Sodium Citrate, Citric Acid, Tetrasodium Iminodisuccinate, Tetrasodium Etidronate | ad 100 |
| Frescolat MGA | Menthone Glycerin Acetale | 2.0 |
| 1,2-heptanediol | 1,2-heptanediol | 0.5 |
| 2,3-nonanediol | 2,3-nonanediol | 0.05 |
| Menthol | Menthol | 0.05 |
| Extrapone Glacier Water GW | Glycerin, Aqua | 1.0 |

**Table 81:** Air freshener (A/F spray water based)

| No | Ingredients | Amount |
|---|---|---|
| 1 | Fragrance | 4 |
| 2 | Ethanol | 8 |

(continued)

| No | Ingredients | Amount |
|---|---|---|
| 3 | PEG-40 Hydrogenated Castor Oil | 5 |
| 4 | 1,2-heptanediol | 1 |
| 5 | Aqua | add to 100 |
| Colorless liquid, pH neutral | | |

**Table 82:** All purpose cleaner

| No | Ingredients | Amount |
|---|---|---|
| 1 | Fettalkoholethoxylat | 4 |
| 2 | Sulfonic acid, C 13-17-sec-Alkyl-, Sodium salt | 6.2 |
| 3 | 2-octyl-2H-isothiazol-3-one | 0.1 |
| 4 | Fragrance | 3.5 |
| 5 | 1,2-heptanediol | 1 |
| 6 | Aqua | add to 100 |
| Colorless liquid, pH 7 | | |

**Table 83:** All purpose cleaner

| No | Ingredients | Amount |
|---|---|---|
| 1 | Fettalkoholethoxylat | 4 |
| 2 | Sulfonic acid, C 13-17-sec-Alkyl-, Sodium salt | 6.2 |
| 3 | 2-octyl-2H-isothiazol-3-one | 0.1 |
| 4 | Fragrance | 3.5 |
| 5 | 2,3-octanediol | 0.1 |
| 6 | 1,2-heptanediol | 0.9 |
| 7 | Aqua | add to 100 |
| Colorless liquid, pH 7 | | |

**Table 84:** APC alkaline

| No | Ingredients | Amount |
|---|---|---|
| 1 | Coco-Glycoside | 1 |
| 2 | Benzalkonium Chloride | 0.2 |
| 3 | Sodiumbicarbonat | 0.1 |
| 4 | Tri-sodiumcitrate-dihydrate | 0.1 |
| 5 | Methylglycinediacetic acid | 0.1 |
| 6 | 1,2-Benzisothiazol-3(2H)-on | 0.1 |
| 7 | Fragrance | 0.1 |
| 8 | 1,2-heptanediol | 0.5 |

(continued)

| No | Ingredients | Amount |
|---|---|---|
| 9 | Aqua | add to 100 |
| Colorless liquid, pH 9 | | |

**Table 85:** Cleaner, liquid, citric acid

| No | Ingredients | Amount |
|---|---|---|
| 1 | Sulfonic acid, C 13-17-sec-Alkyl-, Sodium salt | 1 |
| 2 | Citric Acid | 5 |
| 3 | Xanthan Gum | 0.5 |
| 4 | 1,2-heptanediol | 0.5 |
| 5 | Fragrance | 0.3 |
| 6 | Aqua | add 100 |
| Liquid of low viscosity, pH 2 | | |

**Table 86:** Cleaner, liquid, citric acid

| No | Ingredient | Amount |
|---|---|---|
| 1 | Sulfonic acid, C 13-17-sec-Alkyl-, Sodium salt | 1 |
| 2 | Citric Acid | 5 |
| 3 | Xanthan Gum | 0.5 |
| 4 | 1,2-heptanediol/2,3 heptanediol 90 : 10 | 0.5 |
| 5 | Fragrance | 0.3 |
| 6 | Aqua | add 100 |
| Liquid of low viscosity, pH 2 | | |

**Table 87:** Detergent liquid light duty

| No | Ingredients | Amount |
|---|---|---|
| 1 | Cocos fatty acid | 0.85 |
| 2 | potassium hydroxide | 0.45 |
| 3 | 2-octyl-2H-isothiazol-3-one | 0.2 |
| 4 | Sulfonic acid, C 13-17-sec-Alkyl-, Sodium salt | 20 |
| 5 | Sodium Laureth Sulfate | 3 |
| 6 | Trideceth-9 | 5 |
| 7 | Sodium Chloride | 1.3 |
| 8 | 2,3-octanediol | 0.1 |
| 9 | 1,2-heptanediol | 0.9 |
| 10 | Fragrance | 0.5 |
| 11 | Aqua | add to 100 |
| Liquid, pH 7.3 | | |

**Table 88:** Detergent liquid light duty

| No | Ingredients | Amount |
|---|---|---|
| 1 | Cocos fatty acid | 0.85 |
| 2 | potassium hydroxide | 0.45 |
| 3 | 2-octyl-2H-isothiazol-3-one | 0.2 |
| 4 | Sulfonic acid, C 13-17-sec-Alkyl-, Sodium salt | 20 |
| 5 | Sodium Laureth Sulfate | 3 |
| 6 | Trideceth-9 | 5 |
| 7 | Sodium Chloride | 1.3 |
| 8 | 2,3-heptanediol | 0.1 |
| 9 | 1,2-heptanediol | 0.9 |
| 10 | Fragrance | 0.5 |
| 11 | Aqua | add to 100 |
| Liquid, pH 7.3 | | |

**Table 89:** Dishwash liquid manual

| No | Ingredients | Amount |
|---|---|---|
| 1 | Sodium Laureth Sulfate | add 100 |
| 2 | Ethanol | 2.9 |
| 3 | lauramine oxide | 7.7 |
| 4 | propylene glycol | 1.9 |
| 5 | Phenoxyethanol | 0.1 |
| 6 | Benzisothiazolinone, Methylisothiazolinone, Laurylamine Dipropylenediamine | 0.1 |
| 7 | 1,2-heptanediol | 0.5 |
| 8 | sodium chloride | 3.85 |
| 9 | Fragrance | 0.3 |
| Colorless liquid, pH 8.5 | | |

**Table 90:** Fabric softener

| No | Ingredients | Amount |
|---|---|---|
| 1 | Di-(Talg Carboxyethyl) Hydroxyethyl methylammonium-methosulfat | 5.5 |
| 2 | Simethicone | 0.3 |
| 3 | 2-octyl-2H-isothiazol-3-one | 0.1 |
| 4 | 1,2-heptanediol | 0.5 |
| 5 | Fragrance | 0.2 |
| 6 | Aqua | add 100 |
| Liquid, pH 3 | | |

**Table 91:** Fabric softener concentrate, encapsulated

| No | Ingredients | Amount |
|---|---|---|
| 1 | Di-(Talg Carboxyethyl) Hydroxyethyl methylammonium-methosulfat | 16.6 |
| 2 | 2-octyl-2H-isothiazol-3-one | 0.1 |
| 3 | Simethicone | 0.3 |
| 4 | magnesium chloride | 0.8 |
| 5 | encapsulated perfume oil | 0.3 |
| 6 | crosslinked cationic polymer | 0.15 |
| 7 | 1,2-heptanediol | 0.5 |
| 8 | Fragrance | 0.6 |
| 9 | Aqua | add 100 |
| White liquid, pH 2.5 | | |

**Table 92:** Fabric softener concentrate, encapsulated

| No | Ingredients | Amount |
|---|---|---|
| 1 | Di-(Talg Carboxyethyl) Hydroxyethyl methylammonium-methosulfat | 16.6 |
| 2 | 2-octyl-2H-isothiazol-3-one | 0.1 |
| 3 | Simethicone | 0.3 |
| 4 | magnesium chloride | 0.8 |
| 5 | encapsulated perfume oil | 0.3 |
| 6 | crosslinked cationic polymer | 0.15 |
| 7 | 2,3-heptanediol | 0.05 |
| 8 | 1,2-heptanediol | 0.5 |
| 9 | Fragrance | 0.6 |
| 10 | Aqua | add 100 |
| White liquid, pH 2.5 | | |

**Table 93:** Hand soap, liquid

| No | Ingredients | Amount |
|---|---|---|
| 1 | Sodium Laureth Sulfate | 18 |
| 2 | Cocoamidopropylbetaine | 6 |
| 3 | Cocamide DEA | 3 |
| 4 | citric acid | 0.5 |
| 5 | sodium chloride | 1.9 |
| 6 | Glycerol | 2 |
| 7 | Fragrance | 0.2 |
| 8 | 1,2-heptanediol | 0.25 |
| 9 | Aqua | add 100 |
| Slightly colored liquid, pH 6 | | |

**Table 94:** Rim block gel

| No | Ingredients | Amount |
|---|---|---|
| 1 | Sodium Laureth Sulfate | 11 |
| 2 | Trideceth-9 | 2 |
| 3 | Xanthan gum | 2 |
| 4 | Hydroxyethylcellulose | 0.5 |
| 5 | Citric Acid | 0.4 |
| 6 | Ethanol | 6 |
| 7 | Fragrance | 5 |
| 8 | 1,2-heptanediol | 0.5 |
| 9 | Aqua | add 100 |
| Colorless paste/gel, pH = 4.5 | | |

**Table 95:** Scent Lotion with capsules

| No | Ingredients | Amount |
|---|---|---|
| 1 | microcrystalline cellulose/cellulose gum | 1 |
| 2 | PEG-40 hydrogenated Castor oil/ Trideceth-9 | 0.5 |
| 3 | encapsulated perfume oil | 1 |
| 4 | 2-octyl-2H-isothiazol-3-one | 0.1 |
| 5 | Fragrance | 5 |
| 6 | 1,2-heptanediol | 1 |
| 7 | Aqua | add 100 |
| White emulsion, pH neutral | | |

**Table 96:** Cleaner, liquid, lactic acid

| No | Ingredients | Amount |
|---|---|---|
| 1 | Sodium Laureth Sulfate | 1.8 |
| 2 | Trideceth-9 | 2.1 |
| 3 | Xanthan gum | 0.35 |
| 4 | Lactic Acid | 2,8 |
| 5 | 1,2-heptanediol | 1 |
| 6 | Fragrance | 0.3 |
| 7 | Aqua | add 100 |
| Colorless liquid, pH 2,0 | | |

**Table 97:** Cleaner, liquid, citric acid

| No | Ingredients | Amount |
|---|---|---|
| 1 | Sulfonic acid, C 13-17-sec-Alkyl-, Sodium salt | 1 |

(continued)

| No | Ingredients | Amount |
|---|---|---|
| 2 | Xanthan gum | 0-3 |
| 3 | citric acid | 5 |
| 4 | 1,2heptanediol | 0.25 |
| 5 | Fragrance | 0.3 |
| 6 | Aqua | add 100 |
| Liquid, pH 2 | | |

## Claims

**1.** A cosmetic or pharmaceutical composition, comprising:

(a) at least one linear 1,2-alkanediol having a carbon chain of 5 to 13 carbon atoms; and
(b) at least one linear 2,3-alkanediol having a carbon chain of 5 to 13 carbon atoms;

or

a cosmetic or pharmaceutical composition, comprising:

(a) at least one linear 2,3-alkanediol having a carbon chain of 5 to 13 carbon atoms;

with the proviso that the cosmetic or pharmaceutical composition does not comprise
a cooling agent, or
an antioxidant.

**2.** A composition, comprising:

(a) at least one linear 1,2-alkanediol having a carbon chain of 5 to 13 carbon atoms; and
(b) at least one linear 2,3-alkanediol having a carbon chain of 5 to 13 carbon atoms;

or

a composition, comprising:

(a) at least one linear 2,3-alkanediol having a carbon chain of 5 to 13 carbon atoms;

with the proviso that the composition does not comprise
a cooling agent, or
an antioxidant.

**3.** A cosmetic or pharmaceutical composition, consisting of:

(a) at least one linear 1,2-alkanediol having a carbon chain of 5 to 13 carbon atoms; and
(b) at least one linear 2,3-alkanediol having a carbon chain of 5 to 13 carbon atoms.

**4.** A composition, consisting of:

(a) at least one linear 1,2-alkanediol having a carbon chain of 5 to 13 carbon atoms; and
(b) at least one linear 2,3-alkanediol having a carbon chain of 5 to 13 carbon atoms.

**5.** The composition according to any one of claims 1 to 4, wherein

i.component (a) is 1,2-pentanediol and component (b) is 2,3-pentanediol, or
ii.component (a) is 1,2-hexanediol and component (b) is 2,3-hexanediol, or
iii.component (a) is 1,2-heptanediol and component (b) is 2,3-heptanediol, or
iv.component (a) is 1,2-octanediol and component (b) is 2,3-octanediol, or
v.component (a) is 1,2-nonanediol and component (b) is 2,3-nonanediol, or
vi.component (a) is 1,2-decanediol and component (b) is 2,3-decanediol, or
vii.component (a) is 1,2-undecanediol and component (b) is 2,3-undecanediol, or
viii. component (a) is 1,2-dodecanediol and component (b) is 2,3-dodecanediol, or
ix.component (a) is 1,2-tridecanediol and component (b) is 2,3-tridecanediol.

**6.** The composition according any one of claims 1 to 5, wherein

i.component (a) is 1,2-heptanediol and component (b) is selected from the group consisting of 2,3-octanediol and 2,3-nonanediol, or
ii.component (a) is 1,2-octanediol and component (b) is selected from the group consisting of 2,3-heptanediol and 2,3-nonanediol, or
iii.component (a) is 1,2-nonanediol and component (b) is selected from the group consisting of 2,3-heptanediol and 2,3-octanediol.

**7.** The composition according to any one of claims 1 to 6, wherein the weight ratio of the compounds (a) and (b) is in a range of 50 : 50 to 99.9 : 0.1, preferably wherein the weight ratio of the compounds (a) and (b) is in a range of 75 : 25 to 99 : 1, more preferred wherein the weight ratio of the compounds (a) and (b) is in a range of 80 : 20 to 98 : 2, still more preferred wherein the weight ratio of the compounds (a) and (b) is in a range of 90 : 10 to 95 : 5.

**8.** The composition according to any one of claims 1, 2 and 5 to 7, comprising the component (a) and component (b) in an amount of 0.001 to 15.0 % by weight, particularly in an amount of 0.01 to 10.0 % by weight, more preferred in an amount of 0.1 to 5.0 % by weight, still more preferred in an amount of 0.3 to 3.0 % by weight, most preferred in an amount of 0.5 to 1.0 % by weight, based on the total weight of the composition.

**9.** The composition according to any one of claims 1, 2 and 5 to 8, further comprising:
(c) at least one or more insect repellent compounds, in particular an insect repellent compound selected from the group consisting of: 1-(1-Methylpropoxycarbonyl)-2-(2-hydroxyethyl)piperidine, N,N-Diethyl-meta-toluamide, p-Menthane-3,8-diol, ethyl butylacetylaminopropionate, 2-undecanone, as well as essential oils, such as citronella oil, lemongrass oil, lavender oil, neem oil and eucalyptus oil, in particular p-Menthane-3,8-diol, essential oils, such as citronella oil, lemongrass oil, lavender oil, neem oil and eucalyptus oil.

**10.** The composition according to claim 9, comprising the component (c) in an amount of 0.1 to 50.0 % by weight, particularly in an amount of 0.5 to 45.0 % by weight, more particularly in an amount of 1.0 to 40 % by weight, most particularly in an amount of 2.0 to 25.0 % by weight, based on the total weight of the composition.

**11.** The composition according to any one of claims 1, 2 and 5 to 10, further comprising:
(d) at least one cosmetically or pharmaceutically active substance and/or additive.

**12.** The composition according to claim 11, wherein the at least one cosmetically or pharmaceutically active substance and/or additive is selected from the group consisting of antimicrobial compounds, cooling agents, agents against ageing of the skin, antioxidants, chelating agents, emulsifiers, preservatives, green and synthetic polymers, rheology additives, oils, fragrances or perfume oils, polyols and mixtures of two or more of the afore-mentioned substances.

**13.** The composition according to any one of claims 2, and 4 to 12, wherein the composition is a cosmetic or pharmaceutical composition or homecare product.

**14.** Use of the composition according to any one of claims 1 to 13 for enhancing the antioxidative effect of an antioxidant, for improving the sensory properties, i.e. skin sensation, of a liquid lipophilic component, for sebum control, or for malodour management, or for modulating fragrance notes, or for modification of fragrance notes, or for suppression of fragrance notes, or for topical applications, or for coverage of the malodour of 1-octen-3-ol, or for masking insect alluring odours, or for repelling insects.

**15.** Cosmetic product comprising a composition according to any one of claims 1 to 13, wherein the product is selected

from the group consisting of a deodorant and/or antiperspirant, an aerosol-based deo spray, deo pump spray, deo stick, deo roll on, deo cream, deo wipes, deo crystals, pickering emulsions, hydrodispersion gels, skin balms, shampoo, shower gel, foam bath, micellar water, facial cleansing solutions, cleansing wipes, intimate spray, intimate cream, intimate wash lotion, intimate wipes, aerosol-based foot spray, foot pump spray, foot bath, foot balm, soap, liquid washing, shower and bath preparation, bath product, bath capsule, bath oil, bath tablet, bath salt, bath soap, effervescent preparation, mouth wash concentrate, mouth wash ready to use, repellent, antiinsect application, in particular insect repellent lotion, spray, solution or cream, mosquito repellent, human-smell-masking application, perfume composition and tooth paste.

**16.** Pharmaceutical comprising a composition according to any one of claims 1 to 13, for use in the prevention and treatment of a condition of the skin or mucosa.

**17.** Homecare product comprising a composition according to any one of claims 1 to 13, wherein the product is selected from the group consisting of all-purpose cleaners, dishwashing detergents, hard floor and surface cleaners, glass cleaners, carpet cleaners, oven cleaners, laundry detergents, fabric softeners, laundry scent, scent lotions, car shampoo, rim block gel, car shampoo, furniture polish, and air fresheners, all afore-mentioned goods also in encapsulated form.

Figure 1

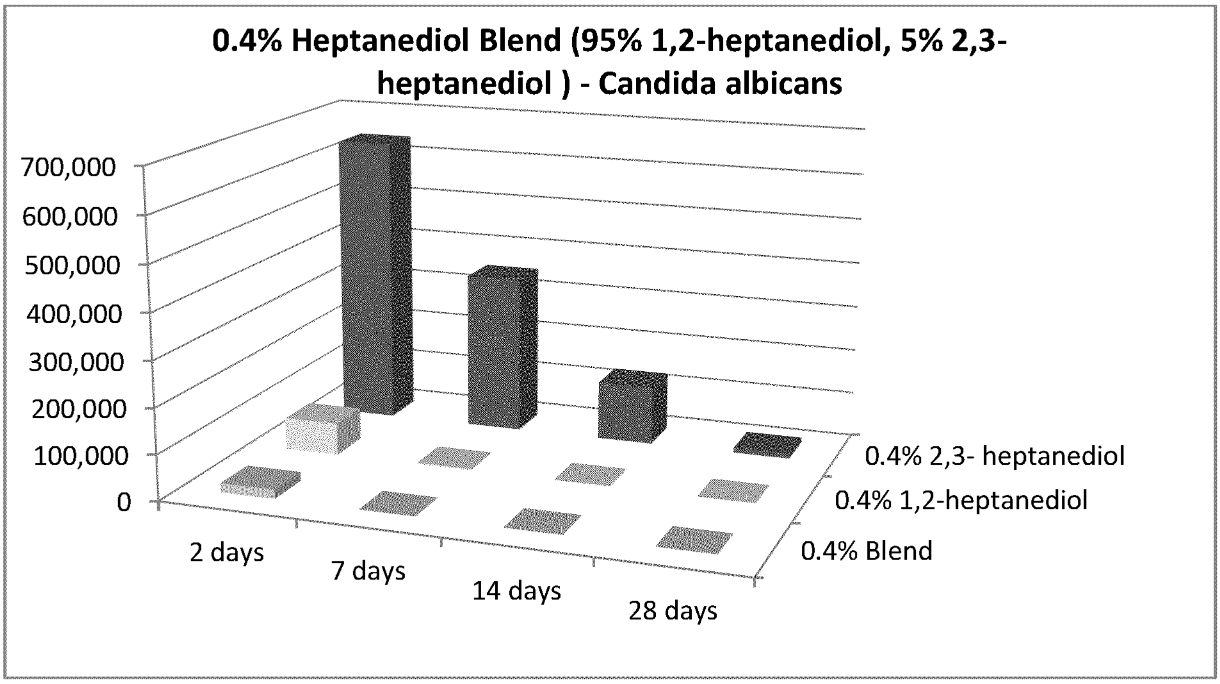
0.4% Heptanediol Blend (95% 1,2-heptanediol, 5% 2,3-heptanediol ) - Candida albicans
700,000
600,000
500,000
400,000
300,000
200,000
100,000
0
2 days
7 days
14 days
28 days
0.4% 2,3- heptanediol
0.4% 1,2-heptanediol
0.4% Blend

Figure 2a

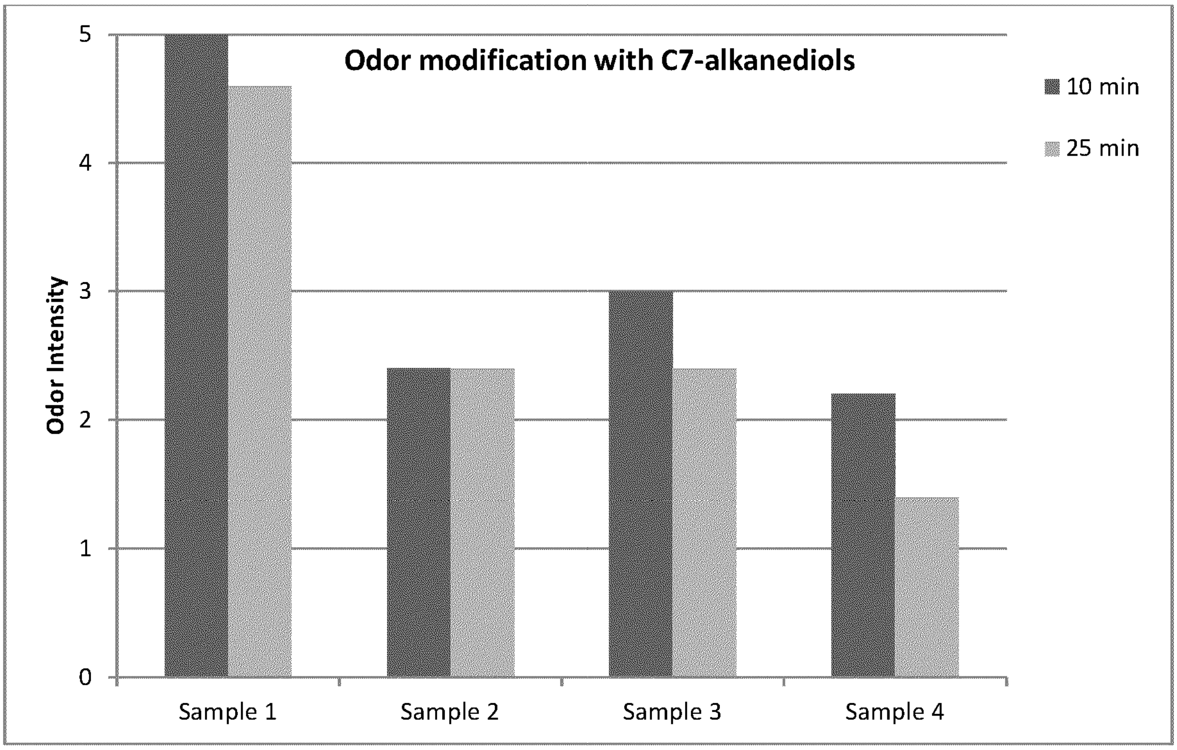


Figure 2b

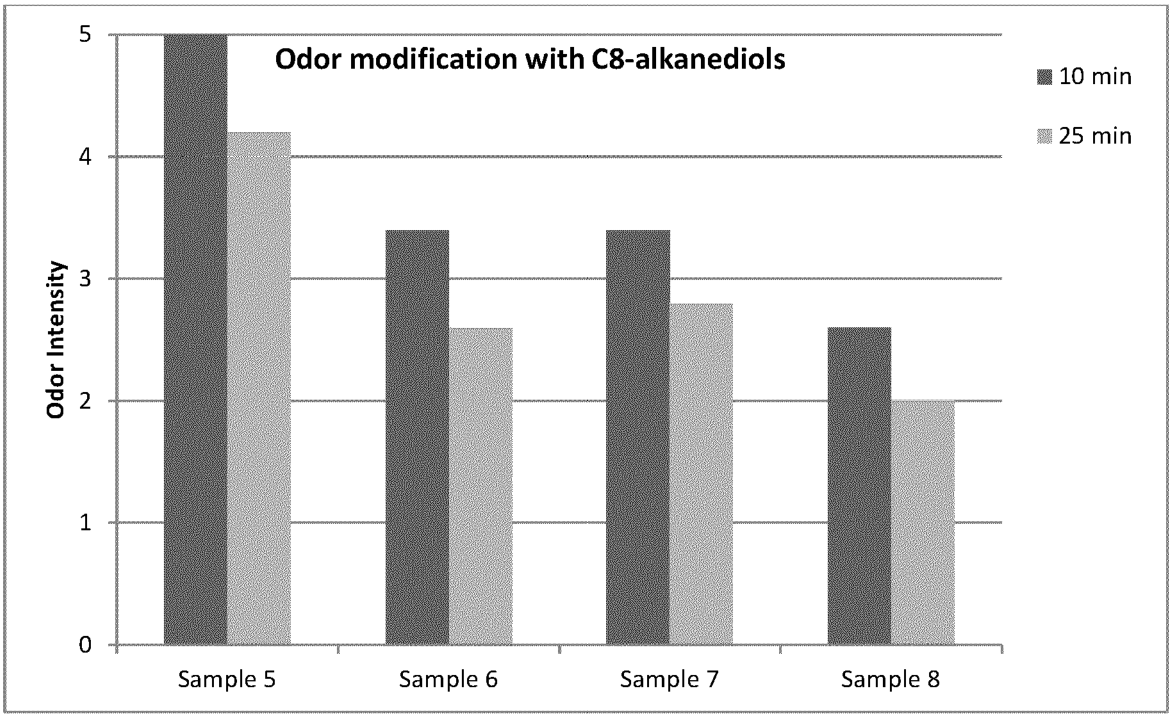

Figure 3

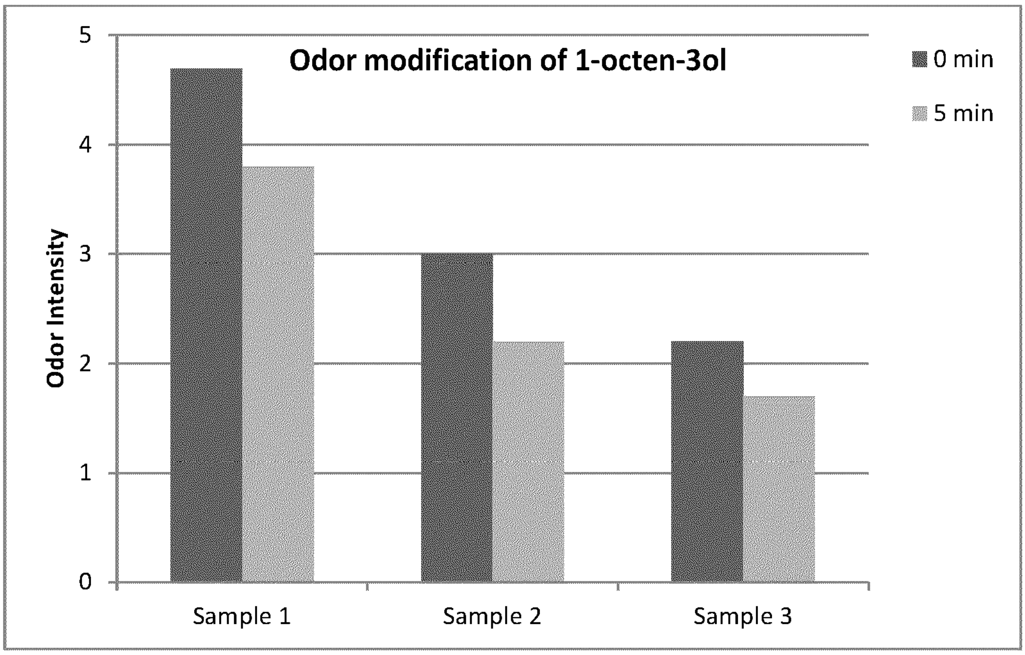
Odor modification of 1-octen-3ol
0 min
5 min
Odor Intensity
5
4
3
2
1
0
Sample 1
Sample 2
Sample 3

Figure 4

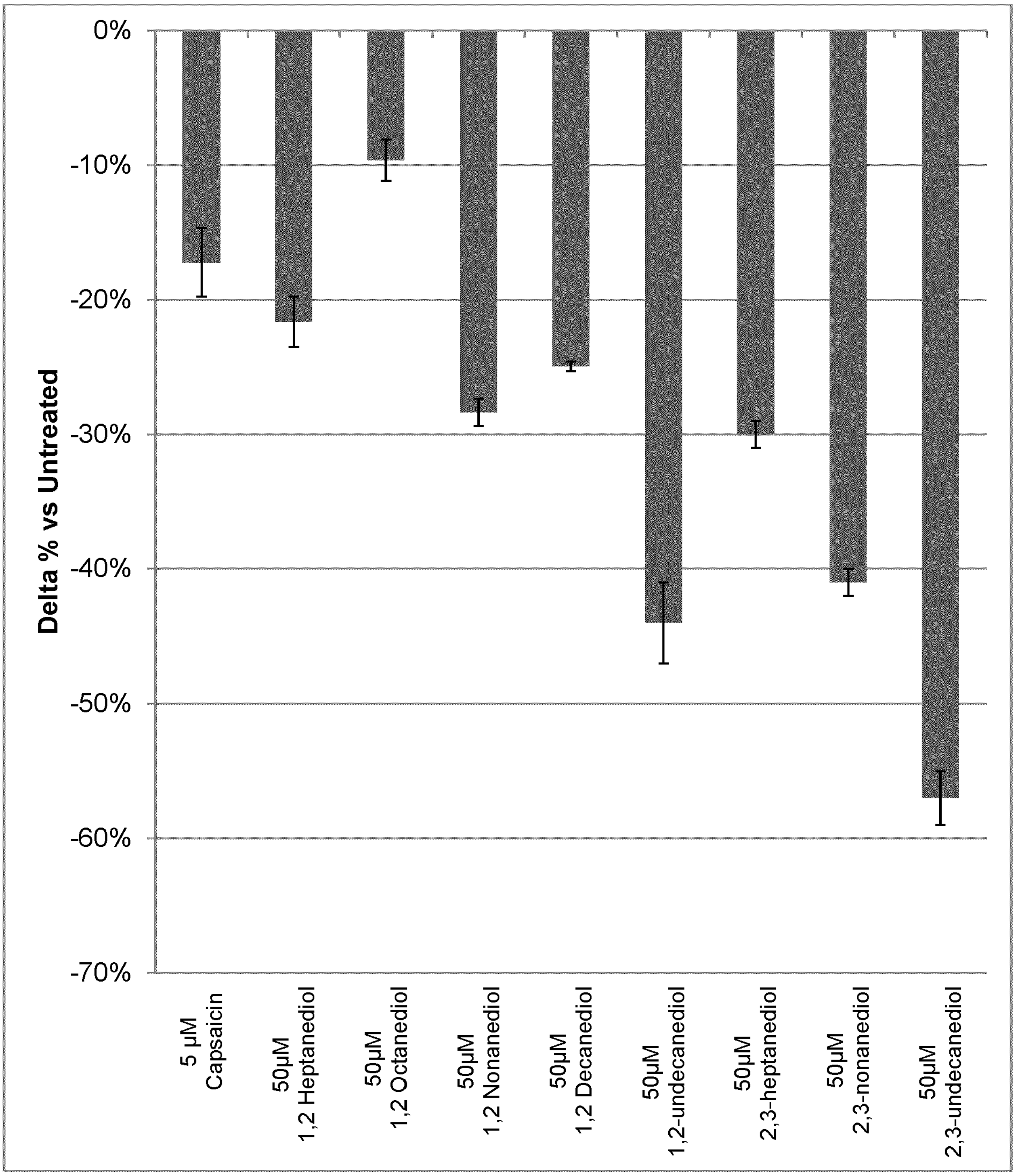
0%
-10%
-20%
-30%
-40%
-50%
-60%
-70%
Delta % vs Untreated
5 µM Capsaicin
50µM 1,2 Heptanediol
50µM 1,2 Octanediol
50µM 1,2 Nonanediol
50µM 1,2 Decanediol
50µM 1,2-undecanediol
50µM 2,3-heptanediol
50µM 2,3-nonanediol
50µM 2,3-undecanediol

Figure 5

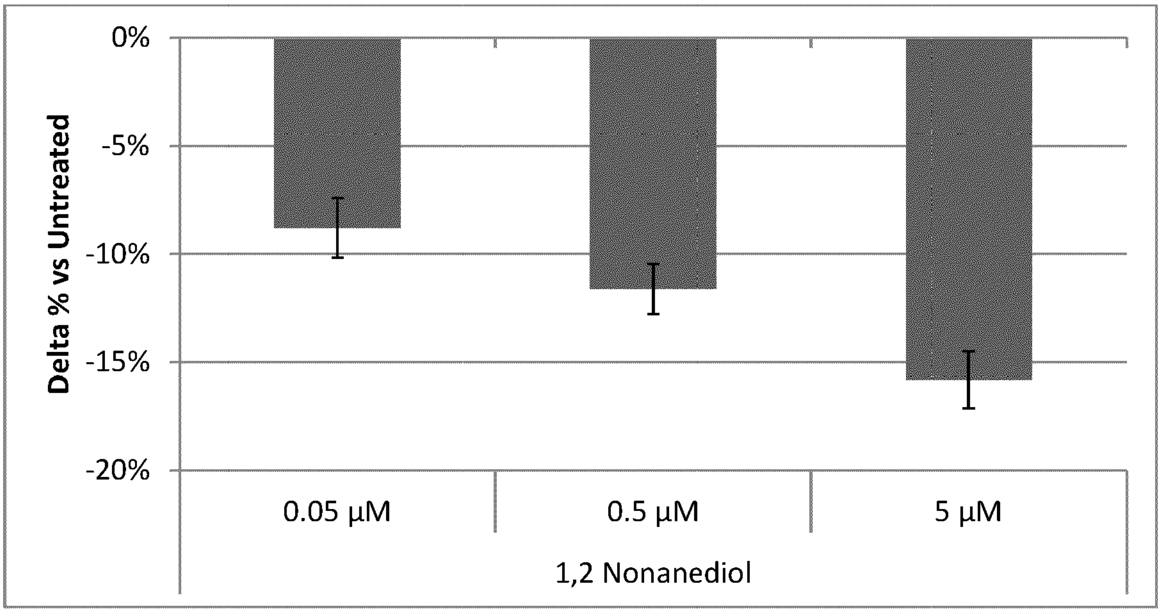

Figure 6a

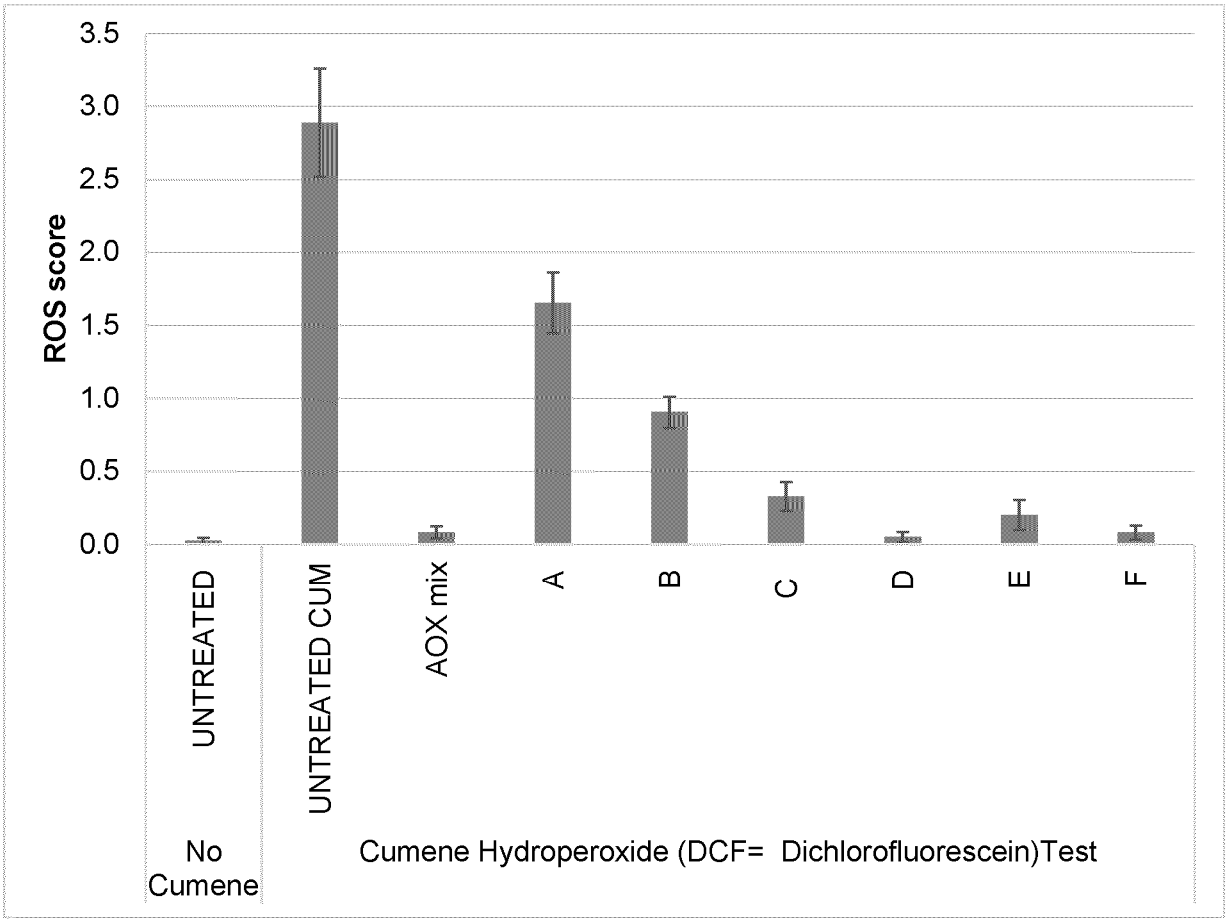

ROS score
3.5
3.0
2.5
2.0
1.5
1.0
0.5
0.0
UNTREATED
UNTREATED CUM
AOX mix
A
B
C
D
E
F
No Cumene
Cumene Hydroperoxide (DCF= Dichlorofluorescein)Test

Figure 6b

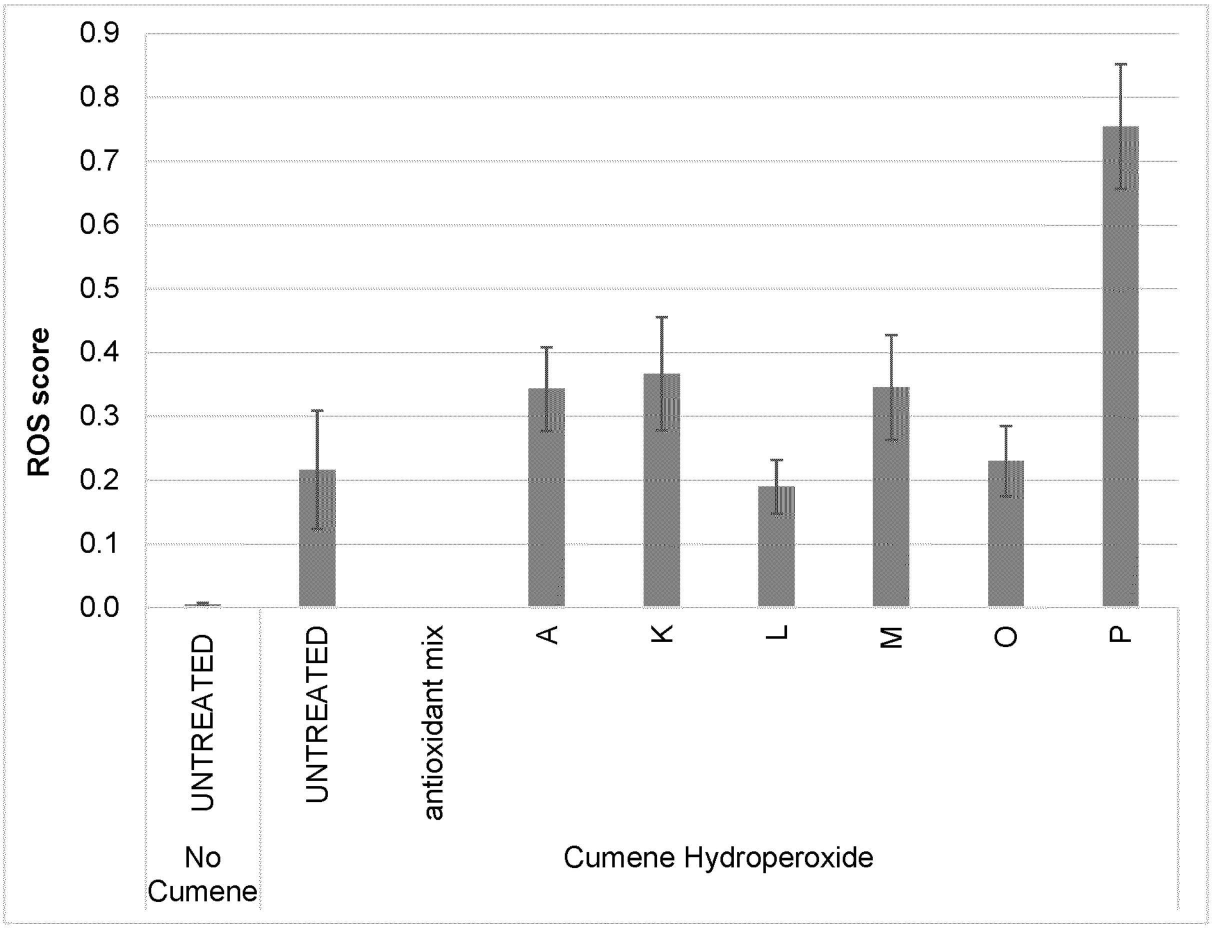
ROS score
0.9
0.8
0.7
0.6
0.5
0.4
0.3
0.2
0.1
0.0
UNTREATED
UNTREATED
antioxidant mix
A
K
L
M
O
P
No Cumene
Cumene Hydroperoxide

Figure 7a

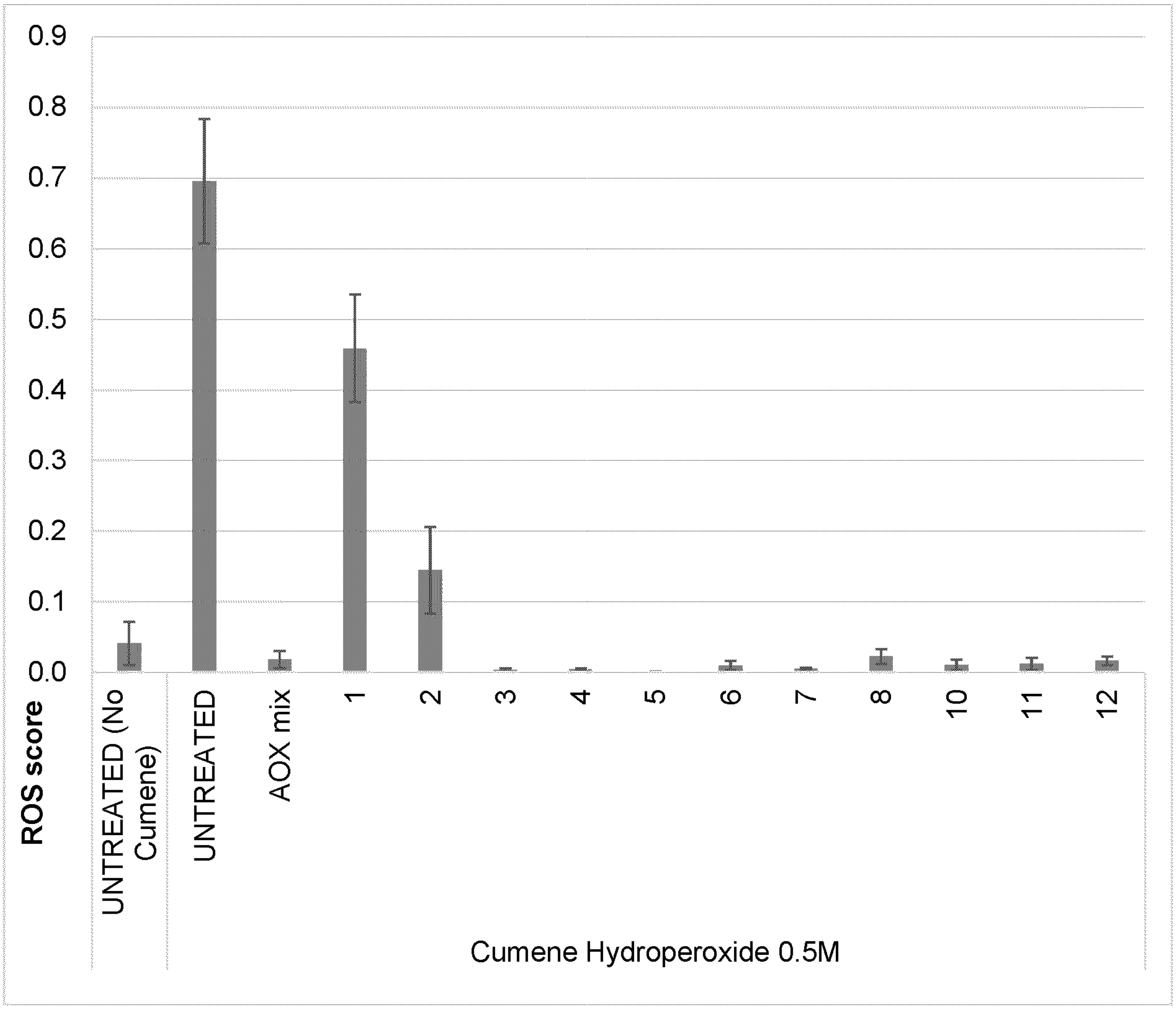
0.9
0.8
0.7
0.6
0.5
0.4
0.3
0.2
0.1
0.0
ROS score
UNTREATED (No Cumene)
UNTREATED
AOX mix
1
2
3
4
5
6
7
8
10
11
12
Cumene Hydroperoxide 0.5M

Figure 7b

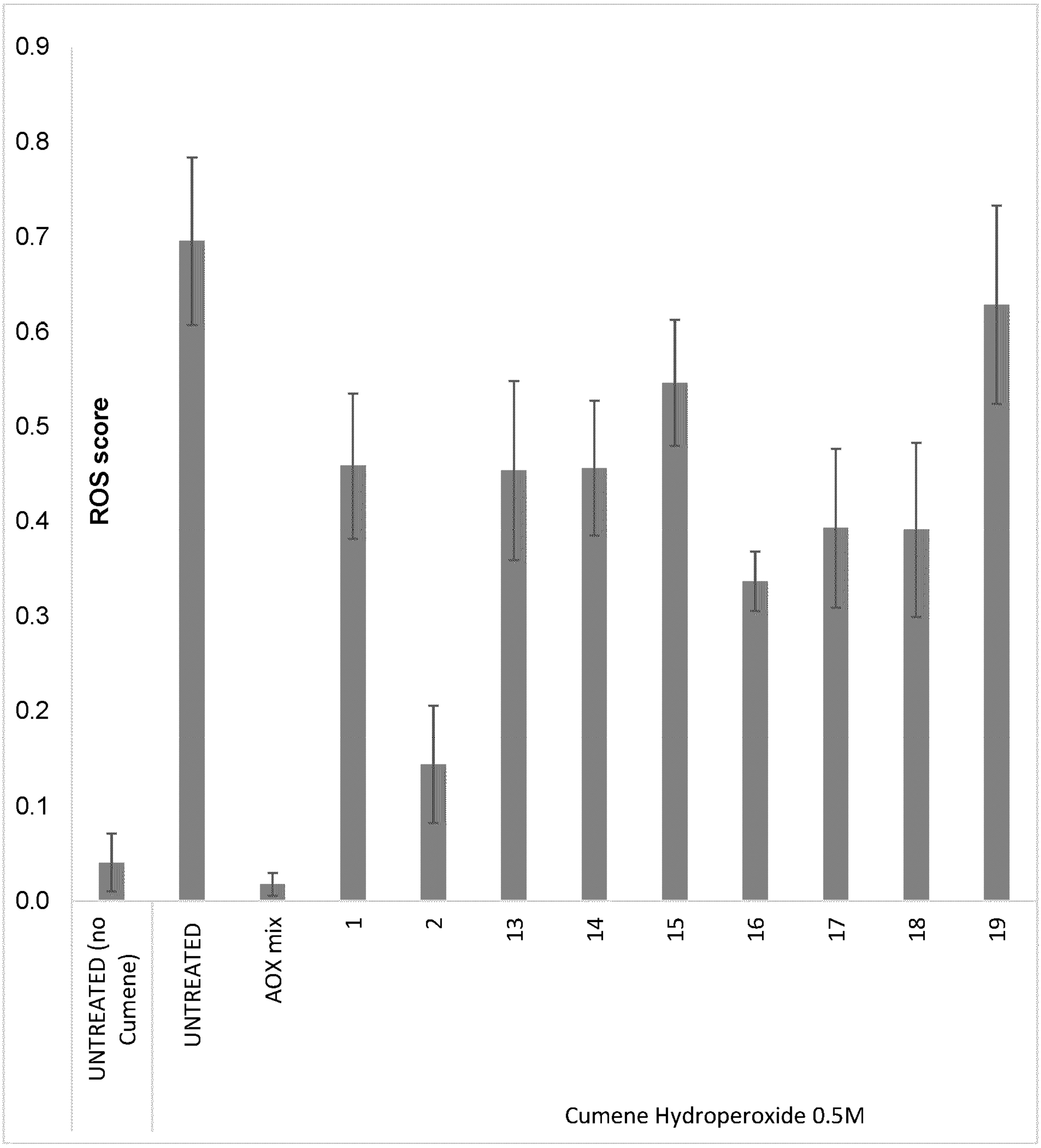
0.9
0.8
0.7
0.6
0.5
0.4
0.3
0.2
0.1
0.0
ROS score
UNTREATED (no Cumene)
UNTREATED
AOX mix
1
2
13
14
15
16
17
18
19
Cumene Hydroperoxide 0.5M

Figure 8

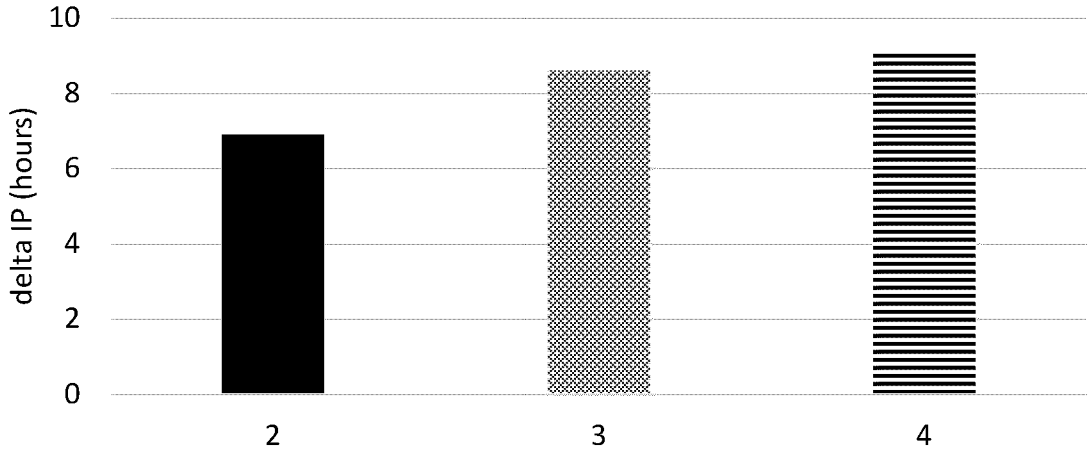


Figure 9

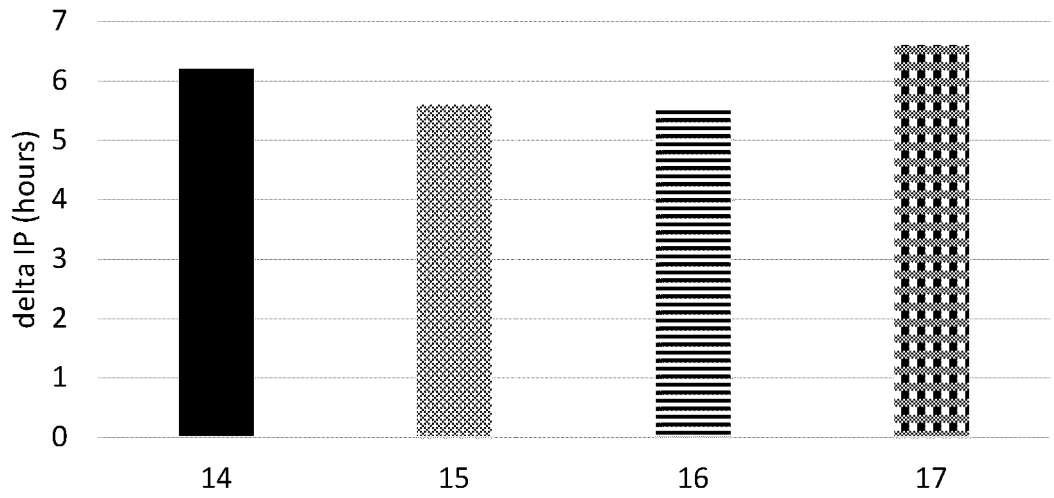

Figure 10

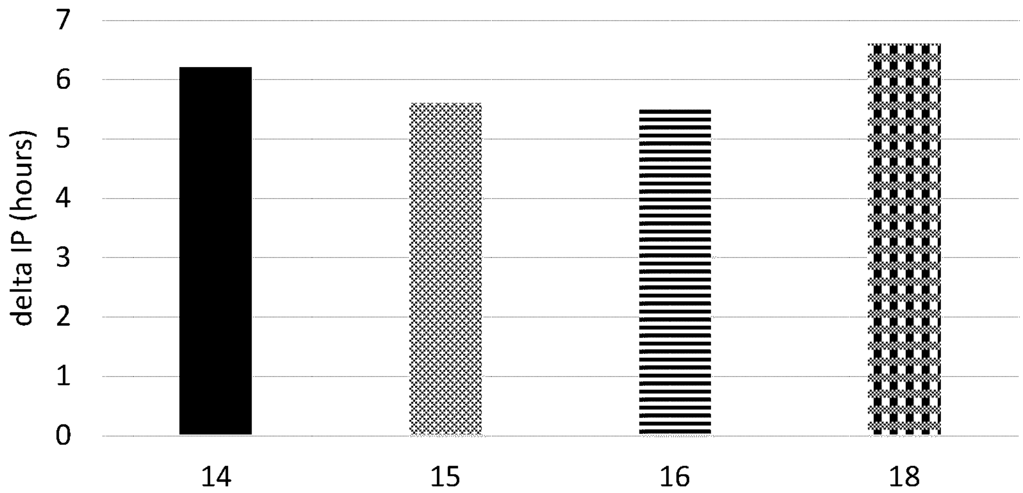


Figure 11

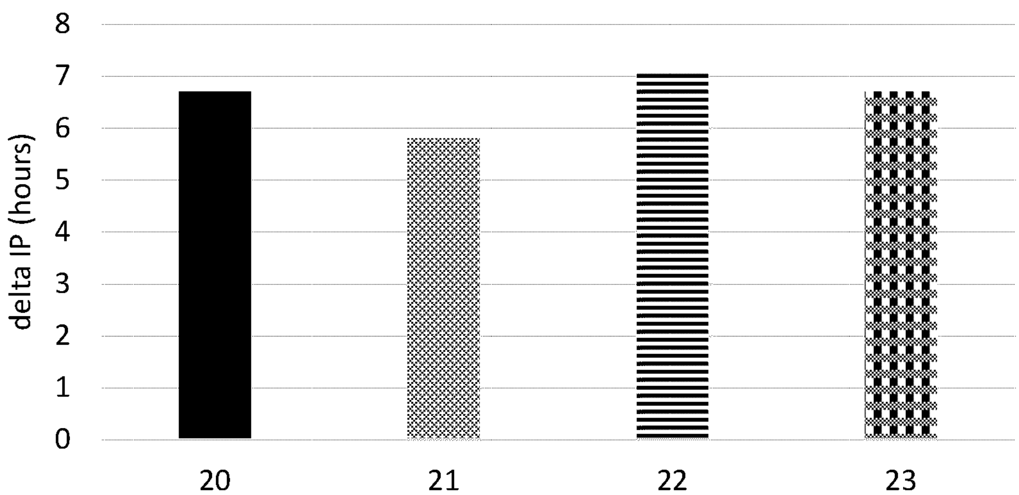

Figure 12

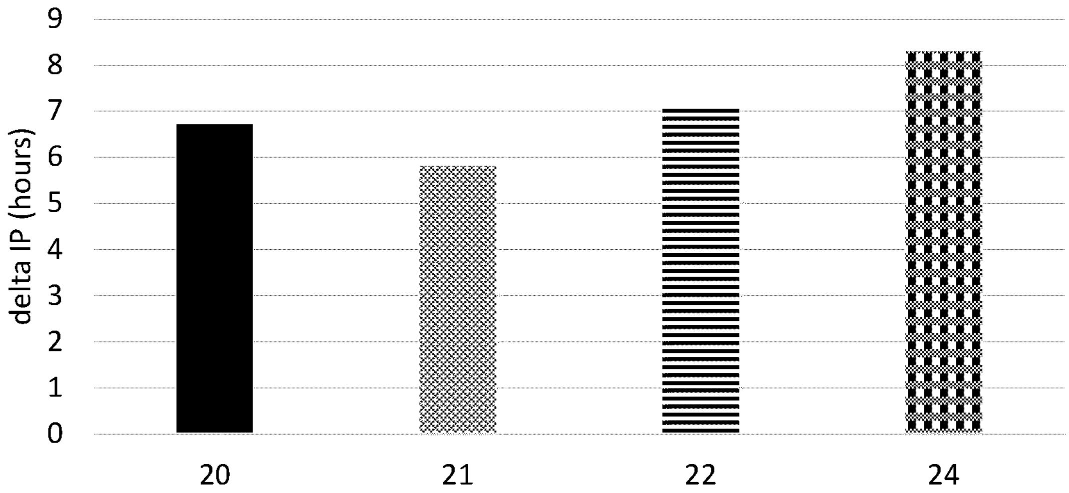


Figure 13

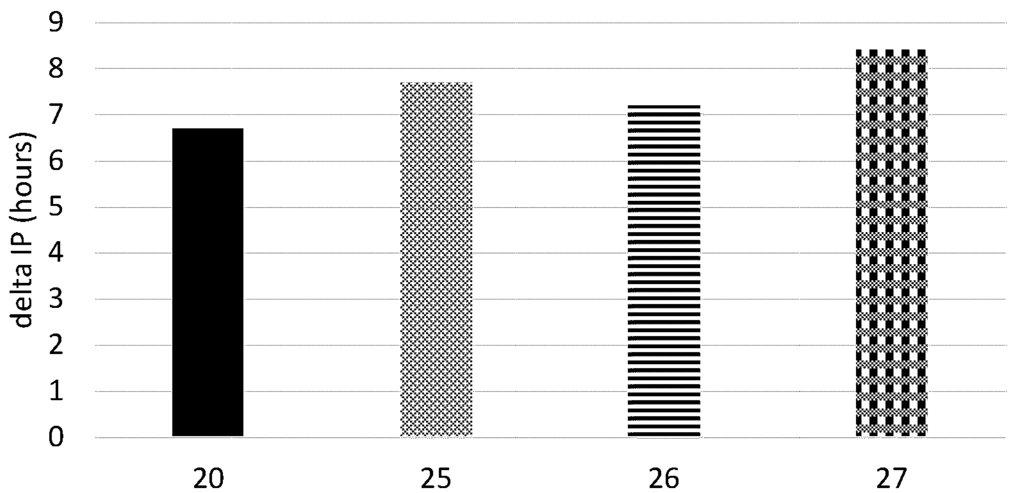

Figure 14

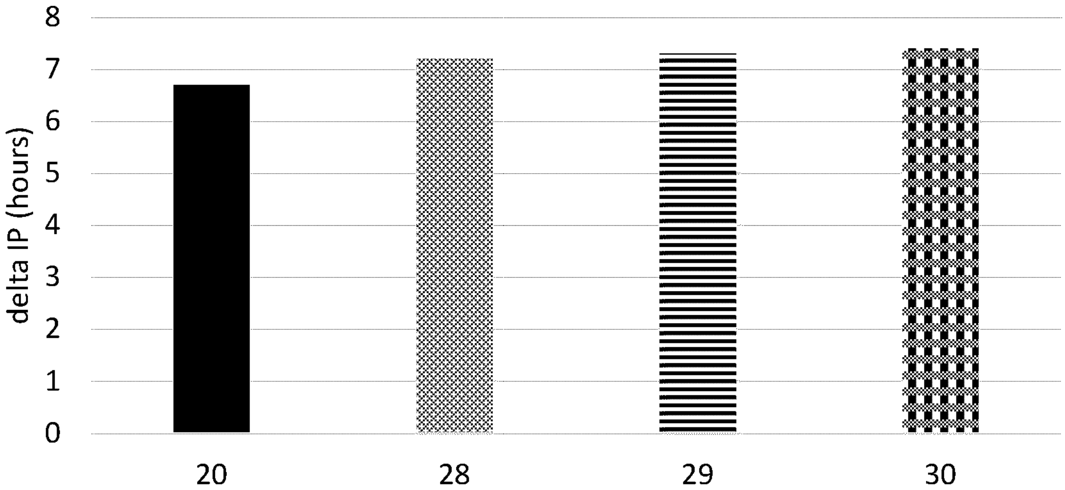


Figure 15

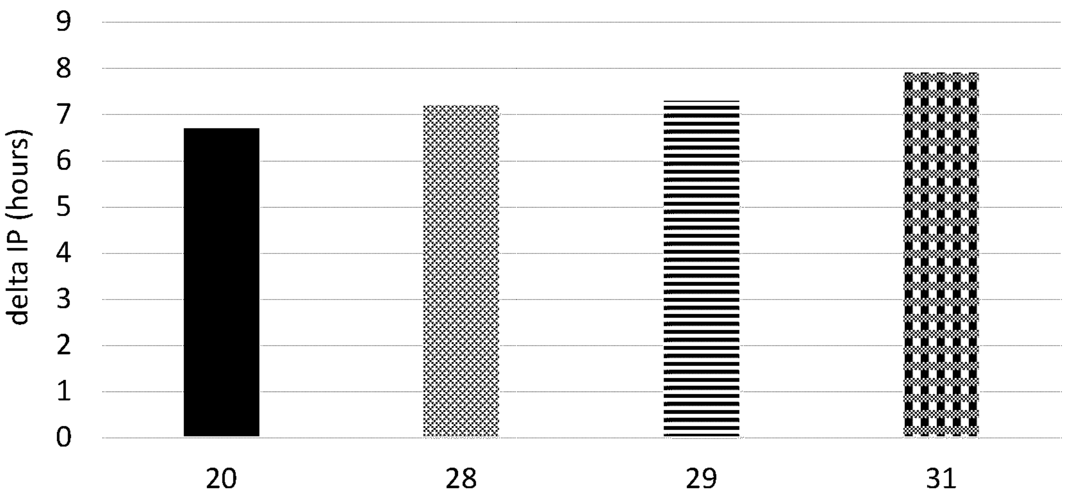

Figure 16

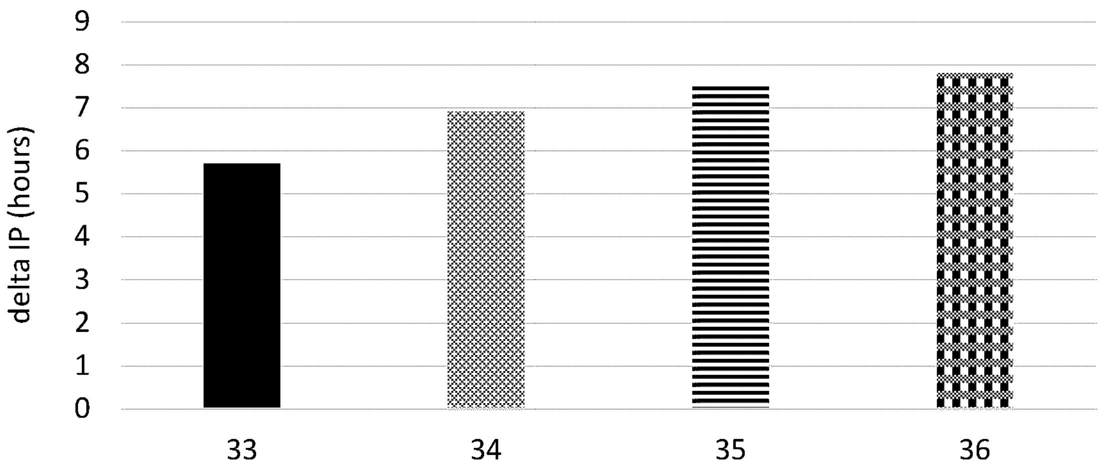


Figure 17

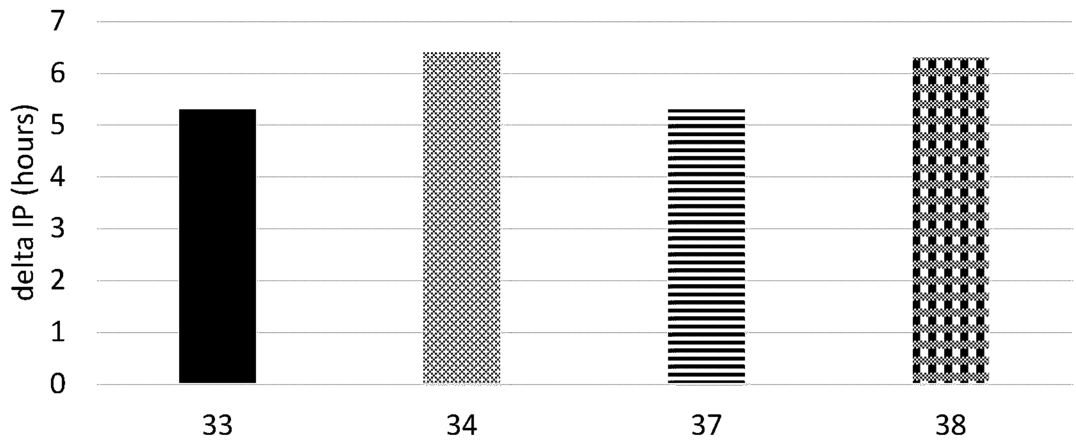

Figure 18

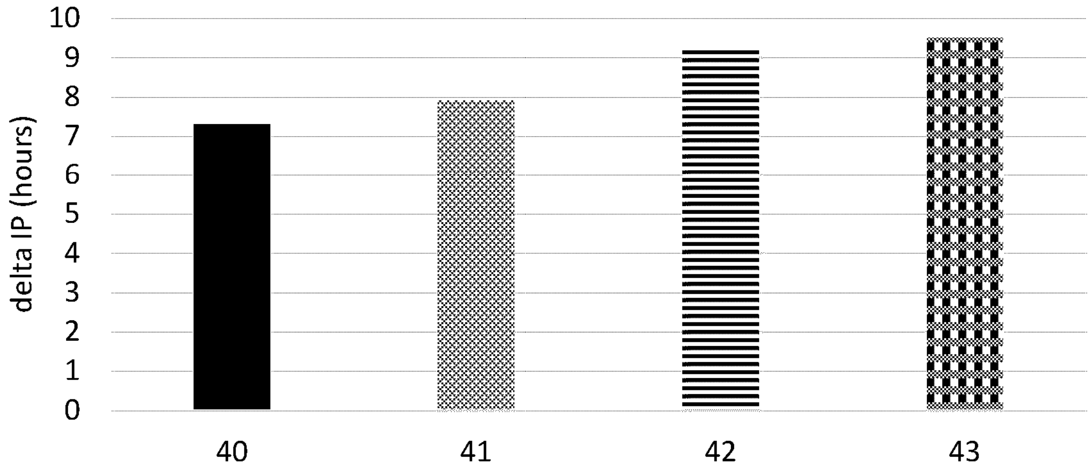


Figure 19

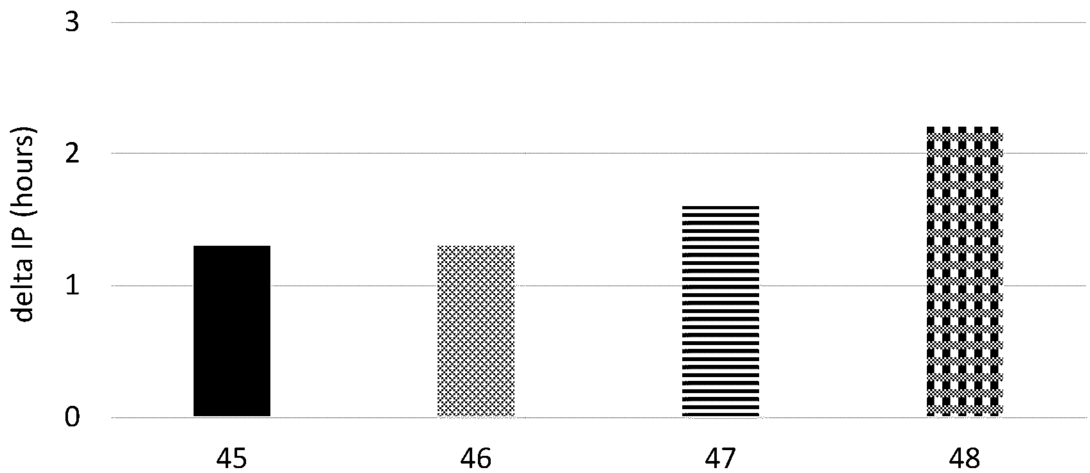

Figure 20

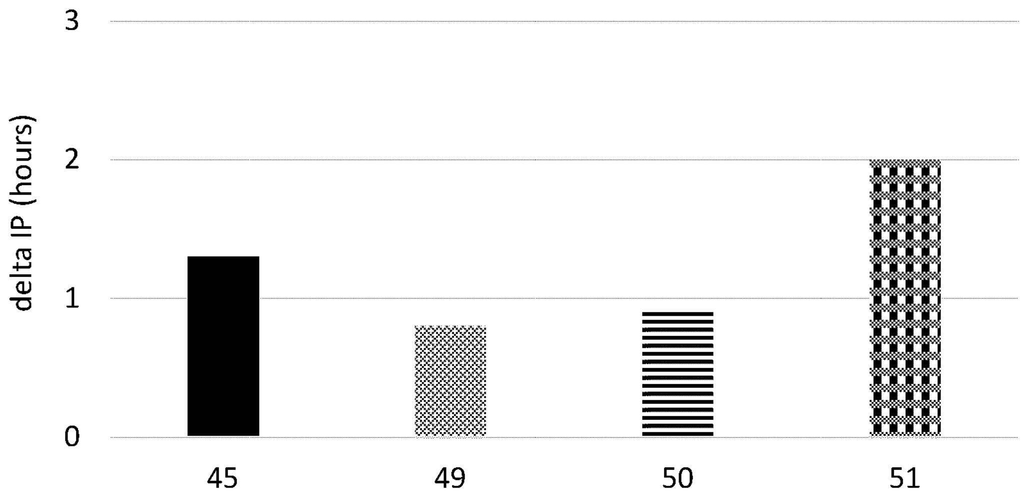


Figure 21

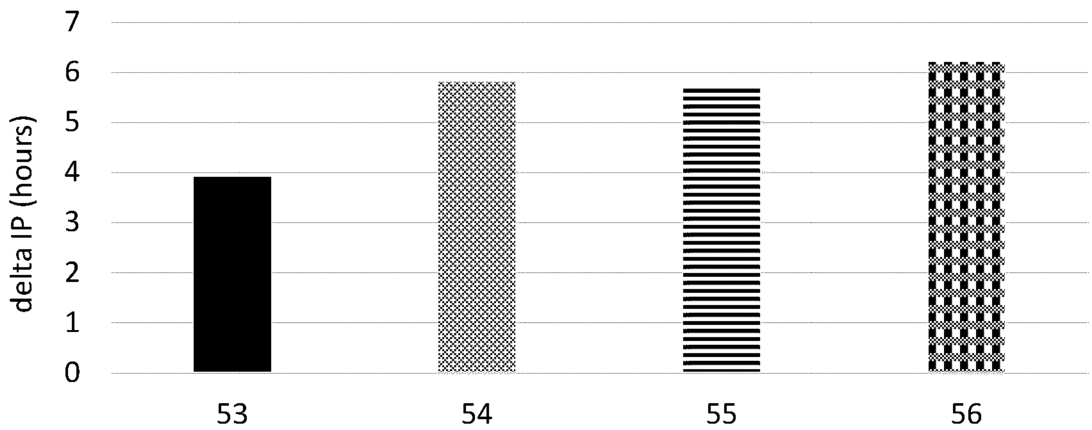

Figure 22

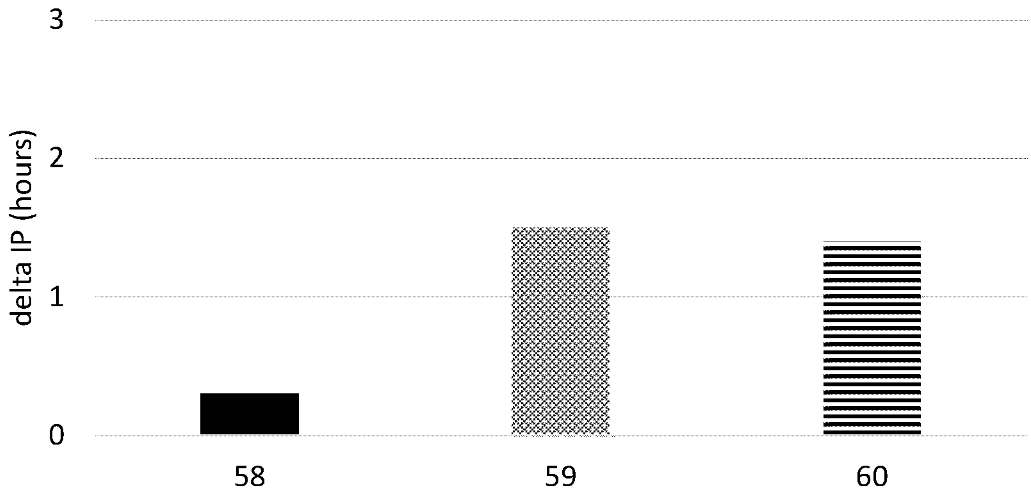


Figure 23

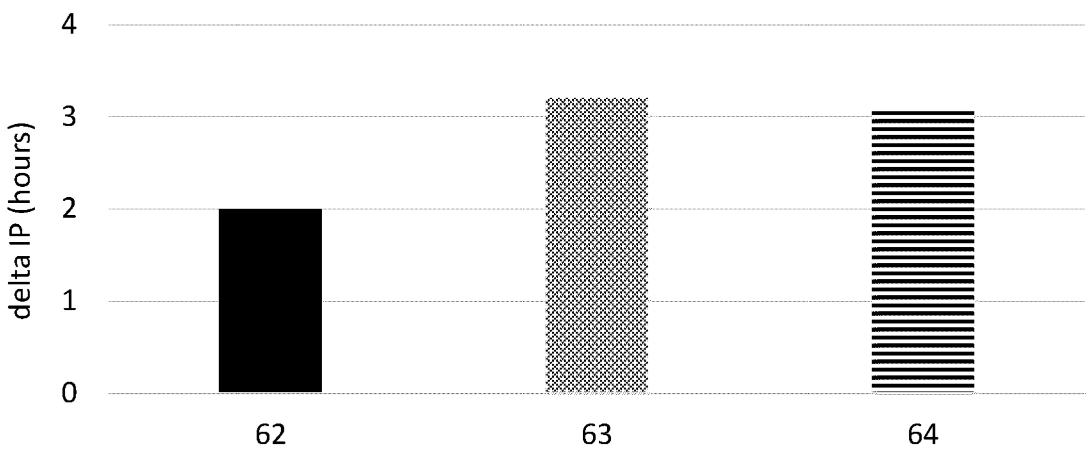

Figure 24

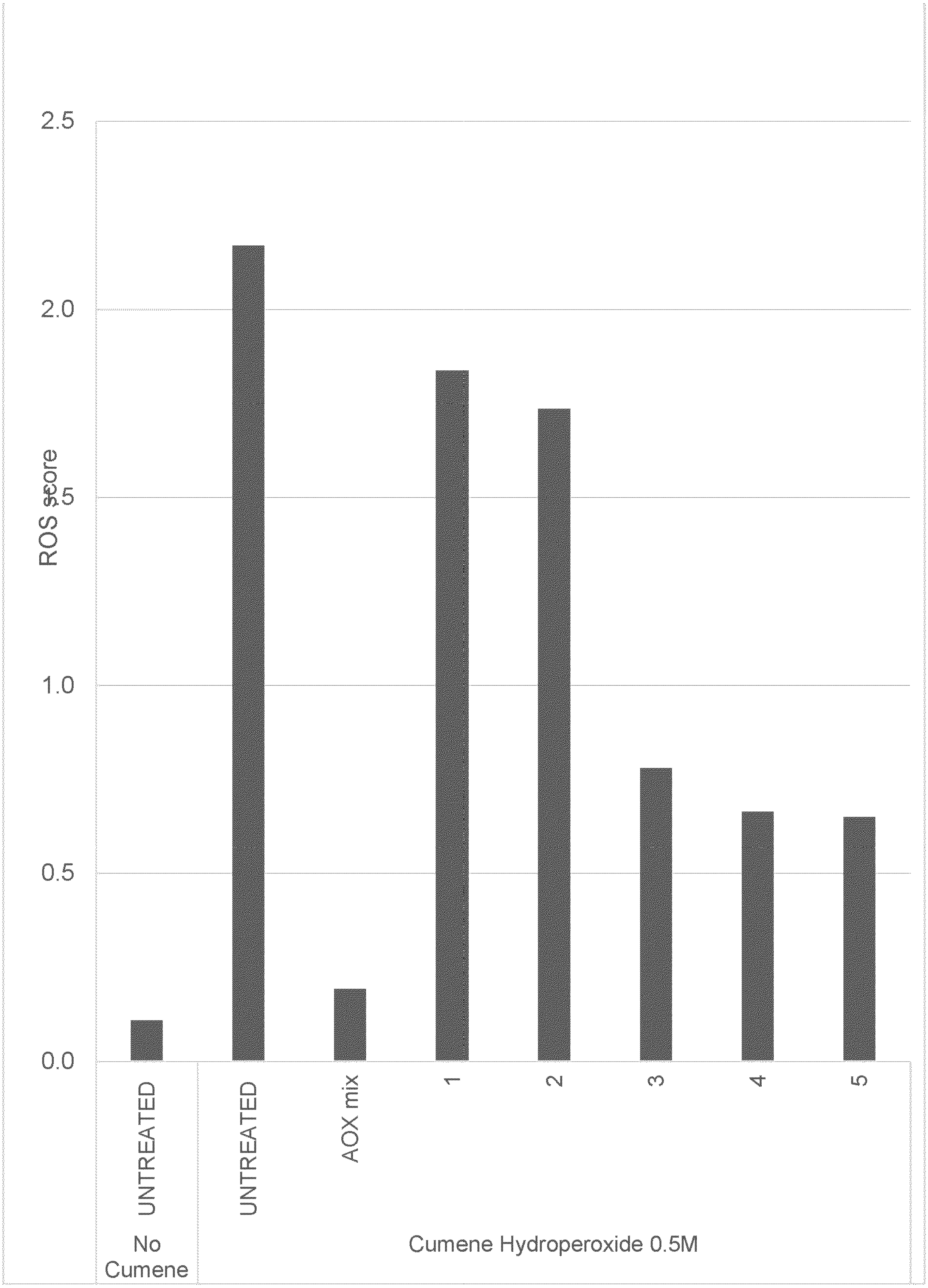
ROS score
2.5
2.0
1.5
1.0
0.5
0.0
UNTREATED
UNTREATED
AOX mix
1
2
3
4
5
No Cumene
Cumene Hydroperoxide 0.5M

Figure 25

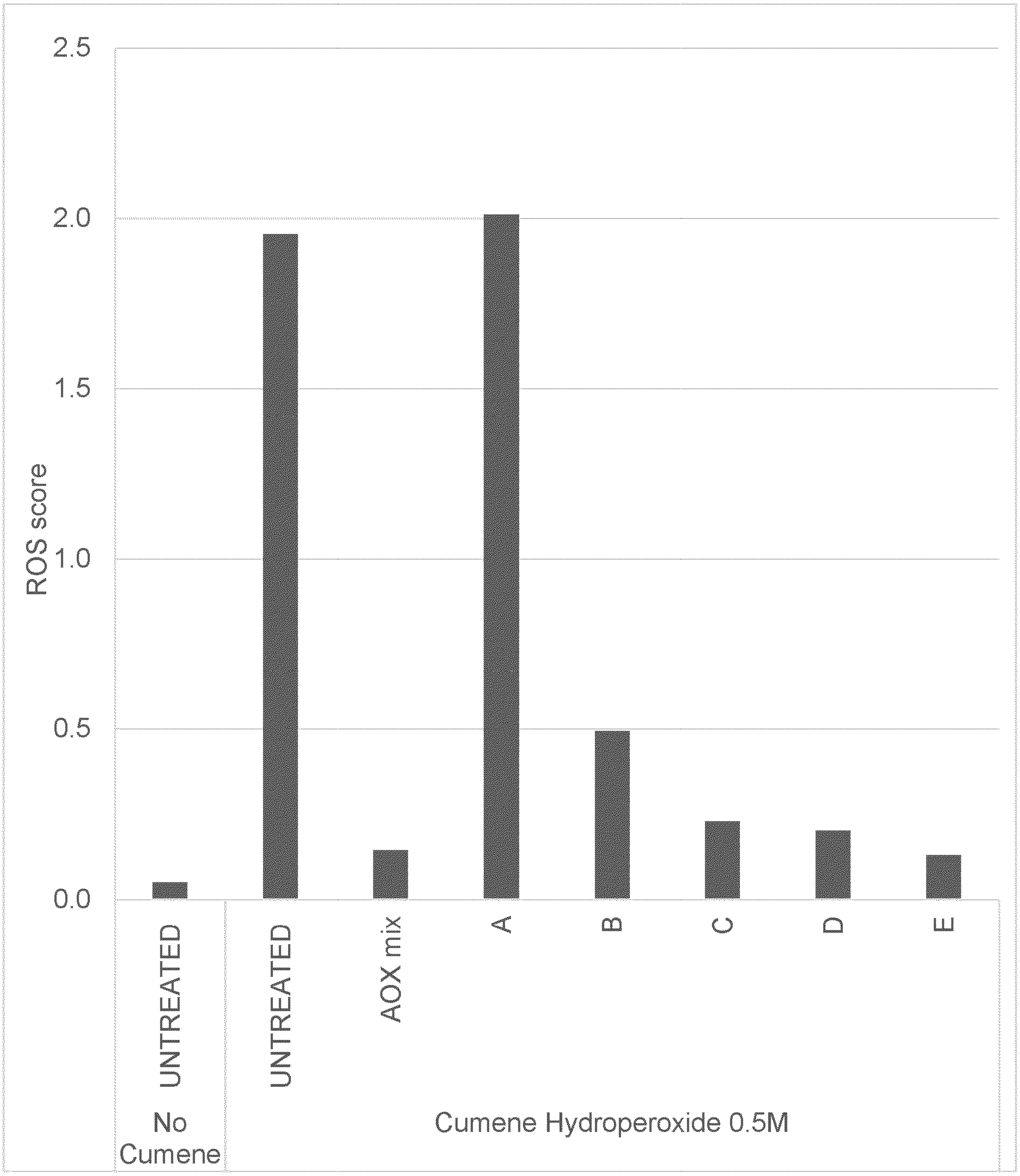
2.5
2.0
1.5
1.0
0.5
0.0
ROS score
UNTREATED
UNTREATED
AOX mix
A
B
C
D
E
No Cumene
Cumene Hydroperoxide 0.5M

Figure 26a

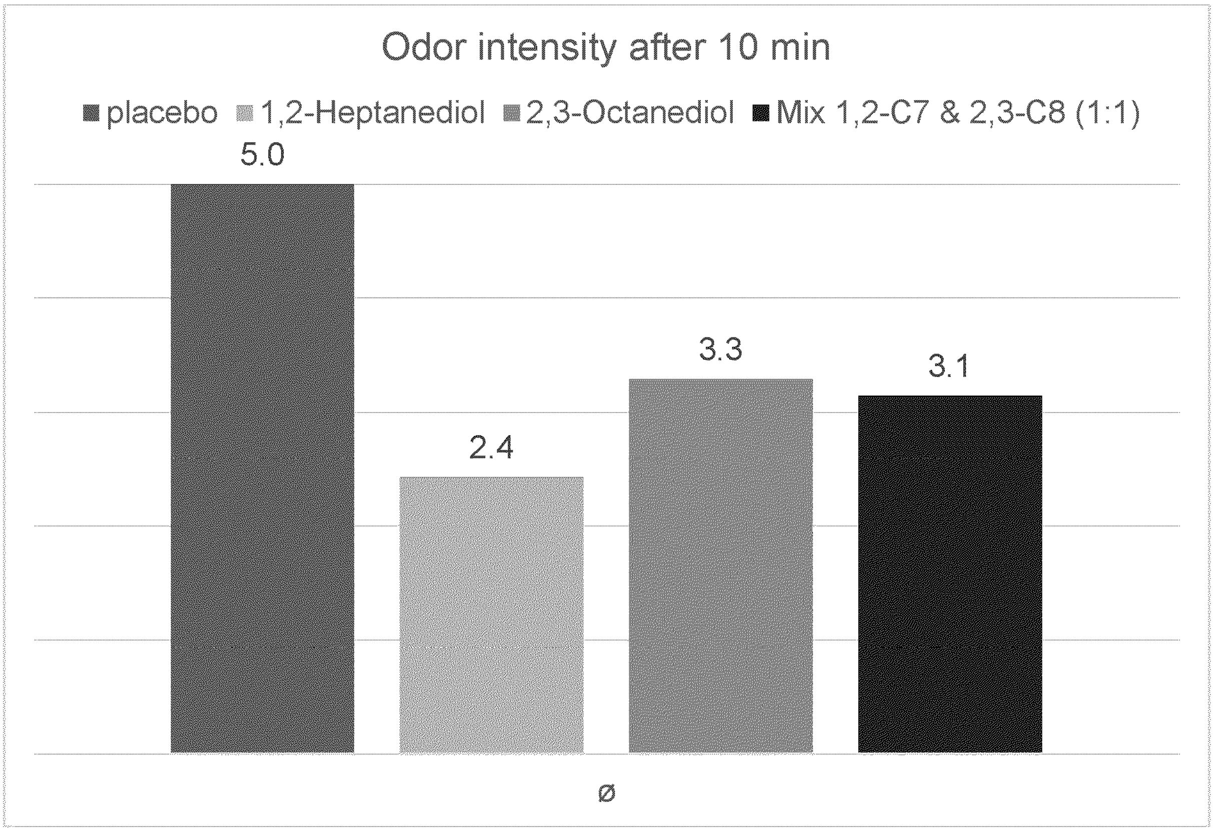
Odor intensity after 10 min
placebo
1,2-Heptanediol
2,3-Octanediol
Mix 1,2-C7 & 2,3-C8 (1:1)
5.0
2.4
3.3
3.1
ø

Figure 26b

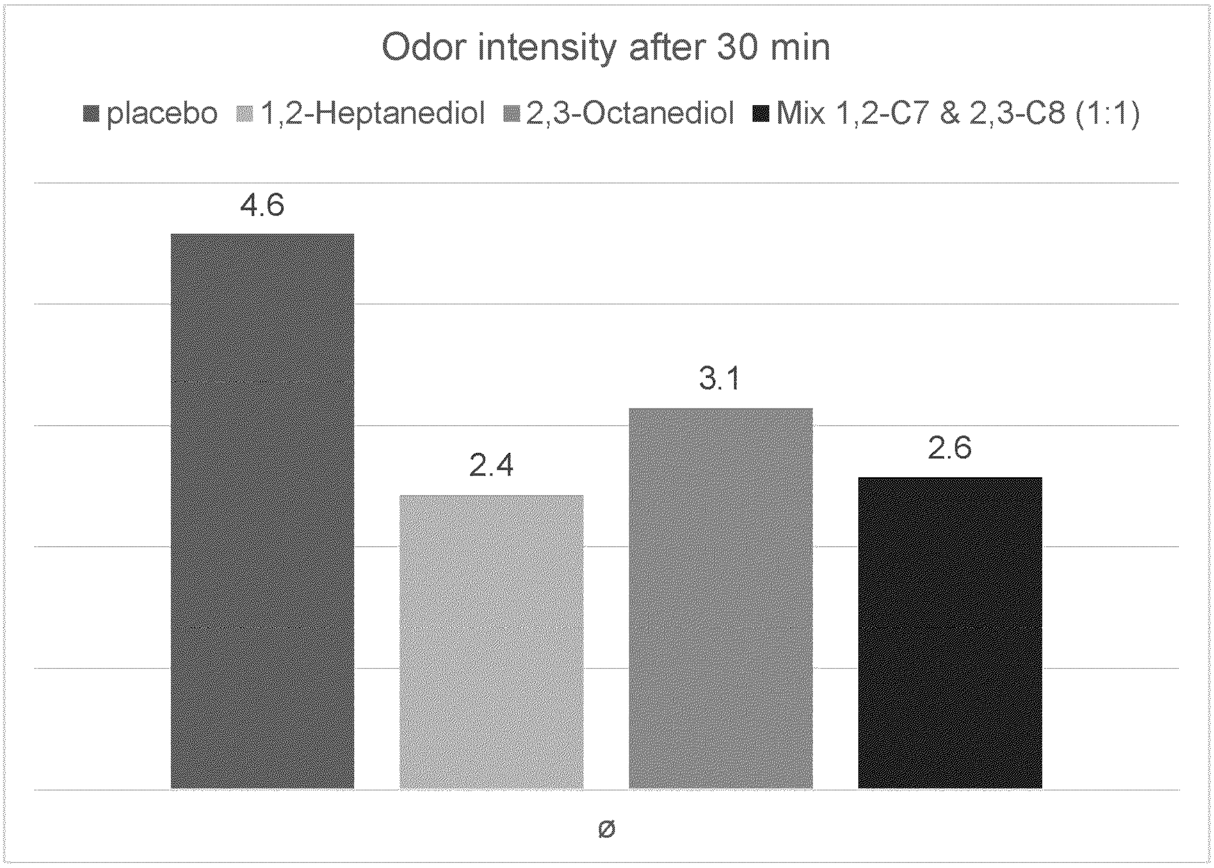
Odor intensity after 30 min
placebo
1,2-Heptanediol
2,3-Octanediol
Mix 1,2-C7 & 2,3-C8 (1:1)
4.6
2.4
3.1
2.6
ø

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008046791 A
- WO 2004047833 A1
- WO 2004026840 A
- EP 0852949 A